(19) Europäisches Patentamt
European Patent Office
Office européen des brevets

(11) **EP 4 356 963 A2**

(12) **EUROPEAN PATENT APPLICATION**

(43) Date of publication:
**24.04.2024 Bulletin 2024/17**

(21) Application number: **23216420.2**

(22) Date of filing: **30.09.2020**

(51) International Patent Classification (IPC):
***A61P 19/02*** (2006.01)

(52) Cooperative Patent Classification (CPC):
**A61K 31/4985; A61K 45/06; A61P 17/06; A61P 19/02**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2019 US 201962908163 P**
**29.10.2019 US 201962927548 P**
**31.01.2020 US 202062968849 P**
**29.05.2020 US 202063032042 P**

(62) Document number(s) of the earlier application(s) in accordance with Art. 76 EPC:
**20793870.5 / 4 037 686**

(71) Applicant: **AbbVie Inc.**
**North Chicago, IL 60064 (US)**

(72) Inventors:
• **PANGAN, Aileen L.**
**La Grange, 60525 (US)**

• **ANDERSON, Jaclyn Kay**
**Gurnee, 60031 (US)**
• **SONG, In-Ho**
**Vernon Hills, 60061 (US)**
• **ENEJOSA, Jose Jeffrey V.**
**Chicago, 60611 (US)**

(74) Representative: **Forresters IP LLP**
**Skygarden**
**Erika-Mann-Straße 11**
**80636 München (DE)**

Remarks:
• This application was filed on 13-12-2023 as a divisional application to the application mentioned under INID code 62.
• Claims filed after the date of receipt of the application (Rule 68(4) EPC).

(54) **TREATING SPONDYLOARTHRITIC AND PSORIATIC CONDITIONS WITH UPADACITINIB**

(57) Provided are methods for treating various spondyloarthritic and psoriatic conditions, including types of axial spondyloarthritis (axSpA), psoriatic arthritis (PsA), and psoriasis (PsO), with the JAKI inhibitor, upadacitinib free base or pharmaceutically acceptable salt thereof. In various aspects, provided are methods for treating active non-radiographic axSpA (nr-axSpA), methods for treating active ankylosing spondylitis (AS), methods for treating active psoriatic arthritis (PsA), and methods for treating active psoriasis (PsO).

FIG. 1

EP 4 356 963 A2

**Description**

**CROSS REFERENCE TO RELATED APPLICATIONS**

This application claims priority to U.S. Provisional Patent Application No. 63/032,042, filed on May 29, 2020; U.S. Provisional Patent Application No. 62/968,849, filed January 31, 2020; U.S. Provisional Patent Application No. 62/927,548, filed October 29, 2019; and U.S. Provisional Patent Application No. 62/908,163, filed September 30, 2019, all of which are herein incorporated by reference in their entirety.

**BACKGROUND**

Axial spondyloarthritis (axSpA) encompasses a spectrum of inflammatory involvement of the axial skeleton. Based on the Assessment of SpondyloArthritis International Society (ASAS) axSpA criteria, the disease can be further divided into 2 categories by radiographic findings: ankylosing spondylitis (AS), and an "early" form of axial SpA, referred to as non-radiographic axial spondyloarthritis (nr-axSpA). Patients with nr-axSpA and AS share common epidemiological, genetic, and clinical disease characteristics, including with regard to disease activity, and similar response to treatment. See, *e.g.,* Poddubnyy and Sieper, Curr Opin Rheumatol. (2014) 26:377-383.

Per international treatment recommendations, nonsteroidal anti-inflammatory drugs (NSAIDs) are the first-line therapy in axSpA. *See, e.g.,* van der Heijde D et al., Ann Rheum Dis. (2017) 76:978-991; Ward et al., Arthritis Rheumatol. (2016) 68:282-298. After failure of two NSAIDs given over a maximum of four weeks, biologic disease-modifying anti-rheumatic drugs (bDMARDs) are the next recommended treatment option. In axSpA, conventional synthetic disease-modifying anti-rheumatic drugs (csDMARDs) and long-term corticosteroids are not efficacious and therefore not recommended for treatment of axial symptoms. *See, e.g.,* van der Heijde D et al., Ann Rheum Dis. (2017) 76:978-991. Furthermore, only approximately 45% to 50% of patients show an Assessment of SpondyloArthritis International Society 40 (ASAS40) response and only approximately 15% to 20% achieve a state of remission in biologic-naive patients, and response rates are even less in axSpA patients who had an inadequate response to bDMARDs. See, *e.g.,* Sieper and Poddubnyy, Lancet (2017) 390:73-84; Sieper et al., Ann Rheum Dis. (2017) 76:571-592; Rudwaleit et al., Arthritis Res Ther. (2010) 12:R117; Deodhar et al., Arthritis Rheumatol. (2019) 71:599-611. To date, other than NSAIDs, there have been no oral targeted therapies approved for the treatment of ankylosing spondylitis (AS) or non-radiographic axSpA.

Psoriatic Arthritis (PsA) is a chronic systemic inflammatory disease classified as a sub-type of spondyloarthritis (SpA) and characterized by the association of arthritis and psoriasis. The course of PsA is usually characterized by flares and remissions. Left untreated, patients with PsA can have persistent inflammation, progressive joint damage, disability, and a reduced life expectancy. Initial treatment of the musculoskeletal symptoms is composed of nonsteroidal anti-inflammatory drugs (NSAIDs) and local corticosteroid injections, while topical therapies are used for the initial treatment of psoriasis. For subjects who experience lack of efficacy or toxicity with these measures, systemic therapy with non-biologic disease modifying anti-rheumatic drugs (non-biologic DMARDs) (*e.g.*, methotrexate [MTX], leflunomide [LEF], sulfasalazine [SSZ]) and ciclosporin A, followed by anti-tumor necrosis factor (TNF) therapy in subjects who do not respond adequately, is recommended. Other biologic therapies (*e.g.*, IL-12 /23 or IL-17 inhibitors) are also recommended as alternatives to anti-TNF inhibitors in selected PsA subjects. *See, e.g.,* Gossec et al., Ann Rheum Dis. (2016) 75:499-510; Coates et al., Arthritis Rheumatol. (2016) 68:1060-71. However, despite the beneficial results achieved with currently available biologic agents, approximately 40% of patients do not have at least 20% improvement in American College of Rheumatology (ACR) scores and only 58% to 61% of patients with PsA who receive them are able to achieve clinical remission after 1 year of treatment, with only approximately 43% achieving sustained remission for at least 1 year. *See, e.g.,* Gossec et al., Ann Rheum Dis. (2016) 75:499-510; Alamanos et al., J Rheumatol. (2003) 30:2641-2644; Savolainen et al., J Rheumatol. (2003) 30:2460-8; Sandborn, Dig Dis. (2010) 28:536-42; Saber et al., Arthritis Res Therapy (2010) 12: R94; Perrotta et al., J Rheumatol. (2016) 43:350-5.

Thus, there continues to remain a clear medical need for additional therapeutic options for the treatment of non-radiographic axial spondyloarthritis (nr-axSpA), ankylosing spondylitis (AS), psoriatic arthritis (PsA), and psoriasis (PsO), including PsO as a skin manifestation of PsA.

**SUMMARY**

The present disclosure addresses the above needs and provides methods for treating axial spondyloarthritis (axSpA), including non-radiographic axSpA (nr-axSpA) and ankylosing spondylitis (AS), and for treating psoriatic arthritis (PsA) and psoriasis (PsO), including PsO as a skin manifestation of PsA.

The below recited Embodiments 1-77 set forth certain aspects of the methods as described herein.

**Embodiment 1**: In certain aspects, provided is a method of treating active ankylosing spondylitis (AS) in a subject in need thereof, the method comprising orally administering to the subject once a day for at least 14 weeks a dose of upadacitinib freebase, or a pharmaceutically acceptable salt thereof, in an amount sufficient to deliver 15 mg of upadacitinib freebase equivalent, wherein the subject achieves an Assessment of SpondyloArthritis International Society 40 (ASAS40) response within 14 weeks of administration of the first dose.

**Embodiment 2:** The method of Embodiment 1, wherein when the method is used to treat a population of subjects, at least 10%, at least 15%, at least 20%, at least 25%, at least 30%, at least 35%, at least 40%, or at least 45% of the subjects in the treated population achieve an ASAS40 response within 14 weeks of administration of the first dose. In certain embodiments of the method of Embodiment 1, wherein when the method is used to treat a population of subjects, a statistically significant population of the subjects in the treated population achieves an ASAS40 response within 14 weeks of administration of the first dose.

**Embodiment 3:** The method of Embodiment 1 or 2, wherein the subject or subjects in the treated population suffering from active AS at baseline further achieve within 14 weeks of administration of the first dose at least one result selected from the group consisting of:

a. improvement from baseline in Ankylosing Spondylitis Disease Activity Score (ASDAS);
b. improvement from baseline in magnetic resonance imaging (MRI) Spondyloarthritis Research Consortium of Canada (SPARCC) score for spine (MRI-Spine SPARCC);
c. ASAS partial remission (PR);
d. Bath Ankylosing Spondylitis Disease Activity Index 50 (BASDAI50) response;
e. improvement from baseline in Bath Ankylosing Spondylitis Functional Index (BASFI);
f. ASDAS low disease activity (LDA);
g. ASDAS inactive disease (ID);
h. ASDAS major improvement (MI); and
i. ASDAS clinically important improvement (CII).

In certain embodiments of the method of Embodiment 1 or 2, when the method is used to treat a population of subjects, at least 10%, at least 15%, at least 20%, at least 25%, at least 30%, at least 35%, at least 40%, or at least 45% of the subjects in the treated population achieve at least one of these results within 14 weeks of administration of the first dose. In other embodiments of the method of Embodiment 1 or 2, when the method is used to treat a population of subjects, a statistically significant population of subjects in the treated population achieves at least one result within 14 weeks of administration of the first dose.

**Embodiment 4:** The method of Embodiment 3, wherein the subject or subjects in the treated population suffering from active AS at baseline further achieve within 14 weeks of administration of the first dose each result.

**Embodiment 5:** The method of any one of Embodiments 1-4, wherein the subject or subjects in the treated population fulfill the 1984 modified New York Criteria for ankylosing spondylitis at baseline.

**Embodiment 6:** The method of any one of Embodiments 1-5, wherein the subject or subjects in the treated population fulfill the 2009 ASAS classification criteria at baseline.

**Embodiment 7:** The method of any one of Embodiments 1-6, wherein the subject or subjects in the treated population meet at least one criteria at baseline selected from the group consisting of:

a. a Bath Ankylosing Spondylitis Disease Activity Index (BASDAI) score ≥4;
b. an Ankylosing Spondylitis Disease Activity Score (ASDAS) of ≥2.1; and
c. a Patient's Assessment of Total Back Pain (Total Back Pain score) of ≥4 based on a 0-10 numerical rating scale.

**Embodiment 8:** The method of any one of Embodiments 1-7, wherein the subject or subjects in the treated population are biologic disease-modifying anti-rheumatic drug (bDMARD) naive at baseline.

**Embodiment 9:** The method of any one of Embodiments 1-7, wherein the subject or subjects in the treated population have had an inadequate response or intolerance to a biologic disease-modifying anti-rheumatic drug (bDMARD) at baseline.

**Embodiment 10:** The method of Embodiment 9, wherein prior to administration of the first dose, the subject or subjects in the treated population have been administered one bDMARD, and discontinued use of the bDMARD due to intolerance or lack of efficacy.

**Embodiment 11:** The method of Embodiment 10, wherein the bDMARD is a tumor necrosis factor (TNF) inhibitor or an interleukin (IL)-17 inhibitor.

**Embodiment 12:** The method of any one of Embodiments 1-11, wherein the subject or subjects in the treated population have had an inadequate response or intolerance to at least two NSAIDs, intolerance to NSAIDS, and/or contraindication for NSAIDs at baseline.

**Embodiment 13:** In other aspects, provided is a method of treating active non-radiographic axial spondyloarthritis in a subject in need thereof, the method comprising orally administering to the subject once a day for at least 14 weeks a dose of upadacitinib freebase, or a pharmaceutically acceptable salt thereof, in an amount sufficient to deliver 15 mg of upadacitinib freebase equivalent, wherein the subject achieves an ASAS40 response within 14 weeks of administration of the first dose. In certain embodiments, when the method is used to treat a population of subjects, at least 10%, at least 15%, at least 20%, at least 25%, at least 30%, at least 35%, at least 40%, or at least 45% of the subjects in the treated population achieve an ASAS40 response within 14 weeks of administration of the first dose. In certain embodiments, a statistically significant population of subjects in the treated population achieve an ASAS40 response within 14 weeks of administration of the first dose.

**Embodiment 14:** In yet other aspects, provided is a method of treating active non-radiographic axial spondyloarthritis in a subject in need thereof, the method comprising orally administering to the subject once a day for at least 52 weeks a dose of upadacitinib freebase, or a pharmaceutically acceptable salt thereof, in an amount sufficient to deliver 15 mg of upadacitinib freebase equivalent, wherein the subject achieves an ASAS40 response within 52 weeks of administration of the first dose. In certain embodiments, when the method is used to treat a population of subjects, at least 10%, at least 15%, at least 20%, at least 25%, at least 30%, at least 35%, at least 40%, or at least 45% of the subjects in the treated population achieve an ASAS40 response within 52 weeks of administration of the first dose. In certain embodiments, a statistically significant population of subjects in the treated population achieves an ASAS40 response within 52 weeks of administration of the first dose.

**Embodiment 15:** The method of Embodiment 13 or 14, wherein the subject or subjects in the treated population fulfill at baseline the 2009 ASAS classification criteria for axial spondyloarthritis, but does not meet the radiologic criteria of the 1984 modified New York criteria for ankylosing spondylitis.

**Embodiment 16:** The method of Embodiment 13 or 14, wherein the subject or subjects in the treated population meet at least one criteria at baseline selected from the group consisting of:

> a. Bath Ankylosing Spondylitis Disease Activity Index (BASDAI) score of $\geq$ 4;
> b. an Ankylosing Spondylitis Disease Activity Score (ASDAS) of $\geq$2.1;
> c. a Patient's Assessment of Total Back Pain (Total Back Pain score) of $\geq$ 4 based on a 0 - 10 numerical rating scale; and
> d. an objective sign of inflammatory activity selected from the group consisting of:
>
>> i. an objective sign of active inflammation on MRI of sacroiliac (SI) joints, and
>> ii. high-sensitivity C reactive protein > upper limit of normal (ULN).

**Embodiment 17:** The method of any one of Embodiments 13-16, wherein the subject or subjects in the treated population are bDMARD naive at baseline.

**Embodiment 18:** The method of any one of Embodiments 13-16, wherein the subject or subjects in the treated population have had an inadequate response or intolerance to a bDMARD at baseline.

**Embodiment 19:** The method of Embodiment 18, wherein prior to administration of the first dose, the subject or subjects in the treated population have been administered one bDMARD, and discontinued use of the bDMARD due to intolerance or lack of efficacy.

**Embodiment 20:** The method of Embodiment 19, wherein the bDMARD is a tumor necrosis factor (TNF) inhibitor or an interleukin (IL)-17 inhibitor.

**Embodiment 21:** The method of any one of Embodiments 13-20, wherein the subject or subjects in the treated population have had an inadequate response or intolerance to at least 2 NSAIDs, has an intolerance to NSAIDS, and/or has a contraindication for NSAIDs at baseline.

**Embodiment 22:** The method of any one of Embodiments 13-21, wherein the subject or subjects in the treated population achieve within 14 weeks of administration of the first dose at least one additional result selected from the group consisting of:

> a. improvement from baseline in Ankylosing Spondylitis Disease Activity Score (ASDAS);
> b. improvement from baseline in magnetic resonance imaging (MRI) Spondyloarthritis Research Consortium of Canada (SPARCC) score for SI joints (MRI-SI joints SPARCC);
> c. ASAS partial remission (PR);
> d. Bath Ankylosing Spondylitis Disease Activity Index (BASDAI) 50 response;
> e. improvement from baseline in Bath Ankylosing Spondylitis Functional Index (BASFI);
> f. improvement from baseline in Ankylosing Spondylitis Quality of Life (ASQoL);
> g. improvement from baseline in ASAS Health Index (HI);
> h. improvement from baseline in Maastricht Ankylosing Spondylitis Enthesitis Score (MASES); and
> i. improvement from baseline in Linear Bath Ankylosing Spondylitis Metrology Index (BASMI$_{lin}$).

In certain embodiments of the method of any one of Embodiments 13-21, wherein when the method is used to treat a population of subjects, at least 10%, at least 15%, at least 20%, at least 25%, at least 30%, at least 35%, at least 40%, or at least 45% of the subjects in the treated population achieve at least one result within 14 weeks of administration of the first dose. In certain embodiments of the method of any one of Embodiments 13-21, wherein when the method is used to treat a population of subjects, a statistically significant population of subjects in the treated population achieves at least one result within 14 weeks of administration of the first dose.

**Embodiment 23:** In yet other aspects, provided is a method of treating active psoriatic arthritis (PsA) in a subject in need thereof, the method comprising orally administering to the subject once a day for at least 12 weeks a dose of upadacitinib freebase, or a pharmaceutically acceptable salt thereof, in an amount sufficient to deliver 15 mg of upadacitinib freebase equivalent, wherein the subject achieves an American College of Rheumatology 20% (ACR20) response within 12 weeks of administration of the first dose.

**Embodiment 24:** In still yet other aspects, provided is a method of treating active psoriatic arthritis (PsA) in a subject in need thereof, the method comprising orally administering to the subject once a day for at least 12 weeks a dose of upadacitinib freebase, or a pharmaceutically acceptable salt thereof, in an amount sufficient to deliver 30 mg of upadacitinib free base equivalent, wherein the subject achieves an American College of Rheumatology 20% (ACR20) response within 12 weeks of administration of the first dose.

**Embodiment 25:** The method of Embodiment 23 or 24, wherein when the method is used to treat a population of subjects, at least 10%, at least 15%, at least 20%, at least 25%, at least 30%, at least 35%, at least 40%, or at least 45% of the subjects in the treated population achieve an ACR20 response within 12 weeks of administration of the first dose. In other embodiments of the method of Embodiment 23 or 24, when the method is used to treat a population of subjects, a statistically significant population of subjects in the treated population achieves an ACR20 response within 12 weeks of administration of the first dose.

**Embodiment 26:** The method of any one of Embodiments 23-25, wherein the subject or subjects in the treated population suffering from active PsA at baseline further achieve at least one result selected from the group consisting of:

a. improvement from baseline in Health Assessment Questionnaire - Disability Index (HAQ-DI) within 12 weeks of administration of the first dose;
b. achieve Static Investigator Global Assessment (sIGA) of Psoriasis of 0 or 1 and at least a 2-point improvement from baseline at within 16 weeks of administration of the first dose (for subjects with baseline sIGA $\geq$ 2);
c. achieve Psoriasis Area Severity Index (PASI) 75 response within 16 weeks of administration of the first dose (for subjects with $\geq$ 3% BSA psoriasis at baseline);
d. improvement from baseline in Sharp/van der Heijde Score (SHS) within 24 weeks of administration of the first dose;
e. achieve Minimal Disease Activity (MDA) within 24 weeks of administration of the first dose;
f. improvement from baseline in Leeds Enthesitis Index (LEI) within 24 weeks of administration of the first dose, preferably wherein the improvement is a resolution of enthesitis (LEI = 0) within 24 weeks of administration of the first dose (for subjects with baseline presence of enthesitis (LEI >0));
g. achieve ACR 20 response within 12 weeks of administration of the first dose (non-inferiority of upadacitinib vs adalimumab);
h. improvement from baseline in 36-Item Short Form Health Survey (SF-36) within 12 weeks of administration of the first dose; and
i. improvement from baseline in Functional Assessment of Chronic Illness Therapy-Fatigue (FACIT-F) Questionnaire within 12 weeks of administration of the first dose.

In certain embodiments, wherein when the method of Embodiments 23-25 is used to treat a population of subjects, at least 10%, at least 15%, at least 20%, at least 25%, at least 30%, at least 35%, at least 40%, or at least 45% of the subjects in the treated population achieve at least one result within 14 weeks of administration of the first dose. In other embodiments, wherein when the method of Embodiments 23-25 is used to treat a population of subjects, a statistically significant population of subjects in the treated population achieve at least one result within 14 weeks of administration of the first dose.

**Embodiment 27:** The method of Embodiment 26, wherein the subject or subjects in the treated population suffering from active PsA at baseline further achieve each result.

**Embodiment 28:** The method of anyone of Embodiments 23-27, wherein the subject or subjects in the treated population suffering from active PsA at baseline further achieve at least one result selected from the group consisting of:

a. ACR 20 response and superiority over adalimumab (40 mg every other week) within 12 weeks of administration of the first dose; and
b. improvement from baseline in Leeds Dactylitis Index (LDI) within 24 weeks of administration of the first dose, preferably wherein the improvement is a resolution of dactylitis (LDI = 0) within 24 weeks of administration of the first dose (for subjects with baseline presence of dactylitis (LDI >0)).

In certain embodiments of Embodiment 28, wherein when the method is used to treat a population of subjects, at least 10%, at least 15%, at least 20%, at least 25%, at least 30%, at least 35%, at least 40%, or at least 45% of the subjects in the treated population achieve at least one result within 14 weeks of administration of the first dose. In other embodiments of Embodiment 28, wherein when the method is used to treat a population of subjects, a statistically significant population of subjects in the treated population achieve at least one result within 14 weeks of administration of the first dose.

**Embodiment 29:** The method of Embodiment 28, wherein the subject or subjects in the treated population suffering from active PsA further achieve ACR 20 response and superiority over adalimumab (40 mg every other week) within 12 weeks of administration of the first dose. In certain embodiments of Embodiment 29, a statistically significant population of subjects in the treated population achieves an ACR20 response and superiority over adalimumab within 12 weeks of administration of the first dose. In certain embodiments of Embodiment 29, at least 10%, at least 15%, at least 20%, at least 25%, at least 30%, at least 35%, at least 40%, or at least 45% of the subjects in the treated population achieve an ACR20 response and superiority over adalimumab within 12 weeks of administration of the first dose.

**Embodiment 30:** The method of Embodiment 29, wherein the subject or subjects in the treated population suffering from active PsA are orally administered once a day for at least 12 weeks a dose of upadacitinib freebase, or a pharmaceutically acceptable salt thereof, in an amount sufficient to deliver 30 mg of upadacitinib freebase equivalent.

**Embodiment 31:** The method of any one of Embodiments 23-30, wherein the subject or subjects in the treated population achieve an ACR 50% response (ACR50) within 12 weeks of administration of the first dose. In certain embodiments of Embodiment 31, a statistically significant population of subjects in the treated population achieves an ACR50 response within 12 weeks of administration of the first dose. In certain embodiments of Embodiment 31, at least 10%, at least 15%, at least 20%, at least 25%, at least 30%, at least 35%, at least 40%, or at least 45% of subjects in the treated population achieve an ACR50 response within 12 weeks of administration of the first dose.

**Embodiment 32:** The method of any one of Embodiments 23-30, wherein the subject or subjects in the treated population achieve an ACR 70% response (ACR70) within 12 weeks of administration of the first dose. In certain embodiments of Embodiment 32, a statistically significant population of subjects in the treated population achieves an ACR70 response within 12 weeks of administration of the first dose. In certain embodiments of Embodiment 32, at least 10%, at least 15%, at least 20%, at least 25%, at least 30%, at least 35%, at least 40%, or at least 45% of the subjects in the treated population achieve an ACR70 response within 12 weeks of administration of the first dose.

**Embodiment 33:** The method of any one of Embodiments 23-32, wherein the subject or subjects in the treated population fulfill the Classification Criteria for Psoriatic Arthritis (CASPAR) criteria at baseline.

**Embodiment 34:** The method of any one of Embodiments 23-33, wherein the subject or subjects in the treated population have at least one criteria selected from the group consisting of $\geq$ 3 tender joints (based on 68 joint counts) and $\geq$ 3 swollen joints (based on 66 joint counts) at baseline.

**Embodiment 35:** The method of Embodiment 34, wherein the subject or subjects in the treated population have $\geq$ 5 tender joints (based on 68 joint counts) and $\geq$ 5 swollen joints (based on 66 joint counts) at baseline.

**Embodiment 36:** The method of any one of Embodiments 23-35, wherein the subject or subjects in the treated population have at least one criteria selected from the group consisting of $\geq$1 erosion on x-ray as determined by central imaging review, and hs-CRP > laboratory defined upper limit of normal (ULN) at baseline.

**Embodiment 37:** The method of any one of Embodiments 23-36, wherein the subject or subjects in the treated population have a diagnosis of active plaque psoriasis, or the subject has a documented history of plaque psoriasis at baseline.

**Embodiment 38:** The method of any one of Embodiments 23-36, wherein the subject or subjects in the treated population have had an inadequate response or intolerance to at least one biologic disease-modifying anti-rheumatic drug (bDMARD) at baseline.

**Embodiment 39:** The method of Embodiment 38, wherein the subject or subjects in the treated population have discontinued all bDMARDs prior to administration of the first dose.

**Embodiment 40:** The method of any one of Embodiments 23-36, wherein the subject or subjects in the treated population have had an inadequate response or intolerance to previous or concurrent treatment with at least one non-biologic DMARD, or an intolerance to or contraindication for non-biologic DMARDs at baseline.

**Embodiment 41:** The method of any one of Embodiments 23-40, wherein the subject or subjects in the treated population have moderately to severely active psoriatic arthritis at baseline.

**Embodiment 42:** In yet other aspects, provided is a method of treating active psoriasis in a subject in need thereof, the method comprising orally administering to the subject once a day for at least 16 weeks a dose of upadacitinib freebase, or a pharmaceutically acceptable salt thereof, in an amount sufficient to deliver 15 mg of upadacitinib freebase equivalent, wherein the subject achieves a Psoriasis Area Severity Index (PASI) 75 response within 16 weeks of administration of the first dose.

**Embodiment 43:** In yet other aspects, provided is a method of treating active psoriasis in a subject in need thereof, the method comprising orally administering to the subject once a day for at least 16 weeks a dose of upadacitinib freebase, or a pharmaceutically acceptable salt thereof, in an amount sufficient to deliver 30 mg of upadacitinib freebase equivalent, wherein the subject achieves a Psoriasis Area Severity Index (PASI) 75 response within 16 weeks of administration of the first dose.

**Embodiment 44:** The method of Embodiment 42 or 43, wherein when the method is used to treat a population of subjects, a portion of the subjects in the treated population achieve a PASI 75 response within 16 weeks of administration of the first dose. In certain embodiments of Embodiment 44, at least 10%, at least 15%, at least 20%, at least 25%, at least 30%, at least 35%, at least 40%, or at least 45% of the subjects in the treated population achieve a PASI 75 response within 16 weeks of administration of the first dose. In other embodiments of Embodiment 44, a statistically significant population of the subjects in the treated population achieve a PASI 75 response within 16 weeks of administration of the first dose.

**Embodiment 45:** The method of any one of Embodiments 1-44 or 46-77, wherein the subject is an adult subject, or the subjects in the treated population are adult subjects.

**Embodiment 46:** The method of any one of Embodiments 1-12, wherein the ASAS40 response is maintained or improved after Week 14 by continuing to administer the daily dose. In one aspect, the ASAS40 response is maintained or improved up to and including Week 64.

**Embodiment 47:** The method of any one of Embodiments 1-12, wherein the subject or subjects in the treated population further achieve ASAS40 within 2 weeks of administration of the first dose.

**Embodiment 48:** The method of any one of Embodiments 1-12, wherein the subject or subjects in the treated population further achieved ASAS40 within 2 weeks of administration of the first dose, and wherein the ASAS40 is maintained or improved after Week 14 by continuing to administer the daily dose.

**Embodiment 49:** In another aspect, provided is a method of treating active ankylosing spondylitis in a subject in need thereof, the method comprising orally administering to the subject once a day for at least 14 weeks a dose of upadacitinib freebase, or a pharmaceutically acceptable salt thereof, in an amount sufficient to deliver 15 mg of upadacitinib freebase equivalent, wherein the subject achieves ASAS partial remission (PR), ASDAS low disease activity (LDA), ASDAS inactive disease (ID), ASDAS major improvement (MI), and/or ASDAS clinically important improvement (CII) within 14 weeks of administration of the first dose.

**Embodiment 50:** The method of Embodiment 49, wherein when the method is used to treat a population of subjects, a portion of the subjects in the treated population achieve ASAS partial remission (PR), ASDAS low disease activity (LDA), ASDAS inactive disease (ID), ASDAS major improvement (MI), and/or ASDAS clinically important improvement (CII) within 14 weeks of administration of the first dose. In certain embodiments of Embodiment 50, at least 10%, at least 15%, at least 20%, at least 25%, at least 30%, at least 35%, at least 40%, or at least 45% of the subjects in the treated population achieve ASAS partial remission (PR), ASDAS low disease activity (LDA), ASDAS inactive disease (ID), ASDAS major improvement (MI), and/or ASDAS clinically important improvement (CII) within 14 weeks of administration of the first dose. In certain embodiments of Embodiment 50, a statistically significant population of subjects in the treated population achieves ASAS partial remission (PR), ASDAS low disease activity (LDA), ASDAS inactive disease (ID), ASDAS major improvement (MI), and/or ASDAS clinically important improvement (CII) within 14 weeks of administration of the first dose.

**Embodiment 51:** The method of Embodiment 49 or 50, wherein the subject or subjects in the treated population further achieve within 14 weeks of administration of the first dose each result.

**Embodiment 52:** The method of any one of Embodiments 49-51, wherein the subject or subjects in the treated population fulfill the 1984 modified New York Criteria for ankylosing spondylitis at baseline.

**Embodiment 53:** The method of any one of Embodiments 49-51, wherein the subject or subjects in the treated population fulfill the 2009 ASAS classification criteria at baseline.

**Embodiment 54:** The method of any one of Embodiments 49-53, wherein the subject or subjects in the treated population meet at least one criteria at baseline selected from the group consisting of:

a. a Bath Ankylosing Spondylitis Disease Activity Index (BASDAI) score ≥4;
b. an Ankylosing Spondylitis Disease Activity Score (ASDAS) of ≥2.1; and
c. a Patient's Assessment of Total Back Pain (Total Back Pain score) of ≥4 based on a 0-10 numerical rating scale.

**Embodiment 55:** The method of any one of Embodiments 49-54, wherein the subject or subjects in the treated population are biologic disease-modifying anti-rheumatic drug (bDMARD) naive at baseline.

**Embodiment 56:** The method of any one of Embodiments 49-55, wherein the subject or subjects in the treated population have had an inadequate response or intolerance to a biologic disease-modifying anti-rheumatic drug (bDMARD) at baseline.

**Embodiment 57:** The method of Embodiments 56, wherein prior to administration of the first dose, the subject or subjects in the population have been administered one bDMARD, and discontinued use of the bDMARD due to intolerance or lack of efficacy.

**Embodiment 58:** The method of Embodiments 57, wherein the bDMARD is a tumor necrosis factor (TNF) inhibitor or an interleukin (IL)-17 inhibitor.

**Embodiment 59:** The method of any one of Embodiments 49-58, wherein the subject or subjects in the population have had an inadequate response or intolerance to at least two NSAIDs, intolerance to NSAIDS, and/or contraindication for NSAIDs at baseline.

**Embodiment 60:** The method of any one of Embodiments 49-59, wherein the ASAS partial remission (PR), ASDAS low disease activity (LDA), ASDAS inactive disease (ID), ASDAS major improvement (MI), and/or ASDAS clinically important improvement (CII) is maintained or improved after Week 14 by continuing to administer the daily dose.

**Embodiment 61:** The method of any one of Embodiments 49-60, wherein the subject or subjects in the treated population further achieve ASAS partial remission (PR), ASDAS low disease activity (LDA), ASDAS inactive disease (ID), ASDAS major improvement (MI), and/or ASDAS clinically important improvement (CII) within 2 weeks of administration of the first dose. In certain embodiments of Embodiment 61, a statistically significant population of subjects in the treated population achieve ASAS partial remission (PR), ASDAS low disease activity (LDA), ASDAS inactive disease (ID), ASDAS major improvement (MI), and/or ASDAS clinically important improvement (CII) within 2 weeks of administration of the first dose. In certain embodiments of Embodiment 61, at least 10%, at least 15%, at least 20%, at least 25%, at least 30%, at least 35%, at least 40%, or at least 45% of the subjects in the treated population achieve ASAS partial remission (PR), ASDAS low disease activity (LDA), ASDAS inactive disease (ID), ASDAS major improvement (MI), and/or ASDAS clinically important improvement (CII) within 2 weeks of administration of the first dose.

**Embodiment 62:** The method of any one of Embodiments 23-41, wherein the ACR score is maintained or improved after Week 12 by continuing to administer the daily dose.

**Embodiment 63:** The method of any one of Embodiments 23-41 or 62, wherein the subject or subjects in the treated population further achieve ACR20 within 2 weeks of administration of the first dose.

**Embodiment 64:** The method of any one of Embodiments 42-44, wherein the subject or subjects in the treated population achieve a Psoriasis Area Severity Index (PASI) 90 response within 16 weeks of administration of the first dose.

**Embodiment 65:** The method of any one of Embodiments 42-44 or 64, wherein the PASI response is maintained or improved after Week 16 by continuing to administer the daily dose.

**Embodiment 66:** In another aspect, provided is a method of treating active psoriatic arthritis in a subject in need thereof, the method comprising orally administering to the subject once a day for at least 24 weeks a dose of upadacitinib freebase, or a pharmaceutically accept thereof, in an amount sufficient to deliver 15 mg of upadacitinib freebase equivalent, wherein the subject achieves Minimal Disease Activity (MDA) within 24 weeks of administration of the first dose.

**Embodiment 67:** In another aspect, provided is a method of treating active psoriatic arthritis in a subject in need thereof, the method comprising orally administering to the subject once a day for at least 24 weeks a dose of upadacitinib freebase, or a pharmaceutically acceptable salt thereof, in an amount sufficient to deliver 30 mg of upadacitinib freebase equivalent, wherein the subject achieves Minimal Disease Activity (MDA) within 24 weeks of administration of the first dose.

**Embodiment 68:** The method of Embodiment 66 or 67, wherein when the method is used to treat a population of subjects, a portion of the subjects in the treated population achieve Minimal Disease Activity (MDA) within 24 weeks of administration of the first dose. In certain embodiments of Embodiment 68, a statistically significant population of the subjects in the treated population achieve Minimal Disease Activity (MDA) within 24 weeks of administration of the first dose. In certain embodiments of Embodiment 68, at least 10%, at least 15%, at least 20%, at least 25%, at least 30%, at least 35%, at least 40%, or at least 45% of the subjects in the treated population achieve Minimal Disease Activity (MDA) within 24 weeks of administration of the first dose.

**Embodiment 69:** The method of any one of Embodiments 66-68, wherein the subject or subjects in the treated population further achieve a Psoriasis Area Severity Index (PASI) response selected from a PASI 75 response, a PASI 90 response, and a PASI 100 response, within 16 weeks of administration of the first dose, and the PASI response is maintained or improved after Week 16 by continuing to administer the daily dose. In certain embodiments of Embodiment 69, a statistically significant population of subjects in the treated population achieve a PASI 75 response, a PASI 90 response, and a PASI 100 response within 16 weeks of administration of the first dose. In certain embodiments of Embodiment 69, at least 10%, at least 15%, at least 20%, at least 25%, at least 30%, at least 35%, at least 40%, or at least 45% of the subjects in the treated population achieve a PASI 75 response, a PASI 90 response, and a PASI 100 response within 16 weeks of administration of the first dose.

**Embodiment 70:** The method of any one of Embodiments 23-42, wherein the subject or subjects in the treated population achieve a Psoriasis Area Severity Index (PASI) 75 response within 16 weeks of administration of the first dose. In certain embodiments of Embodiment 70, a statistically significant population of subjects in the treated population achieve a PASI 75 response within 16 weeks of administration of the first dose. In certain embodiments of Embodiment 70, at least 10%, at least 15%, at least 20%, at least 25%, at least 30%, at least 35%, at least 40%, or at least 45% of the subjects in the treated population achieve a PASI 75 response within 16 weeks of administration of the first dose.

**Embodiment 71:** The method of any one of Embodiments 23-42 or 70, wherein the subject or subjects in the treated population achieve a Psoriasis Area Severity Index (PASI) 90 response within 16 weeks of administration of the first dose.

**Embodiment 72:** The method of any one of Embodiments 23-42 or 70-71, wherein the subject or subjects in the treated population achieve a Psoriasis Area Severity Index (PASI) 100 response within 16 weeks of administration of the first dose.

**Embodiment 73:** In another aspect, provided is a method of treating active psoriatic arthritis in a subject in need thereof, the method comprising orally administering to the subject once a day for at least 16 weeks a dose of upadacitinib freebase, or a pharmaceutically acceptable salt thereof, in an amount sufficient to deliver 15 mg of upadacitinib freebase equivalent, wherein the subject achieves a Psoriasis Area Severity Index (PASI) 75 response within 16 weeks of administration of the first dose.

**Embodiment 74:** In another aspect, provided is a method of treating active psoriatic arthritis in a subject in need thereof, the method comprising orally administering to the subject once a day for at least 16 weeks a dose of upadacitinib freebase, or a pharmaceutically acceptable salt thereof, in an amount sufficient to deliver 30 mg of upadacitinib free base equivalent, wherein the subject achieves a Psoriasis Area Severity Index (PASI) 75 response within 16 weeks of administration of the first dose.

**Embodiment 75:** The method of Embodiment 73 or 74, wherein when the method is used to treat a population of subjects, a portion subjects in the treated population achieve a PASI 75 response within 16 weeks of administration of the first dose. In certain embodiments of Embodiment 75, a statistically significant population of subjects in the treated population achieve a PASI 75 response within 16 weeks of administration of the first dose. In certain embodiments of Embodiment 75, at least 10%, at least 15%, at least 20%, at least 25%, at least 30%, at least 35%, at least 40%, or at least 45% of the subjects in the treated population achieve a PASI 75 response within 16 weeks of administration of the first dose.

**Embodiment 76:** The method of any one of Embodiments 73-75, wherein the subject or subjects in the treated population achieve a Psoriasis Area Severity Index (PASI) 90 response within 16 weeks of administration of the first dose.

**Embodiment 77:** The method of any one of Embodiments 73-76, wherein the subject or subjects in the treated population achieve a Psoriasis Area Severity Index (PASI) 100 response within 16 weeks of administration of the first dose.

## BRIEF DESCRIPTION OF THE DRAWINGS

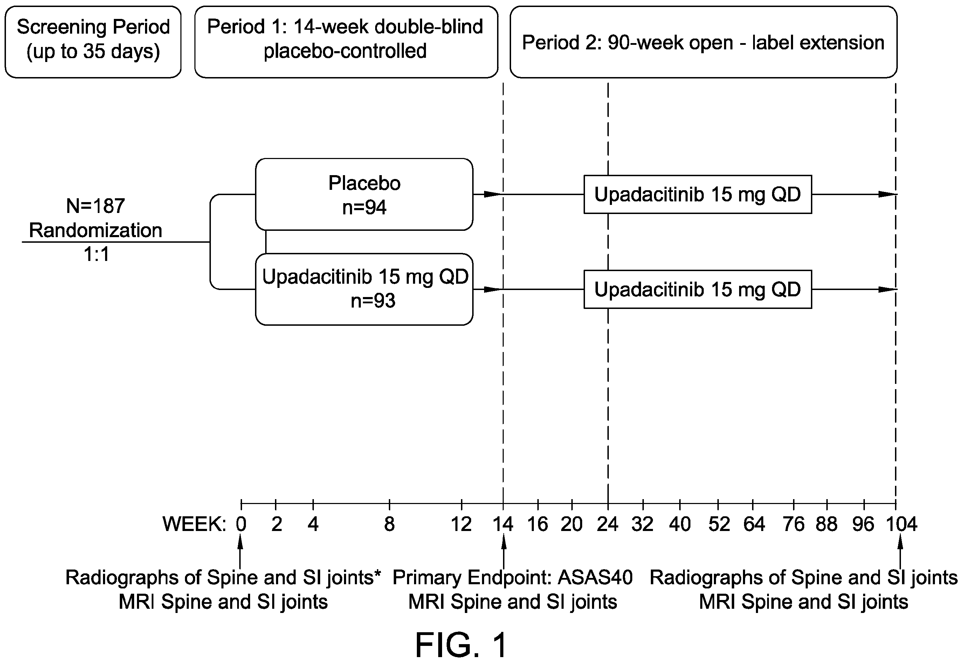

**FIG. 1** depicts the Phase 2/3 Ankylosing Spondylitis (SELECT-AXIS 1) clinical study plan. Asterisk (*) indicates radiographs were conducted during the screening period. ASAS40=Assessment of SpondyloArthritis international Society 40% response. MRI=magnetic resonance imaging. QD=once daily. SI=sacroiliac.

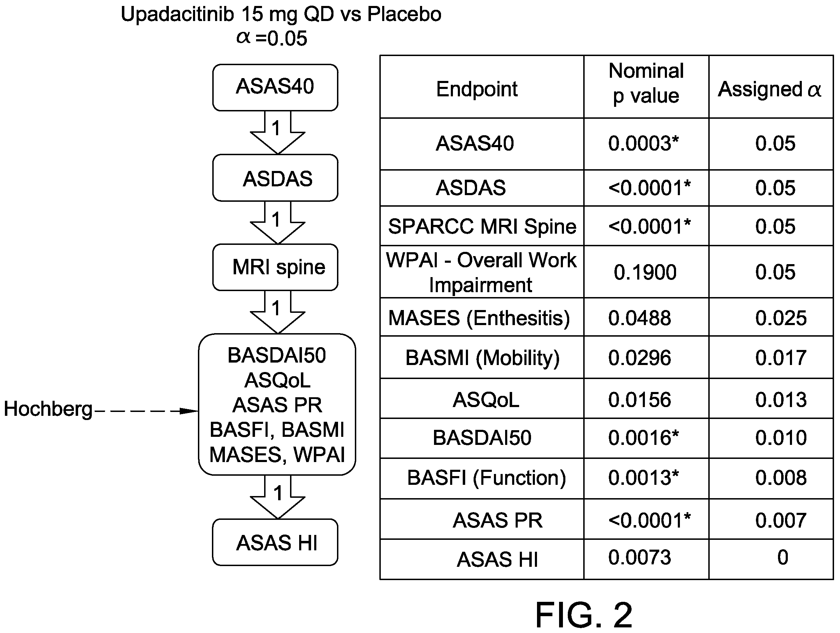

**FIG. 2** depicts the multiplicity-controlled analysis used in the Phase 2/3 Ankylosing Spondylitis (SELECT-AXIS 1) clinical trial using the Hochberg Procedure. Asterisk (*) indicates result was statistically significant in multiplicity-controlled analysis, otherwise nominal p values are shown. The multiplicity-controlled endpoints are tested in a sequential manner with initially assigned $\alpha$=0.05. Statistical significance (p <0.05) can be claimed for a lower ranked endpoint only if the previous endpoint in the sequence meets the requirements of statistical significance. ASAS HI can be evaluated only if the group of endpoints tested by Hochberg procedure are all statistically significant. Within the Hochberg procedure, BASDAI50, BASFI, and ASAS PR achieved the required statistical significance; however, WPAI, MASES, BASMI, and ASQoL did not meet the requirement of statistical significance, so ASAS HI was not tested. Per Hochberg procedure, all endpoints are tested using assigned $\alpha$ according to the magnitude of nominal p value starting from the largest one. If an endpoint is rejected, all endpoints with smaller p values are rejected. If an endpoint fails, then the procedure advances to the next endpoint. ASAS=Assessment of SpondyloArthritis International Society. ASAS40=ASAS 40% response. ASAS HI=ASAS Health Index. ASAS PR=ASAS Partial Remission. ASDAS=Ankylosing Spondylitis Disease Activity Score. ASQoL=Ankylosing Spondylitis Quality of Life. BASDAI50=50% improvement from baseline in Bath Ankylosing Spondylitis Disease Activity Index. BASFI=Bath Ankylosing Spondylitis Functional Index. BASMI=Bath Ankylosing Spondylitis Metrology Index. MASES=Maastricht Ankylosing Spondylitis Enthesitis Score. MRI=magnetic resonance imaging. QD=once daily. SPARCC=Spondyloarthritis Research Consortium of Canada. WPAI=Work Productivity and Activity Impairment.

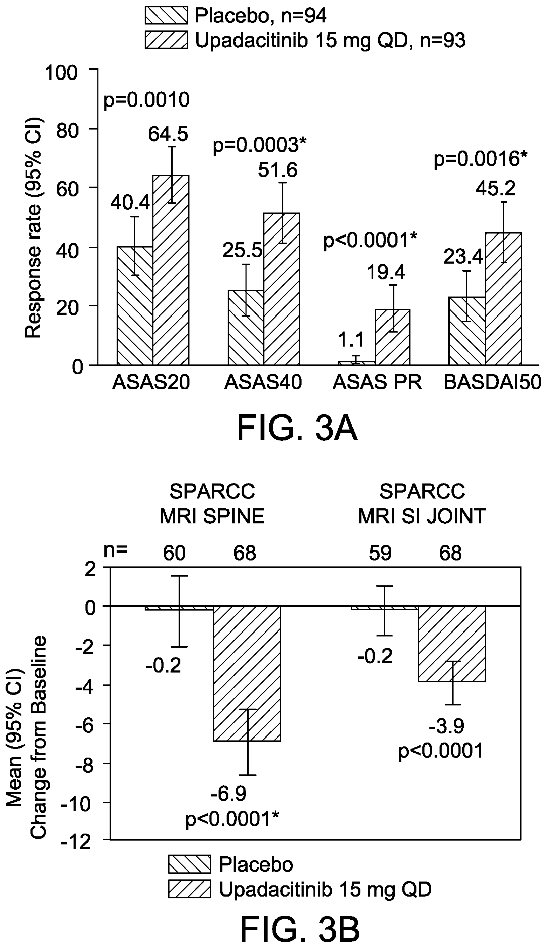

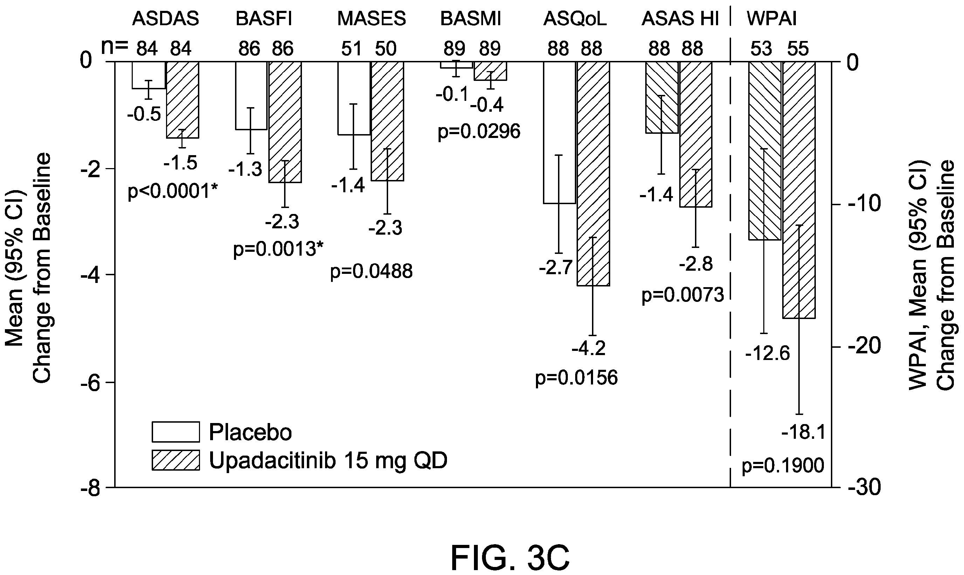

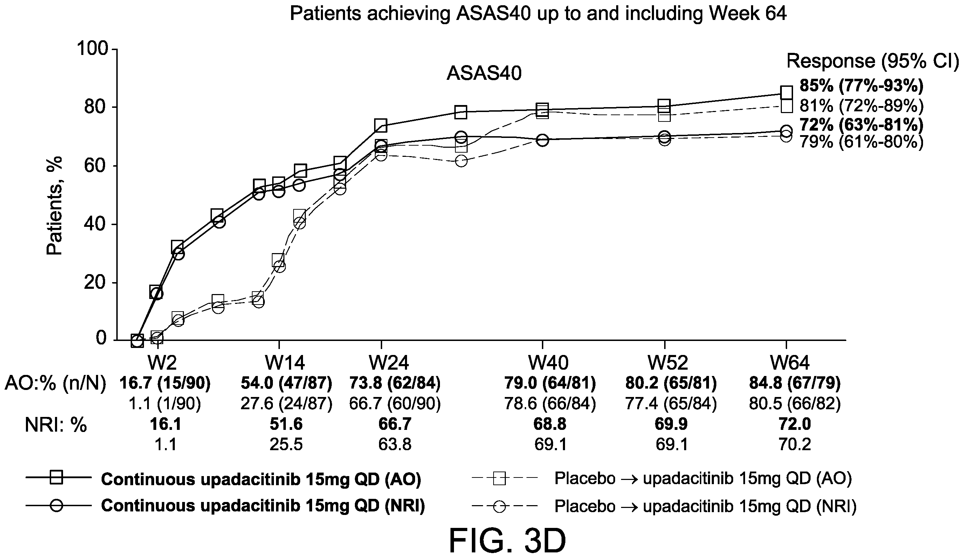

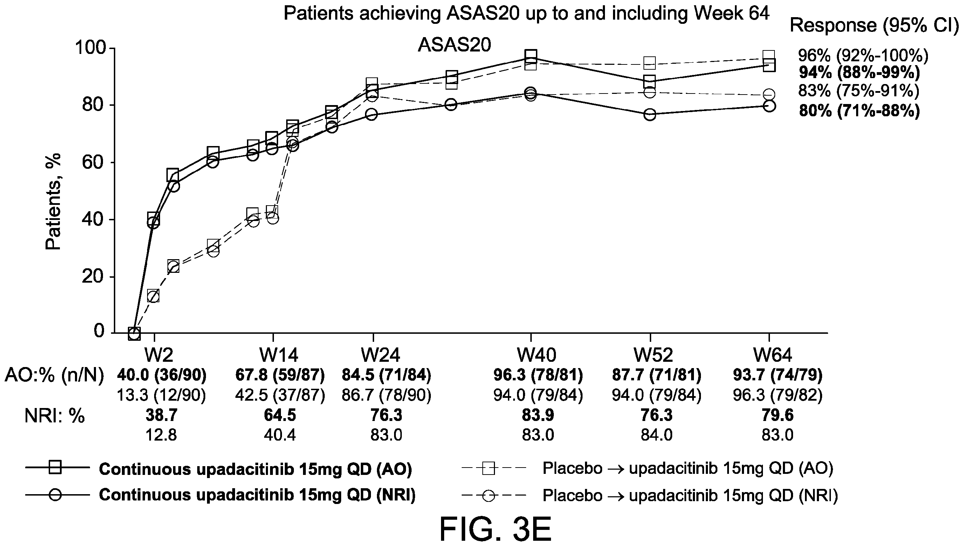

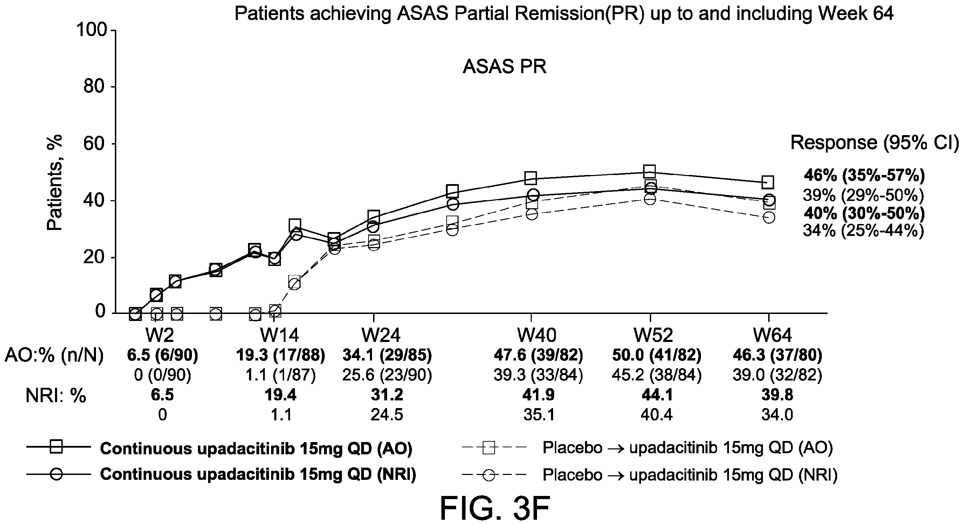

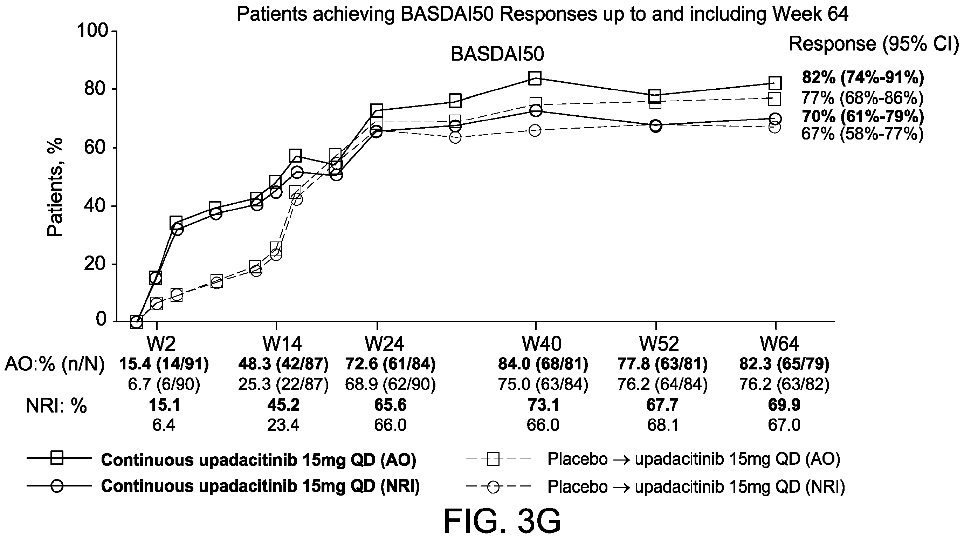

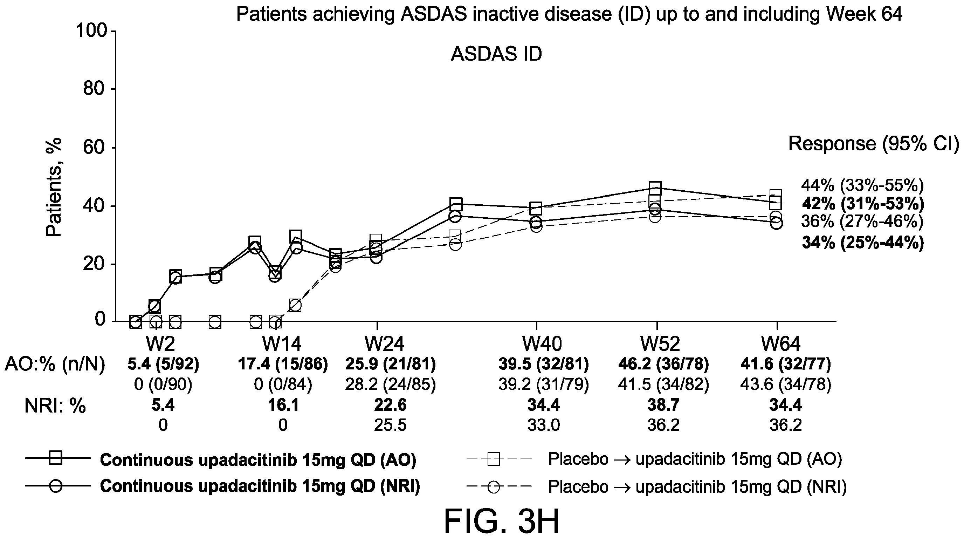

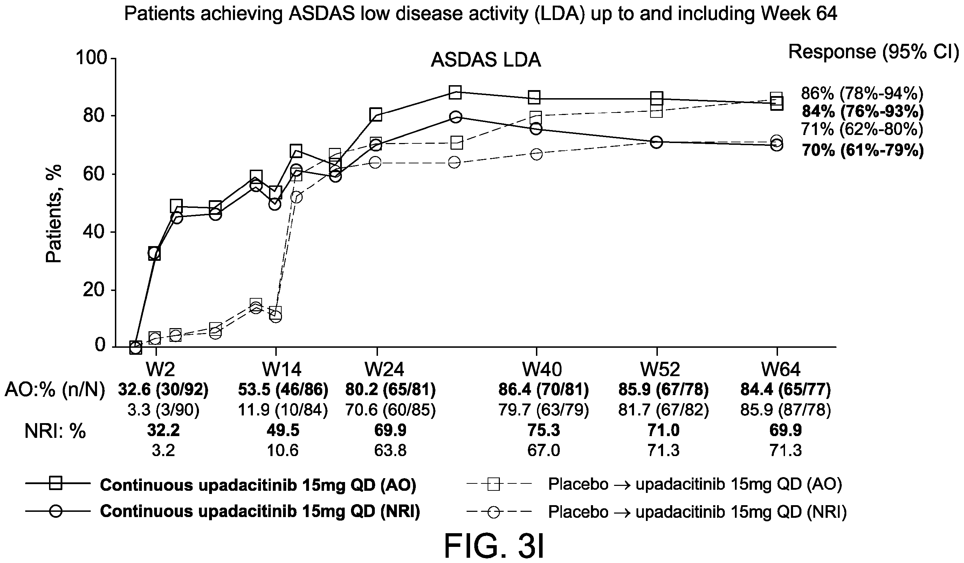

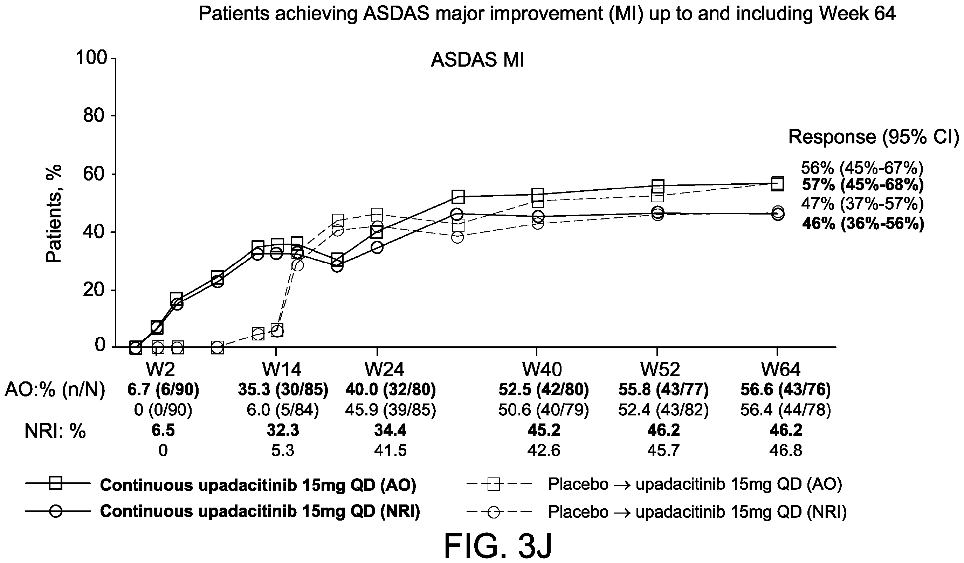

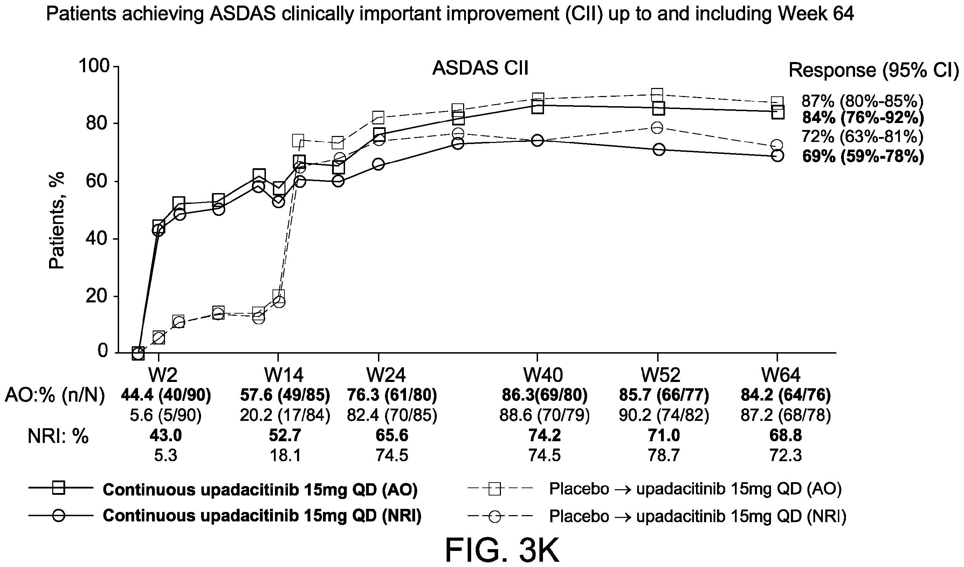

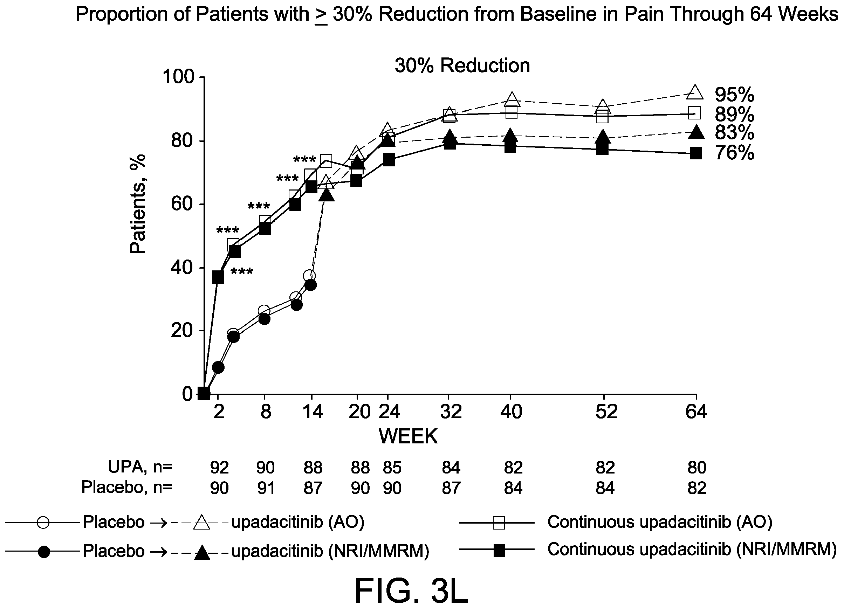

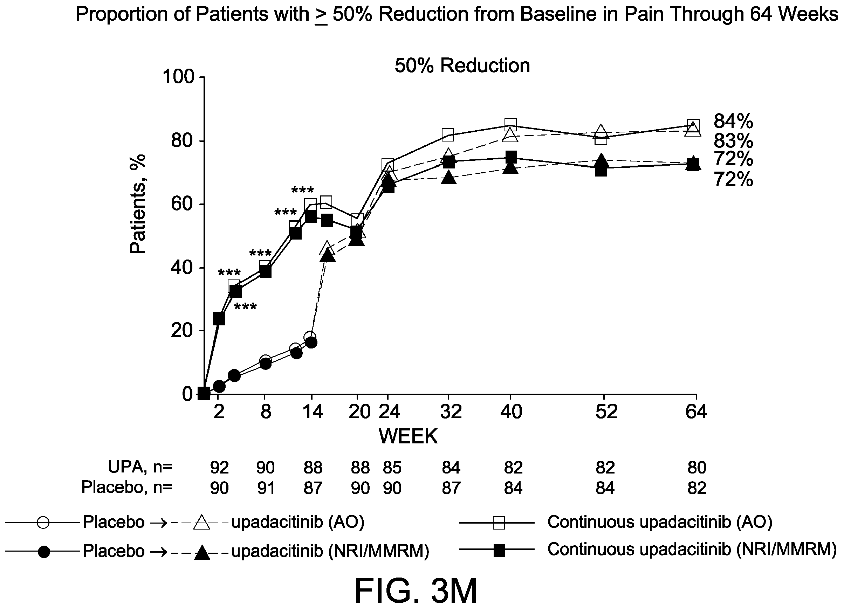

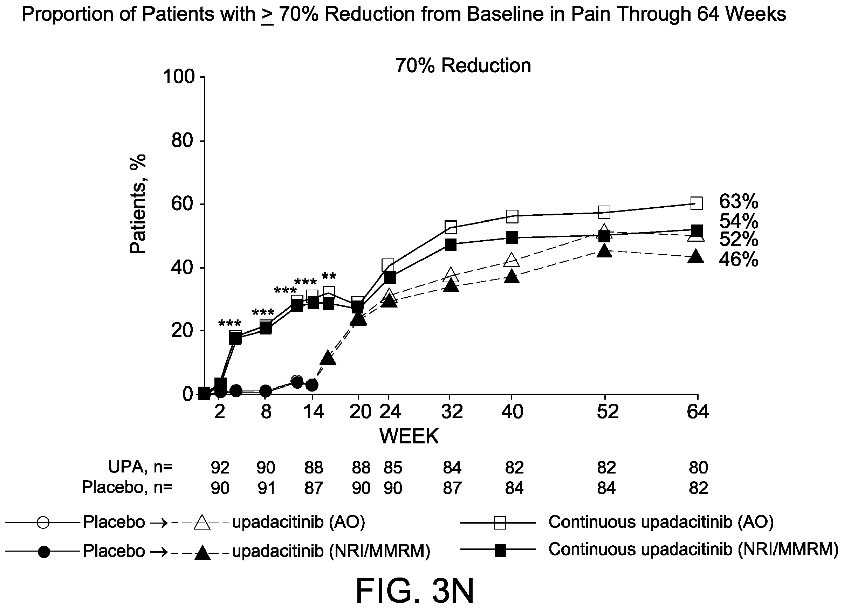

**FIGS. 3A-3C** and **FIGS. 3D-3N** respectively depict the Week 14 results of the Phase 2/3 Ankylosing Spondylitis (SELECT-AXIS 1) clinical trial for key clinical efficacy endpoints, and time course of key endpoints up to and including Week 64. **FIG. 3A** depicts the ASAS20, ASAS40, ASAS PR, and BASDAI50 Responses at Week 14; **FIG. 3B** depicts the change From Baseline in SPARCC MRI Spine and SI Joint Scores at Week 14; and **FIG. 3C** depicts other Multiplicity-Controlled Key Secondary Efficacy Endpoints at Week 14. The results demonstrate the study met its primary endpoint, with statistically significantly more patients treated with upadacitinib freebase versus placebo achieving ASAS40 response at Week 14 (48/93 [51.6%] vs 24/94 [25.5%]; p=0.0003) with a treatment difference (95% CI) of 26.1% (12.6-39.5%). All endpoints were multiplicity controlled, except for ASAS20 and SPARCC MRI SI joint. The multiplicity-controlled secondary endpoints were tested in a sequential manner: ASDAS, SPARCC MRI Spine, group of endpoints tested by Hochberg procedure (BASDAI50, ASQoL, ASAS PR, BASFI, BASMI, MASES, and WPAI), and ASAS HI. For **FIGS. 3L-3N,** Asterisk (***) indicates P<0.001, Asterisk (**) *P<0.01;* and Asterisk (*) *P*<0.05. For other **FIGS.,** Asterisk (*) indicates statistically significant in multiplicity-controlled analysis, otherwise nominal p values are shown. Accounting for multiplicity adjustment, change from baseline to Week 14 in ASDAS (**FIG. 3C**), SPARCC MRI spine (**FIG. 3B**), and BASFI (**FIG. 3C**) and proportion of patients who achieved BASDAI50 (**FIG. 3A**) and ASAS PR (**FIG. 3A**) were statistically significant for upadacitinib freebase versus placebo. **FIGS. 3D-3K** depict the time course of the ASAS40 (**FIG. 3D**), ASAS20 (**FIG. 3E**), ASAS partial remission (PR) (**FIG. 3F**), BASDAI50 Responses (**FIG. 3G**), ASDAS inactive disease (ID) (**FIG. 3H**), ASDAS low disease activity (LDA) (**FIG. 3I**), ASDAS major improvement (MI) (**FIG. 3J**), and ASDAS clinically important improvement (CII) (**FIG. 3K**) up to and including Week 64. At week 14, the placebo group was rescued and administered 15 mg upadacitinib free base (Placebo → Upadacitinib 15 mg QD). Patients who switched from placebo to upadacitinib at week 14 showed a similar efficacy response compared with those who received continuous upadacitinib free base from Day 0. The data suggests upadacitinib 15 mg QD, showing achievement of efficacy at Week 14, and sustaining or even improving upon this efficacy up to and including Week 64, will help to address an unmet need for patients with AS (as well as in patients with non-radiographic axial spondyloarthritis (nr-axSpA)), especially in those patients who have active disease and have inadequately responded to NSAIDs. A significantly higher proportion of patients receiving upadacitinib versus placebo achieved ≥30% (**FIG. 3L**) and ≥50% reduction (**FIG. 3M**) in Patient's Global Assessment of pain (PtPain) as early as week 2, and ≥70% reduction (**FIG. 3N**) as early as week 4, and efficacy achieved was sustained thereafter. Patients who switched from placebo to open-label upadacitinib at week 14 generally reached the same level of pain reduction after week 14 as those initially randomized to upadacitinib. MASES assessment includes patients with baseline enthesitis; WPAI assessment includes patients currently employed; SPARCC MRI assessment population as pre-specified in the statistical analysis plan (baseline included MRI data ≤3 days after first dose of study drug, and Week 14 included MRI data up to first dose of Period 2 study drug). ASAS20=Assessment of SpondyloArthritis international Society 20 response. ASAS40=Assessment of SpondyloArthritis international Society 40 response. ASQoL=Ankylosing Spondylitis Quality of Life score. ASDAS=Ankylosing Spondylitis Disease Activity Score. BASDAI50=50% improvement from baseline in Bath Ankylosing Spondylitis Disease Activity Index. BASFI=Bath Ankylosing Spondylitis Functional Index. BASMI=Bath Ankylosing Spondylitis Metrology Index. HI=Health Index. MASES=Maastricht Ankylosing Spondylitis Enthesitis Score. MRI=magnetic resonance imaging. MMRM=mixed model for repeated measures. NRI=non-responder imputation. AO = As Observed. PR=partial remission. QD=once daily. SI, sacroiliac. SPARCC=Spondyloarthritis Research Consortium of Canada. WPAI=Work Productivity and Activity Impairment.

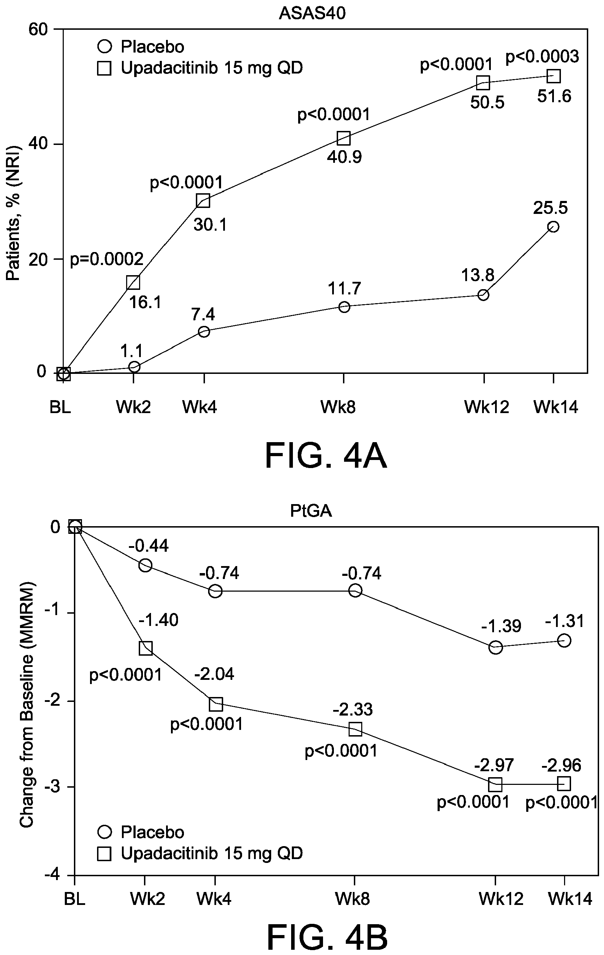

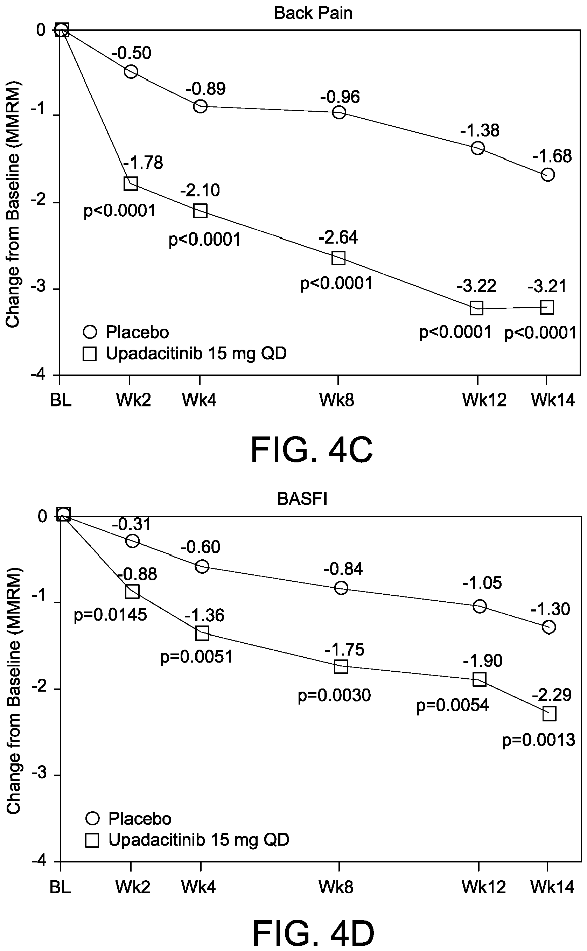

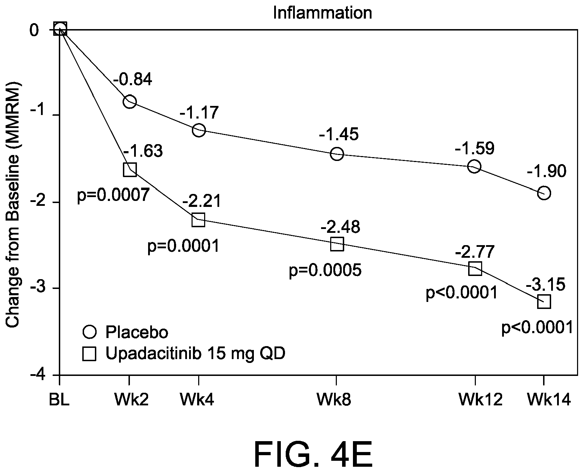

**FIGS. 4A-4E** depict data for ASAS domains (ASAS40, PtGA, Back Pain, BASFI, and Inflammation) measured at Weeks 2, 4, 8, 12, and 14 in the Phase 2/3 Ankylosing Spondylitis (SELECT-AXIS 1) clinical trial. A significant difference for upadacitinib freebase versus placebo in ASAS40 (**FIG. 4A**) and the mean change for each of its four individual domains (**FIG. 4B-4E**) was observed as early as the first post-baseline visit (Week 2), and this difference was maintained consistently through Week 14, with Week 14 achieving a statistically significant difference in multiplicity-controlled analysis. Back pain defined on a numerical rating scale (0-10) based on the following question, "What is the amount of back pain that you experienced at any time during the last week? Inflammation is defined as mean of Questions 5 and 6 of the BASDAI. BASDAI=Bath Ankylosing Spondylitis Disease Activity Index. BASFI=Bath Ankylosing Spondylitis Functional Index. BL=baseline. LSM=least squares mean. MMRM=mixed model for repeated measures. NRI=non-responder imputation. PtGA=Patient Global Assessment of disease activity. QD=once daily.

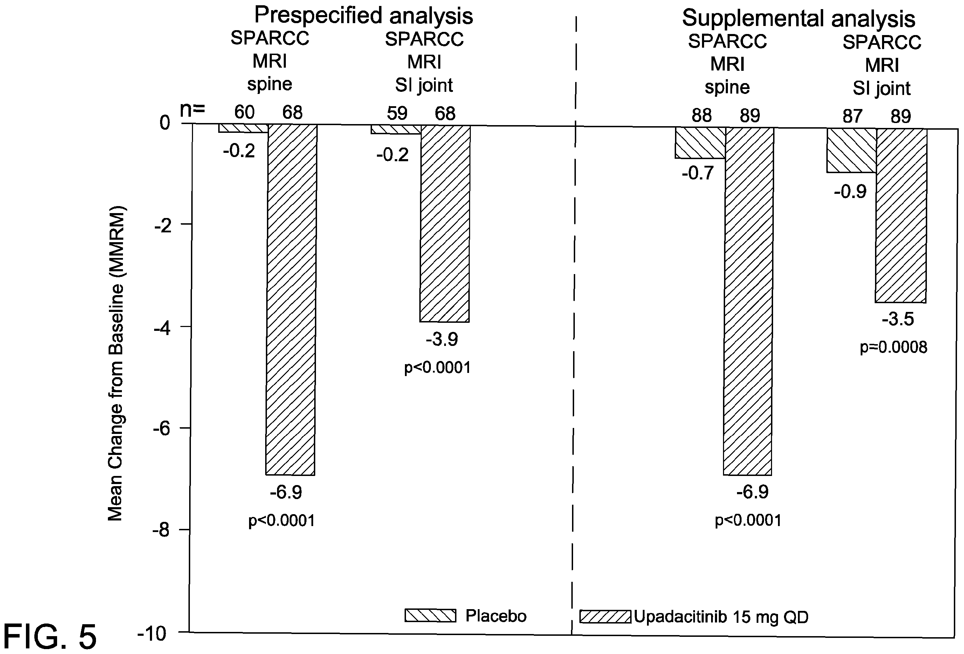

**FIG. 5** depicts the pre-specified and supplemental SPARCC MRI Analysis of the Phase 2/3 Ankylosing Spondylitis (SELECT-AXIS 1) clinical trial. The SPARCC MRI assessment population was pre-specified in the statistical analysis plan (baseline included MRI data ≤3 days after first dose of study drug, and Week 14 included MRI data up to first dose of period 2 study drug). The supplemental SPARCC MRI analysis included all MRI data collected at nominal visits at baseline and Week 14, and confirmed the results of the primary SPARCC MRI analysis for both the spine and SI joints. MMRM=mixed model for repeated measures. MRI=magnetic resonance imaging. QD=once daily. SI=sacroiliac. SPARCC=Spondyloarthritis Research Consortium of Canada.

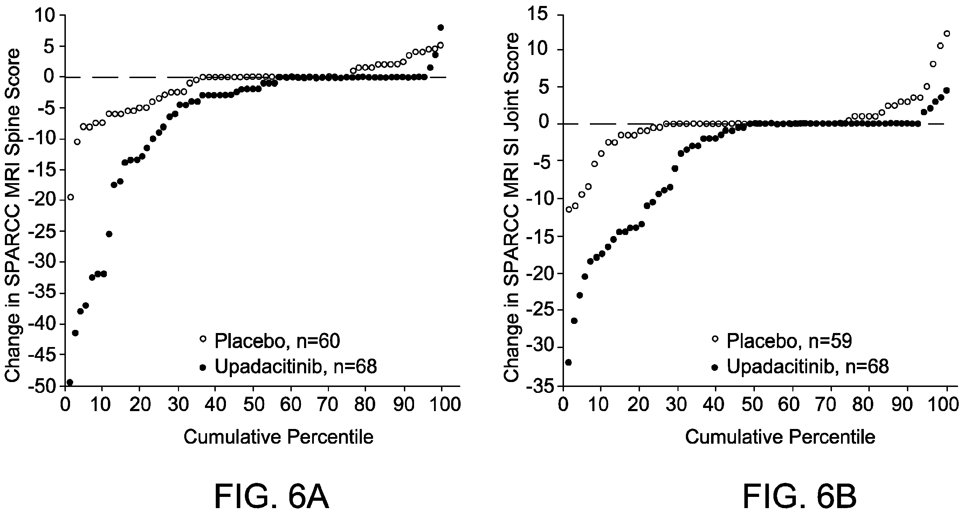

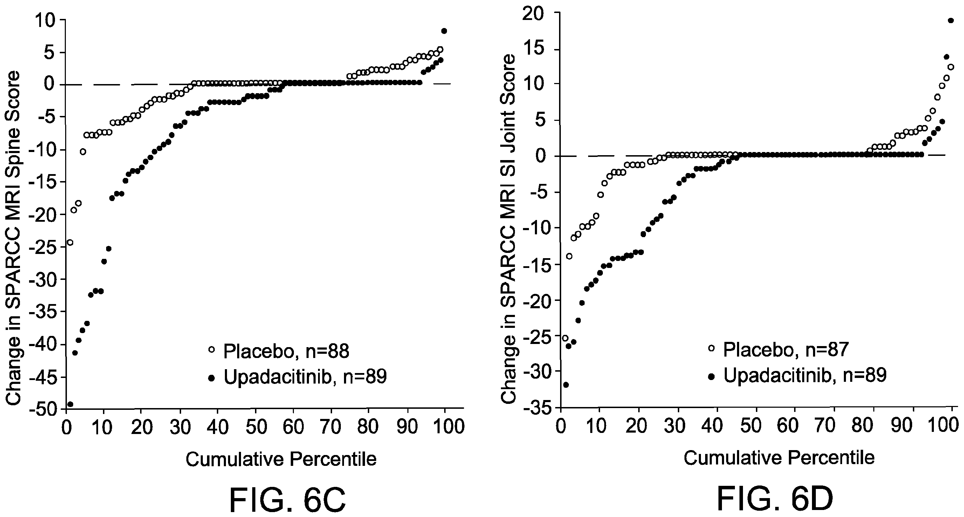

**FIGS. 6A-6D** depict the cumulative probability plots of change in SPARCC scores as described in **FIG. 5** for the Phase 2/3 Ankylosing Spondylitis (SELECT-AXIS 1), demonstrating that the SPARCC MRI spine and SI joint scores improved from baseline to week 14 to a greater extent in patients receiving upadacitinib compared with placebo. Results for the primary MRI analyses (**FIG. 6A-6B**) and supplemental MRI analyses (**FIG. 6B-6C**) were consistent.

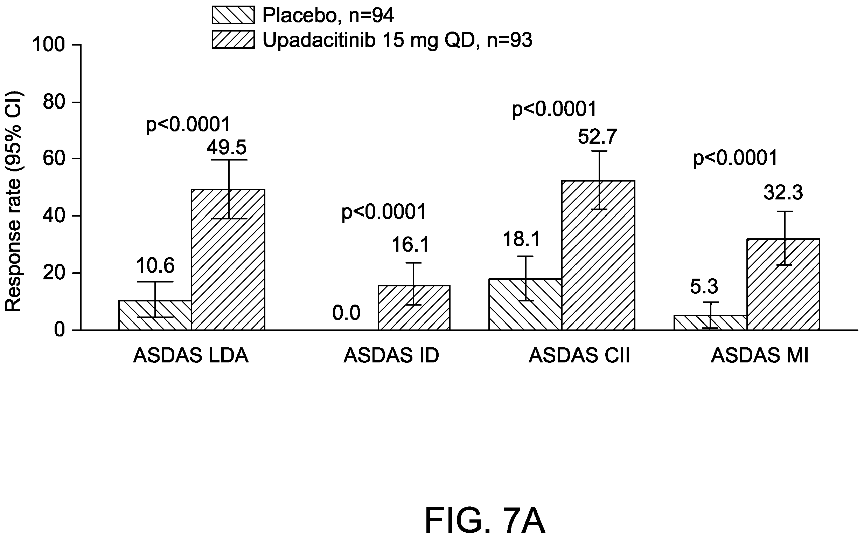

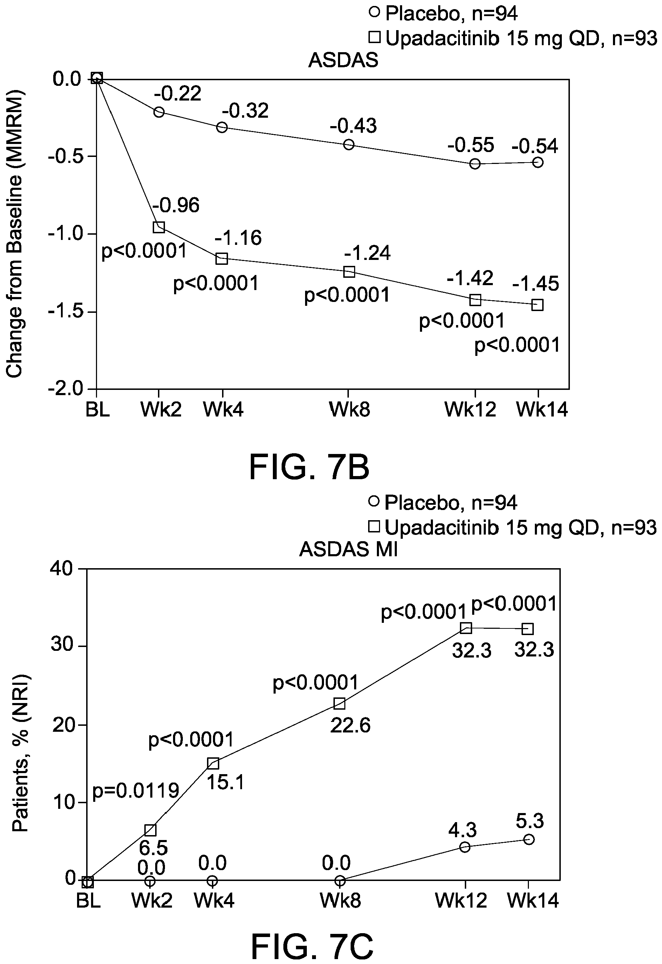

**FIGS. 7A-7C** depict the percentage of patients achieving ASDAS LDA, ASDAS ID, ASDAS CII, and ASDAS MI at Week 14 (**FIG. 7A**), Change From Baseline in Mean ASDAS Over Time (**FIG. 7B**), and ASDAS MI Over Time (**FIG. 7C**) in the Phase 2/3 Ankylosing Spondylitis (SELECT-AXIS 1) clinical trial. The proportions of patients who achieved ASDAS LDA, ASDAS ID, ASDAS CII, and ASDAS MI were greater (nominal p<0.0001) for upadacitinib freebase versus placebo at Week 14 (**FIG. 7A**). ASDAS=Ankylosing Spondylitis Disease Activity Score. BL=baseline. CII=Clinically Important Improvement (≥1·1-point decrease from baseline). ID=Inactive Disease (score <1·3). LDA=low disease activity (<2.1). MI=Major Improvement (>_2-point decrease from baseline). MMRM=mixed model for repeated measures. NRI=non-responder imputation. QD=once daily.

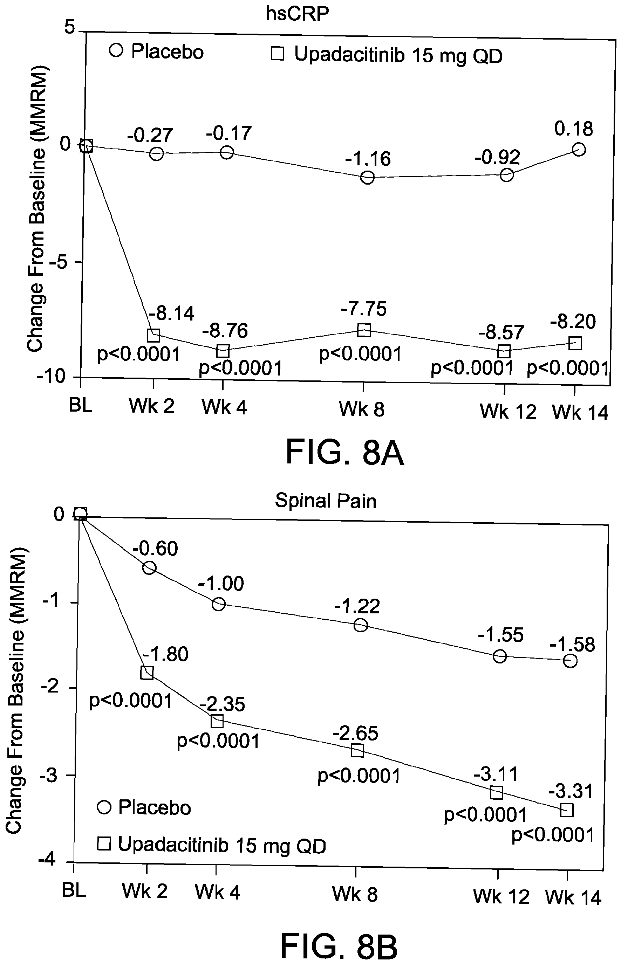

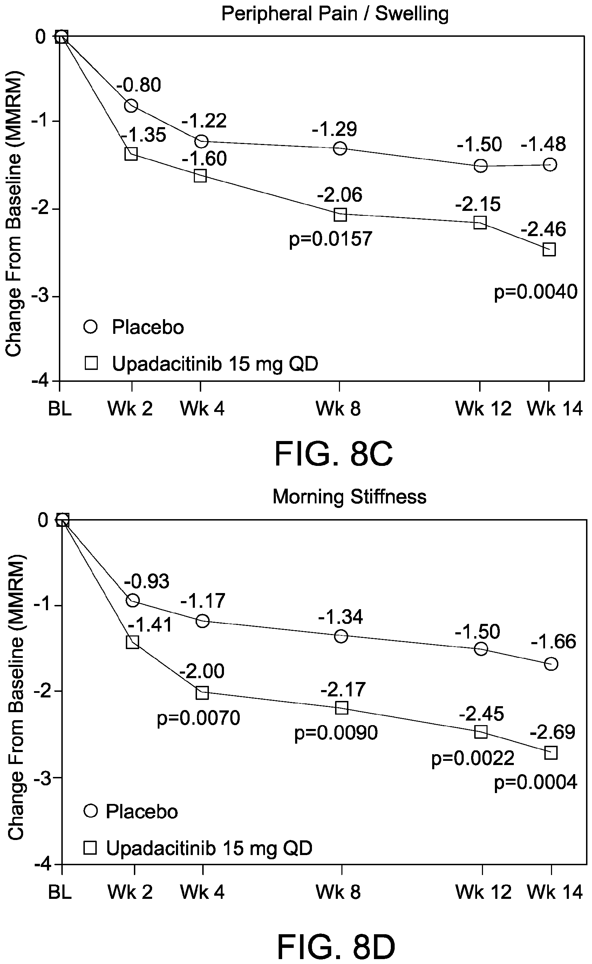

**FIGS. 8A-8D** depict the least squares mean (LSM) change from baseline in individual ASDAS components over time in the Phase 2/3 Ankylosing Spondylitis (SELECT-AXIS 1) clinical trial. Improvement in the mean ASDAS (**FIG. 7B**) and the individual ASDAS components (**FIG. 8A-8D**) was seen as early as Week 2 with continued improvement up to Week 14 with upadacitinib freebase. Spinal pain = BASDAI Question 2. Peripheral pain/swelling = BASDAI Question 3. Duration of morning stiffness = BASDAI Question 6. ASDAS=Ankylosing Spondylitis Disease Activity Score. BASDAI=Bath Ankylosing Spondylitis Disease Activity Index. BL=baseline. hsCRP=high-sensitivity C-reactive protein. MMRM=mixed model for repeated measures. PtGA=Patient Global Assessment of disease activity. QD=once daily.

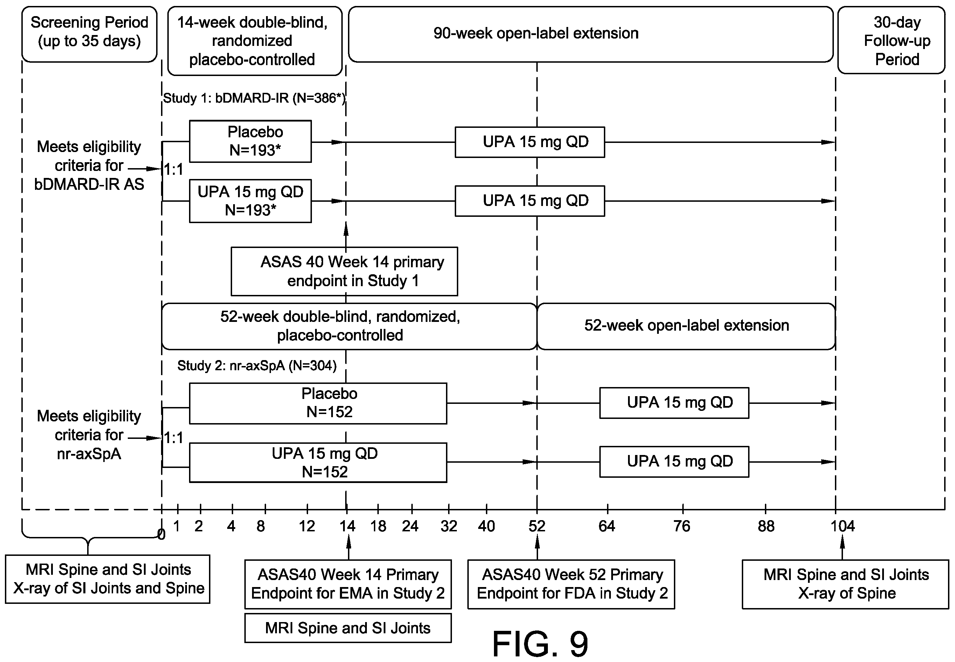

**FIG. 9** depicts the Phase 3 study design for the treatment of AS and nr-AxSpA subjects. AS = ankylosing spondylitis; ASAS = Assessment of SpondyloArthritis International Society; bDMARD-IR = biologic disease-modifying antirheumatic drug inadequate responder; EMA = European Medicines Agency; FDA = Food and Drug Administration; MRI = magnetic resonance imaging; nr-axSpA = non-radiographic axial spondyloarthritis; QD = once daily; SI = sacroiliac; UPA = upadacitinib freebase.

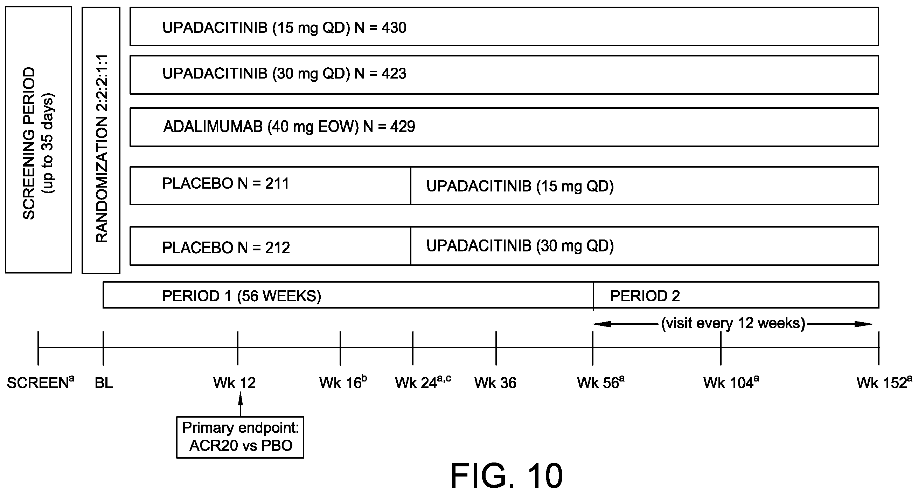

**FIG. 10** depicts the Phase 3 Study design for SELECT-PSA1 in subjects with active PsA and a previous inadequate response to at least one non-biologic DMARD (PsA DMARD-IR). a. All subjects will receive x-rays of hands and feet at Screening, Week 24, Week 56, Week 104, and Week 152. b. At Week 16 rescue therapy will be offered to subjects classified as non-responders (defined as not achieving at least 20% improvement in either or both tender joint count (TJC) and swollen joint count (SJC) at both Week 12 and Week 16). c. At Week 24, all placebo subjects will switch to upadacitinib freebase 15 mg QD or 30 mg QD (1:1 ratio) regardless of response.

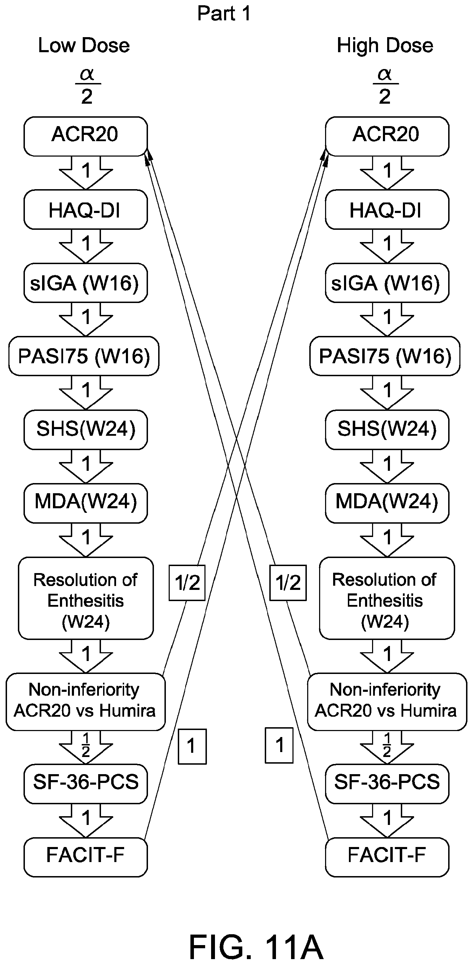

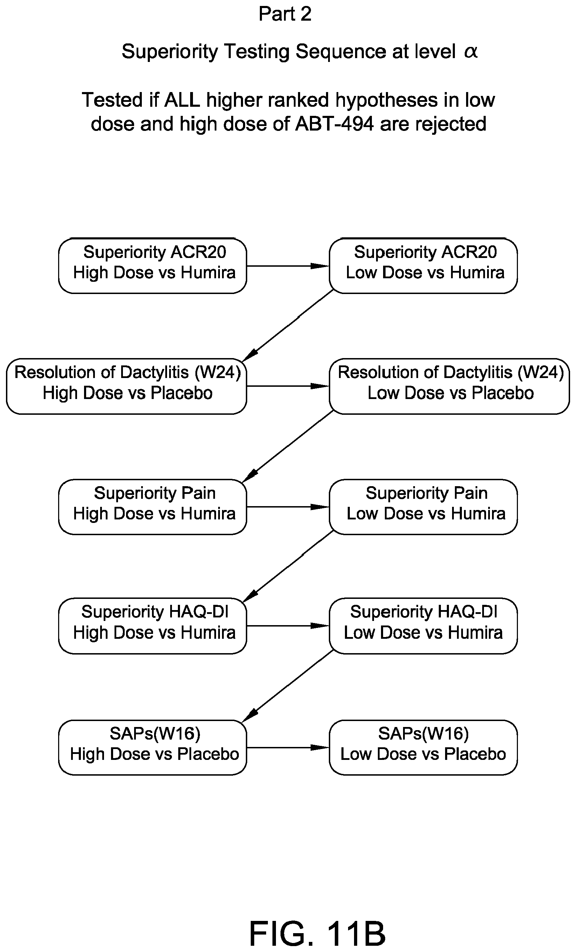

**FIGS. 11A-11B** depict the graphical testing procedure in the Phase 3 SELECT-PSA1 (PsA DMARD-IR) for the primary endpoint (ACR20 response at Week 12) and ranked secondary endpoints. The overall type I error rate was controlled at the 2-sided 0.05 level (including a 0.0001 α for the interim futility analysis). **FIG. 11A,** Part 1, describes the testing sequence of the adequately powered ranked secondary endpoints in PsA symptoms and radiographic progression. **FIG. 11B,** Part 2, describes the testing sequence of additional ranked secondary endpoints including superiority tests versus adalimumab. Part 2 endpoints are tested only after statistical significance of all Part 1 endpoints are achieved.

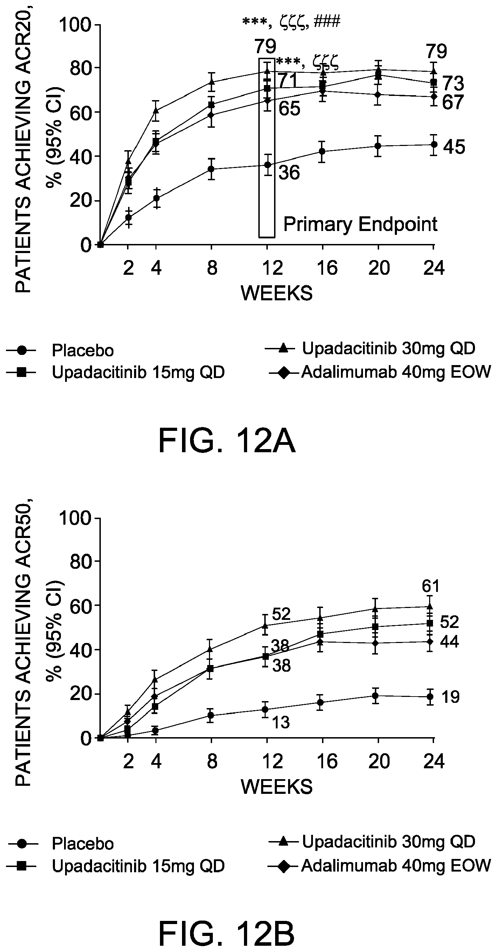

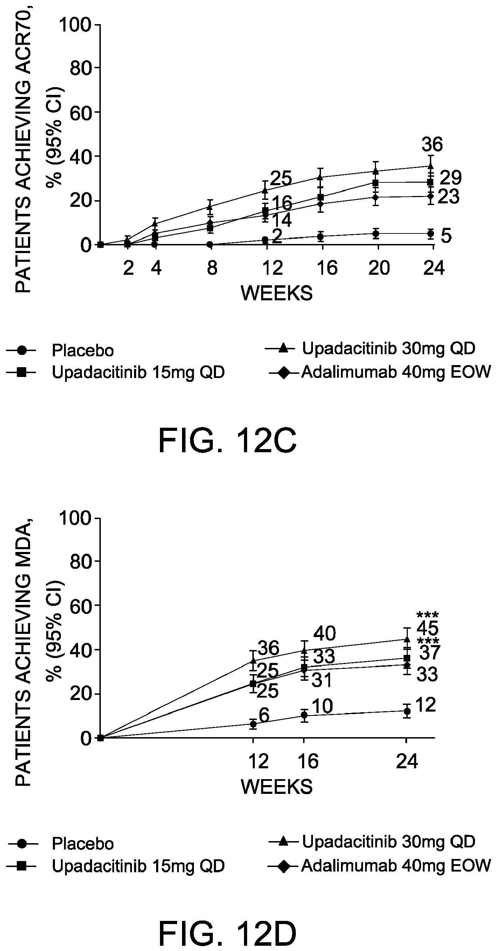

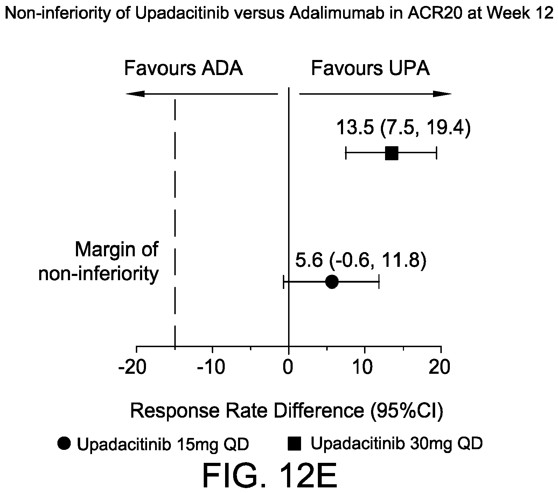

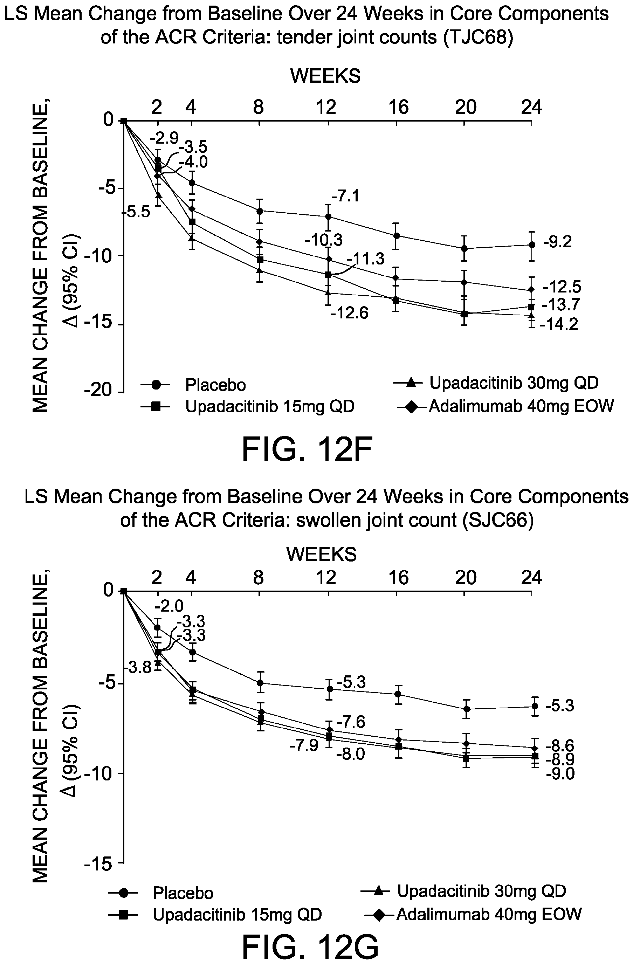

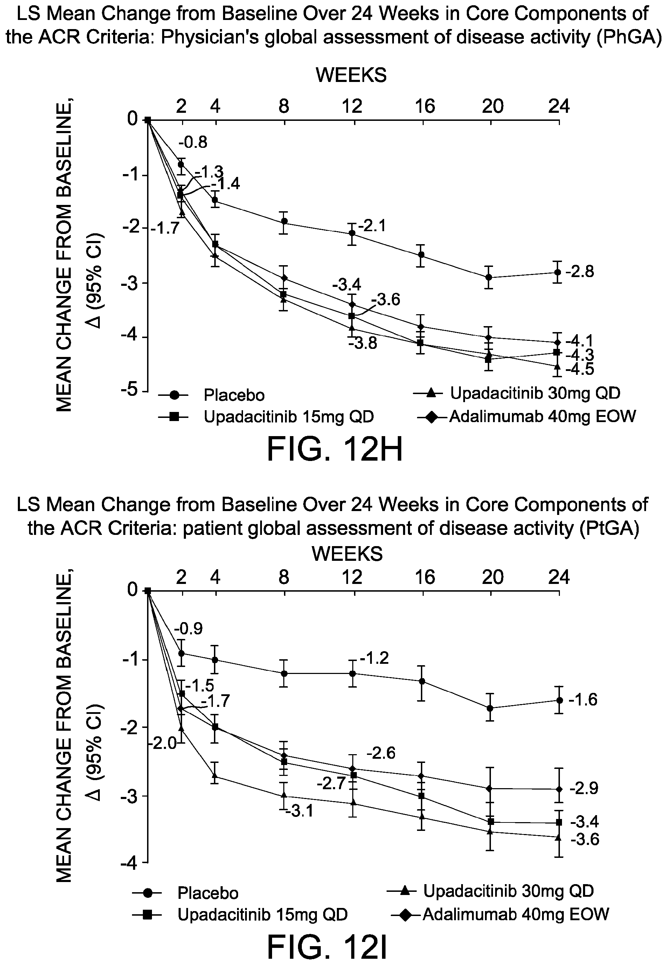

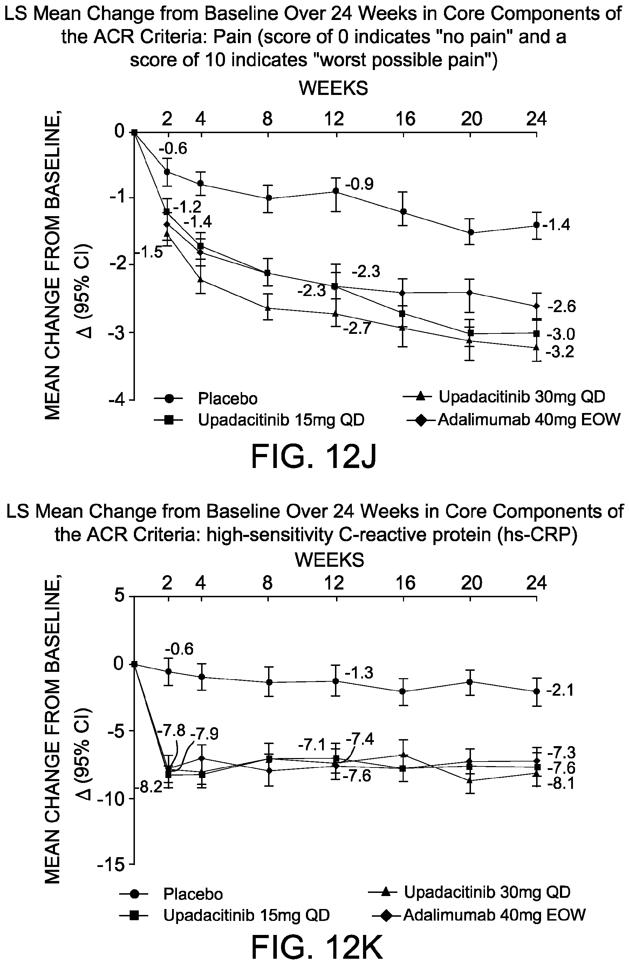

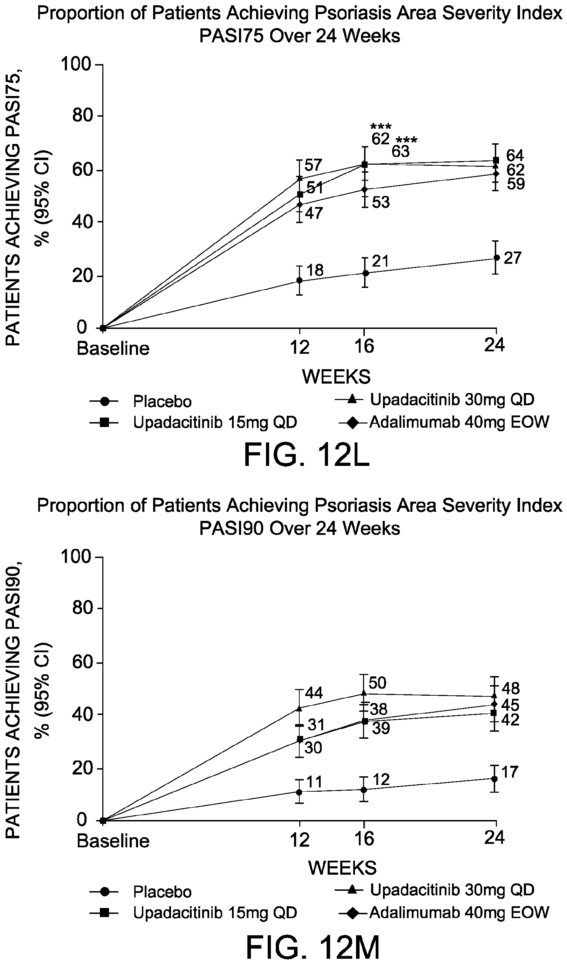

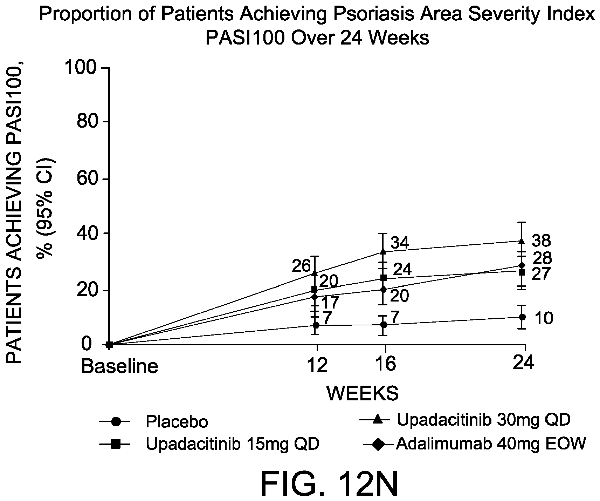

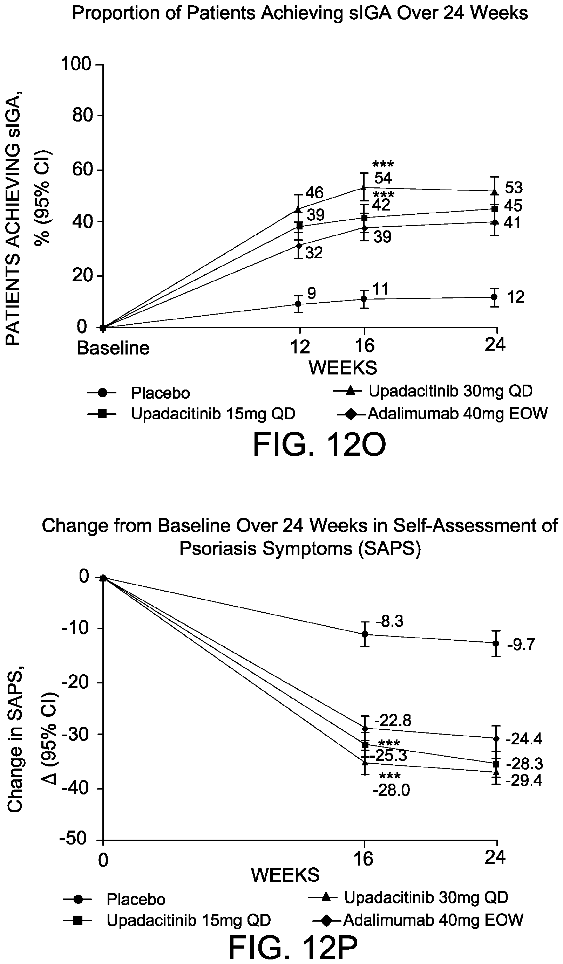

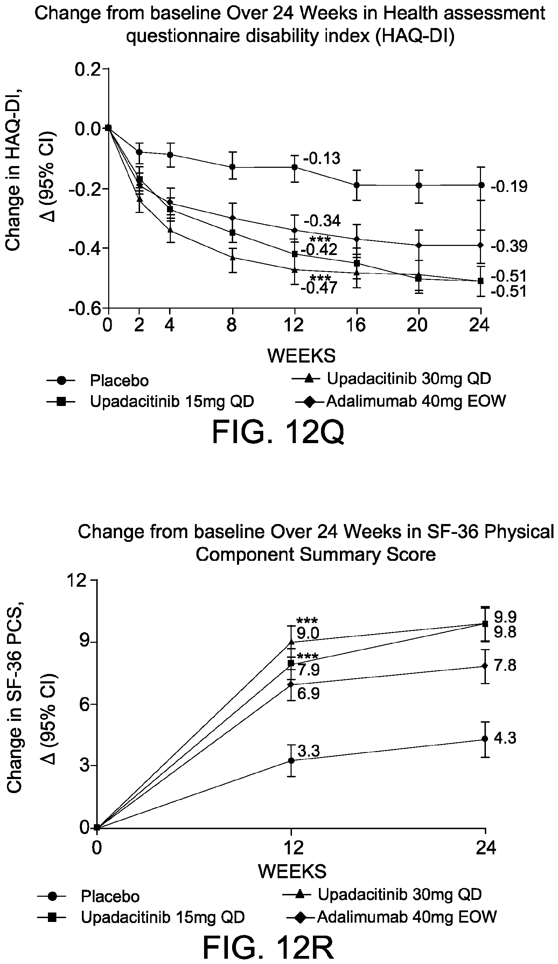

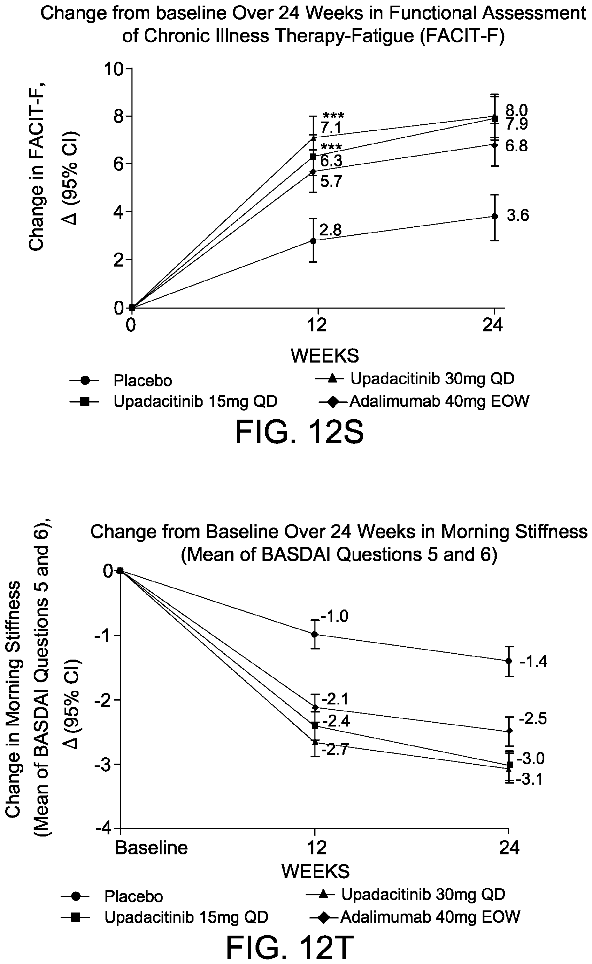

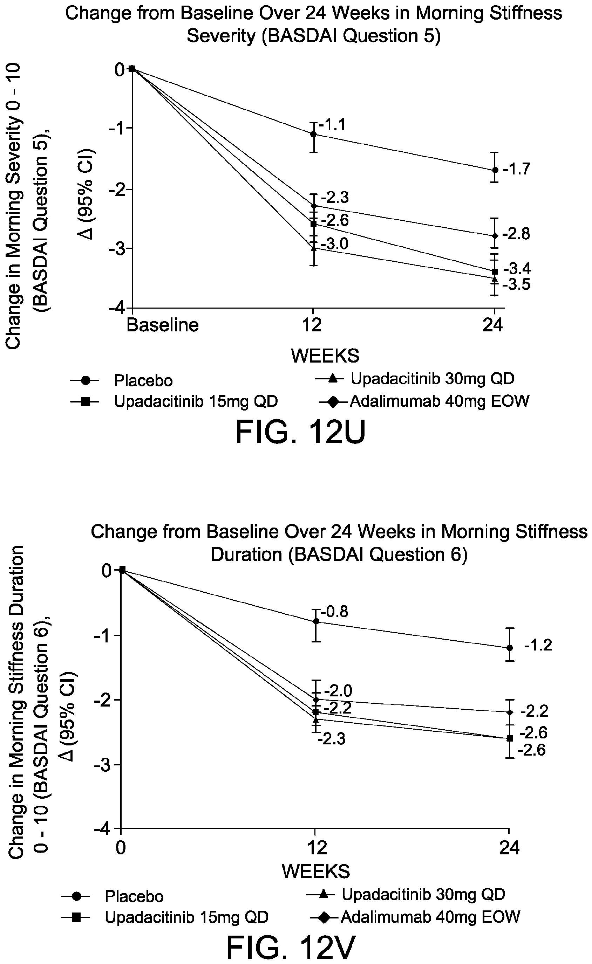

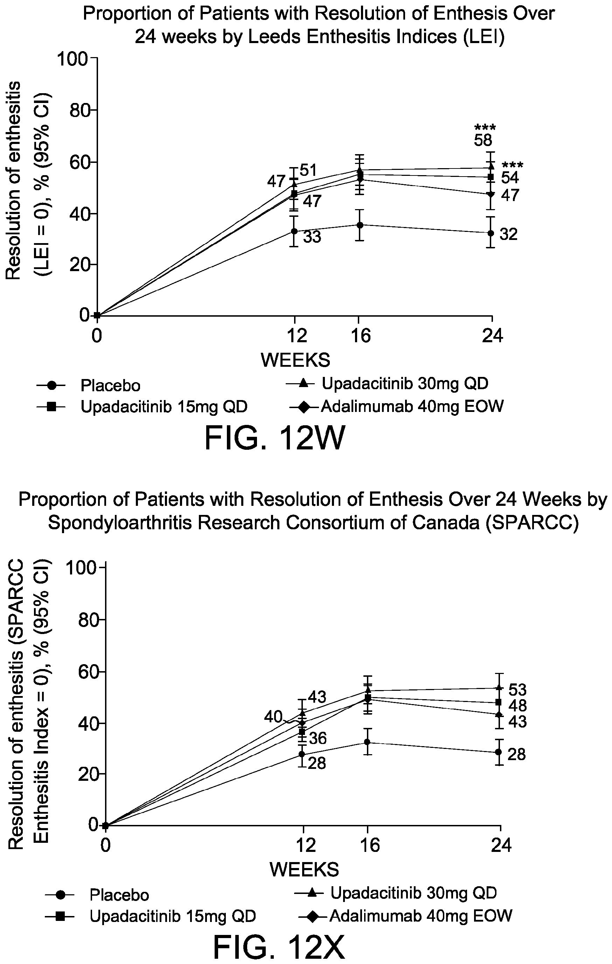

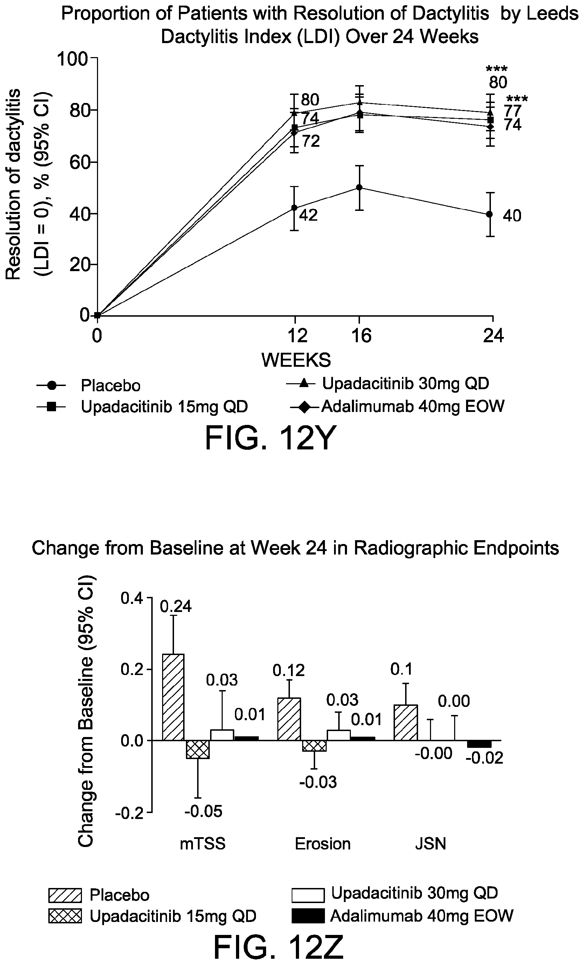

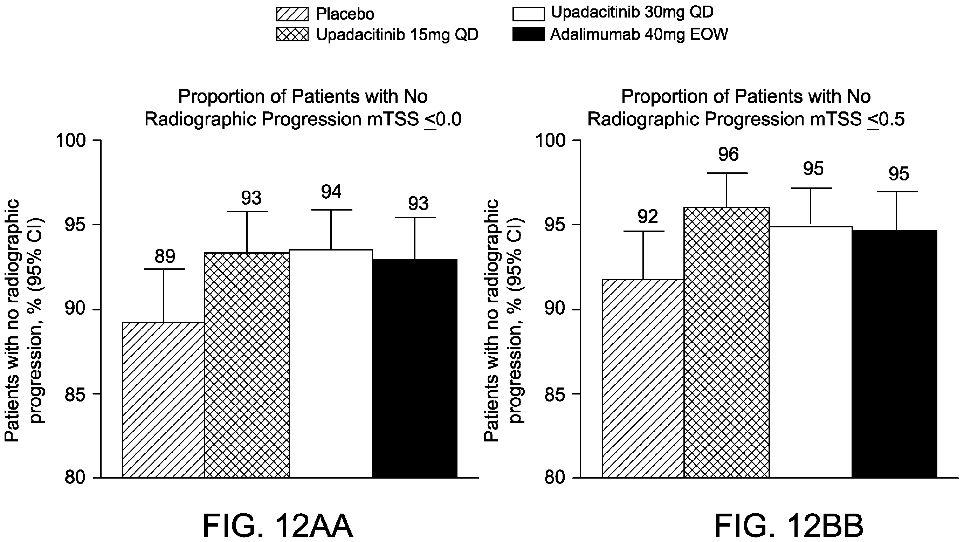

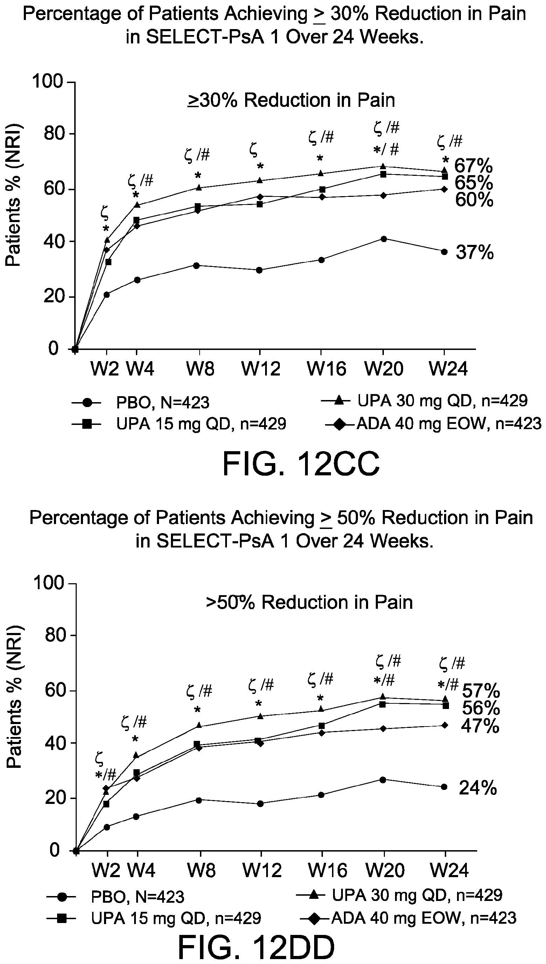

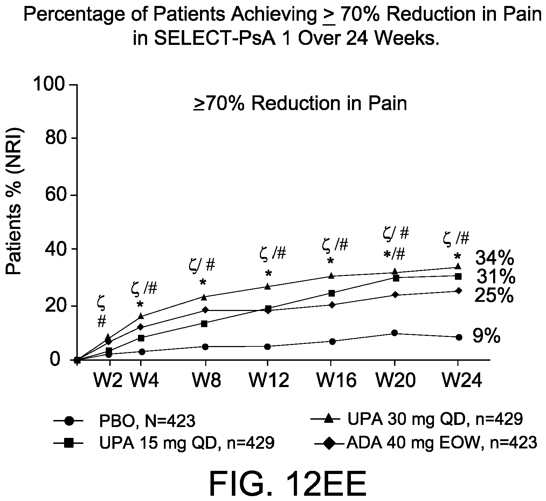

**FIGS. 12A-12EE** provide a summary of primary and key secondary efficacy results for the SELECT-PSA1 (PsA DMARD-IR) Phase 3 clinical trial. **FIG. 12A** depicts the time course of the ACR20 response and associated 95% confidence interval (CI) up to and including Week 24. Response rate (%) with 95% CI is presented by visit. Nominal p-value < 0.05 for both UPA doses vs placebo for all visits. UPA = upadacitinib freebase; ADA = adalimumab; QD = once daily; EOW = every other week. Symbols: ***, P<0.001 for upadacitinib vs. placebo; ###, P<0.001 for upadacitinib vs. adalimumab; ζζζ, P<0.001 for non-inferiority upadacitinib vs. adalimumab. Additional key secondary efficacy results include: patients achieving ACR50 response (**FIG. 12B**); patients achieving ACR70 response (**FIG. 12C**); patients achieving Minimal Disease Activity (MDA) (**FIG. 12D**); patients achieving Non-inferiority of Upadacitinib versus Adalimumab in ACR20 at Week 12 (**FIG. 12E**); patients achieving LS mean change from baseline over 24 weeks in core components of the ACR criteria: tender joint count (TJC68) (**FIG. 12F**), swollen joint count (SJC66) (**FIG. 12G**), Physician's global assessment of disease activity (PhGA) (**FIG. 12H**), patient's global assessment of disease activity (PtGA) (**FIG. 12I**), Pain (PtPain) (score of 0 indicates "no pain" and a score of 10 indicates "worst possible pain") (**FIG. 12J**), and high-sensitivity C-reactive protein (hs-CRP) (**FIG. 12K**); proportion of patients achieving Psoriasis Area Severity Index PASI 75 (**FIG. 12L**), PASI 90 (**FIG. 12M**), and PASI 100 (**FIG. 12N**) over 24 weeks, wherein after week 16 assessments were performed and patients were able to use concomitant treatments specifically for psoriasis per investigator judgment; proportions of patients achieving Static Investigator Global Assessment (sIGA) over 24 weeks (**FIG. 12O**); change from baseline over 24 weeks in Self-Assessment of Psoriasis Symptoms (SAPS) (**FIG. 12P**); patients achieving change from baseline over 24 weeks in health assessment questionnaire disability index (HAQ-DI) (**FIG. 12Q**) and SF-36 Physical Component Summary Score (the PCS is one of two summary scores calculated from the eight SF-36 domains. A linear algorithm is applied to calculate the PCS which has normative mean value of 50, with higher scores indicating better outcomes) (**FIG. 12R**); patients achieving change from baseline over 24 weeks in Functional Assessment of Chronic Illness Therapy-Fatigue (FACIT-F) (the FACIT-F score ranges from 0-52, with higher scores indicating less fatigue) (**FIG. 12S**); patients achieving change from baseline over 24 weeks in Morning Stiffness (Mean of BASDAI Questions 5 and 6) (**FIG. 12T**), Morning Stiffness Severity (BASDAI Question 5) (**FIG. 12U**), and Morning Stiffness Duration (BASDAI Question 6) (**FIG. 12V**); Proportion of Patients with Resolution of Enthesis Over 24 Weeks by Leeds Enthesitis Indices (LEI) (**FIG. 12W**), by Spondyloarthritis Research Consortium of Canada (SPARCC) (**FIG. 12X**), and Proportion of Patients with Resolution of Dactylitis by Leeds Dactylitis Index (LDI) (**FIG. 12Y**); patients achieving change from baseline at Week 24 in radiographic endpoints (mTSS = modified total Sharp/van der Heijde Score, JSN = joint space narrowing score) (**FIG. 12Z**); and proportion of patients with no radiographic progression mTSS ≤0.0 ((**FIG. 12AA**) and mTSS ≤0.5 at Week 24 (**FIG. 12BB**). A significantly higher proportion of patients receiving upadacitinib freebase (15 mg QD or 30 mg QD) versus placebo achieved ≥30% and ≥50% reduction in Patient's Global Assessment of pain (PtPain) as early as week 2, and efficacy achieved was sustained thereafter (see **FIGS. 12CC** and **12DD**). A significantly higher proportion of patients receiving upadacitinib freebase versus placebo achieved ≥70% reduction in PtPain as early as week 2 (30 mg QD upadacitinib) or as early as week 4 (15 mg QD upadacitinib), and efficacy achieved was sustained thereafter (see **FIG. 12EE**). Symbols: *, statistically significant at 0.05 level (UPA 15 mg QD); ζ, statistically significant at 0.05 level (UPA 30 mg QD); #, statistically significant at 0.05 level (ADA 40 mg EOW);***, P<0.001 for upadacitinib vs. placebo; ###, P<0.001 for upadacitinib vs. adalimumab; ζζζ, P<0.001 for non-inferiority upadacitinib vs. adalimumab; PBO = placebo; ADA = adalimumab; UPA = upadacitinib freebase; EOW = every other week; QD = once daily;CI = confidence interval.

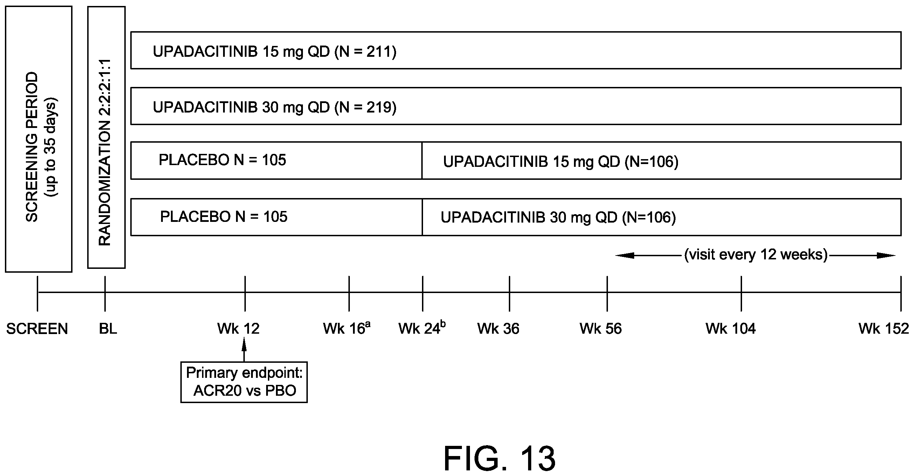

**FIG. 13** depicts the Phase 3 Study design for SELECT-PSA2 in subjects with active PsA and a previous inadequate response to at least one bDMARD (PsA bDMARD-IR). a. At Week 16 rescue therapy will be offered to subjects classified as non-responders (defined as not achieving at least 20% improvement in either or both tender joint count (TJC) and swollen joint count (SJC) at both Week 12 and Week 16). b. At Week 24, all placebo subjects will switch to upadacitinib freebase 15 mg QD or 30 mg QD (1:1 ratio) regardless of response.

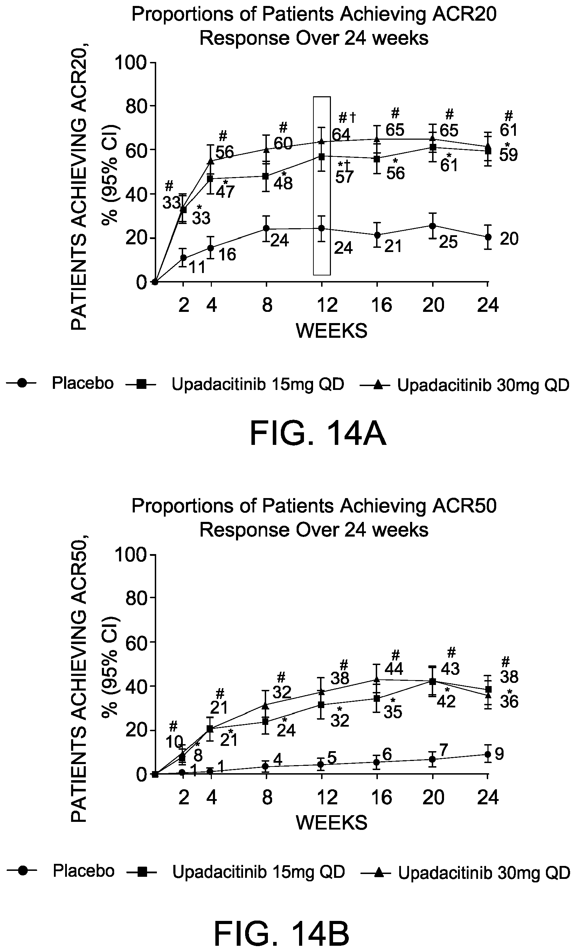

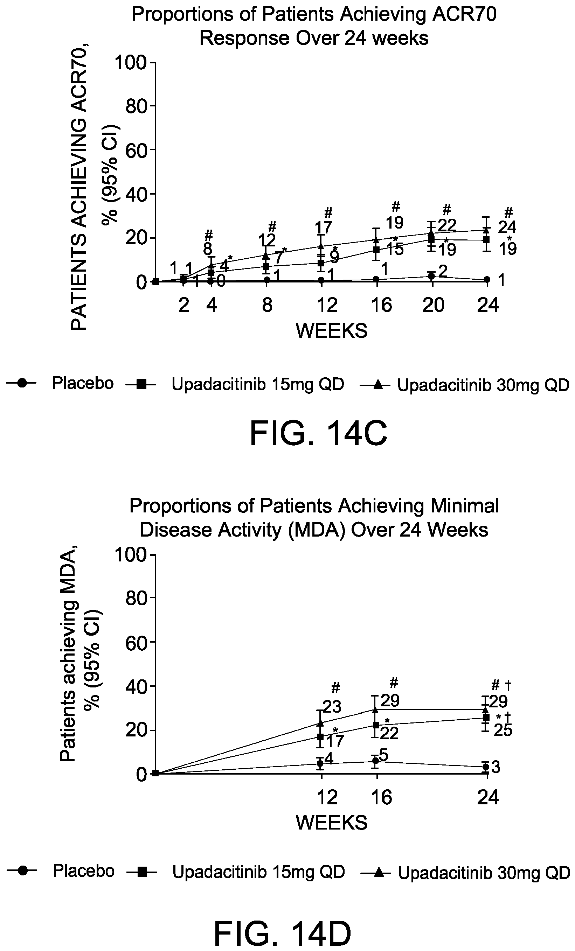

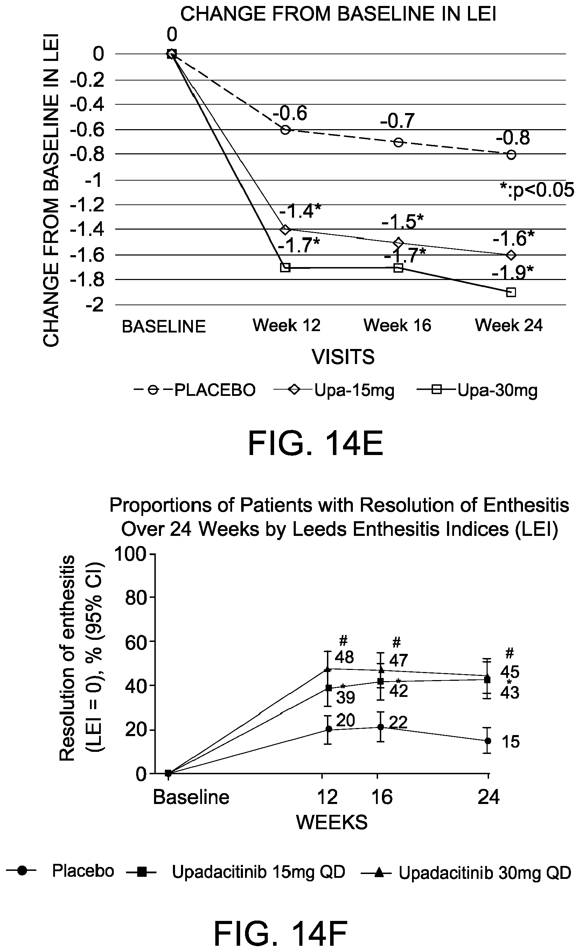

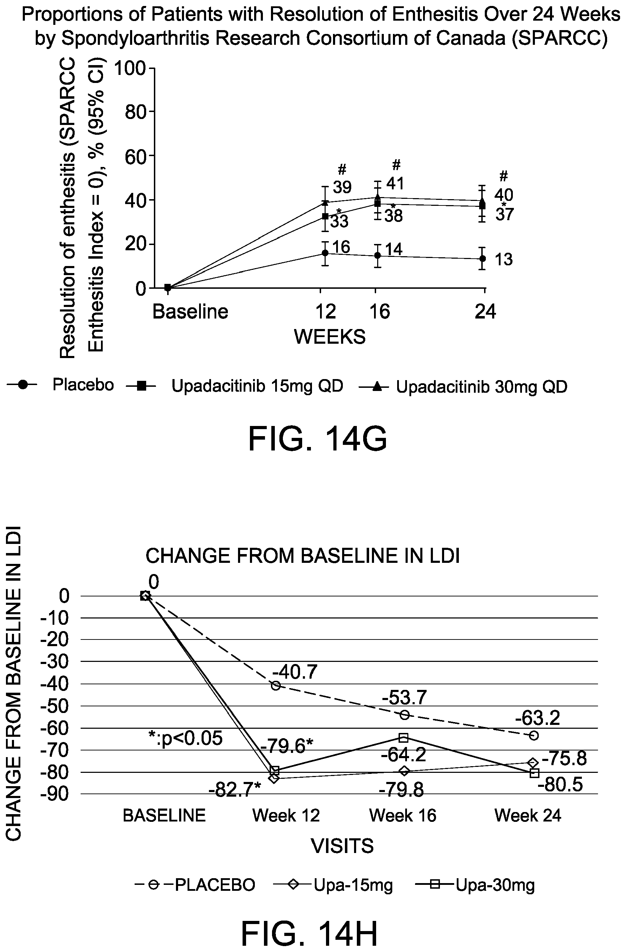

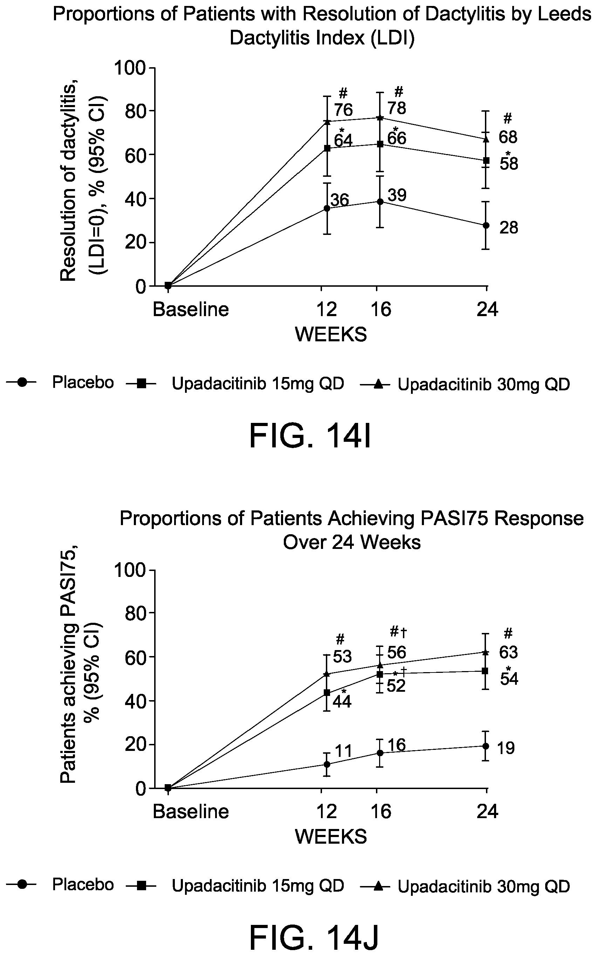

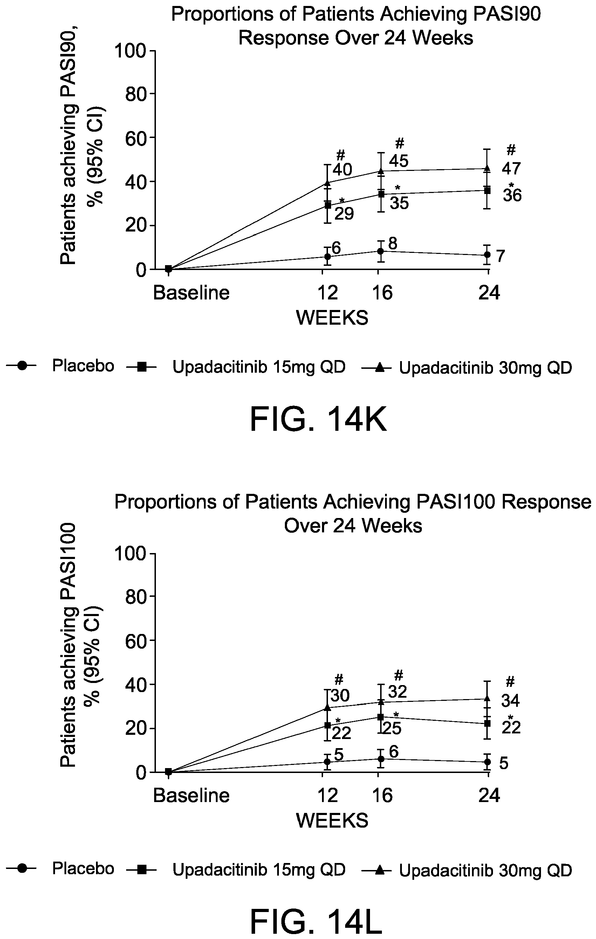

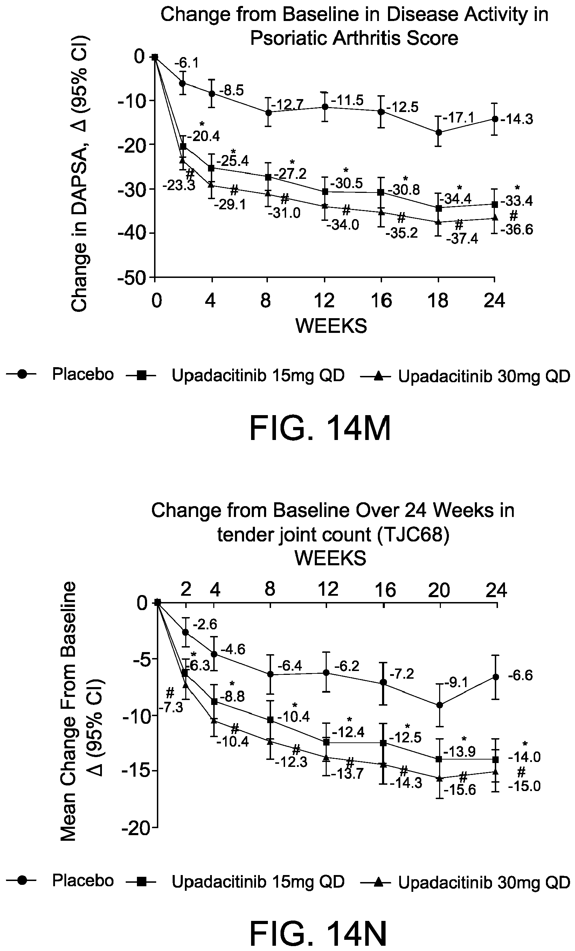

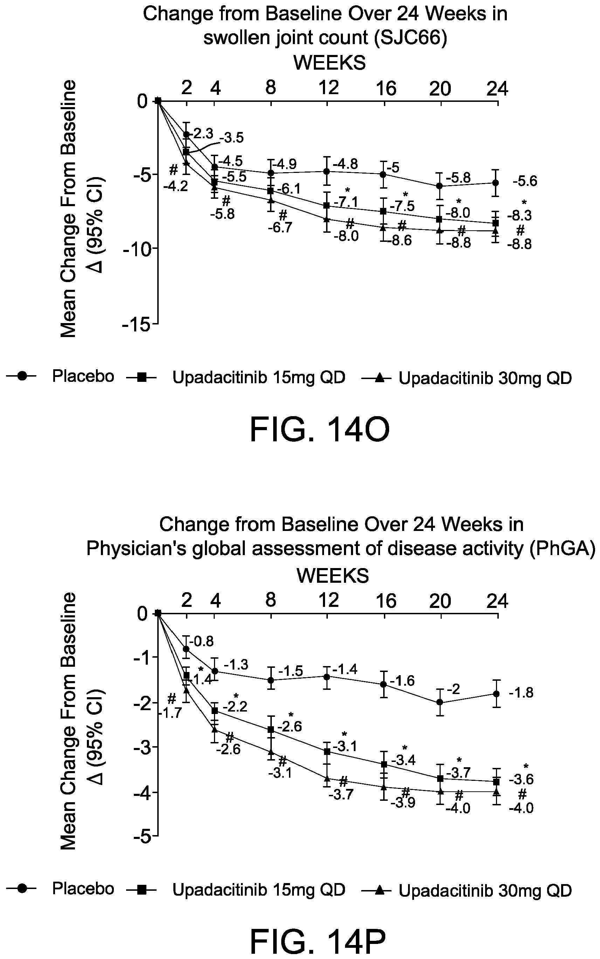

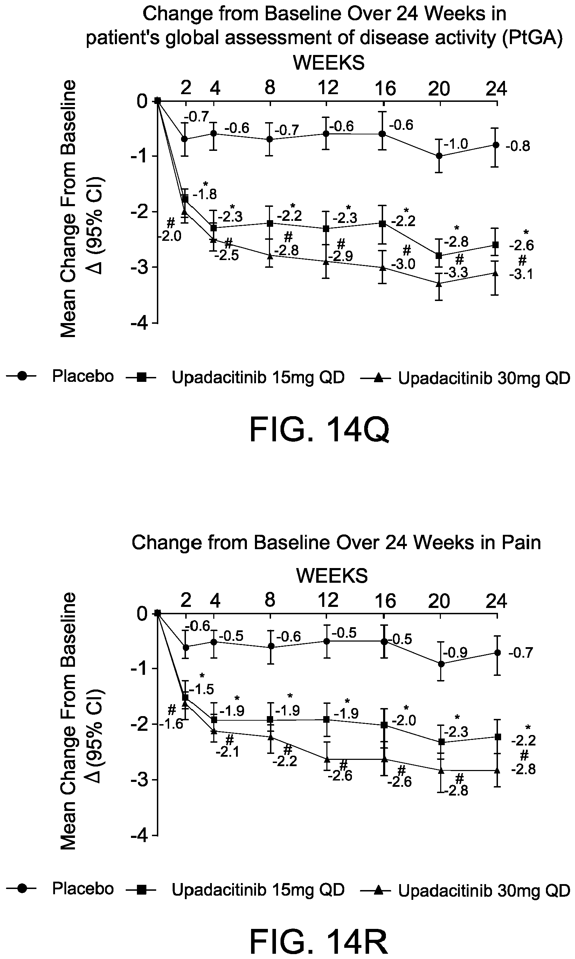

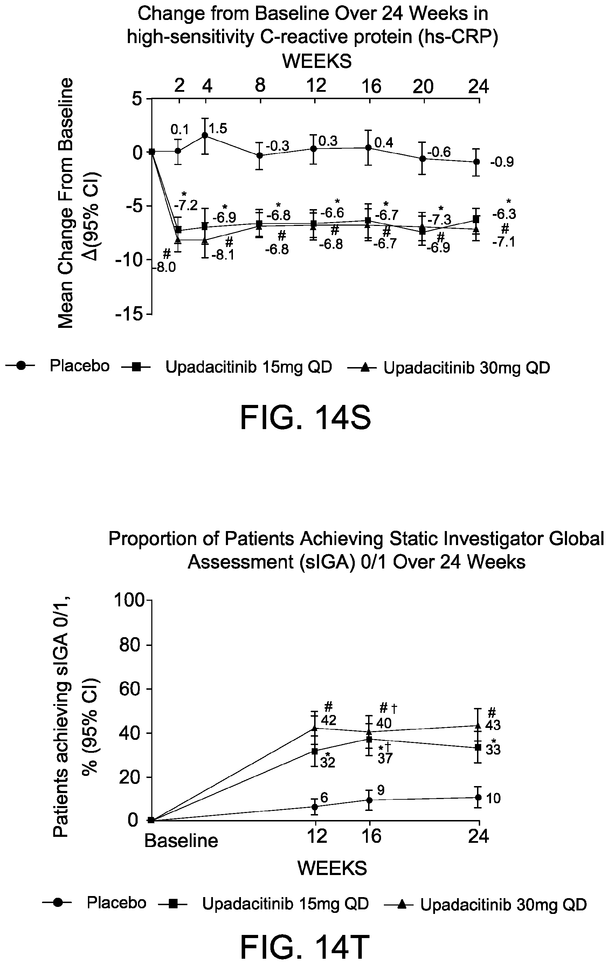

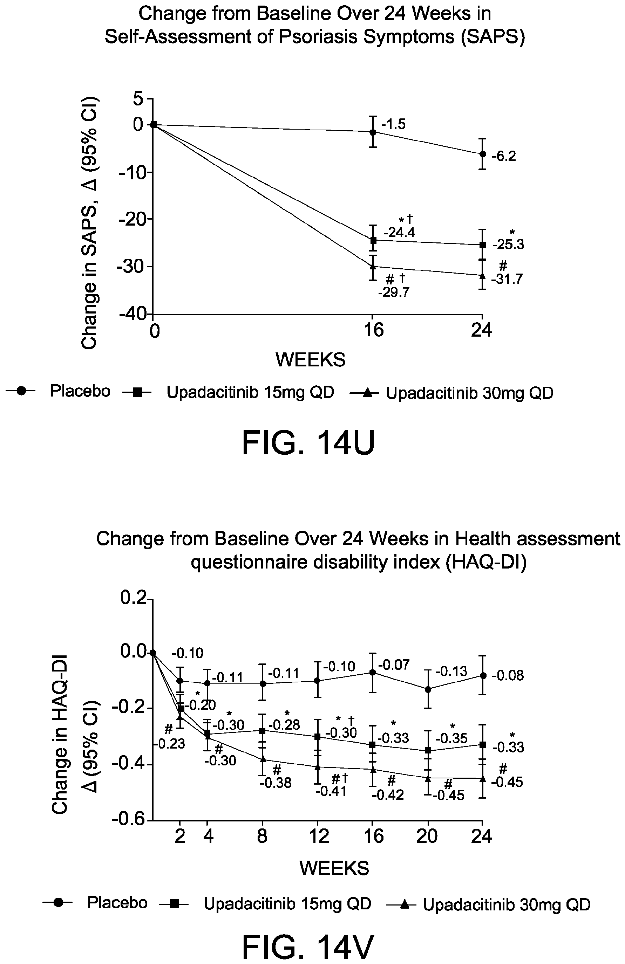

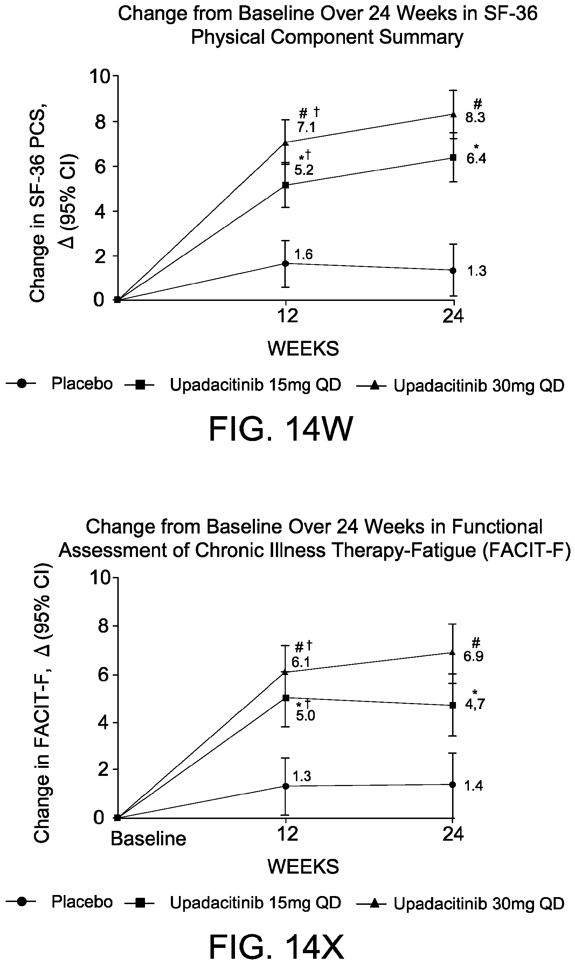

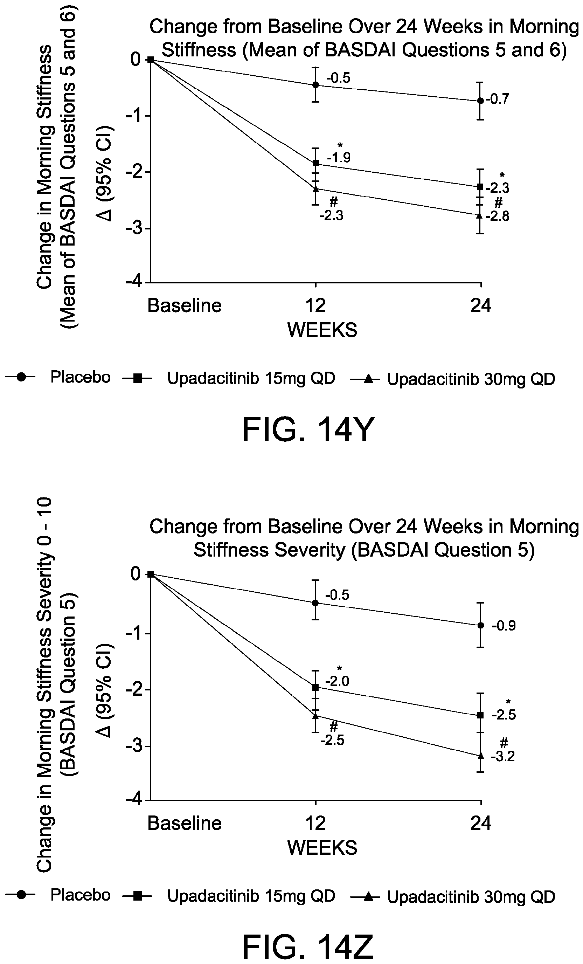

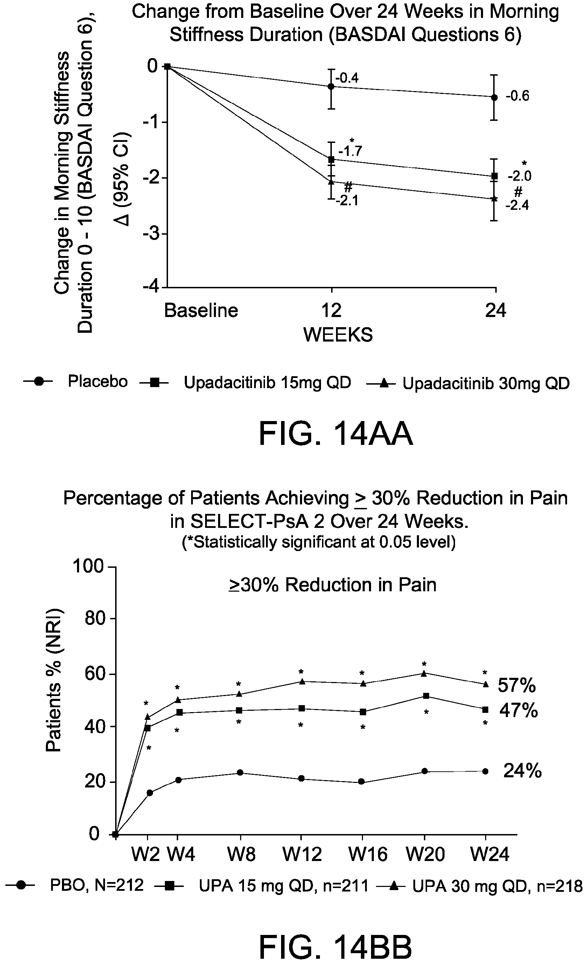

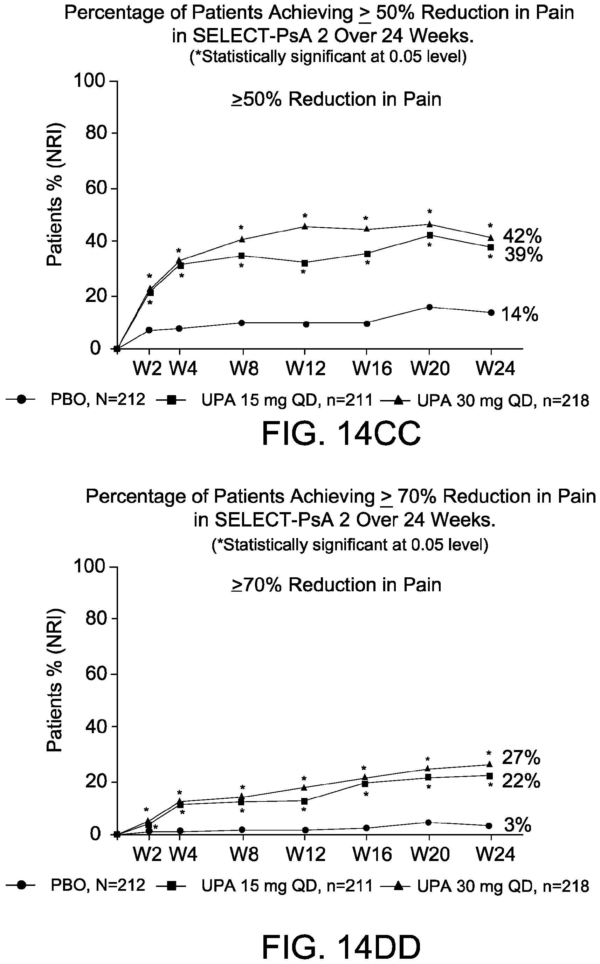

**FIGS. 14A-14DD** provides a summary of primary and key secondary efficacy results for the SELECT-PSA2 (PsA bDMARD-IR) Phase 3 clinical trial. **FIG. 14A** depicts the time course up to and including Week 24 of the primary endpoint ACR20. Response rate (%) with 95% CI is presented by visit. Nominal p-value < 0.05 for both UPA doses vs placebo for all visits. UPA = upadacitinib freebase. Symbols: * = p≤0.05 for comparison of upadacitinib 15mg QD versus placebo; # = p≤0.05 for comparison of upadacitinib 30mg QD versus placebo; † = significant in the multiplicity-controlled analysis. Additional key secondary efficacy results include: patients achieving ACR50 response (**FIG. 14B**); patients achieving ACR70 response (**FIG. 14C**); proportion of patients achieving Minimal Disease Activity (MDA) over 24 weeks (**FIG. 14D**); patients achieving change in baseline in Leeds Enthesitis Index (LEI) (**FIG. 14E**); patients achieving resolution of enthesitis (LEI = 0) (**FIG. 14F**); proportion of patients with resolution of enthesitis over 24 weeks by Spondyloarthritis Research Consortium of Canada (SPARCC) (**FIG. 14G**); patients achieving change in baseline in Leeds Dactylitis Index (LDI) (**FIG. 14H**); patients achieving resolution of dactylitis (LDI = 0) (**FIG. 14I**); proportion of patients achieving Psoriasis Area Severity Index PASI 75 (**FIG. 14J**), PASI 90 (**FIG. 14K**), and PASI 100 Response (**FIG. 14L**) over 24 weeks; patients achieving change from baseline in disease activity in psoriatic arthritis (DAPSA) score (**FIG. 14M**); patients achieving change from baseline over 24 weeks in core components of the ACR criteria: tender joint count (TJC68) (**FIG. 14N**), swollen joint count (SJC66) (**FIG. 14O**), physician's global assessment of disease activity (PhGA) (**FIG. 14P**), patient's global assessment of disease activity (PtGA) (**FIG. 14Q**), Pain (PtPain) (a score of 0 indicates "no pain" and a score of 10 indicates "worst possible pain") (**FIG. 14R**), high-sensitivity C-reactive protein (hs-CRP) (**FIG. 14S**); proportions of patients achieving static investigator global assessment (sIGA) 0/1 over 24 weeks (**FIG. 14T**); patients achieving change from baseline over 24 weeks in self-assessment of psoriasis symptoms (SAPS) (**FIG. 14U**); patients achieving change from baseline over 24 weeks in health assessment questionnaire disability index (HAQ-DI) (**FIG. 14V**); patients achieving change from baseline over 24 weeks in SF-36 Physical Component Summary (**FIG. 14W**); patients achieving change from baseline over 24 weeks in Functional Assessment of Chronic Illness Therapy-Fatigue (FACIT-F) (the FACIT-F score ranges from 0-52, with higher scores indicating less fatigue) (**FIG. 14X**); patients achieving change from baseline over 24 weeks in morning stiffness (mean of BASDAI Questions 5 and 6) (**FIG. 14Y**); patients achieving change from baseline over 24 weeks in morning stiffness severity (BASDAI Question 5) (**FIG. 14Z**); and patients achieving change from baseline over 24 weeks in morning stiffness duration (BASDAI Question 6) (**FIG. 14AA**). A significantly higher proportion of patients receiving upadacitinib freebase (15 mg QD or 30 mg QD) versus placebo achieved ≥30%, ≥50%, and ≥70% reduction in Patient's Global Assessment of pain (PtPain) as early as week 2, and efficacy achieved was sustained thereafter (see **FIGS. 14BB-14DD**). UPA = upadacitinib; PBO = placebo; QD = once daily; NRI = non-responder imputation.

## DEFINITIONS

Section headings as used in this section and the entire disclosure are not intended to be limiting.

1. Clinical Endpoint Definitions

American College of Rheumatology (ACR) Criteria

ACR criteria is a composite measurement calculated based on the improvement over a set of core measurements. ACR20 is defined as at least 20% improvement (compared to baseline values) in tender and swollen joint counts (TJC and SJC) and at least 20% improvement in 3 of the remaining 5 core set measures (subject assessment of pain, subject global assessment of disease activity, physician global assessment of disease activity, subject assessment of physical function and acute phase reactant hsCRP). ACR50 and ACR70 are similarly defined with at least 50% and 70% improvement, respectively. A subject will be classified as an ACR20 (ACR50, or ACR70) responder, if the following conditions are met:

1. $\geq$ 20% (50%, or 70%) improvement from baseline in tender joint count (TJC68) and
2. $\geq$ 20% (50%, or 70%) improvement from baseline in swollen joint count (SJC66) and
3. $\geq$ 20% (50%, or 70%) improvement from baseline in at least 3 of the following 5:

    a. Patient's Global Assessment of Pain (Pt Pain)
    b. Patient's Global Assessment of Disease Activity (PtGA)
    c. Physician's Global Assessment of Disease Activity (PGA)
    d. Patient's self-assessment of physical function (*i.e.*, measured by Health Assessment Questionnaire HAQ-DI score)
    e. Acute-phase reactant value high-sensitivity CRP (hsCRP)

Assessment of SpondyloArthritis International Society (ASAS), ASAS20, ASAS40, ASAS-PR, and ASAS 5/6 Responses.

Domains used for the ASAS responses are as follows:

    a. Patient's Global Assessment - Represented by the PtGA-disease activity (NRS score 0 - 10)
    b. Pain - Represented by the Patient's Assessment of Total Back Pain (Total Back Pain, NRS score 0 - 10)
    c. Function - Represented by the BASFI (NRS score 0-10)
    d. Inflammation - Represented by the mean of the 2 morning stiffness-related BASDAI (mean of Questions 5 and 6 of the BASDAI NRS score 0-10)

ASAS20 Response: Improvement of $\geq$ 20% and absolute improvement of $\geq$ 1 unit (on a scale of 0 to 10; 0 = no pain and 10 = worst possible pain) from Baseline in $\geq$ 3 of the above 4 domains above, with no deterioration (defined as a worsening of $\geq$ 20% and a net worsening of $\geq$ 1 unit) in the potential remaining domain.

ASAS40 Response: Improvement of $\geq$ 40% and absolute improvement of $\geq$ 2 units (on a scale of 0 to 10; 0 = no pain and 10 = worst possible pain) from Baseline in $\geq$ 3 of the above 4 domains above, with no deterioration (defined as a net worsening of > 0 units) in the potential remaining domain.

ASAS partial remission (PR): an absolute score of $\leq$ 2 units (on a scale of 0 to 10; 0 = no pain and 10 = worst possible pain) from Baseline for each of the 4 domains above.

ASAS 5/6 Response: Improvement of $\geq$ 20% from Baseline in 5 out of the following 6 domains: BASFI, Patient's Assessment of Total Back Pain, PtGA-disease activity, inflammation (mean of Questions5 and 6 of the BASDAI]), lateral lumbar flexion from BASMI, and hs-CRP.

ASAS Health Index (HI)

The ASAS HI is a linear composite measure with a dichotomous response option: "I agree" and "I do not agree" to a listing of 17 Questions. Each statement on the ASAS HI is given a score of "1" = "I agree" or "0" = "I do not agree." The total sum of the ASAS HI ranges from 0-17, with a lower score indicating a better health status. Questions 7 and 8 are not applicable to all patients. For those patients who ticked the response "not applicable," the sum score is analyzed based on n = 16 or n = 15 respectively. A total score can be analyzed if no more than 20% of the data (*i.e.*, 3 Questions) are missing. The total score is calculated as follows for respondents with up to a maximum of three missing responses: Sum.score = x/(17-m)* 17, where x is the Question summation score and m is the number of missing Questions and m $\leq$ 3. Cases with more than three missing responses (m > 3) cannot be allocated a total score and the total score will be set as missing. The 17 ASAS Health Index Questions are as follows:

    1. Pain sometimes disrupts my normal activities.

2. I find it hard to stand for long.
3. I have problems running.
4. I have problems using toilet facilities.
5. I am often exhausted.
6. I am less motivated to do anything that requires physical effort.
7. I have lost interest in sex.
8. I have difficulty operating the pedals in my car.
9. I am finding it hard to make contact with people.
10. I am not able to walk outdoors on flat ground.
11. I find it hard to concentrate.
12. I am restricted in traveling because of my mobility.
13. I often get frustrated.
14. I find it difficult to wash my hair.
15. I have experienced financial changes because of my rheumatic disease.
16. I sleep badly at night.
17. I cannot overcome my difficulties.

Ankylosing Spondylitis Disease Activity Score (ASDAS)

Parameters used for the calculation of ASDAS:

1. Patient's Assessment of Total Back Pain (BASDAI Question 2 NRS score 0 - 10),
2. Duration of morning stiffness (BASDAI Question 6 NRS score 0 - 10),
3. Patient global assessment of disease activity (PtGA NRS score 0-10),
4. Peripheral pain/swelling (BASDAI Question 3 NRS score 0 - 10), and
5. high-sensitivity C reactive protein (hsCRP) (in mg/mL) or erythrocyte sedimentation rate (ESR).

Calculation of ASDAS:

$$ASDAS_{hs\text{-}CRP} = 0.121 \times \text{total back pain} + 0.110 \times PtGA + 0.073 \times \text{peripheral pain/swelling} + 0.058 \times \text{duration of morning stiffness} + 0.579 \times \text{Ln(hs-CRP+1)}.$$

$$ASDAS_{ESR} = 0.113 \times \text{patient global} + 0.293 \times \sqrt{ESR} + 0.086 \times \text{peripheral pain/swelling} + 0.069 \times \text{duration of morning stiffness} + 0.079 \times \text{total back pain}.$$

To calculate observed ASDAS scores, the observed component value will be calculated first. Then the components will be included in the calculation per the ASDAS formula. If any observed component is missing in a window, then the observed ASDAS score will be missing.

When the conventional CRP is below the limit of detection or when the high sensitivity CRP is < 2 mg/L, the constant value of 2 mg/L should be used to calculate ASDAS-CRP.

ASDAS score is categorized by the following ASDAS Disease Activity States:

- ASDAS Inactive Disease (ID):

$$ASDAS < 1.3$$

- ASDAS Moderate Disease:

$$1.3 \leq ASDAS < 2.1$$

- ASDAS Low Disease Activity (LDA):

$$ASDAS < 2.1$$

- ASDAS High Disease:

$$2.1 \leq ASDAS \leq 3.5$$

- ASDAS Very High Disease:

$$ASDAS > 3.5$$

ASDAS Response categories are defined as follows:

- ASDAS Major Improvement (MI) (a change from baseline ≤ -2.0; ≥2-point decrease from baseline)
- ASDAS Clinically Important Improvement (CII) (a change from baseline ≤ -1.1; (≥1.1-point decrease from baseline)

Ankylosing Spondylitis Quality of Life Questionnaire (ASQoL)

Each of the 18 statements on the ASQoL (provided below) is given a score of "1" (yes) or "0" (no). Concepts measured include activities of daily life, emotional functioning, pain, fatigue, and sleep problems. A score of "1" is given where the Question is affirmed (with a "yes" answer), indicating adverse QoL. All Question scores are summed to give a total score or index. Scores can range from 0 (good QoL) to 18 (poor QoL), with higher scores equaling worsening functioning. Cases with more than three missing responses (i. e., more than 20%) cannot be allocated a total score. For cases with between one and three missing responses, the total score is calculated as follows: T=18x/18-m where: T is the total score, x is the total score for the Questions affirmed and m is the number of missing Questions.

1. My condition limits the places I can go

2. I sometimes feel like crying

3. I have difficulty dressing

4. I struggle to do jobs around the house

5. It's impossible to sleep

6. I am unable to join in activities with my friends/family

7. I am tired all the time

8. I have to keep stopping what I am doing to rest

9. I have unbearable pain

10. It takes a long time to get going in the morning

11. I am unable to do jobs around the house

12. I get tired easily

13. I often get frustrated

14. The pain is always there

15. I feel I miss out on a lot

16. I find it difficult to wash my hair

17. My condition gets me down

18. I worry about letting people down

Bath Ankylosing Spondylitis Disease Activity Index (BASDAI), BASDAI 50 Response, and the Morning Stiffness Score

The BASDAI consists of a 1 through 10 scale (1 being no problem and 10 being the worst problem) and is used to answer 6 questions pertaining to the 5 symptoms: Fatigue, Spinal pain, Joint pain/swelling, Areas of localized tenderness (also called enthesitis, or inflammation of tendons and ligaments), Morning stiffness duration, and Morning stiffness severity. A lower score indicates less disease activity.
The six BASDAI Questions (Components) are as follows:

Q1. How would you describe the overall level of fatigue/tiredness you have experienced?
Q2. How would you describe the overall level of AS neck, back or hip pain you have had?
Q3. How would you describe the overall level of pain/swelling in joints, other than neck, back or hips you have had?
Q4. How would you describe the overall level of discomfort you have had from any areas tender to touch or pressure?
Q5. How would you describe the overall level of morning stiffness you have had from the time you wake up?
Q6. How long does your morning stiffness last from the time you wake up?

Questions 1 through 5 have responses that can range from 0 (none) to 10 (very severe); Question 6 have response range from 0 (0 hr) to 10 (2 or more hrs), with 5 representing 1 hr.
Scoring of the BASDAI: BASDAI will be reported 0 to 10. The score has a maximum value of 10 and is calculated as follows:

$$\text{BASDAI Score} = 0.2\,(Q1 + Q2 + Q3 + Q4 + Q5/2 + Q6/2)$$

If one of the 5 Questions (Questions 1 - Question 4, inflammation) is missing, then the score is the mean of the 4 non-missing Questions (total of 4 non-missing Questions divided by 4). If more than 1 of the 5 Questions is missing, then the BASDAI score is missing. Question 5 and Question 6 jointly constitute Question 5 (inflammation). If both Questions 5 and 6 are missing, and questions 1 through 4 are non-missing, then only one Question will be considered missing. The BASDAI score can still be calculated as the mean of Questions 1-4. However, if, for example, both Question 6 and Question 1 are missing, then 2 Questions will be considered missing, as the inflammation calculation would be incomplete. The BASDAI score would then be considered missing in this case.
A BASDAI 50 response is a categorical response based on BASDAI that represents an at least 50% improvement from baseline in BASDAI.
The Morning Stiffness Score is the average of BASDAI Questions 5 and 6, and it ranges from 0 - 10.
A "change from baseline in BASDAI and BASDAI Questions (Components), including change from baseline in mean of Question 5 and 6 of the BASDAI" means (1) a change from baseline from the BASDAI Score, (2) a change from baseline in all of the BASDAI Questions, and (3) a change from baseline of the mean of Questions 5 and 6 (which represent inflammation).

Bath Ankylosing Spondylitis Functional Index (BASFI)

The BASFI consists of the following 10 questions, assessing ability to perform activities such as dressing, bending, reaching, turning, and climbing steps, each with a response ranging from 0 (easy) to 10 (impossible):

1. Putting on your socks or tights without help or aids (*e.g.*, sock-aid).
2. Bending forward from the waist to pick up a pen from the floor without an aid.
3. Reaching up to a high shelf without help or aids (*e.g.*, helping hand).
4. Getting up out of an armless dining room chair without using your hands or any other help.
5. Getting up off the floor without help from lying on your back.
6. Standing unsupported for 10 minutes without discomfort.
7. Climbing 12 to 15 steps without using a handrail or walking aid. One foot on each step.
8. Looking over your shoulder without turning your body.
9. Doing physically demanding activities (*e.g.*, physiotherapy, exercises, gardening, or sports).

10. Doing a full day's activities whether at home or at work.

See, *e.g.,* Sieper et al., Ann Rheum Dis (2009) 68 (Suppl II): ii1-ii44. doi: 1 0.1 1 36/ard.2008. 1 040 18.

Scoring of BASFI. The BASFI score will be derived based on the average of Questions 1 through 10. If up to 2 Questions are missing, corresponding scores will be replaced with the mean of the remaining non-missing Questions. If 3 or more Questions are missing, BASFI will be considered missing.

Bath Ankylosing Spondylitis Metrology Linear Index (BASMI$_{lin}$)

The Linear BASMI (BASMI$_{lin}$) composite score will be calculated using the BASMI components. The table below presents the components of BASMI$_{lin}$ and assessment ranges for score.

| Table 1. | | | |
|---|---|---|---|
| **Components of BASMI$_{lin}$** | **0** | **Between 0 and 10** | **10** |
| Lateral Lumbar flexion (cm) | $A \geq 21.1$ | $(21.1 - A)/2.1$ | $A \leq 0.1$ |
| Tragus to wall distance (cm) | $A \leq 8$ | $(A - 8)/3$ | $A \geq 38$ |
| Lumbar flexion (modified Schober) (cm) | $A \geq 7.4$ | $(7.4 - A)/0.7$ | $A \leq 0.4$ |
| Intermalleolar distance (cm) | $A \geq 124.5$ | $(124.5 - A)/10$ | $A \leq 24.5$ |
| Cervical rotation (°) | $A \geq 89.3$ | $(89.3 - A)/8.5$ | $A \leq 4.3$ |
| BASMI$_{lin}$ = Assessment measurements for tragus to wall, cervical rotation and lateral lumbar flexion are the means of the left and right measurement; A = assessment measurement | | | |

Scores for each assessment range from 0 to 10, and the BASMI$_{lin}$ total score will be the average of the 5 assessment scores. If 1 Question is missing, the BASMI$_{lin}$ will be calculated as the mean of remaining 4 Questions. Hence, the range of the BASMI$_{lin}$ total score should be between 0 and 10. If 2 or more Questions are missing, then the BASMI$_{lin}$ score will be considered missing. See *e.g.,* van der Heijde et al., Arth. Care & Res. (2012) 64:1919-1922 and van der Heijde et al., Ann Rheum Dis (2008) 67:489-93.

Body Surface Area - Psoriasis (BSA-PS)

The subject's right or left hand should be selected as the measuring device. For purposes of clinical estimation, the total surface of the palm plus five digits will be assumed to be approximately equivalent to 1%. Measurement of the total area of involvement is aided by imagining if scattered plaques were moved so that they were next to each other and then estimating the total area involved. See, *e.g.,* See, *e.g.,* Bozek and Reich, Adv. Clin. Exp. Med. (2017) 26:851-856.

Disease Activity in Psoriatic Arthritis (DAPSA) Score

DAPSA is a continuous endpoint that measures the disease activity in psoriatic arthritis. DAPSA consists of five components: Tender Joint Count 68, Swollen Joint Count 66, Patient's Global Assessment of Pain (Pt Pain) (0-10 NRS), PtGA of Disease Activity (0-10 NRS), and hsCRP (in mg/dL). Calculation of the DAPSA score is as follows:

$$DAPSA = SJC66 + TJC68 + Pt\ Pain\ (0\text{-}10\ NRS) + PtGA\ (0\text{-}10\ NRS) + hsCRP\ (in\ mg/dL)$$

To calculate observed DAPSA scores, the observed component value will be calculated first. Then the components will be included in the calculation per the DAPSA formula. If any observed component is missing in a window, then the observed DAPSA score will be missing.

Disease Activity Score (DAS28)

DAS28 (CRP) and DAS28 (ESR) are composite indices to assess disease activity in PsA using high-sensitivity c-reactive protein lab value (hsCRP) or erythrocyte sedimentation rate (ESR) measurement, respectively. The DAS28 provides a score between 0 and 10, indicating arthritis disease activity at the time of measurement. DAS28 (CRP) and DAS28 (ESR) are calculated based on Tender Joint Count, Swollen Joint Count, PtGA of Disease Activity (0-100), and hsCRP (in mg/L) or ESR (mm/hr). As PtGA of Disease Activity is collected with the scale of 0-10 NRS, the variable needs to be multiplied by 10 before being used the DAS28 formula. To calculate observed DAS28 scores, the observed component value will be calculated first. Then the components will be included in the calculation per the DAS formula selected. If any observed component is missing in a window, then the observed DAS28 score will be missing. Calculation of DAS28 (CRP) and DAS28 (ESR) are provided by the following equations:

$$DAS28\,(CRP) = 0.56 \times \sqrt{(TJC28)} + 0.28 \times \sqrt{(SJC28)} + 0.36 \times \ln(hsCRP + 1) + 0.014 \times PtGA + 0.96$$

$$DAS28\,(ESR) = 0.56 \times \sqrt{(TJC28)} + 0.28 \times \sqrt{(SJC28)} + 0.70 \times \ln(ESR) + 0.014 \times PtGA$$

where $\sqrt{}$ is square root and ln is natural log; TJC28 refers to the Subject's total Tender Joint Count out of the provided 28 evaluated joints; SJC28 refers to the Subject's total Swollen Joint Count out of the provided 28 evaluated joints; hsCRP unit in the DAS28 (CRP) equation is expressed as mg/L; ESR unit in the DAS28 (ESR) equation is expressed as mm/hr; and PtGA refers to the Patient's Global Assessment of Disease Activity.

| Table 2. Anatomical Joints for DAS28 (CRP) Calculation | | | |
|---|---|---|---|
| Shoulder | Elbow | Wrist | Thumb Interphalangeal |
| Metacarpophalangeal I | Metacarpophalangeal II | Metacarpophalangeal III | Metacarpophalangeal IV |
| Metacarpophalangeal V | Proximal Interphalangeal II | Proximal Interphalangeal III | Proximal Interphalangeal IV |
| Proximal Interphalangeal V | Knee | | |

Leeds Dactylitis Index (LDI), Dactylitis Count and Tender Dactylitis Count

The Dactylitis scores will be the presence of Dactylitis at baseline, the Dactylitis Count (out of subjects with baseline presence of Dactylitis) ("total dactylitis count" or "tender dactylitis count") and the resolution of Dactylitis (out of subjects with baseline presence of Dactylitis). The presence of Dactylitis at baseline is defined as the following: at least one affected and tender digit with circumference increase over reference digit ≥ 10%. The Dactylitis Count will be calculated as the number of digits (hands and feet) with presence of dactylitis, and ranges from 0 to 20.

The Leeds Dactylitis Index (LDI) is a score based on finger circumference and tenderness, assessed and summed across all dactylitic digits. The assessment should begin with visual inspection of the hands and feet. For each pair of digits in which one or both digits appear dactylitic, the circumference of the affected digits (both right and left side) is assessed using a dactylometer. Additionally, the affected digit pairs are assessed for tenderness by squeezing the digital shaft mid-way between the metacarpophalangeal and proximal interphalangeal joints and is recorded as tenderness, yes or no. Tenderness should not be assessed by squeezing the joint lines. For each of 20 digits of a subject, a digit final score needs to be calculated first. For an unaffected digit, the digit final score is set to be 0. For an affected digit, the digit final score is calculated as (A/B-1)*100*C if A/B ≥ 1.1, and digit finals score = 0 if A/B < 1.1, where A denotes the circumference of the digit, B the reference circumference, and C the tenderness score. The reference circumference can be either the circumference of the unaffected contralateral digit if available, or from a reference table if otherwise. For any digit without an available dactylometer measurement the standard reference value will be utilized in calculation of the LDI. LDI is the sum of the digit final scores over all 20 digits. The proportion of subjects with Resolution of Dactylitis is defined as the proportion of subjects with LDI = 0. Digits injected with corticosteroid will be considered non-evaluable for 90 days from the time of the injection. If a digit is missing and its contralateral digit is dactylitic, "digit absent" will be recorded for the missing digit.

Enthesitis Scoring: Spondyloarthritis Research Consortium of Canada (SPARCC) Enthesitis Index, Leeds Enthesitis Index (LEI), Total Enthesitis Count, and Maastricht Ankylosing Spondylitis Enthesitis Score (MASES)

For the Spondyloarthritis Research Consortium of Canada (SPARCC) Enthesitis Index, 16 sites are evaluated as indicated in rows 1- 8 in the table below. Tenderness on examination is recorded as either present (coded as 1), absent (coded as 0), or not assessed (NA) for each site. The SPARCC enthesitis index is calculated by taking the sum of the scores from the 16 sites. The SPARCC score ranges from 0 to 16.

The Leeds Enthesitis Index evaluates enthesitis at the 6 entheseal sites indicated in rows 2, 7 and 9 in the table below. Tenderness on examination is recorded as either present (coded as 1), absent (coded as 0), or not assessed (NA) for each of the 6 sites. The LEI is calculated by taking the sum of the scores from the 6 sites. The LEI ranges from 0 to 6.

The Total Enthesitis Count is calculated by taking the sum of the tenderness scores from all 18 sites in the table below.

The proportion of subjects with resolution of enthesitis sites included in the LEI is defined as the proportion of subjects with LEI = 0; the proportion with resolution of the SPARCC Enthesitis Index and of the total enthesitis count are similarly defined (score = 0).

Present = 1; Absent = 0; NA = Not assessed

| Table 3. | | Tenderness in Left | | | Tenderness in Right | | |
|---|---|---|---|---|---|---|---|
| | | Present | Absent | NA | Present | Absent | NA |
| 1 | Medial epicondyle | | | | | | |
| 2 | Lateral epicondyle | | | | | | |
| 3 | Supraspinatus insertion into the greater tuberosity of humerus | | | | | | |
| 4 | Greater trochanter | | | | | | |
| 5 | Quadriceps insertion into superior border of patella | | | | | | |
| 6 | Patellar ligament insertion into inferior pole of patella or tibial tubercle | | | | | | |
| 7 | Achilles tendon insertion into calcaneum | | | | | | |
| 8 | Plantar fascia insertion into calcaneum | | | | | | |
| 9 | Medial femoral condyle | | | | | | |

The Maastricht Ankylosing Spondylitis Enthesitis Score (MASES) will be measured to assess the presence (1) or absence (0) of enthesitis at 13 different sites (first costochondral joint left/right, seventh costochondral joint left/right, posterior superior iliac spine left/right, anterior superior iliac spine left/right, iliac crest left/right, fifth lumbar spinous process, and proximal insertion of Achilles tendon left/right), noting the subjects' responses, yielding a total score ranging 0 - 13. If one or more locations are missing, the score will be calculated using available data. If all locations are missing, then MASES is set to be missing.

EuroQoL-5D (EQ-5D-5L)

The EQ-5D-5L questionnaire is one of the most commonly used questionnaires to measure health-related quality of life. It consists of a questionnaire and a visual analogue scale (VAS). The self-assessment questionnaire measures 5 dimensions of health status (mobility, selfcare, usual activities, pain/discomfort, and anxiety/depression). The AS subject is asked to grade their own current level of function in each dimension into 1 of 3 degrees of disability (severe, moderate, or none). The PsA subject is asked to grade their own current level of function in each dimension into 5 levels per dimension (no problems, slight problems, moderate problems, severe problems, and extreme problems corresponding to Level 1 to Level 5 respectively) and includes the EQ Visual Analogue Scale (EQ VAS). The 5 dimensions of health status are converted into a single index value. Using the VAS, subjects record perceptions of current perceived health status with a grade ranging from 0 (the worst possible health status) to 100 (the best possible health status).

FACIT-Fatigue Questionnaire (FACIT-F)

The FACIT Fatigue Questionnaire is a 13-Question tool that measures an individual's level of fatigue during their usual daily activities over the past week. The level of fatigue is measured on a four point scale (4 = not at all fatigued to 0 = very much fatigued). The Fatigue scale ranges from 0 to 52, with higher scores indicating less fatigue. Question score for each Question is calculated by either subtracting from 4 or adding 0 depending on whether it is a reversal Question or not. FACIT Fatigue Scale is then calculated by adding up all Question scores, multiplying by 13 and dividing by the number of Questions answered. If less than 7 Questions are answered, the scale will not be computed.

1. I feel fatigued
2. I feel weak all over
3. I feel listless ("washed out")
4. I feel tired
5. I have trouble starting things because I am tired
6. I have trouble finishing things because I am tired
7. I have energy
8. I am able to do my usual activities
9. I need to sleep during the day
10. I am too tired to eat
11. I need help doing my usual activities
12. I am frustrated by being too tired to do the things I want to do
13. I have to limit my social activity because I am too tired

Health Assessment Questionnaire Disability Index (HAQ DI)

HAQ-DI is a self-reported patient outcome measurement. It is calculated as the mean of the scores from 8 following categories with a range 0 - 3: Dressing and Grooming, Rising, Eating, Walking, Hygiene, Reach, Grip, and Activities. Higher scores reflect greater disability. The maximum score for all the questions in each category is considered as the score for the category. The HAQ-DI takes into account the subject's use of aids or devices or assistance in the scoring algorithm for a disability category. For each category there is an AIDS OR DEVICES companion variable that is used to record the type of assistance, if any, a subject uses for his/her usual activities. If aids or devices and/or assistance from another person are checked for a category, the score for this category is set to 2 (much difficulty), if the original score is 0 (no difficulty) or 1 (some difficulty). The HAQ-DI is then calculated by summing the adjusted categories scores and dividing by the number of categories answered. The HAQ-DI cannot be calculated if the subject does not have scores for at least 6 categories.

High-Sensitivity C Reactive Protein (hs-CRP)

C-reactive protein (CRP), which is measured in blood plasma, is an acute phase protein that appears in blood circulation in response to inflammation, and serves as a biomarker for systemic inflammation. However, routine methods of CRP detection (turbidimetric, nephelometric) demonstrated poor sensitivity in detecting concentrations of CRP below 6-10 mg/litre. See, *e.g.,* Poddubnyy et al., Ann. Rheum. Dis. (2010) 69:1338-1341.

The high-sensitivity C-reactive protein (hs-CRP) assay is a more precise measurement than routine CRP. Several different tests may be used to measure the hs-CRP normal range versus abnormal value for the subject to be treated; thus the upper limit of normal (ULN) will be determined by the laboratory for the hs-CRP test used and may differ from laboratory to laboratory.

Health Resource Utilization (HRU) Questionnaire for PsA

The HRU questionnaire contains three questions regarding health care utilization in the following categories: unscheduled health care professional visits, emergency room visits, and hospital admissions. The data gathered from the HRU questionnaire will be used to calculate the individual cumulative number of utilizations per unit of time (*e.g.,* subject-year) under observation in each variable (*i.e.,* the number of unscheduled PsA-related health care professional visits, the number of emergency room visits, the number of hospital admissions and the total number of days in hospital) as follows: (i) time under observation for a subject will be defined as "date of last visit with non-missing HRU - date of baseline visit." and (ii) the number of utilizations after baseline will be summed up for each subject.

Insomnia Severity Index (ISI)

ISI is a 7 Question questionnaire that assess, (1) difficulty with sleep onset, (2) difficulty with sleep maintenance (3) problem with early awakening, (4) satisfaction with sleep pattern, (5) interference with daily functioning as a result of sleep problems, (6) noticeability of sleep problem to others, and (7) degree of distress caused by sleep problem. Question 1 (the severity of insomnia Question) is rated on a Likert type of scale, (0) "None," 1- "Mild," 2- "Moderate," 3-"Severe" and 4- "Very Severe." The remaining Questions on satisfaction, noticeability on impairment, worry/distress about sleep and interference are similarly rated on 5-point likert type scale form. See an ISI example below:

1. Please rate the current (*i.e.*, last 2 weeks) severity of your insomnia problems:

> a. Difficulty falling asleep
> b. Difficulty staying asleep
> c. Problem waking up too early

2. How satisfied/dissatisfied are you with your current sleep pattern? (0 = very satisfied; 1 = satisfied; 2 = moderately satisfied; 3 = dissatisfied; 5 = very dissatisfied)

3. How noticeable to others do you think your sleep problem is in terms of impairing the quality of your life? (0 = not noticeable at all; 1 = a little; 2 = somewhat; 3 = much; 5 = very much noticeable)

4. How worried/distressed are you about your current sleep problem? (0 = not at all worried; 1 = a little; 2 = somewhat; 3 = much; 4 = very much worried)

5. To what extent do you consider your sleep problem to interfere with your daily functioning (*e.g.*, daytime fatigue, mood, ability to function at work/daily chores, concentration, memory, mood, etc.)? (0 = not at all interfering; 1 = a little; 2 = somewhat; 3 = much; 4 = very much interfering).

Guidelines for Scoring/Interpretation: Add the scores for all seven Questions. If there is one or two missing Questions, their value can be replaced with the average score of the remaining Questions. If there are more than two Questions with no responses, it is preferred to consider the total score missing. Total score categories:

> 0 - 7 = No clinically significant insomnia
> 8 - 14 = Subthreshold insomnia
> 15 - 21 = Clinical insomnia (moderate severity)
> 22 - 28 = Clinical insomnia (severe)

Joint Count Assessment: SJC and TJC

Swollen Joint Count Assessment (SJC or SJC66): An assessment of 66 joints will be done by physical examination. The joints to be examined for swelling are the same as those examined for tenderness, except the hip joints are excluded. Joint swelling will be classified as present ("1"), absent ("0"), replaced ("9"), or no assessment ("NA"). Joints injected with corticosteroid will be considered non-evaluable for 90 days from the time of the injection. The range for SJC66 will be 0 to 66.

Tender Joint Count Assessment (TJC or TJC68): An assessment of 68 joints will be done for tenderness by pressure manipulation on physical examination. Joint pain/tenderness will be classified as: present ("1"), absent ("0"), replaced ("9"), or no assessment ("NA"). Joints injected with corticosteroid will be considered non-evaluable for 90 days from the time of the injection. The range for TJC68 will be 0 to 68.

Anatomical joints are evaluated for swelling and tenderness at every study visit. The 34 anatomical joints in the below table are assessed in this study for both the left and right side of the body.

a. Hip joints are not assessed for swelling.

| Table 4. Anatomical Joints Assessed for Calculation of Tender and Swollen Joint Counts (TJC68 and SJC66) | | | |
|---|---|---|---|
| Temporomandibular | Sternoclavicular | Acromio-clavicular | Shoulder |
| Elbow | Wrist | Metacarpophalangeal I | Metacarpophalangeal II |
| Metacarpophalangeal III | Metacarpophalangeal IV | Metacarpophalangeal V | Thumb Interphalangeal |
| Proximal Interphalangeal II | Proximal Interphalangeal III | Proximal Interphalangeal IV | Proximal Interphalangeal V |
| Distal Interphalangeal II | Distal Interphalangeal III | Distal Interphalangeal IV | Distal Interphalangeal V |
| Hip[a] | Knee | Ankle | Tarsus |
| Metatarsophalangeal I | Metatarsophalangeal II | Metatarsophalangeal III | Metatarsophalangeal IV |
| Metatarsophalangeal V | Great Toe/Hallux | Interphalangeal II | Interphalangeal III |
| Interphalangeal IV | Interphalangeal V | | |

Modified Stoke Ankylosing Spondylitis Spine Score (mSASSS)

The mSASSS is a scoring method that measure radiographic progression in the spine of patients with ankylosing spondylitis. The mSASSS has a range of 0 to 72, which is derived from scoring the anterior site of the lumbar spine from the lower border of T 12 to the upper border of S 1 and the anterior site of the cervical spine from the lower border of C2 to the upper border of T1 as either 0 (normal), 1 (erosion, sclerosis, or squaring), 2 (syndesmophyte), 3 (bridging syndesmophyte), or NA vertebral body not evaluable. X-ray of spinal films will be analyzed for radiographic progression (from Baseline to the follow-up timepoint).

Minimal Disease Activity for Psoriatic Arthritis (MDA-PsA)

A patient is classified as having minimal disease activity (MDA) for PsA when 5 of the following 7 criteria are met (responder), or if at least 3 of the 7 criteria are not met (non-responder): TJC68 $\leq$ 1; SJC66 $\leq$ 1; PASI $\leq$ 1 or BSA-Ps $\leq$ 3%; Patient's Global Assessment of Pain (Pt Pain)_$\leq$ 1.5 (0 - 10 NRS); Patient's Global Assessment of disease activity $\leq$ 2 (0 - 10 NRS); HAQ-DI score $\leq$ 0.5; Leeds Enthesitis Index $\leq$ 1.

Modified Psoriatic Arthritis Response Criteria (Modified PsARC)

The modified PsARC is a PsA-specific composite responder index. To achieve response, a subject must achieve 2 of the following 4 Questions, one of which has to be a Tender Joint Count 68 or Swollen Joint Count 66, and no worsening of any measure: $\geq$ 30% improvement in TJC68; >_ 30% improvement in SJC66; Improvement in PtGA of Disease Activity NRS; Improvement in PGA of Disease Activity NRS.

NSAID Score

To calculate the NSAID Score, the following information is collected: (1) the type of NSAID and weight factor; (2) the dose (mg/administration); (3) the frequency (days of administration per week); (4) days of administration (NSAID end date - NSAID start date + 1); and (5) number of days in the study (last visit date - baseline date + 1).
The NSAID score may then be calculate using the following equation:

$$= (\text{Equivalent NSAID Score}) \times (\text{days of administration})/(\text{number of days in the study})$$

wherein Equivalent NSAID Score is calculated by the following equivation:
= (Weight factor) x (dose) x (frequency)
The weight factor of the NSAID is determined by the maximum dose of each NSAID to achieve a score of 100, in which 0 equals no administration, and 100 equals 150 mg diclofenac, or 1000 mg naproxen, or 200 mg aceclofenac, or 400 mg celecoxib etc. per daily dose. For example, 150 mg Diclofenac is equivalent to 1000 mg Naproxen, so the weight factor for Diclofenac is 100/150, and the weight factor for Naproxen is 100/1000. See also Dougados et al. Ann Rheum Dis (2011) 70:249-251.

- Diclofenac, Weight factor: 100/150
- Naproxen, Weight factor: 100/1000
- Aceclofenac, Weight factor: 100/200
- Celecoxib, Weight factor: 100/400
- Etodolac, Weight factor: 100/600
- Etoricoxib, Weight factor: 100/90
- Flurbiprofen, Weight factor: 100/200
- Ibuprofen, Weight factor: 100/2400
- Indometacin, Weight factor: 100/150
- Ketoprofen, Weight factor: 100/200
- Meloxicam, Weight factor: 100/15
- Nimesulide, Weight factor: 100/200
- Phenylbutazone, Weight factor: 100/400
- Piroxicam, Weight factor: 100/20
- Tenoxicam, Weight factor: 100/20

Patient's Assessment of Nocturnal Back Pain (Nocturnal Back Pain), Patient's Assessment of Total Back Pain (Total Back Pain score), and Patient's Global Assessment of Pain (Pt Pain or "Pain") Numerical Rating Scales (NRS)

Pain will be measured using 0-10 numerical rating scale (NRS) scale Questions for Nocturnal Back Pain NRS (0 = no pain and 10 = worst possible pain), Total Back Pain (0 = no pain and 10 = severe pain), and Pain (*i.e.*, overall pain) (0 = no pain and 10 = severe pain).

ASAS (e.g., ASAS20, ASAS40, ASAS PR) assesses the Patient's Assessment of Total Back Pain (Total Back Pain score) using the above described scoring method. For ASDAS, Patient's Assessment of Total Back Pain assesses total back pain by answering BASDAI Question 2.

Patient's Global Assessment of Disease Activity Numeric Rating Scale (PtGA-NRS)

The subject will assess his/her disease activity using the Patient's Global Assessment of Disease NRS. The range is 0 to 10 with no activity being indicated by 0 and severe activity by 10.

Physician's Global Assessment of Disease Activity Numeric Rating Scale (PGA or PhGA-Disease Activity NRS)

The PGA-Disease Activity will be conducted to assess the subject's current disease activity, taking into consideration both arthritis and psoriasis activity, independent of the subject's self-assessment, using a 0 - 10 NRS. The range is 0 to 10 with no activity being indicated by 0 and severe activity by 10.

Physical Activity Assessment

The Physical Activity Assessment measures step count and physical activity (e.g., as measured by a digital health technology wearable device) and spinal range of motion tasks (e.g., by measurement with an inclinometer).

Psoriatic Arthritis Disease Activity Score (PASDAS)

PASDAS is a continuous scale of combined joint, dactylitis and enthesitis assessments, physician and patient global assessments for arthritis, SF36-PCS, and hsCRP measurements. PASDAS is calculated based on the following equation:

$$= (((0.18 \sqrt{(PGA)}) + 0.159 \sqrt{(PtGA)} - 0.253 \sqrt{(SF36\text{-}PCS)} + 0.101 \ln (SJC66 + 1) + 0.048 \ln (TJC68 + 1) + 0.23 \ln (LEI + 1) + 0.37 \ln (\text{Tender Dactylitis Count} + 1) + 0.102 \ln (hsCRP + 1) + 2) * 1.5$$

wherein $\sqrt{}$ is square root and ln is natural log; PtGA is on the scale of 0 - 100; PGA is on the scale of 0 - 100 (as PtGA and PGA are collected with the scale of 0-10 NRS, their values need to be multiplied by 10 before being used in the PASDAS formula); SF36-PCS is the physical component scale in the SF36 instrument; the unit for hsCRP is mg/L; and LEI ranges from 0 to 6.

Psoriasis Area Severity Index (PASI)

The PASI is a measure of psoriasis severity. Four anatomic sites - head, upper extremities, trunk, and lower extremities - are assessed for erythema, induration and desquamation using a 5-point scale (0 = no symptoms; 1 = slight; 2 = moderate; 3 = marked; 4 = very marked). Based on the extent of lesions in a given anatomic site, the area affected is assigned a numerical value (0 = no involvement; 1 = < 10%; 2 = 10% - 29%; 3 = 30% - 49%; 4 = 50% - 69%; 5 = 70% - 89%; 6 = 90% - 100%). Since the head, upper extremities, trunk and lower extremities correspond to approximately 10, 20, 30 and 40% of body surface area, respectively; the PASI score is calculated using the formula:

$$= 0.1(E_h + I_h + D_h)A_h + 0.2(E_u + I_u + D_u)A_u + 0.3(E_t + I_t + D_t)A_t + 0.4(E_l + I_l + D_l)A_l$$

where E, I, D, and A denote erythema, induration, desquamation, and area, respectively, and h, u, t, and l denote head, upper extremities, trunk, and lower extremities, respectively.

PASI scores range from 0.0 to 72.0 with the highest score representing complete erythroderma of the severest possible degree. Typically scores of 3 or less represent mild disease, scores over 3 and up and including 15 represent moderate disease and scores over 15 are considered to be associated with severe disease. If a Question is missing, PASI is not scored. PASI 75 (PASI 50, PASI 90, PASI 100) response is achieved if there is at least a 75% (50%, 90%, 100%) reduction in PASI score (>_ PASI 75/50/90/100 response) at a visit relative to the Baseline PASI score. See, *e.g.,* Feldman et al., J Invest Dermatol. 1996;106(1):183-6.

Self-Assessment of Psoriasis Symptoms (SAPS)

The Self-Assessment of Psoriasis Symptoms (SAPS) contains 11 symptom-focused Questions, as provided below. Each Question is scored from 0 to 10, with 0 being least severe and 10 being most severe. The total score is generated by summing the 11 Questions. The total score ranges from 0 to 110.

1. Over the past 24 hours, what was the worst pain you experienced in the areas affected by psoriasis?
2. Over the past 24 hours, what was the worst itching you experienced in the areas affected by psoriasis?
3. Over the past 24 hours, what was the worst redness you experienced in the areas affected by psoriasis?
4. Over the past 24 hours, what was the worst scaling you experienced in the areas affected by psoriasis?
5. Over the past 24 hours, what was the worst flaking (scales falling off your skin) you experienced in the areas affected by psoriasis?
6. Over the past 24 hours, what was the worst bleeding you experienced in the areas affected by psoriasis?
7. Over the past 24 hours, what was the worst burning you experienced in the areas affected by psoriasis?
8. Over the past 24 hours, what was the worst stinging you experienced in the areas affected by psoriasis?
9. Over the past 24 hours, what was the worst tenderness you experienced in the areas affected by psoriasis?
10. Over the past 24 hours, what was the worst pain you experienced due to skin cracking in the areas affected by psoriasis?
11. Over the past 24 hours, what was the worst joint pain you experienced due to psoriasis?

Sharp/van der Heijde Score (SHS) score (equivalent to the modified total Sharp score, mTSS)

Radiographic outcomes will be assessed and scored according to Sharp's method (van der Heijde modification for PsA). To obtain the total SHS score, scores for erosions and JSN in both the hands and feet will be added together. The range of scores is summarized below. A finding of No Radiographic Progression of PsA is no change or improvement from baseline in SHS ($\leq$0).

| Table 5. Range of Total SHS Score, Erosion Score and Joint Space Narrowing | | | |
|---|---|---|---|
| | Hands | Feet | Total (Hands and Feet) |
| Erosion Score Range | 0 - 200 | 0 - 120 | 0 - 320 |
| Joint Space Narrowing Range | 0 - 160 | 0 - 48 | 0 - 208 |
| Total SHS Range for Erosion and JSN | 0 - 360 | 0 - 168 | 0 - 528 |

Erosion Assessment. Erosions will be assessed in each hand (20 locations per hand) and foot (6 locations per foot). The locations assessed in the SHS method include: 4 Distal interphalangeal joints (2 - 5); 5 Metacarpo-Phalangeal Joints (1 - 5); 4 Proximal Inter-Phalangeal Joints (2 - 5); Inter-Phalangeal Joint of the thumb; Proximal first Metacarpal Bone; Radius Bone; Ulnar Bone; Trapezium and Trapezoid (as one unit; multangular); Navicular Bone; Lunate Bone; 5 Metatarso-phalangeal joints (1 - 5); Inter-phalangeal joint of the first toe.

Joint Space Narrowing Assessment. Joint space narrowing (JSN) will be assessed in each hand (20 locations per hand) and foot (6 locations per foot). The locations assessed in the SHS method include: 4 Distal inter-phalangeal joints (2 - 5); 4 Proximal inter-phalangeal joints (2 - 5); 5 Metacarpo-phalangeal joints (1 - 5); Interphalangeal Joint of thumb (IP); 3 Carpo-metacarpal joints (3 - 5); Radio-carpal joint; Multangular-navicular joint; Capitate-navicular-lunate joint; 5 Metatarso-phalangeal joints; Inter-phalangeal joint of first toe.

For each Joint and Bone assessed, scores range as follows: Erosions: 0 - 5 (hands/wrists) or 0 - 10 (feet) to characterize the extent of erosions (where 0 denotes no erosion); Joint Space Narrowing: 0 - 4 to characterize the extent of Joint Space Narrowing (JSN) (where 0 denotes no narrowing).

SpondyloArthritis Research Consortium of Canada (SPARCC) Assessment for Spine (MRI SPARCC - Spine or MRI-Spine SPARCC) and Sacroiliac (SI) Joints (MRI SPARCC - Joints or MRI-SI Joints SPARCC)

SPARCC scores for spine and sacroiliac (SI) joints are calculated by adding up the dichotomous outcomes from evaluations of the presence, depth and intensity of bone marrow edema lesions of the spine and SI joints, respectively.

In the MRI SPARCC score of Spine, the entire spine is evaluated for active inflammation (bone marrow edema) using the Short-TI Inversion Recovery (STIR) image sequence. 23 discovertebral units (DVUs) are assessed, and the six most severely affected DVUs are selected and used to calculate the MRI Spine SPARCC score. For each of the six DVUs, 3 consecutive sagittal slices are assessed in four quadrants in order to evaluate the extent of inflammation in all three dimensions:

1. Each quadrant is scored for presence of increased signal on STIR (1 = increased signal; 0 = normal signal)
2. Presence, on each of the sagittal slices, of a lesion exhibiting high signal intensity (comparable to cerebrospinal fluid) in any disco-vertebral unit is given an additional score of 1.
3. Slices that included a lesion demonstrating continuous increased signal of depth $\geq$ 1 cm extending from the endplate are to be scored as +1 per slice.

The maximum possible score for any individual slice is 6, with a maximum score for all 6 discovertebral units being 108.

The MRI SPARCC score of SI joints is conducted on 6 consecutive slices of the STIR image sequence. All lesions within the iliac bone and within the sacrum up to the sacral foramina are to be scored. The SI joint is divided into 4 quadrants: upper iliac, lower iliac, upper sacral and lower sacral. Each consecutive slice is scored separately for the right and left joint in all four quadrants as follows:

1. Each quadrant is scored for presence of increased signal on STIR (1 = increased signal; 0 = normal signal)
2. Joints that include a lesion exhibiting intense signal on the STIR sequence are scored as +1 per slice.
3. Joints that included a lesion demonstrating continuous increased signal of depth $\geq$ 1 cm from the articular surface are be scored as +1 per slice.

The maximum possible score for any individual slice is 12, with a maximum score for all 6 slices being 72.

Static Investigator Global Assessment of Psoriasis (sIGA)

The sIGA is a 5-point score ranging from 0 to 4, based on assessment of the average elevation, erythema, and scaling of all psoriatic lesions. The assessment is considered "static" which refers to the patients disease state at the time of the assessments, without comparison to any of the patient's previous disease states, whether at baseline or at a previous visit. A lower score indicates less severe psoriasis (0 = clear, 1 = almost clear, 2 = mild, 3 = moderate and 4 = severe). A binary clinical endpoint for the PsA-1 and PsA-2 clinical studies based on sIGA is the proportion of subjects achieving a sIGA score of 0 or 1 and at least a 2-point improvement from baseline. This endpoint is calculated among the subjects with baseline sIGA score $\geq$ 2.

Work Productivity and Activity Impairment Questionnaire for Psoriatic Arthritis (WPAI-PsA) and

Axial Spondyloarthritis (WPAI-Axial SpA)

The Work Productivity and Activity Impairment Questionnaire (WPAI) was developed to measure the effect of overall health and specific symptoms on productivity at work and outside of work. It consists of 6 questions. A lower WPAI score indicates an improvement. The 4 main impairment scores (S1 to S4) are expressed as percent impairment based on the 6 questions.

S0. Employment: defined below
S1. Absenteeism: Percent work time missed due to PsA or Axial SpA:

$$100 \times \left[ \frac{Q2}{Q2 + Q4} \right]$$

S2. Presenteeism: Percent impairment while working due to PsA or Axial SpA:

$$100 \times \left[ \frac{Q5}{10} \right]$$

S3. Percent overall work impairment due to PsA or Axial SpA:

$$100 \times \left[ \frac{Q2}{Q2+Q4} + \left\{ 1 - \frac{Q2}{Q2+Q4} \right\} \times \frac{Q5}{10} \right]$$

S4. Percent activity impairment due to PsA or Axial SpA:

$$100 \times \left[ \frac{Q6}{10} \right]$$

S5. Did subject miss work (defined below). This is needed to derive the proportion of subjects who missed work.

When calculating the WPAI scores, the following computational notes should be followed.

∘ Define Employment as a binary YES or NO variable where YES corresponds to "Employed" and NO corresponds to "Not Employed."
∘ A subject will be considered "employed" at a given visit if Q1 = YES or Q2 > 0 or Q4 > 0.
∘ A subject will be considered "unemployed" at a given visit if Q 1 = NO and no positive hours recorded under Q2 and Q4 (*i.e.,* if Q1 = NO AND Q2 ≤ 0 AND Q4 ≤ 0, then UNEMPLOYED).
∘ Employment status for a subject will be considered "missing" at a given visit if Q1 = missing and no positive hours recorded under Q2 and Q4.
∘ If a subject is "unemployed" or employment status is "missing," then S1, S2, and S3 will be set to "missing."
∘ If Q2 = 0 and Q4 = 0 or missing then Q2/(Q2 + Q4) = missing *(i.e.,* S1 = missing).
∘ If Q2 = 0 and Q4 = 0, then set S3 to missing.
∘ If Q2 is missing or Q4 is missing, then set S1 and S3 to missing.
∘ If Q4 = missing, then DO NOT set Q5 = missing.
∘ If Q5 is missing, then apply the following rules:
∘ If Q2 > 0, Q4 = 0, and Q5 = missing, then S3 = 100%.
∘ If Q2 = 0, Q4 > 0, and Q5 = missing, then S3 is missing.
∘ If Q2 > 0, Q4 > 0, and Q5 = missing, then S3 is missing.
∘ Determine if a subject missed work (based on Q2) in order to analyze the proportion of subjects who missed work:

  ∘ Create a binary (yes or no) "missed work" variable.
  ∘ A subject will be considered as yes to missed work if Q2 is greater than 0.
  ∘ If Q2 = missing, then MISSED WORK = missing.
  ∘ If Q2 > 0, then MISSED WORK = "yes."
  ∘ If Q2 = 0, then MISSED WORK = "no."
  ∘ Therefore, the proportion of subjects who missed work will be counted based on the number of subjects with MISSED WORK = YES. 36-Item Short Form Health Survey (Form SF-36)

The 36-Item Short Form, Version 2 (SF-36v2) (Quality Metric) health survey consists of 36 general health questions. It has 2 components: physical and mental. For each component, a transformed summary score is calculated using 8 sub domains: physical functioning, role-physical, bodily pain, general health, vitality, social functioning, role-emotional, and mental health. The range is from 0 to 100, with higher scores indicating better outcomes. The coding and scoring for the SF-36 will use the software provided by the vendor.

2. Additional Definitions

Where a numeric range is recited, each intervening number within the range is explicitly contemplated with the same degree of precision. For example, for the range 6 to 9, the numbers 7 and 8 are contemplated in addition to 6 and 9, and for the range 6.0 to 7.0, the numbers 6.0, 6.1, 6.2, 6.3, 6.4, 6.5, 6.6, 6.7, 6.8, 6.9 and 7.0 are explicitly contemplated. In the same manner, all recited ratios also include all sub-ratios falling within the broader ratio.

The singular forms "a," "an" and "the" include plural referents unless the context clearly dictates otherwise.

The term "about" generally refers to a range of numbers that one of skill in the art would consider equivalent to the recited value *(i.e.,* having the same function or result). In many instances, the term "about" may include numbers that are rounded to the nearest significant figure.

A "subject" means a human. The terms "patient" and "subject" are used interchangeably herein.

An "adult subject" means a subject 18 years or older.

A "juvenile" or "pediatric" subject means a subject 1 to < 18 years old. Juvenile subjects to be treated are subjects diagnosed with juvenile AS (JAS), juvenile PsA (JPsA), and/or juvenile PsO (JPsO) and in need of treatment as determined by a physician ("active juvenile AS" and "active juvenile PsA", "active juvenile PsO", respectively). Juvenile AS may be classified per International League of Associations for Rheumatology (ILAR) (defining 7 discrete categories of arthritis starting before the age of 18 years: systemic arthritis, oligoarthritis, polyarthritis (rheumatoid factor [RF]-negative), polyarthritis (RF-positive), PsA, enthesitis-related JIA (or juvenile enthesitis-related arthritis [ERA]), and undifferentiated arthritis). See, *e.g.,* Petty et al., J Rheumatol. (2004)31:390-2. Juvenile PsA may be classified per pediatric International League of Associations for Rheumatology (ILAR) and/or adult criteria [Classification criteria for Psoriatic Arthritis (CASPAR)]. See *e.g.,* Aviel et al., Pediatric Rheumatology (2013) 11:11; Zisman et al., J. Rheum. (2017) 44:342-351.

The "2009 ASAS classification criteria" for the classification of a subject with axial spondyloarthritis (axial SpA, or axSpA) is described in Rudwaleit et al., Ann. Rheum. Dis. (2009) 68:777-783. The criteria require chronic back pain (≥3 months) in the subject and age at onset < 45 years, with the subject also having the following conditions (1) the presence of sacroiliitis by radiography or by magnetic resonance imaging (MRI) plus at least one SpA feature ("imaging arm") or (2) the presence of human leukocyte antigen (HLA) B27 plus at least two SpA features ("clinical arm"). Sacroiliitis on imaging refers to active (acute) inflammation on MRI highly suggestive of sacroiliitis associated with SpA, or definite radiographic sacroiliitis. SpA features are selected from the group consisting of inflammatory back pain, arthritis, enthesitis (heel), uveitis, dactylitis, psoriasis, Crohn's disease or ulcerative colitis, good response to NSAISs (24-48 hours after a full dose of an NSAID the back pain is not present any more or is much better), family history for SpA, positive HLA-B27, and elevated C-reactive protein (above the upper normal limit in the presence of back pain, and after exclusion of other reasons for elevation). *See also* Deodhar et al., Arth. & Rheum. (2014) 66:2649-2656.

The "1984 modified New York criteria" for the classification of a subject with ankylosing spondylitis (AS), is described in van der Linden et al., Arthritis and Rheumatism (1984) 27:361-368 , and has two components: diagnosis and grading; the diagnosis component further has two criteria: clinical and radiologic. The clinical criteria require: (i) low back pain and stiffness for more than 3 months which improves with exercise, but is not relieved by rest; (ii) limitation of motion of the lumbar spine in both the sagittal and frontal planes, and (iii) limitation of chest expansion relative to normal values corrected for age and sex. The radiologic criterion requires sacroiliitis grade ≥2 bilaterally or sacroiliitis grade 3-4 unilaterally. The grading component requires: (i) definite ankylosing spondylitis if the radiologic criterion is associated with at least 1 clinical criterion; and (ii) probable ankylosing spondylitis if 3 clinical criteria are present, and the radiologic criterion is present without any signs or symptoms satisfying the clinical criteria. *See also* Deodhar et al., Arth. & Rheum. (2014) 66:2649-2656.

The term "axial Spondyloarthritis" (axial SpA or axSpA) encompasses both "ankylosing spondylitis" (AS) and "non-radiographic axial spondyloarthritis" (nr-axial SpA, or nr-axSpA). A subject with "active axial Spondyloarthritis" (active axSpA) means a subject with a clinical diagnosis of active AS or active nr-axial SpA, and in need of treatment as determined by a physician.

A subject with "active ankylosing spondylitis" (active AS) means a subject with a clinical diagnosis of AS and in need of treatment as determined by a physician. In certain embodiments, the subject diagnosed as suffering from AS is further classified (*e.g.*, in the United States) as fulfilling the 1984 modified New York Criteria for AS and/or as fulfilling the 2009 ASAS classification criteria. In certain embodiments, the subject with a high disease activity of AS has a Bath Ankylosing Spondylitis Disease Activity Index score ≥4 and/or ASDAS ≥2.1 and/or a Patient's Assessment of Total Back Pain (Total Back Pain score) ≥4 based on a 0-10 numerical rating scale at baseline. *See, e.g.,* van der Heijde et al., Ann Rheum Dis. (2017)76:978-991; Sieper and Poddubnyy, Lancet (2017) 73-84.

A subject with "active non-radiographic axial spondyloarthritis" (active nr-axial SpA or active nr-axSpA) means a subject with a clinical diagnosis of nr-axial SpA and in need of treatment as determined by a physician. In certain embodiments, the subject diagnosed as suffering from nr-axial SpA is further classified (*e.g.*, in the United States) as fulfilling the 2009 ASAS classification criteria for axSpA but not meeting the radiologic criterion of the 1984 modified New York criteria for AS. In certain embodiments, the subject with high disease activity of nr-axial SpA has a Bath Ankylosing Spondylitis Disease Activity Index score ≥ 4 and/or an ASDAS ≥2.1 and/or a Patient's Assessment of Total Back Pain Score (Total Back Pain score) >_ 4 based on a 0 - 10 numerical rating scale at baseline; and an objective sign of inflammatory activity selected from the group consisting of (i) an objective sign of active inflammation on MRI of SI joints or (ii) hsCRP > upper limit of normal (ULN) at baseline. See, *e.g.,* van der Heijde et al., Ann Rheum Dis. (2017) 76:978-991Sieper et al. Ann. Rheum. Dis. (2009) 68 Suppl 2:ii1-44. doi: 10.1136/ard.2008.104018; Van der Heijde et al. Ann Rheum Dis. (2017) 76:978-991; Sieper and Poddubnyy, Lancet (2017) 73-84.

The "CASPAR criteria" (Classification criteria for Psoriatic Arthritis (see, *e.g.,* Taylor et al., Arthritis and Rheumatism (2006) 54:2665-2673)) requires the subject to have inflammatory articular disease (joint, spine, or entheseal) with ≥ 3 points from the following 5 categories at baseline:

1. Evidence of current psoriasis, a personal history of psoriasis, or a family history of psoriasis (one of the following).

a. Current psoriasis is defined as psoriatic skin or scalp disease present today as judged by a rheumatologist or dermatologist. Current psoriasis is assigned a score of 2; all other features are assigned a score of 1.
b. A personal history of psoriasis is defined as a history of psoriasis that may be obtained from a patient, family physician, dermatologist, rheumatologist, or other qualified health care provider.
c. A family history of psoriasis is defined as a history of psoriasis in a first- or second-degree relative according to a patient report.

2. Typical psoriatic nail dystrophy including onycholysis, pitting, and hyperkeratosis observed on current physical examination.
3. A negative test result for the presence of rheumatoid factor by any method except latex but preferably by enzyme-linked immunosorbent assay or nephelometry, according to the local laboratory reference range.
4. Either current dactylitis, defined as swelling of an entire digit, or a history of dactylitis recorded by a rheumatologist.
5. Radiographic evidence of juxtaarticular new bone formation, appearing as ill-defined ossification near joint margins (but excluding osteophyte formation) on plain radiographs of the hand or foot.

A subject with "active psoriatic arthritis" (active PsA) means a subject with a clinical diagnosis of PsA and in need of treatment as determined by a physician. In certain embodiments, the subject diagnosed as suffering from PsA is further classified (*e.g.*, in the United States) as fulfilling the Classification Criteria for PsA (CASPAR) criteria at baseline. In certain embodiments, the subject may have ≥ 3 tender joints (based on 68 joint counts) and ≥ 3 swollen joints (based on 66 joint counts) at baseline. In certain embodiments, the subject may have ≥ 5 tender joints (based on 68 joint counts) and ≥ 5 swollen joints (based on 66 joint counts) at baseline. In certain embodiments, the subject may have ≥3% Body Surface Area with Psoriasis (BSA-PS). In certain embodiments, subjects with Minimal Disease Activity (responders and non-responders) for PsA are excluded. "Active PsA" includes subjects who are, as determined by a physician, functioning normally while suffering from active PsA and subjects who are not, as determined by a physician, functioning normally while suffering from active PsA. For example, a subject with psoriasis on the abdomen may be considered to be manifesting active PsA but able to function normally, while a subject with psoriasis on the face may be considered to be manifesting active PsA but not able to function normally (*e.g.*, due to emotional distress). "Moderately to severely active PsA" is a subset of "active PsA" and involves the determination by a physician that the subject is not able to function normally while suffering from active PsA.

A subject with "active psoriasis" (active PsO) means a subject with a clinical diagnosis of PsO and in need of treatment as determined by a physician. In certain embodiments, the active psoriasis is active plaque psoriasis. In certain embodiments, the subject has a documented history of plaque psoriasis. "Active PsO" includes subjects who are, as determined by a physician, functioning normally while suffering from active PsO and subjects who are not, as determined by a physician, functioning normally while suffering from active PsO. For example, a subject with psoriasis on the abdomen may be considered to be manifesting active PsO but able to function normally, while a subject with psoriasis on the face may be considered to be manifesting active PsO but not able to function normally (*e.g.*, due to emotional distress). "Moderately to severely active PsO" is a subset of "active PsO" and involves the determination by a physician that the subject is not able to function normally while suffering from active PsO. In certain embodiments, the subject may have ≥3% Body Surface Area with Psoriasis (BSA-PS) at baseline.

The abbreviation "AS" refers to ankylosing spondylitis.

A result being achieved "within X weeks" of administration of the first dose of the JAK1 inhibitor wherein X is a integer greater than 0 (*e.g.,* 1, 2, 4, 6, 8, 10, 12, 14, 16, 18, 20, 22, 24, 52,...64 weeks, *etc.*) means the result occurs within the time frame beginning at the time of the administration of the first dose (Week 0) of the JAK1 inhibitor, and ending on and including the last day of the given specified week. A measurement or score used to determine if a result is achieved "at week X" (e.g., at week 1, 2, 4, 6, 8, 10, 12, 14, 16, 18, 20, 22, 24, 52,...64 weeks, *etc.*) may be taken at any point during, as well as on and including the first and last day, of given week X for the subject.

The abbreviation "axSpA" refers to axial spondyloarthritis.

The abbreviations "bDMARDs" and "biologic DMARDs" refer to biologic Disease Modifying Anti-Rheumatic Drugs. Examples of bDMARDs include, but are not limited to, biologic tumor necrosis factor inhibitors (*e.g.*, adalimumab, etanercept) and interleukin (IL)-17 inhibitors (*e.g.*, secukinumab, ixekizumab).

The term "bDMARD-IR" refers to a subject who is a bDMARD inadequate responder. bDMARD-IR subjects include those who have had an inadequate response to treatment with at least one bDMARD, or who have an intolerance to or contraindication for bDMARDs. Subjects who are bDMARD-IR include subjects who have discontinued treatment with at least one bDMARD due to intolerance or lack of efficacy.

The term "bDMARD naive" refers to a subject who has not had prior exposure to any biologic therapy, including any bDMARD, that may potentially have a therapeutic impact on the disorder or condition that is being treated.

The term "baseline" or "BL" refers to the time immediately before first dosing with the JAK1 inhibitor. Baseline measurements (*i.e.,* on the "Baseline Visit" or on the "Screening Visit") are collected prior to administration of the first dose of the JAK1 inhibitor (*i.e.,* upadacitinib freebase or a pharmaceutically acceptable salt thereof), and may include a measurement taken the day of but prior to first dosing with the JAK1 inhibitor.

The term "change from baseline" for a particular score or measurement means the score or measurement has improved (*e.g.*, demonstrating a positive clinical effect in the subject or population of subjects) as compared to the score or measurement taken at baseline.

The abbreviation "CII" means Clinically Important Improvement.

The phrase "concomitant administration" or "concomitant treatment" when referencing a therapy in addition to administration of the JAK1 inhibitor means the additional therapy is occurring at baseline and/or during treatment with the JAK1 inhibitor.

The abbreviations "DMARDs" and "non-biologic DMARDs" refer to non-biologic Disease Modifying Anti-Rheumatic Drugs. Non-biologic DMARDs include, but are not limited to, methotrexate (MTX), sulfasalazine (SSZ), leflunomide (LEF), apremilast, hydroxychloroquine (HCQ), bucillamine, and iguratimod. "Non-biologic DMARDs" and "conventional-synthetic disease modifying anti-rheumatic drugs" (csDMARDs) are used interchangeably herein.

The term "DMARD-IR" or "non-biologic DMARD-IR" refers a subject who is a non-biologic DMARD inadequate responder. DMARD-IR subjects include those who have had an inadequate response to treatment with at least one non-biologic DMARD, or who have an intolerance to or contraindication for non-biologic DMARDs.

The abbreviation "EMA" means European Medicines Agency.

The abbreviation "FDA" means Food and Drug Administration.

The abbreviation "hsCRP" means high-sensitivity C-reactive protein.

The abbreviation "ID" means Inactive Disease.

The phrase "improving physical function" in a subject with active PsA means an improvement in activities or tasks compared to baseline.

"In need of treatment" or "in need of treatment... as determined by a physician" refers to the physician's opinion that, at baseline, the condition is not sufficiently well-controlled, such as by other medical management (*e.g.*, by other therapy or therapies previously administered to treat the condition).

The phrase "inhibiting the progression of structural damage" or "preventing structural progression" in a subject with active PsA means demonstrating prevention of bony changes on x-ray compared to baseline.

"JAK1 inhibitor" refers to the compound upadacitinib ((3S,4R)-3-ethyl-4-(3H-imidazo[1,2-a]pyrrolo[2,3-e]pyrazin-8-yl)-N-(2,2,2-trifluoroethyl)pyrrolidine-1-carboxamide) freebase or a pharmaceutically acceptable salt thereof. Solid state forms of the JAK1 inhibitor are further described herein.

The abbreviation "LDA" means low disease activity.

The abbreviation "MI" means major improvement.

The abbreviation "MRI" means magnetic resonance imaging.

A result is considered "non-inferior" (NI) as compared to adalimumab if administration of the JAK1 inhibitor preserves at least 50% of the placebo-subtracted adalimumab effect.

The abbreviation "nr-axSpA" refers to non-radiographic axial spondyloarthritis.

The abbreviation "NRI" means non-responder imputation.

The abbreviation "NSAIDs" refers to non-steroidal anti-inflammatory drugs. Examples NSAIDs include, but are not limited to, traditional NSAIDs (*e.g.*, ibuprofen) and salicylates (*e.g.*, aspirin).

The term "pharmaceutically acceptable" (such as in the recitation of a "pharmaceutically acceptable salt" or a "pharmaceutically acceptable diluent") refers to a material that is compatible with administration to a human subject, *e.g.,* the material does not cause an undesirable biological effect. Examples of pharmaceutically acceptable salts are described in "Handbook of Pharmaceutical Salts: Properties, Selection, and Use" by Stahl and Wermuth (Wiley-VCH, Weinheim, Germany, 2002). Examples of pharmaceutically acceptable excipients are described in the "Handbook of Pharmaceutical Excipients," Rowe et al., Ed. (Pharmaceutical Press, 7th Ed., 2012).

"Pharmaceutically acceptable salts" refers to those salts which retain the biological effectiveness and properties of the free bases and which are obtained by reaction with inorganic acids, for example, hydrochloric acid, hydrobromic acid, sulfuric acid, nitric acid, and phosphoric acid or organic acids such as sulfonic acid, carboxylic acid, organic phosphoric acid, methane sulfonic acid, ethane sulfonic acid, p-toluene sulfonic acid, citric acid, fumaric acid, maleic acid, succinic acid, benzoic acid, salicylic acid, lactic acid, mono-malic acid, mono oxalic acid, tartaric acid such as mono tartaric acid (*e.g.,* (+) or (-)-tartaric acid or mixtures thereof), amino acids (*e.g.,* (+) or (-)-amino acids or mixtures thereof), and the like. These salts can be prepared by methods known to those skilled in the art.

A "population of subjects" refers to the group of subjects participating in a clinical trial, with all subjects suffering from the same disease or symptom to be treated, wherein the clinical trial comprises a treatment arm (a subgroup of the subjects treated with the JAK1 inhibitor), and a placebo arm (a subgroup of the subjects not treated with the JAK1 inhibitor). When used in connection with the treatment of a population of subjects, the phrase "at least X% of the subjects in the treated population achieve" a particular response refers to the placebo corrected X% response (subjects treated - subjects not treated).

The abbreviation "PR" means partial remission.

The abbreviation "PsA" refers to psoriatic arthritis.

The abbreviation "PsO" refers to psoriasis. Psoriasis includes psoriasis as a skin manifestation of PsA.

The abbreviation "QD" means once daily.

The phrase "reducing signs and symptoms" means an improvement in disease activity, function, and/or quality of life compared to baseline.

The abbreviation "SI" means sacroiliac.

"Statistically significant" means when observed p value < alpha for a given hypothesis testing. The pre-specified significance level, alpha, is the probability of rejecting the null hypothesis given that it is true. Alpha is also called type I error or false positive rate. It is usually set at or below 0.05. The observed p value is the probability, under the null hypothesis, of observing an effect of the same magnitude or more extreme. When observed p value < alpha, the null hypothesis is rejected, and statistical significance is claimed. In a multiplicity-controlled analysis, when the adjusted p value < alpha, the result is statistically significant. In the fixed sequence of a multiplicity-controlled analysis, statistical significance can be claimed for a lower ranked endpoint only if the previous endpoint in the sequence meets the requirements of statistical significance.

A result is considered "superior" as compared to adalimumab if the multiplicity adjusted p value for the null hypothesis testing of the treatment difference between the JAK1 inhibitor and adalimumab is less than pre-specified significance level.

"Total spinal ankylosis" refers to bridging syndesmophytes (fusion) in a total sum of $\geq$5 segments of the C2-T1 or T12-S1 spine (*e.g.*, 2 segments fused in the cervical and 3 segments fused in the lumbar spine would be considered positive for total spinal ankylosis).

The terms "treating", "treatment", and "therapy" and the like, as used herein, are meant to include but not limited to alleviation or relief of one or more symptoms of the condition from which the subject is suffering (*i.e.*, axial spondyloarthritis (axSpA) (*e.g.*, non-radiographic axSpA (nr-axSpA), ankylosing spondylitis (AS)), psoriatic arthritis (PsA), psoriasis (PsO)), including the slowing or cessation of the progression of the condition, such as slowing or cessation of the progression of structural damage associated with the condition, the structural progression of the condition, and/or improving the physical function of a subject suffering from the condition.

The term "upadacitinib freebase" refers to freebase (non-salt, neutral) forms of upadacitinib. Examples of upadacitinib freebase solid state forms include amorphous upadacitinib freebase and crystalline freebases of upadacitinib, such as crystalline freebase solvates, crystalline freebase hydrates, crystalline freebase hemihydrates, and crystalline freebase anhydrates of upadacitinib. Specific examples of upadacitinib freebase solid state forms include but are not limited to Amorphous Upadacitinib Freebase, Upadacitinib Freebase Solvate Form A, Upadacitinib Freebase Hydrate Form B, Upadacitinib Freebase Hydrate Form C (which is a hemihydrate), and Upadacitinib Freebase Anhydrate Form D, each as described in WO 2017/066775 and WO 2018/165581.

The term "upadacitinib freebase equivalent" refers to the amount of the neutral upadacitinib freebase (active ingredient) administered, and not including any coformer (*e.g.*, solvent or water molecule(s)) of a solvate or hydrate (including hemihydrate), and not including any pharmaceutically acceptable salt counteranions of a pharmaceutically acceptable salt. For example, 15.4 mg of crystalline upadacitinib freebase hemihydrate (which includes 1/2 of a water conformer molecule per upadacitinib freebase molecule) delivers 15 mg of upadacitinib freebase equivalent, while 30.7 mg of crystalline upadacitinib freebase hemihydrate (which includes 1/2 of a water conformer molecule per upadacitinib freebase molecule) delivers 30 mg of upadacitinib freebase equivalent.

## DETAILED DESCRIPTION

Ankylosing Spondylitis (AS) is a chronic, inflammatory rheumatic disease primarily affecting the axial skeleton, characterized by chronic back pain (including nocturnal back pain), morning stiffness, enthesitis, peripheral arthritis, and extra-articular manifestations. The "early" form of this disease (non-radiographic axial spondyloarthritis (nr-axSpA)) shares many of AS disease characteristics.

Due to the longstanding debilitating nature of AS, irreversible structural damage often occurs, negatively impacting patients' lives. No cure for AS exists, thus the primary goal of treatment is to maximize patients' quality of life through controlling the signs and symptoms of disease, preventing structural damage, and maintaining physical function, ideally by achieving sustained clinical remission or, at minimum, low disease activity. Nonsteroidal anti-inflammatory drugs (NSAIDs) are the first-line treatment for AS, followed by biologic disease-modifying antirheumatic drugs (bDMARDs), such as tumor necrosis factor (TNF) inhibitors or interleukin-17 (IL-17) inhibitors, in patients who do not sufficiently respond to NSAIDs. TNF inhibitors and IL-17 inhibitors are efficacious in some patients with AS, but there are still patients for whom neither of these approved therapies address individual treatment goals. AS is a difficult disease to treat, as shown based on low efficacy achieved with IL-6 inhibitors tocilizumab and sarilumab, as well as IL-12/23 inhibitor ustekinumab and T cell blockade inhibitor abatacept. See, *e.g.,* Sieper et al., Ann. Rheum. Dis. 2014 73:95-100, Sieper et al., Ann. Rheum. Dis. 2015 74:1051-1057; Deodhar et al., Arthritis and Rheumatology 2019 71:258-270, and Song et al., Ann. Rheum. Dis. 2011 70:1108-1110.

The JAK family is composed of 4 members: JAK1, 2, 3, and tyrosine kinase 2 (Tyk2). These cytoplasmic tyrosine kinases act in tandem to activate the Signal Transducer and Activator of Transcription (STAT) that transduce cytokine-mediated signals and are associated with multiple membrane cytokine receptors such as common gamma-chain (CGC) receptors and the glycoprotein 130 trans-membrane proteins. JAK3 and JAK1 are components of the CGC cytokine receptor complexes that are responsible for the signaling of the inflammatory cytokines IL-2, -4, -7, -9, -15 and -21; whereas IL-12 and IL-23 signal through JAK2 and Tyk2. See Ghoreschi, et al., Immunol Rev. (2009), 228:273-87. Propagation of these signals is important in the amplification of inflammatory responses in axial spondyloarthritis (axSpA). Upadacitinib, a JAK inhibitor engineered for increased selectivity for JAK1 over JAK2, JAK3, and tyrosine kinase 2, has been investigated for the treatment of bDMARD-naive patients with AS who had an inadequate response to non-steroidal anti-inflammatory drugs (NSAIDs) in the randomized, placebo-controlled phase 2/3 SELECT-AXIS 1 study. See Example 2, herein. A second study (SELECT-AXIS-2) is underway, expanding the scope of enrollment to non-radiographic axial spondyloarthritis (nr-axSpA) patients and AS bDMARD-IR patients. See Example 3.

The SELECT-AXIS 1 met its primary endpoint of significantly greater achievement of Assessment of SpondyloArthritis International Society (ASAS40) response at Week 14, as well as several disease activity measures (ASDAS, BASDAI, ASAS, and their components), inflammation (based on MRI of spine and sacroiliac joints as well as hsCRP), physical function (BASFI), quality of life (ASQoL, ASAS HI), and other aspects of disease (BASMI, MASES), reflecting significant improvement in outcomes for upadacitinib versus placebo. Furthermore, a review of the placebo corrected data for upadacitinib at Week 14, biologics Ixekizumab and Adalimumab at Week 16, and JAK small molecule inhibitors Tofacitinib and Filgotinib at Week 12 for key primary and secondary endpoints, while not a head to head comparison, suggests that upadacitinib 15 mg QD shows decided promise for the more difficult to achieve endpoints ASAS PR, ASDAS ID, and ASDAS LDA versus the other two JAK small molecule inhibitors, with a remarkable efficacy only comparable to that demonstrated with the biologics. See Example 2, and van der Heijde et al. Lancet (2018) 392: 2441-2451, van der Heijde D, et al. Ann. Rheum. Dis. (2017) 1-8; van der Heijde et al. Lancet (2018) 2378-2387. Furthermore, this efficacy, once achieved at Week 14, was sustained or improved over time, with long term efficacy in these difficult to achieve endpoints (including ASDAS major improvement (MI) and ASDAS clinically important improvement (CII)), sustained or improved up to and including Week 64. In patients who switched from placebo to upadacitinib at Week 14, a similar speed of onset and magnitude of efficacy response was observed up to and including Week 64 compared with those who received continuous upadacitinib starting at Week 0. Based on the results of phase 2/3 study SELECT-AXIS 1 and the consistent safety data from other upadacitinib clinical trials, the benefit-risk profile of upadacitinib in AS (particularly compared to the risk profile of other small molecule JAK inhibitors), and viewed in the context of the benefit-risk of TNF inhibitors and IL-17 inhibitors, presents a promising oral targeted treatment option for patients with AS, especially for those AS (as well as nr-axSpA patients) who have active disease and inadequate response to NSAIDs.

Psoriatic arthritis (PsA) is a systemic inflammatory disease with heterogeneous clinical manifestations such as plaque psoriasis, arthritis, dactylitis, and enthesitis, and is interrelated with AS, as it shares genetic and clinical features (e.g., axial involvement with back pain, peripheral arthritis or enthesitis, genetic association with HLA-B27 as well as presence of extra-articular manifestations). Multiple cytokines such as IL-1, -6, -12, -17, -20, and -23 are thought to be involved in the activation and proliferation of epidermal keratinocytes in psoriatic lesions. *See e.g.,* Nestle, et al., N. Engl. J. Med. (2009) 361:496-509. The IL-17/IL-23 cytokine axis is also thought to be important in PsA pathogenesis. *See e.g.,* Mease, Curr. Opin. Rheumatol. (2015) 27:127-33. Thus, blockade of JAK1 could inhibit the response of central cytokine signals thought to be important in the pathogenesis of PsA. Current treatment guidelines for PsA vary, recommending conventional synthetic disease-modifying anti-rheumatic drugs (DMARDs) such as methotrexate as initial therapy, followed by biologic DMARDs or targeted synthetic DMARDs, such as tofacitinib, or TNFi initially, followed by other approved therapies. While multiple therapeutic choices are available, additional options are needed in order to reach the more difficult to achieve endpoints, such as achievement of minimal disease activity (MDA), as well as treatment of the psoriasis as a skin manifestation of PsA, with achievement of PASI 75 or PASI 90. The JAK1 inhibitor upadacitinib has been investigated for the treatment of patients with active Psoriatic Arthritis and a previous inadequate response to at least one non-biologic Disease Modifying Anti-Rheumatic Drug (non-biologic DMARD) (SELECT PSA1) or previous inadequate response to at least one biologic Disease Modifying Anti-Rheumatic Drug (bDMARD) (SELECT PSA2). See Examples 4 and 5. In these two trials, greater efficacy was demonstrated for once daily upadacitinib 15 mg and 30 mg versus placebo for clinical manifestations of psoriatic arthritis including musculoskeletal symptoms (peripheral arthritis, enthesitis, dactylitis, and spondylitis), psoriasis, physical function, pain, fatigue, and quality of life. Efficacy was observed as early as week 2. Furthermore, a review of the placebo corrected data for upadacitinib and the JAK small molecule inhibitor Tofacitinib (approved for the lower (5 mg BID) dose in the treatment of PsA) for key primary and secondary endpoints, while not a head to head comparison, suggests that upadacitinib 15 mg QD and 30 mg QD shows decided promise for the more difficult to achieve endpoints of minimal disease activity (MDA), as well as psoriasis endpoints PASI 75 or PASI 90, as well as certain ACR components (*e.g.*, ACR20/50/70). See Examples 4 and 5, as well as Gladman et al., New England Journal of Medicine (2017) 377:1525-1536 and Mease P et al. N Engl J Med (2017) 377:1537-1550. Furthermore, it was observed that this efficacy, once achieved, was sustained or improved over time during the course of the daily treatment.

Thus, in one aspect, provided is a method for treating various spondyloarthritic and psoriatic conditions, including types of axial spondyloarthritis (axSpA), psoriatic arthritis (PsA), and psoriasis (PsO) by administering the JAK1 inhibitor, upadacitinib free base or a pharmaceutically acceptable salt, to a subject in need thereof. In various aspects, provided is a method for treating active non-radiographic axSpA (nr-axSpA), methods for treating active ankylosing spondylitis (AS), and methods for treating active psoriatic arthritis (PsA) and active psoriasis (PsO), including PsO as a skin manifestation of PsA.

In one embodiment, the JAK1 inhibitor useful in the methods disclosed herein is upadacitinib freebase. Upadacitinib freebase solid state forms include amorphous upadacitinib freebase and crystalline freebases of upadacitinib. Crystalline freebases of upadacitinib include those selected from the group consisting of crystalline freebase solvates of upadacitinib, crystalline freebase hydrates of upadacitinib (*e.g.*, crystalline freebase hemihydrates of upadacitinib), and crystalline freebase anhydrates of upadacitinib. In one embodiment, the crystalline freebase of upadacitinib is a crystalline freebase hemihydrate of upadacitinib. In one embodiment, the crystalline freebase of upadacitinib is Upadacitinib Freebase Hydrate Form C (which is a hemihydrate) as described in WO 2018/165581 and WO 2017/066775. Other specific examples of solid state forms of the JAK1 inhibitor suitable for use in the methods disclosed herein include those selected from the group consisting of Amorphous Upadacitinib Freebase, Upadacitinib Freebase Solvate Form A, Upadacitinib Freebase Hydrate Form B, Upadacitinib Freebase Anhydrate Form D, and Upadacitinib Tartrate Hydrate, each as described in WO 2018/165581 and WO 2017/066775.

## Methods of Treating Axial Spondyloarthritis

Provided herein are methods of treating axial spondyloarthritis (axSpA). In a particular aspect, provided are methods of treating active axSpA, which encompasses treating subjects with active ankylosing spondylitis (AS) and active non-radiographic axial spondyloarthritis (nr-axSpA), comprising administering orally once a day a dose of the JAK1 inhibitor, upadacitinib freebase, or a pharmaceutically acceptable salt thereof, to a subject in need thereof in certain amounts and/or at certain intervals. In one aspect, the JAK1 inhibitor is upadacitinib freebase. In one aspect, the JAK1 inhibitor is administered orally once a day in an amount sufficient to deliver 15 mg of upadacitinib freebase equivalent. In one aspect, the JAK1 inhibitor is administered orally once a day for at least 14 weeks. In one aspect, the JAK1 inhibitor is administered orally once a day for at least 52 weeks.

Disease activity/severity for axSpA may be measured using a variety of indexes, including the Assessment of SpondyloArthritis International Society (ASAS) responses (*e.g.*, ASAS20, ASAS40, ASAS partial remission (PR), ASAS5/6); the Ankylosing Spondylitis Disease Activity Score (ASDAS), ASDAS low disease activity (LDA), ASDAS inactive disease (ID), ASDAS major improvement (MI), ASDAS clinically important improvement (CII), the magnetic resonance imaging (MRI) Spondyloarthritis Research Consortium of Canada (SPARCC) score for spine (MRI-Spine SPARCC); the MRI SPARCC score for sacroiliac (SI) joints (MRI-SI joints SPARCC); the Bath Ankylosing Spondylitis Disease Activity Index (BASDAI); a BASDAI 50 (BASDAI50) response; the Bath Ankylosing Spondylitis Functional Index (BASFI); the Ankylosing Spondylitis Quality of Life Questionnaire (ASQoL); the ASAS Health Index (HI); the Maastricht Ankylosing Spondylitis Enthesitis Score (MASES) (enthesitis); the Linear Bath Ankylosing Spondylitis Metrology Index (BASMI$_{lin}$) (mobility); the Work Productivity and Activity Impairment Questionnaire - Axial Spondyloarthritis (WPAI-Axial SpA); high-sensitivity C-reactive protein levels (hsCRP); the Functional Assessment of Chronic Illness Therapy-Fatigue (FACIT-F) Questionnaire; the Insomnia Severity Index (ISI); the Modified Stroke Ankylosing Spondylitis Spine Score (mSASSS); the Patient's Assessment of Total Back Pain (Total Back Pain score); the Patient's Assessment of Nocturnal Back Pain (Nocturnal Back Pain); the Patient's Global Assessment of Pain (Pt Pain); the Physician's Global Assessment of Disease Activity (PGA-Disease Activity); Inflammation (mean of Questions 5 and 6 of the BASDAI); Patient's Assessment of Total Back Pain (Question 2 of BASDAI); Peripheral pain/swelling (Question 3 of BASDAI); duration of morning stiffness (Question 6 of BASDAI); Patient's Global Assessment of Disease Activity (PtGA); tender joint count (TJC68) and swollen joint count (SJC66); resolution of dactylitis; total dactylitis count; EuroQoL-5D-5L (EQ-5D-5L) Questionnaire; 36-Item Short Form Health Survey (SF-36); Physical Activity Assessment, and NSAID score. These indexes are described in detail in the Clinical Endpoint Definitions and Examples.

In one aspect, provided is a method of treating axSpA, including active axSpA, comprising administering a dose of the JAK1 inhibitor to a subject in need thereof in certain amounts and/or at certain intervals as described herein, wherein the subject achieves an Assessment of SpondyloArthritis International Society 40 (ASAS40) response following administration of the JAK1 inhibitor. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 14 weeks. In one aspect, the JAK1 inhibitor is administered orally once a day for at least 52 weeks.

In one aspect, the subject achieves an ASAS40 response within 14 weeks of administration of the first dose of the JAK1 inhibitor (including at week 14). In one aspect, the subject achieves an ASAS40 response within 14 weeks of administration of the first dose of the JAK1 inhibitor (including at week 14), and the response is maintained or improved after week 14 by continuing to administer the daily dose. In one aspect, the subject achieves an ASAS40 response within 2 weeks of administration of the first dose of the JAK1 inhibitor (including at week 2). In one aspect, the subject achieves an ASAS40 response within 2 weeks of administration of the first dose of the JAK1 inhibitor (including at week 2), and the response is maintained or improved after week 2 by continuing to administer the daily dose. In one aspect, the subject achieves an ASAS40 response within 52 weeks of administration of the first dose of the JAK1 inhibitor (including at week 52). In one aspect, the subject achieves an ASAS40 response within 52 weeks of administration of the first dose of the JAK1 inhibitor (including at week 52), and the response is maintained or improved after week 52 by continuing to administer the daily dose. In one aspect, the subject achieves an ASAS40 response within 2 weeks, within 4 weeks, within 8 weeks, within 12 weeks, within 14 weeks, within 18 weeks, within 24 weeks, within 32 weeks, within 40 weeks, and/or within 52 weeks of administration of the first dose of the JAK1 inhibitor (including at week 2, week 4, week 8, week 12, week 14, week 18, week 24, week 32, week 40, and/or week 52). In one embodiment, the subject achieves an ASAS40 response within 2 weeks of administration of the first dose (including at week 2) and maintains the ASAS40 response until at least 14 weeks after administration of the first dose (e.g., until at least week 14). In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 2 weeks, for at least 4 weeks, for at least 8 weeks, for at least 12 weeks, for at least 14 weeks, for at least 18 weeks, for at least 24 weeks, for at least 32 weeks, for at least 40 weeks, and/or for at least 52 weeks. In one embodiment, the JAK1 inhibitor is administered orally once a day for at least 14 weeks. In one aspect, the JAK1 inhibitor is administered orally once a day for at least 52 weeks.

In another aspect, provided is a method of treating axSpA, including active axSpA, comprising administering the JAK1 inhibitor to a subject in need thereof in certain amounts and/or at certain intervals as described herein, wherein the subject achieves an ASAS40 response at a certain interval as described herein, and additionally achieves at least one of the results set forth hereinafter for treatment of ankylosing spondylitis (AS) and/or non-radiographic axial spondyloarthritis (nr-axSpA) following administration of the JAK1 inhibitor. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 14 weeks. In one aspect, the JAK1 inhibitor is administered orally once a day for at least 52 weeks.

In another aspect, provided is a method of treating axSpA, including active axSpA, in a population of subjects in need thereof, the method comprising administering a dose of the JAK1 inhibitor to the subjects in certain amounts and/or at certain intervals as described herein, wherein a portion of subjects in the treated population achieve an ASAS40 response following administration of the JAK1 inhibitor (e.g., a statistically significant population of the subjects in the treated population, and/or at least 10%, at least 15%, at least 20%, at least 25%, at least 30%, at least 35%, at least 40%, or at least 45% of the subjects in the treated population achieve the response). In one aspect, the subjects in the treated population achieve an ASAS40 response within 14 weeks of administration of the first dose of the JAK1 inhibitor (including at week 14). In one aspect, subjects in the treated population achieve an ASAS40 response within 14 weeks of administration of the first dose of the JAK1 inhibitor (including at week 14), and the response is maintained or improved after week 14 by continuing to administer a daily dose of the JAK1 inhibitor to the subjects. In one aspect, subjects in the treated population achieve an ASAS40 response within 52 weeks of administration of the first dose of the JAK1 inhibitor (including at week 52). In one aspect, subjects in the treated population achieve an ASAS40 response within 52 weeks of administration of the first dose of the JAK1 inhibitor (including at week 52), and the response is maintained or improved after week 52 by continuing to administer a daily dose of the JAK1 inhibitor to the subjects. In one aspect, the subjects in the treated population achieve an ASAS40 response within 2 weeks of administration of the first dose of the JAK1 inhibitor (including at week 2). In one aspect, subjects in the treated population achieve an ASAS40 response within 2 weeks of administration of the first dose of the JAK1 inhibitor (including at week 2), and the response is maintained or improved after week 2 by continuing to administer a daily dose of the JAK1 inhibitor to the subjects. In one aspect, subjects in the treated population achieve at least one of the results set forth hereinafter for treatment of ankylosing spondylitis (AS) and/or non-radiographic axial spondyloarthritis (nr-axSpA) following administration of the JAK1 inhibitor (*e.g.*, a statistically significant population of subjects in the treated population, and/or at least 10%, at least 15%, at least 20%, at least 25%, at least 30%, at least 35%, at least 40%, or at least 45% of the subjects in the treated population achieve at least one of the results). In one aspect, the JAK1 inhibitor is upadacitinib freebase. In one aspect, a dose of the JAK1 inhibitor is administered to the population in an amount sufficient to deliver 15 mg of upadacitinib freebase equivalent. In one aspect, the JAK1 inhibitor is administered to the subjects orally once a day for at least 14 weeks. In one aspect, the JAK1 inhibitor is administered to the subjects orally once a day for at least 52 weeks.

Further provided are methods of reducing the signs and symptoms of axSpA. In one aspect, provided is a method of reducing the signs and symptoms of axSpA, including active axSpA, comprising administering a dose of the JAK1 inhibitor to a subject in need thereof in certain amounts and/or at certain intervals. In one aspect, the JAK1 inhibitor is upadacitinib freebase or a pharmaceutically acceptable salt thereof. In one aspect, the JAK1 inhibitor is administered in an amount sufficient to deliver 15 mg of upadacitinib freebase equivalent. In one aspect, the JAK1 inhibitor is administered once a day for 14 weeks. In one aspect, the JAK1 inhibitor is administered once a day for 52 weeks.

In one aspect, the signs and symptoms of axSpA, including active axSpA, are reduced following administration of the JAK1 inhibitor when the subject achieves, within 14 weeks of administration of the first dose of the JAK1 inhibitor (including at week 14), at least one result selected from the group consisting of: an ASAS40 response; a change (improvement) from baseline in ASDAS; a change (improvement) from baseline in MRI SPARCC score for spine (MRI-Spine SPARCC); ASAS partial remission (PR); a BASDAI50 response; a change (improvement) from baseline in BASFI; a change (improvement) from baseline in ASQoL; a change (improvement) from baseline in ASAS Health Index (HI); a change (improvement) from baseline in MASES (enthesitis); a change (improvement) from baseline in BASMI$_{lin}$ (mobility); a change (improvement) from baseline in WPAI-Axial SpA; and a change (improvement) from baseline in MRI SPARCC score for SI joints (MRI-SI joints SPARCC); or when the subject achieves, within 52 weeks of administration of the first dose of the JAK1 inhibitor (including at week 52), an ASAS40 response. In one aspect, the subject achieves the response within 14 weeks of administration of the first dose of the JAK1 inhibitor (including at week 14), and the response is maintained or improved after week 14 by continuing to administer a daily dose of the JAK1 inhibitor. In another aspect, the subject achieves the response within 52 weeks of administration of the first dose of the JAK1 inhibitor (including at week 52), and the response is maintained or improved after week 52 by continuing to administer a daily dose of the JAK1 inhibitor. In one aspect, the subject achieves the response within 2 weeks of administration of the first dose of the JAK1 inhibitor (including at week 2), and the response is maintained or improved after week 2 by continuing to administer a daily dose of the JAK1 inhibitor.

For any of the methods of treating axSpA and/or methods for reducing the signs and symptoms of axSpA described herein, the subject and/or subjects in the treated population i) may be biologic disease-modifying anti-rheumatic drug (bDMARD) naive or ii) may have had an inadequate response or intolerance to a bDMARD (bDMARD-IR) at baseline. In certain embodiments, the subject (or subjects in the treated population) may have had a prior inadequate response to, intolerance to, or contraindication to NSAIDs at baseline.

Methods of Treating Ankyolosing Spondylitis (AS)

Further provided are methods of treating ankylosing spondylitis (AS). For example, in one aspect, provided is a method of treating AS, including active AS, comprising administering orally once a day a dose of the JAK1 inhibitor to a subject in need thereof in certain amounts and/or at certain intervals. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 14 weeks. In one aspect, the subject is bDMARD naive. In one aspect, the subject is bDMARD-IR.

Disease activity/severity for AS may be measured using a variety of indexes, including those set forth above for the treatment of axSpA. In one particular aspect, provided is a method of treating AS, including active AS, comprising administering a dose of the JAK1 inhibitor to a subject in need thereof in certain amounts and/or at certain intervals as described herein, wherein the subject achieves an Assessment of SpondyloArthritis International Society 40 (ASAS40) response following administration of the JAK1 inhibitor. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 14 weeks. In one aspect, the subject is bDMARD naive. In one aspect, the subject is bDMARD-IR. In one aspect, the subject is an adult.

In one aspect, the subject achieves an ASAS40 response within 14 weeks of administration of the first dose of the JAK1 inhibitor (including at week 14). In one aspect, the subject achieves an ASAS40 response within 14 weeks of administration of the first dose of the JAK1 inhibitor (including at week 14), and the response is maintained or improved after week 14 by continuing to administer a daily dose of the JAK1 inhibitor. In one aspect, the subject achieves an ASAS40 response within 2 weeks of administration of the first dose of the JAK1 inhibitor (including at week 2). In one aspect, the subject achieves an ASAS40 response within 2 weeks of administration of the first dose of the JAK1 inhibitor (including at week 2), and the response is maintained or improved after week 2 by continuing to administer a daily dose of the JAK1 inhibitor. In one aspect, the subject achieves an ASAS40 response within 2 weeks, within 4 weeks, within 8 weeks, within 12 weeks, within 14 weeks, within 16 weeks, within 18 weeks, within 20 weeks, within 24 weeks, within 32 weeks, within 40 weeks, within 52 weeks, within 64 weeks, within 76 weeks, within 88 weeks, within 96 weeks, and/or within 104 weeks (including at week 2, week 4, week 8, week 12, week 14, week 16, week 18, week 20, week 24, week 32, week 40, week 52, week 64, week 76, week 88, week 96, and/or week 104) of administration of the first dose of the JAK1 inhibitor. In one aspect, the subject achieves an ASAS 40 response within 14 weeks of administration of the first dose of the JAK1 inhibitor (including at week 14), and the ASAS40 response is maintained or improved until at least 64 weeks after administration of the first dose (e.g., up to and including week 64). In one embodiment, the subject achieves an ASAS 40 response within 2 weeks of administration of the first dose (including at week 2), and maintains or improves the ASAS40 response until at least 14 weeks after administration of the first dose (*e.g.*, until at least week 14). In one embodiment, the subject achieves an ASAS 40 response within 2 weeks of administration of the first dose of the JAK1 inhibitor (including at week 2), and the ASAS40 response is maintained or improved until at least 64 weeks after administration of the first dose (e.g., up to and including week 64). In one aspect, the subject alternately or additionally achieves within 14 weeks of administration of the first dose of the JAK1 inhibitor (including at week 14) at least one additional result selected from the group consisting of: ASAS partial remission (PR); BASDAI50 response; change (improvement) from baseline in MRI SPARCC score for spine (MRI-Spine SPARCC); change (improvement) from baseline in ASDAS; change (improvement) from baseline in BASFI; ASDAS low disease activity (LDA); ASDAS inactive disease (ID); ASDAS major improvement (MI); and ASDAS clinically important improvement (CII). In one aspect, the subject achieves the result within 14 weeks of administration of the first dose of the JAK1 inhibitor (including at week 14), and the result is maintained or improved after week 14 by continuing to administer a daily dose of the JAK1 inhibitor. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 2 weeks, for at least 4 weeks, for at least 8 weeks, for at least 12 weeks, for at least 14 weeks, for at least 16 weeks, for at least 18 weeks, for at least 20 weeks, for at least 24 weeks, for at least 32 weeks, for at least 40 weeks, for at least 52 weeks, for at least 64 weeks, for at least 76 weeks, for at least 88 weeks, for at least 96 weeks, and/or for at least 104 weeks. In one embodiment, the JAK1 inhibitor is administered orally once a day for at least 14 weeks.

Further provided are methods of treating AS, including active AS, in a subject in need thereof, comprising administering orally once a day a dose of a JAK1 inhibitor to a subject in need thereof in certain amounts and/or at certain intervals as described herein, wherein the subject achieves ASAS partial remission (PR), ASDAS low disease activity (LDA), ASDAS inactive disease (ID), ASDAS major improvement (MI), and/or ASDAS clinically important improvement (CII) following administration of the JAK1 inhibitor. In one embodiment, the subject achieves ASAS PR, ASDAS LDA, ASDAS ID, ASDAS MI, and/or ASDAS CII within 14 weeks of administration of the first dose of the JAK1 inhibitor (including at week 14). In one embodiment, the subject achieves each result (e.g., ASAS PR, ASDAS LDA, ASDAS ID, ASDAS MI, and ASDAS CII) within 14 weeks of administration of the first dose of the JAK1 inhibitor (including at week 14). In one aspect, the subject achieves ASAS PR, ASDAS LDA, ASDAS ID, ASDAS MI, and/or ASDAS CII within 2 weeks of administration of the first dose of the JAK1 inhibitor (including at week 2). In one aspect, the subject achieves ASAS PR, ASDAS LDA, ASDAS ID, ASDAS MI, and/or ASDAS CII within 2 weeks, within 4 weeks, within 8 weeks, within 12 weeks, within 14 weeks, within 16 weeks, within 18 weeks, within 20 weeks, within 24 weeks, within 32 weeks, within 40 weeks, within 52 weeks, within 64 weeks, within 76 weeks, within 88 weeks, within 96 weeks, and/or within 104 weeks (including at week 2, week 4, week 8, week 12, week 14, week 16, week 18, week 20, week 24, week 32, week 40, week 52, week 64, week 76, week 88, week 96, and/or week 104) of administration of the first dose of the JAK1 inhibitor. In one aspect, the subject achieves ASAS PR, ASDAS LDA, ASDAS ID, ASDAS MI, and/or ASDAS CII within 14 weeks of administration of the first dose of the JAK1 inhibitor (including at week 14), and the response is maintained or improved after week 14 by continuing to administer a daily dose of the JAK1 inhibitor. In one aspect, the subject achieves ASAS PR, ASDAS LDA, ASDAS ID, ASDAS MI, and/or ASDAS CII within 2 weeks of administration of the first dose of the JAK1 inhibitor (including at week 2), and the response is maintained or improved after week 2 by continuing to administer the daily dose of the JAK1 inhibitor. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 2 weeks, for at least 4 weeks, for at least 8 weeks, for at least 12 weeks, for at least 14 weeks, for at least 16 weeks, for at least 18 weeks, for at least 20 weeks, for at least 24 weeks, for at least 32 weeks, for at least 40 weeks, for at least 52 weeks, for at least 64 weeks, for at least 76 weeks, for at least 88 weeks, for at least 96 weeks, and/or for at least 104 weeks. In one embodiment, the JAK1 inhibitor is administered orally once a day for at least 14 weeks In one aspect, the subject is bDMARD naive.

In one aspect, the subject is bDMARD-IR. In one aspect, the subject is an adult.

In one aspect, provided is a method of treating AS, including active AS, in a population of subjects in need thereof, the method comprising administering a dose of the JAK1 inhibitor to the subjects in certain amounts and/or at certain intervals as described herein, wherein a portion of subjects in the treated population (e.g., a statistically significant population of subjects in the treated population, and/or at least 10%, at least 15%, at least 20%, at least 25%, at least 30%, at least 35%, at least 40%, or at least 45% of the subjects in the treated population) achieve an ASAS40 response following administration of the JAK1 inhibitor. In one aspect, subjects in the treated population achieve an ASAS40 response within 14 weeks of administration of the first dose of the JAK1 inhibitor (including at week 14). In one aspect, subjects in the treated population achieve an ASAS40 response within 14 weeks of administration of the first dose of the JAK1 inhibitor (including at week 14), and the response is maintained or improved after week 14 by continuing to administer a daily dose of the JAK1 inhibitor. In one aspect, subjects in the treated population achieve an ASAS40 response within 2 weeks of administration of the first dose of the JAK1 inhibitor (including at week 2). In one aspect, subjects in the treated population of the subjects achieve an ASAS40 response within 2 weeks of administration of the first dose of the JAK1 inhibitor (including at week 2), and the response is maintained or improved after week 2 by continuing to administer a daily dose of the JAK1 inhibitor. In one aspect, subjects in the treated population achieve an ASAS40 response within 2 weeks, within 4 weeks, within 8 weeks, within 12 weeks, within 14 weeks, within 16 weeks, within 18 weeks, within 20 weeks, within 24 weeks, within 32 weeks, within 40 weeks, within 52 weeks, within 64 weeks, within 76 weeks, within 88 weeks, within 96 weeks, and/or within 104 weeks (including at week 2, week 4, week 8, week 12, week 14, week 16, week 18, week 20, week 24, week 32, week 40, week 52, week 64, week 76, week 88, week 96, and/or week 104) of administration of the first dose of the JAK1 inhibitor. In one embodiment, subjects in the treated population achieve an ASAS 40 response within 14 weeks of administration of the first dose of the JAK1 inhibitor (including at week 14), and the ASAS40 response is maintained or improved until at least 64 weeks after administration of the first dose (e.g., up to and including week 64). In one embodiment, subjects in the treated population achieve an ASAS 40 response within 2 weeks of administration of the first dose (including at week 2), and maintains or improves the ASAS40 response until at least 14 weeks after administration of the first dose (e.g., until at least week 14). In one aspect, subjects in the treated population alternately or additionally achieve within 14 weeks of administration of the first dose of the JAK1 inhibitor (including at week 14) at least one additional result selected from the group consisting of: ASAS partial remission (PR); BASDAI50 response; change (improvement) from baseline in MRI SPARCC score for spine (MRI-Spine SPARCC); change (improvement) from baseline in ASDAS; change (improvement) from baseline in BASFI; ASDAS low disease activity (LDA); ASDAS inactive disease (ID); ASDAS major improvement (MI); and ASDAS clinically important improvement (CII) . In one aspect, subjects in the treated population achieve the result within 14 weeks of administration of the first dose of the JAK1 inhibitor (including at week 14), and the result is maintained or improved after week 14 by continuing to administer a daily dose of the JAK1 inhibitor. In certain embodiments, for any of the aforementioned results achieved, a statistically significant population of the subjects in the treated population, and/or at least 10%, at least 15%, at least 20%, at least 25%, at least 30%, at least 35%, at least 40%, or at least 45% of the subjects in the treated population, achieve the result. In one aspect, the JAK1 inhibitor is upadacitinib freebase. In one aspect, a dose of the JAK1 inhibitor is administered to the population in an amount sufficient to deliver 15 mg of upadacitinib freebase equivalent. In one aspect, the JAK1 inhibitor is administered to the population orally once a day for at least 14 weeks. In one aspect, the subjects in the population are bDMARD naive. In one aspect, the subjects in the population are bDMARD-IR.

Further provided are methods of treating AS, including active AS, in a population of subjects in need thereof, the method comprising administering orally once a day a dose of the JAK1 inhibitor to a subject in need thereof in certain amounts and/or at certain intervals as described herein, wherein a portion of the subjects in the treated population (e.g., a statistically significant population of subjects in the treated population, and/or at least 10%, at least 15%, at least 20%, at least 25%, at least 30%, at least 35%, at least 40%, or at least 45% of the subjects in the treated population) achieve ASAS partial remission (PR), ASDAS low disease activity (LDA), ASDAS inactive disease (ID), ASDAS major improvement (MI), and/or ASDAS clinically important improvement (CII) following administration of the JAK1 inhibitor. In one aspect, subjects in the treated population achieve ASAS PR, ASDAS LDA, ASDAS ID, ASDAS MI, and/or ASDAS CII within 14 weeks of administration of the first dose of the JAK1 inhibitor (including at week 14). In one aspect, subjects in the treated population achieve each result (e.g., ASAS PR, ASDAS LDA, ASDAS ID, ASDAS MI, and ASDAS CII) within 14 weeks of administration of the first dose of the JAK1 inhibitor (including at week 14). In one aspect, subjects in the treated population achieve ASAS PR, ASDAS LDA, ASDAS ID, ASDAS MI, and/or ASDAS CII within 2 weeks of administration of the first dose of the JAK1 inhibitor (including at week 2). In one aspect, subjects in the treated population achieve ASAS PR, ASDAS LDA, ASDAS ID, ASDAS MI, and/or ASDAS CII within 2 weeks, within 4 weeks, within 8 weeks, within 12 weeks, within 14 weeks, within 16 weeks, within 18 weeks, within 20 weeks, within 24 weeks, within 32 weeks, within 40 weeks, within 52 weeks, within 64 weeks, within 76 weeks, within 88 weeks, within 96 weeks, and/or within 104 weeks (including at week 2, week 4, week 8, week 12, week 14, week 16, week 18, week 20, week 24, week 32, week 40, week 52, week 64, week 76, week 88, week 96, and/or week 104) of administration of the first dose of the JAK1 inhibitor. In one aspect, subjects in the treated population achieve ASAS PR, ASDAS LDA, ASDAS ID, ASDAS MI, and/or ASDAS CII within 14 weeks of administration of the first dose of the JAK1 inhibitor (including at week 14), and the response is maintained or improved after week 14 by continuing to administer the daily dose of the JAK1 inhibitor. In one aspect, subjects in the treated population achieve ASAS PR, ASDAS LDA, ASDAS ID, ASDAS MI, and/or ASDAS CII within 2 weeks of administration of the first dose of the JAK1 inhibitor (including at week 2), and the response is maintained or improved after week 2 by continuing to administer the daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve the result. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 2 weeks, for at least 4 weeks, for at least 8 weeks, for at least 12 weeks, for at least 14 weeks, for at least 16 weeks, for at least 18 weeks, for at least 20 weeks, for at least 24 weeks, for at least 32 weeks, for at least 40 weeks, for at least 52 weeks, for at least 64 weeks, for at least 76 weeks, for at least 88 weeks, for at least 96 weeks, and/or for at least 104 weeks. In one embodiment, the JAK1 inhibitor is administered orally once a day for at least 14 weeks. In one aspect, the subjects in the population are bDMARD naive. In one aspect, the subjects in the population are bDMARD-IR. In one aspect, the subjects are adults.

Further provided are methods of reducing the signs and symptoms of AS. In one aspect, provided is a method of reducing the signs and symptoms of AS, including active AS, the method comprising administering a dose of the JAK1 inhibitor to a subject in need thereof in certain amounts and/or at certain intervals. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 14 weeks. In one aspect, the subject is bDMARD naive. In one aspect, the subject is bDMARD-IR.

In one aspect of a method of reducing the signs and symptoms of AS, including active AS, wherein the subject achieves within 14 weeks of administration of the first dose of the JAK1 inhibitor (including at week 14), at least one result selected from the group consisting of: an ASAS40 response; a change (improvement) from baseline in ASDAS; a change (improvement) from baseline in MRI-Spine SPARCC; ASAS partial remission (PR); a BASDAI50 response; a change (improvement) from baseline in BASFI; a change (improvement) from baseline in ASQoL; a change (improvement) from baseline in ASAS Health Index (HI); a change (improvement) from baseline in MASES (enthesitis); a change (improvement) from baseline in $BASMI_{lin}$ (mobility); and a change (improvement) from baseline in WPAI-Axial SpA. In certain embodiments, for any of the aforementioned results achieved, a statistically significant population of the subjects in the treated population, and/or at least 10%, at least 15%, at least 20%, at least 25%, at least 30%, at least 35%, at least 40%, or at least 45% of the subjects in the treated population, achieve the result. In certain embodiments, for any of the aforementioned results achieved, the subject (or subjects in the treated population) achieve the result or results within 14 weeks of administration of the first dose of the JAK1 inhibitor (including at week 14), and the result (or results) is maintained or improved after week 14 by continuing to administer a daily dose of the JAK1 inhibitor.

In another aspect, the subject has (or subjects in the treated population have) active AS at baseline. In one aspect, the subject (or subjects in the treated population) fulfills the 1984 modified New York Criteria for AS at baseline. In another aspect, the subject (or subjects in the treated population) fulfills the 2009 ASAS classification criteria at baseline. In yet another aspect, the subject (or subjects in the treated population) fulfills the 1984 modified New York Criteria for AS and the 2009 ASAS classification criteria at baseline. In one embodiment, the subject (or subjects in the treated population) meets at least one criteria selected from the group consisting of: (i) a BASDAI score ≥4; (ii) an ASDAS of ≥2.1; and (iii) a Patient's Assessment of Total Back Pain (Total Back Pain score) of ≥4 (based on a 0-10 numerical rating scale) at baseline. In one embodiment, the subject (or subjects in the treated population) has both a BASDAI score ≥4 and a Patient's Assessment of Total Back Pain (Total Back Pain score) of ≥4 at baseline. In another embodiment, the subject (or subjects in the treated population) has both a BASDAI score ≥4 and an ASDAS of ≥2.1 at baseline. In certain embodiments, the subject (or subjects in the treated population) has (i) a BASDAI score ≥4; (ii) an ASDAS of ≥2.1; and (iii) a Patient's Assessment of Total Back Pain (Total Back Pain score) of ≥4 (based on a 0-10 numerical rating scale) at baseline. In one aspect, the subject (or subjects in the treated population) does not have total spinal ankylosis at baseline. In one aspect, the subject (or subjects in the treated population) is an adult subject. In another aspect, the subject (or subjects in the treated population) is a juvenile subject.

In one aspect, the subject (or subjects in the treated population) is bDMARD naive at baseline. Exemplary bDMARDs include, but are not limited to, a biologic tumor necrosis factor inhibitors (*e.g.*, adalimumab, etanercept) and interleukin IL)-17 inhibitors (*e.g.*, secukinumab, ixekizumab).

In one aspect, the subject (or subjects in the treated population) is bDMARD naive at baseline, and further has had i) an inadequate response or intolerance to at least two NSAIDs (*e.g.*, over at least a four week period at the maximum recommended or tolerated doses); ii) intolerance to NSAIDs; and/or iii) contraindication for NSAIDs, as determined by a physician. Examples of NSAIDs include, but are not limited to, traditional NSAIDs (*e.g.*, ibuprofen) and salicylates (*e.g.*, aspirin).

In certain aspects, the subject (or subjects in the treated population) to be treated is bDMARD naive at baseline, has had an inadequate response or intolerance to at least two NSAIDS (as described above), or an intolerance to or contraindication for NSAIDS, and is further receiving at least one additional therapy. Additional therapies include, but are not limited to concomitant administration of non-biologic DMARDs, NSAIDs, corticosteroids, and combinations thereof. Suitable additional therapies for use in combination with the methods described herein include:

1) Concomitant administration of non-biologic DMARDs, including methotrexate (MTX), leflunomide (LEF), sulfasalazine (SSZ), and/or hydroxychloroquine (HCQ). In one embodiment, the subject is on a stable dose of MTX ($\leq$ 25 mg/week), SSZ ($\leq$ 3 g/day), hydroxychloroquine ($<$ 400 mg/day), and/or leflunomide ($\leq$ 20 mg/day) for at least 28 days prior to baseline. In some embodiments, a combination of up to two background non-biologic DMARDs is allowed, except the combination of MTX and leflunomide. In one embodiment, the subject has not received any non-biologic DMARDs (other than MTX, LEF, SSZ, and/or HCQ), thalidomide, or apremilast within 28 days or five half-lives (whichever is longer) prior to baseline.

2) Concomitant administration of oral corticosteroids. In one embodiment, the subject is on a stable dose of prednisone ($<$ 10 mg/day), or oral corticosteroid equivalents, for at least 14 days prior to baseline.

3) Concomitant administration of NSAIDs, tramadol, a combination of acetaminophen and codeine or hydrocodone, and/or non-opioid analgesics. In one embodiment, the subject is on stable dose(s) for at least 14 days prior baseline.

In another aspect, the subject (or subjects in the treated population) is bDMARD-IR at baseline. In one aspect, the subject has had an inadequate response or intolerance to a bDMARD at baseline. Subjects who are bDMARD-IR include those subjects who have had prior exposure to one bDMARD (either 1 tumor necrosis factor (TNF) inhibitor (*e.g.*, adalimumab, etanercept) or 1 interleukin (IL)-17 inhibitor (*e.g.*, secukinumab, ixekizumab)), and have discontinued the bDMARD due to either intolerance or lack of efficacy (*e.g.,* as determined by a physician). In one embodiment, the subject (or subjects in the treated population) has not had prior exposure to a second bDMARD, if the reason for discontinuation was not due to lack of efficacy. In one embodiment, the subject (or subjects in the treated population) has not discontinued both a TNF inhibitor and an IL-17 inhibitor due to lack of efficacy.

In certain embodiments, the subject (or subjects in the treated population) has discontinued the bDMARD prior to receiving the first dose of the JAK1 inhibitor for:

$\geq$ 4 weeks for etanercept;

$\geq$ 8 weeks for adalimumab, infliximab, certolizumab, golimumab, abatacept, tocilizumab, and ixekizumab;

$\geq$ 12 weeks for ustekinumab;

$\geq$ 16 weeks for secukinumab;

$\geq$ 1 year for rituximab or $\geq$ 6 months if B cells have returned to pre-treatment level or normal reference range (central lab) if pre-treatment levels are not available; or

$\geq$ 12 weeks or at least 5 times the mean terminal elimination half-life, whichever is longer, for other bDMARDs.

In one aspect, the subject (or subjects in the treated population) is bDMARD-IR a baseline, and further has had i) an inadequate response or intolerance to at least two NSAIDs (*e.g.*, over at least a four week period at the maximum recommended or tolerated doses); ii) intolerance to NSAIDs; and/or iii) contraindication for NSAIDs. In one aspect, the subject (or population of subjects) is bDMARD-IR, and has had an inadequate response to at least two NSAIDS or intolerance to and/or contraindication for NSAIDs. Examples of NSAIDs include, but are not limited to, traditional NSAIDs (*e.g.*, ibuprofen) and salicylates (*e.g.*, aspirin).

In certain aspects, the subject (or subjects in the treated population) to be treated is bDMARD-IR at baseline, has had an inadequate response or intolerance to at least two NSAIDS (as described above), and/or an intolerance to NSAIDs and/or contraindication for NSAIDS, and is further receiving at least one additional therapy. Additional therapies include, but are not limited to concomitant administration of non-biologic DMARDs, NSAIDs, corticosteroids, and combinations thereof. Suitable additional therapies for use in combination with the methods described herein include:

1) Concomitant administration of non-biologic DMARDs, including methotrexate (MTX), leflunomide, sulfasalazine (SSZ), hydroxychloroquine, chloroquine, and/or apremilast. In one embodiment, the subject is on a stable dose of MTX ($\leq$ 25 mg/week), SSZ ($\leq$ 3 g/day), hydroxychloroquine ($\leq$ 400 mg/day), chloroquine ($\leq$ 400 mg/day); leflunomide ($\leq$ 20 mg/day), or apremilast ($\leq$ 60 mg/day)), for at least 28 days prior to baseline. In some embodiments, a combination of up to two background non-biologic DMARDs is allowed, except the combination of MTX and leflunomide.

2) Concomitant administration of oral corticosteroids. In one embodiment, the subject is on a stable dose of prednisone ($<$ 10 mg/day), or oral corticosteroid equivalents, for at least 14 days prior to baseline.

3) Concomitant administration of NSAIDs, tramadol, a combination of acetaminophen/paracetamol and codeine or combination of acetaminophen/paracetamol and hydrocodone, and/or non-opioid analgesics. In one embodiment, the subject is on stable dose(s) for at least 14 days prior to baseline.

In one embodiment, the subject (or subjects in the treated population) is bDMARD-IR at baseline, has not been exposed to any JAK inhibitor, and has not had any of the following treatments/conditions within the specified time frame prior to baseline:

1) Intra-articular joint injections, spinal/paraspinal injection(s), or parenteral administration of corticosteroids within 28 days prior to baseline (not including inhaled or topical corticosteroids);

2) Any other non-biologic DMARDs (other than those mentioned above for concomitant treatment), including thalidomide, within 28 days or 5 half-lives (whichever is longer) of the drug prior to baseline;

3) Opioid analgesics (except for combination of acetaminophen/paracetamol and codeine or combination of acetaminophen/paracetamol and hydrocodone) within 14 days prior to the Baseline Visit:

4) No live vaccine within 28 days (or longer if required locally) prior to the first dose of JAK1 inhibitor, or have expected need of live vaccination during treatment with the JAK1 inhibitor, including at least 30 days (or longer if required locally) after the last dose of the JAK1 inhibitor;

5) No systemic use of known strong cytochrome P450 3A (CYP3A) inhibitors during administration of the JAK1 inhibitor, or strong CYP3A inducers 30 days prior to administration of the JAK1 inhibitor through the end of treatment;

6) Herbal therapies or other traditional medicines with unknown effects on CYP3A during treatment;

7) Investigational drug of chemical or biologic nature within a minimum of 30 days or 5 half-lives of the drug (whichever is longer) prior to the first dose of the JAK1 inhibitor; or

8) History of an allergic reaction or significant sensitivity to constituents of the JAK1 inhibitor (and its excipients) and/or other products in the same class.

In one embodiment, the subject (or subjects in the treated population) is bDMARD naive or bDMARD-IR, and has not been previously exposed to any JAK inhibitor at baseline.

In one aspect of the methods of treating AS described herein, the subject (or subjects in the treated population) is bDMARD naive at baseline, and achieves an ASAS40 response within 14 weeks of administration of the first dose (including at week 14). In another embodiment, the subject (or subjects in the treated population) is bDMARD naive at baseline, and achieves an ASAS40 response within 2 weeks of administration of the first dose (including at week 2). In one embodiment, the subject (or subjects in the treated population) is bDMARD naive at baseline, and achieves an ASAS40 response within 2 weeks of administration of the first dose (including at week 2) and maintains the ASAS40 response until at least 14 weeks after administration of the first dose (*i.e.,* including until at least week 14). In one aspect, the subject (or subjects in the treated population) further achieves ASAS PR, ASDAS LDA, ASDAS ID, ASDAS MI, and/or ASDAS CII within 2 weeks of administration of the first dose (including at week 2). In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve the result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD naive at baseline, and achieves within 14 weeks of administration of the first dose (including at week 14) an improvement of ≥ 40% and absolute improvement of ≥ 2 units (on a scale of 0 to 10) from baseline in each of the following 4 (ASAS40) domains:

a) Patient Global Assessment of disease activity (PtGA) as assessed on a numeric rating scale (NRS 0-10);

b) Patient's Assessment of Total Back Pain (Total Back Pain score) as assessed on a numeric rating scale (NRS 0-10);

c) Bath Ankylosing Spondylitis Functional Index (BASFI); and

d) inflammation, as represented by the mean of Questions 5 and 6 of the Bath Ankylosing Spondylitis Disease Activity Index (BASDAI).

In one embodiment, the above described improvements are achieved within 2 weeks of administration of the first dose (including at week 2). In one embodiment, the improvements are achieved within 14 weeks of administration of the first dose (including at week 14). In certain embodiments, a statistically significant population of subjects in the treated population, and/or at least 10%, at least 15%, at least 20%, at least 25%, at least 30%, at least 35%, at least 40%, or at least 45% of the subjects in the treated population, achieve the result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD naive at baseline, and alternately or additionally achieves within 14 weeks of administration of the first dose (including at week 14) at least one result selected from the group consisting of: a) a change (improvement) from baseline in ASDAS (CRP); b) a change (improvement) from baseline in MRI SPARCC score for spine (MRI-Spine SPARCC); c) ASAS partial remission (PR); d) BASDAI50 response; e) a change (improvement) from baseline in BASFI; f) change from baseline in ASQoL; g) a change (improvement) from baseline in ASAS Health Index (HI); h) a change (improvement) from baseline in MASES (*i.e.,* for subjects with baseline enthesitis); i) a change (improvement) from baseline in BASMIlin (mobility); and j) a change (improvement) from baseline in WPAI-Axial SpA. In one embodiment, the subject achieves the result within 2 weeks of administration of the first dose (including at week 2). In one embodiment, the subject achieves the result within 14 weeks of administration of the first dose (including at week 14). In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD naive at baseline, and alternately or additionally achieves within 14 weeks of administration (including at week 14) of the first dose at least one result selected from the group consisting of: a) a change (improvement) from baseline in ASDAS; b) a change (improvement) from baseline in MRI SPARCC score for spine (MRI-Spine SPARCC); c) ASAS partial remission (PR); d) BASDAI50 response; and e) a change (improvement) from baseline in BASFI. In one embodiment, each of the results are achieved within 14 weeks of administration of the first dose (including at week 14). In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD naive at baseline, and alternately or additionally achieves within 14 weeks of administration of the first dose (including at week 14) at least one result selected from the group consisting of: k) ASAS 20 response; and 1) a change (improvement) from baseline in MRI SPARCC score for sacroiliac (SI) joints (MRI-SI joints SPARCC). In one embodiment, each of the results are achieved within 14 weeks of administration of the first dose (including at week 14). In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD naive at baseline, and alternately or additionally achieves at least one result selected from the group consisting of: m) ASAS20 response; n) ASAS40 response; o) ASAS PR; p) ASAS 5/6 response; q) ASDAS Inactive Disease (based on ASDAS (CRP) and ASDAS (ESR)); r) ASDAS Low Disease; s) ASDAS Major Improvement (based on ASDAS (CRP) and ASDAS (ESR)); t) ASDAS Clinically Important Improvement (based on ASDAS (CRP) and ASDAS(ESR); u) change (improvement) from baseline in ASAS HI; v) change (improvement) from baseline in ASDAS(CRP) and ASDAS (ESR); w) change (improvement) from baseline in ASQoL; x) change (improvement) from baseline in BASDAI; y) change (improvement) from baseline in BASFI; z) change (improvement) from baseline in BASMI$_{lin}$; aa) change (improvement) from baseline in C-reactive protein (CRP); bb) change (improvement) from baseline in FACIT-F; cc) change (improvement) from baseline in ISI; dd) change (improvement) from baseline in MASES (in subjects with baseline MASES >0); ee) change (improvement) from baseline in mASSS (with conventional radiograph); ff) change (improvement) from baseline in MRI SPARCC score of SI joints; gg) change (improvement) from baseline in MRI SPARCC score of spine; hh) change (improvement) from baseline in Patient's Assessment of Total Back Pain score (Total Back Pain score); ii) change (improvement) from baseline in Patient's Assessment of Nocturnal Back Pain (Nocturnal Back Pain); jj) change (improvement) from baseline in Patient's Global Assessment of Pain (Pt Pain); kk) change (improvement) from baseline in Physician's Global Assessment of Disease Activity (PGA-Disease Activity); ll) change (improvement) from baseline in inflammation, as represented by the change (improvement) from baseline in the mean of Questions5 and 6 of the BASDAI; mm) change (improvement) from baseline in the Patient's Assessment of Total Back Pain, as represented by a change (improvement) from baseline in question 2 of BASDAI; nn) change (improvement) from baseline in peripheral pain/swelling, as represented by a change (improvement) in baseline in question 3 of BASDAI; oo) change (improvement) from baseline in duration of morning stiffness, as represented by a change (improvement) in baseline in question 6 of BASDAI; pp) change (improvement) from baseline in Patient's Global Assessment of Disease Activity (PtGA); qq) change (improvement) from baseline in TJC68 and SJC66; rr) change (improvement) from baseline in WPAI-Axial SpA; ss) resolution (improvement) of dactylitis in subjects with baseline presence of dactylitis; and tt) change (improvement) from baseline in total dactylitis count in subjects with baseline presence of dactylitis. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD naive at baseline, and alternately or additionally achieves within 14 weeks of administration (including at week 14) of the first dose at least one result selected from the group consisting of: q) ASDAS Inactive Disease ; r) ASDAS Low Disease; s) ASDAS Major Improvement; and t) ASDAS Clinically Important Improvement. In one embodiment, each of the results are achieved within 14 weeks of administration of the first dose (including at week 14). In certain embodiments, for any of the aforementioned results achieved, a statistically significant population of subjects in the treated population and/or at least 10%, at least 15%, at least 20%, at least 25%, at least 30%, at least 35%, at least 40%, or at least 45% of the subjects in the treated population, achieved at least one result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD naive at baseline, and alternately or additionally achieves at least one result selected from the group consisting of: ASDAS Inactive Disease, ASDAS Moderate Disease, ASDAS Low Disease Activity (LDA), ASDAS High Disease, ASDAS Very High Disease, ASDAS Major Improvement, and ASDAS Clinically Important Improvement. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD-IR at baseline, and achieves an ASAS40 response within 14 weeks of administration of the first dose (including at week 14). In another embodiment, the subject is bDMARD-IR at baseline, and achieves an ASAS40 response within 2 weeks of administration of the first dose (including at week 2). In one embodiment, the subject achieves an ASAS40 response within 2 weeks of administration of the first dose (including at week 2) and maintains the ASAS40 response until at least 14 weeks after administration of the first dose (i.e., including until at least week 14). In one aspect, the subject (or population of subjects) further achieves ASAS PR, ASDAS LDA, ASDAS ID, ASDAS MI, and/or ASDAS CII within 2 weeks of administration of the first dose (including at week 2). In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieved the result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD-IR at baseline, and alternately or additionally achieves within 14 weeks of administration (including at week 14) of the first dose at least one result selected from the group consisting of: a) change (improvement) from baseline in ASDAS; b) change (improvement) from baseline in MRI SPARCC score for spine (MRI-Spine SPARCC); c) ASAS partial remission (PR); d) BASDAI 50 response; e) change (improvement) from baseline in BASFI; f) change (improvement) from baseline in ASQoL; g) change (improvement) from baseline in ASAS Health Index (HI); h) change (improvement) from baseline in MASES (enthesitis); and i) change (improvement) from baseline in BASMIlin (mobility). In one embodiment, the result is achieved within 14 weeks of administration of the first dose (including at week 14). In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD-IR at baseline, and alternately or additionally achieves at least one result selected from the group consisting of: j) ASAS 20 response; and k) change (improvement) from baseline in MRI SPARCC score for SI joints (MRI-SI joints SPARCC). In one embodiment, the result is achieved within 14 weeks of administration of the first dose (including at week 14). In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In certain embodiments, for any of the aforementioned results achieved, the result (or results) is maintained or improved after achieving the result (or results) by continuing to administer a daily dose of the JAK1 inhibitor.

In one aspect of the methods of treating AS described herein, the subject (or subjects in the treated population) is bDMARD-IR at baseline, and alternately or additionally achieves at least one result selected from the group consisting of: 1) ASAS20 response; m) ASAS40 response; n) ASAS PR; o) ASDAS Inactive Disease; p) ASDAS Low Disease; q) ASDAS Major Improvement; r) ASDAS Clinically Important Improvement; s) discontinuation of opioids among subjects with opioid use at baseline; t) change (improvement) from baseline in ASAS HI; u) change (improvement) from baseline in ASDAS; v) change (improvement) from baseline in ASQoL; w) change (improvement) from baseline in BASDAI and BASDAI Questions, including change (improvement) from baseline in mean of question 5 and 6 of the BASDAI; x) change (improvement) from baseline in BASFI; y) change (improvement) from baseline in $BASMI_{lin}$; z) change (improvement) from baseline in high sensitivity C-reactive protein (hsCRP); aa) change (improvement) from baseline in FACIT-F; bb) change (improvement) from baseline in EuroQoL-5D-5L (EQ-5D-5L); cc) change (improvement) from baseline in MASES; dd) change (improvement) from baseline in mSASSS (with conventional radiograph); ee) change (improvement) from baseline in MRI SPARCC score of SI joints; ff) change (improvement) from baseline in MRI SPARCC score of spine; gg) change (improvement) from baseline in Patient's Assessment of Total Back Pain score (Total Back Pain score); hh) change (improvement) from baseline in Patient's Assessment of Nocturnal Back Pain (Nocturnal Back Pain); ii) change (improvement) from baseline in Patient's Global Assessment of Pain (Pt Pain); jj) change (improvement) from baseline in Physician's Global Assessment of Disease Activity (PGA-Disease Activity); kk) change (improvement) from baseline in Patient's Global Assessment of Disease Activity (PtGA); ll) change (improvement) from baseline in SF-36; mm) change (improvement) from baseline in TJC68 and SJC66; nn) change (improvement) from baseline in WPAI-Axial SpA; oo) change (improvement) from baseline in Change of NSAID score; and pp) change (improvement) from baseline in Physical Activity Assessment. In one embodiment, the result is achieved within 14 weeks of administration of the first dose (including at week 14). In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In certain embodiments, for any of the aforementioned results achieved, the result (or results) is maintained or improved after achieving the result (or results) by continuing to administer a daily dose of the JAK1 inhibitor.

Methods of Treating Non-Radiographic Axial Spondyloarthritis (nr-axSpA)

Further provided are methods of treating non-radiographic axial spondyloarthritis (nr-axSpA). In one aspect, provided are methods of treating active nr-axSpA, comprising administering a dose of the JAK1 inhibitor to a subject in need thereof in certain amounts and/or at certain intervals. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 14 weeks. In one aspect, the JAK1 inhibitor is administered orally once a day for at least 52 weeks. In one aspect, the subject is bDMARD naive. In one aspect, the subject is bDMARD-IR.

Disease activity/severity for nr-axSpA may be measured using a variety of indexes, including those set forth above for the treatment of axSpA. In one particular aspect, provided is a method of treating nr-axSpA, including active nr-axSpA, comprising administering a dose of the JAK1 inhibitor to a subject in need thereof in certain amounts and/or at certain intervals as described herein, wherein the subject achieves an Assessment of SpondyloArthritis International Society 40 (ASAS40) response following administration of the JAK1 inhibitor. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 14 weeks. In one aspect, the JAK1 inhibitor is administered orally once a day for at least 52 weeks. In one aspect, the subject is bDMARD naive. In one aspect, the subject is bDMARD-IR.

In one aspect, the subject achieves an ASAS40 response within 14 weeks of administration of the first dose of the JAK1 inhibitor (including at week 14). In one aspect, the subject achieves an ASAS40 response within 14 weeks of administration of the first dose of the JAK1 inhibitor (including at week 14), and the response is maintained or improved after week 14 by continuing to administer a daily dose of the JAK1 inhibitor. In one aspect, the subject achieves an ASAS40 response within 52 weeks of administration of the first dose of the JAK1 inhibitor (including at week 52). In one aspect, the subject achieves an ASAS40 response within 52 weeks of administration of the first dose of the JAK1 inhibitor (including at week 52), and the response is maintained or improved after week 52 by continuing to administer a daily dose of the JAK1 inhibitor. In one aspect, the subject achieves an ASAS40 response within 2 weeks of administration of the first dose of the JAK1 inhibitor (including at week 2). In one aspect, the subject achieves an ASAS40 response within 2 weeks of administration of the first dose of the JAK1 inhibitor (including at week 2), and the response is maintained or improved after week 2 by continuing to administer a daily dose of the JAK1 inhibitor. In one aspect, the subject achieves an ASAS40 response within 2 weeks, within 4 weeks, within 8 weeks, within 12 weeks, within 14 weeks, within 18 weeks, within 24 weeks, within 32 weeks, within 40 weeks, and/or within 52 weeks of administration of the first dose of the JAK1 inhibitor (including at week 2, week 4, week 8, week 12, week 14, week 18, week 24, week 32, week 40, and/or week 52). In one embodiment, the subject achieves an ASAS 40 response within 2 weeks of administration of the first dose (including at week 2), and maintains the ASAS40 response until at least 14 weeks after administration of the first dose (*e.g.*, until at least week 14). In one embodiment, the subject achieves an ASAS 40 response within 2 weeks of administration of the first dose (including at week 2), and maintains the ASAS40 response until at least 52 weeks after administration of the first dose (*e.g.*, until at least week 52). In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 2 weeks, for at least 4 weeks, for at least 8 weeks, for at least 12 weeks, for at least 14 weeks, for at least 18 weeks, for at least 24 weeks, for at least 32 weeks, for at least 40 weeks, and/or for at least 52 weeks. In one embodiment, the JAK1 inhibitor is administered orally once a day for at least 14 weeks. In one aspect, the JAK1 inhibitor is administered orally once a day for at least 52 weeks.

In one aspect, provided is a method of treating nr-axSpA, including active nr-axSpA, in a population of subjects in need thereof, the method comprising administering a dose of the JAK1 inhibitor to the subjects in certain amounts and/or at certain intervals as described herein, wherein a portion of the subjects in the treated population (*e.g.*, a statistically significant population of subjects in the treated population, and/or at least 10%, at least 15%, at least 20%, at least 25%, at least 30%, at least 35%, at least 40%, or at least 45% of the subjects in the treated population) achieve an ASAS40 response following administration of the JAK1 inhibitor. In one aspect, subjects in the treated population achieve an ASAS40 response within 14 weeks of administration of the first dose of the JAK1 inhibitor (including at week 14). In one aspect, subjects in the treated population achieve an ASAS40 response within 14 weeks of administration of the first dose of the JAK1 inhibitor (including at week 14), and the result is maintained or improved after week 14 by continuing to administer a daily dose of the JAK1 inhibitor. In one aspect, subjects in the treated population achieve an ASAS40 response within 52 weeks of administration of the first dose of the JAK1 inhibitor (including at week 52). In one aspectsubjects in the treated population achieve an ASAS40 response within 52 weeks of administration of the first dose of the JAK1 inhibitor (including at week 52), and the result is maintained or improved after week 52 by continuing to administer a daily dose of the JAK1 inhibitor. In one aspect, the JAK1 inhibitor is upadacitinib freebase. In one aspect, a dose of the JAK1 inhibitor is administered to the subjects in an amount sufficient to deliver 15 mg of upadacitinib freebase equivalent. In one aspect, the JAK1 inhibitor is administered to the subjects orally once a day for at least 14 weeks. In one aspect, the JAK1 inhibitor is administered to the subjects orally once a day for at least 52 weeks. In one aspect, the subjects in the population are bDMARD naive. In one aspect, the subjects in the population are bDMARD-IR.

Further provided are methods of reducing the signs and symptoms of nr-axSpA. In one aspect, provided is a method of reducing the signs and symptoms of active nr-axSpA, in particular methods comprising administering a dose of the JAK1 inhibitor to a subject in need thereof in certain amounts and/or at certain intervals as described herein. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered orally once a day for at least 14 weeks. In one aspect, the JAK1 inhibitor is administered orally once a day for at least 52 weeks. In one aspect, the subject is bDMARD naive. In one aspect, the subject is bDMARD-IR.

In one aspect of a method of reducing the signs and symptoms of nr-axSpA, including active nr-axSpA, the subject (or subjects in the treated population) achieves within 14 weeks of administration of the first dose of the JAK1 inhibitor (including at week 14), at least one result selected from the group consisting of: an ASAS40 response; a change (improvement) from baseline in ASDAS; a change (improvement) from baseline in MRI-Spine SPARCC; ASAS partial remission (PR); a BASDAI50 response; a change (improvement) from baseline in BASFI; a change (improvement) from baseline in ASQoL; a change (improvement) from baseline in ASAS Health Index (HI); a change (improvement) from baseline in MASES (enthesitis); and a change (improvement) from baseline in BASMIlin (mobility); or the subject achieves within 52 weeks of administration of the first dose of the JAK1 inhibitor (including at week 52) an ASAS40 response. In one aspect, the subject (or subjects in the treated population) achieves the result within 14 weeks of administration of the first dose of the JAK1 inhibitor (including at week 14), and the result is maintained or improved after week 14 by continuing to administer a daily dose of the JAK1 inhibitor. In one aspect, the subject (or subjects in the treated population) achieves the result within 52 weeks of administration of the first dose of the JAK1 inhibitor (including at week 52), and the result is maintained or improved after week 52 by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect, the subject (or population of subjects) in need of treatment has active nr-axSpA at baseline. In one aspect, the subject (or subjects in the treated population) fulfills the 2009 ASAS classification criteria for axSpA, but does not meet the radiologic criteria of the 1984 modified New York Criteria for AS at baseline. In one embodiment, the subject (or subjects in the treated population) meets at least one criteria at baseline selected from the group consisting of: (i) a BASDAI score ≥4; (ii) an ASDAS of ≥2.1; (iii) a Patient's Assessment of Total Back Pain (Total Back Pain score) of ≥4 (based on a 0-10 numerical rating scale); and (iv) an objective sign of inflammatory activity selected from the group consisting of: a) an objective sign of active inflammation on MRI of sacroiliac (SI) joints; and b) high-sensitivity C reactive protein (hsCRP) > upper limit of normal (ULN). In one aspect, the subject meets criteria (i), (ii), (iii), and (iv). In one aspect, the subject (or subjects in the treated population) is an adult subject. In another aspect, the subject (or subjects in the treated population) is a juvenile subject.

In one aspect, the subject (or subjects in the treated population) is bDMARD naive at baseline. Exemplary bDMARDs include, but are not limited to, a biologic tumor necrosis factor inhibitors (*e.g.*, adalimumab, etanercept) and interleukin IL)-17 inhibitors (*e.g.*, secukinumab, ixekizumab).

In another aspect, the subject (or subjects in the treated population) is bDMARD-IR at baseline. In one aspect, the subject (or subjects in the treated population) has had an inadequate response or intolerance to a bDMARD. Subjects who are bDMARD-IR include those subjects who have had prior exposure to one bDMARD (either 1 tumor necrosis factor (TNF) inhibitor (*e.g.*, adalimumab, etanercept) or 1 interleukin (IL)-17 inhibitor (*e.g.*, secukinumab, ixekizumab)), and have discontinued the bDMARD due to either intolerance or lack of efficacy (*e.g.*, as determined by a physician). In one embodiment, the subject (or subjects in the treated population) has not had prior exposure to a second bDMARD, if the reason for discontinuation was not due to lack of efficacy. In one embodiment, the subject (or subjects in the treated population) has not discontinued both a TNF inhibitor and an IL-17 inhibitor due to lack of efficacy. In certain embodiments, the subject (or subjects in the treated population) has discontinued the bDMARD prior to receiving the first dose of the JAK1 inhibitor for:

≥ 4 weeks for etanercept;
≥ 8 weeks for adalimumab, infliximab, certolizumab, golimumab, abatacept, tocilizumab, and ixekizumab;
≥ 12 weeks for ustekinumab;
≥ 16 weeks for secukinumab;
≥ 1 year for rituximab or ≥ 6 months if B cells have returned to pre-treatment level or normal reference range (central lab) if pre-treatment levels are not available; or
≥ 12 weeks or at least 5 times the mean terminal elimination half-life, whichever is longer, for other bDMARDs.

In one aspect, the subject (or subjects in the treated population) further has had i) an inadequate response or intolerance to at least two NSAIDs (*e.g.*, over at least a four week period at the maximum recommended or tolerated doses); ii) intolerance to NSAIDs; and/or iii) contraindication for NSAIDs at baseline. Examples of NSAIDs include, but are not limited to, traditional NSAIDs (*e.g.*, ibuprofen) and salicylates (*e.g.*, aspirin).

In certain aspects, the subject (or population of subjects) to be treated is further receiving at least one additional therapy. Additional therapies include, but are not limited to concomitant administration of non-biologic DMARDs, NSAIDs, corticosteroids, and combinations thereof. Suitable additional therapies for use in combination with the methods described herein include:

1) Concomitant administration of non-biologic DMARDs, including methotrexate (MTX), leflunomide, sulfasalazine (SSZ), hydroxychloroquine, chloroquine, or apremilast. In one embodiment, the subject is on a stable dose of MTX (≤ 25 mg/week), SSZ (≤ 3 g/day), hydroxychloroquine (≤ 400 mg/day), chloroquine (≤ 400 mg/day); leflunomide (≤ 20 mg/day), or apremilast (≤ 60 mg/day)), for at least 28 days prior to baseline. In some embodiments, a combination of up to two background non-biologic DMARDs is allowed, except the combination of MTX and leflunomide.
2) Concomitant administration of oral corticosteroids. In one embodiment, the subject is on a stable dose of prednisone (<_ 10 mg/day), or oral corticosteroid equivalents, for at least 14 days prior to baseline.
3) Concomitant administration of NSAIDs, tramadol, a combination of acetaminophen/paracetamol and codeine or combination of acetaminophen/paracetamol and hydrocodone, and/or non-opioid analgesics. In one embodiment, the subject is on stable dose(s) for at least 14 days prior to baseline.

In one embodiment, the subject (or subjects in the treated population) has not been previously exposed to any JAK inhibitor at baseline.

In one aspect of the methods of treating nr-axSpA described herein, the subject (or subjects in the treated population) alternately or additionally achieves within 14 weeks of administration of the first dose (including at week 14) at least one result selected from the group consisting of: a) a change (improvement) from baseline in ASDAS; b) a change (improvement) from baseline in MRI SPARCC score for SI joints (MRI-SI joints SPARCC); c) ASAS partial remission (PR); d) BASDAI50 response; e) a change (improvement) from baseline in BASFI; f) change (improvement) from baseline in ASQoL; g) a change (improvement) from baseline in ASAS Health Index (HI); h) a change (improvement) from baseline in MASES (enthesitis); and i) a change (improvement) from baseline in BASMIlin (mobility); In one embodiment, the subject achieves the result within 2 weeks of administration of the first dose (including at week 2). In one embodiment, the subject achieves each of the results within 14 weeks of administration of the first dose (including at week 14). In certain embodiments, for any of the aforementioned results achieved, the result (or results) is maintained or improved after week 2 or week 14 by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of the methods of treating nr-axSpA described herein, the subject (or subjects in the treated population) alternately or additionally achieves within 14 weeks of administration of the first dose (including at week 14) at least one result selected from the group consisting of: j) ASAS 20 response; and k) a change (improvement) from baseline in MRI SPARCC score for spine (MRI-Spine SPARCC). In one embodiment, each of the results is achieved within 14 weeks of administration of the first dose (including at week 14). In certain embodiments, for any of the aforementioned results achieved, the result (or results) is maintained or improved after week 14 by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of the methods of treating nr-axSpA described herein, the subject (or subjects in the treated population) alternately or additionally achieves at least one result selected from the group consisting of: 1) ASAS20 response; m) ASAS40 response; n) ASAS PR; o) ASDAS Inactive Disease; p) ASDAS Low Disease; q) ASDAS Major Improvement; r) ASDAS Clinically Important Improvement; s) discontinuation of opioids among subjects with opioid use at baseline; t) change (improvement) from baseline in ASAS HI; u) change (improvement) from baseline in ASDAS; v) change (improvement) from baseline in ASQoL; w) change (improvement) from baseline in BASDAI and BASDAI Questions, including change (improvement) from baseline in mean of questions 5 and 6 of the BASDAI; x) change (improvement) from baseline in BASFI; y) change (improvement) from baseline in $BASMI_{lin}$; z) change (improvement) from baseline in high sensitivity C-reactive protein (hsCRP); aa) change (improvement) from baseline in FACIT-F; bb) change (improvement) from baseline in EQ-5D-5L; cc) change (improvement) from baseline in MASES; dd) change (improvement) from baseline in mASSS (with conventional radiograph); ee) change (improvement) from baseline in MRI SPARCC score of SI joints; ff) change (improvement) from baseline in MRI SPARCC score of spine; gg) change (improvement) from baseline in Patient's Assessment of Total Back Pain score (Total Back Pain score); hh) change (improvement) from baseline in Patient's Assessment of Nocturnal Back Pain (Nocturnal Back Pain); ii) change (improvement) from baseline in Patient's Global Assessment of Pain (Pt Pain); jj) change (improvement) from baseline in Physician's Global Assessment of Disease Activity (PGA-Disease Activity); kk) change (improvement) from baseline in Patient's Global Assessment of Disease Activity (PtGA); ll) change (improvement) from baseline in SF-36; mm) change (improvement) from baseline in TJC68 and SJC66; nn) change (improvement) from baseline in (WPAI-Axial SpA); and oo) change (improvement) from baseline in Change of NSAID score. In certain embodiments, for any of the aforementioned results achieved, the result (or results) is maintained or improved after the result (or results) is achieved by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

Methods of Treating Psoriatic Arthritis (PsA) and Psoriasis (PsO)

Further provided are methods of treating psoriatic arthritis (PsA) and psoriasis (PsO), including PsO as a skin manifestation of PsA.

In one aspect, provide are methods of treating active PsA comprising administering a dose of the JAK1 inhibitor to a subject in need thereof in certain amounts and/or at certain intervals as described herein. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered in an amount sufficient to deliver 30 mg of upadacitinib freebase equivalent. In one aspect, the JAK1 inhibitor is administered orally once a day for at least 12 weeks. In one aspect, the subject is non-biologic DMARD-IR. In one aspect, the subject is bDMARD-IR. In one aspect, the subject is an adult.

Disease activity/severity for PsA may be measured using a variety of indexes, including the American College of Rheumatology response rates (e.g., ACR20, ACR50, ACR70); the Health Assessment Questionnaire - Disability Index (HAQ-DI); the Static Investigator Global Assessment of Psoriasis (sIGA); the Psoriasis Area Severity Index (PASI) (including PASI 75, PASI 90, and PASI 100); the Sharp/van der Heijde Score (SHS); Minimal Disease Activity (MDA) assessment; Leeds Enthesitis Index (LEI); Leeds Dactylitis Index (LDI); the 36-Item Short Form Health Survey (SF-36); the Patient's Global Assessment of Pain Numerical Rating Scale (NRS); the Functional Assessment of Chronic Illness Therapy-Fatigue (FACIT-F) Questionnaire; the Self-Assessment of Psoriasis Symptoms (SAPS) Questionnaire; tender joint count (TJC68); swollen joint count (SJC66); the Physician's Global Assessment of Disease Activity Numeric Rating Scale (PGA-Disease Activity NRS) (numerical rating scale); the Patient's Global Assessment of Disease Activity (PtGA); high-sensitivity C Reactive Protein levels (hsCRP); dactylitis count; resolution of dactylitis; resolution of enthesitis sites included in the LEI; Spondyloarthritis Research Consortium of Canada (SPARCC) Enthesitis Index; resolution of enthesitis sites included in the SPARCC Enthesitis Index; total enthesitis count; resolution of enthesitis; Body Surface Area with Psoriasis (BSA-PS); Modified Psoriatic Arthritis Response Criteria (PsARC); Disease Activity Score 28 (DAS28) (CRP); DAS28 (Erythrocyte Sedimentation Rate (ERS)); Psoriatic Arthritis Disease Activity Score (PASDAS); Disease Activity in Psoriatic Arthritis (DAPSA) score; EuroQoL-5D-5L (EQ-5D-5L) Questionnaire; Work Productivity and Activity Impairment Questionnaire-Psoriatic Arthritis (WPAI-PsA); Health Resource Utilization (HRU) Questionnaire; the Bath Ankylosing Spondylitis Disease Activity Index (BASDAI); BASDAI 50 response; Morning Stiffness (mean of BASDAI Questions 5 and 6); and Ankylosing Spondylitis Disease Activity Score (ASDAS). These indexes are described in detail in the Clinical Endpoint Definitions and Examples.

In one particular aspect, provided is a method of treating PsA, including active PsA, comprising administering a dose of the JAK1 inhibitor to a subject in need thereof in certain amounts and/or at certain intervals as described herein, wherein the subject achieves an American College of Rheumatology 20% (ACR20) response following administration of the JAK1 inhibitor. In one embodiment, the subject achieves an ACR 50% (ACR50) response following administration of the JAK1 inhibitor. In one embodiment, the subject achieves an ACR 70% (ACR70) response following administration of the JAK1 inhibitor. In one aspect, the subject achieves an ACR20, ACR50, or ACR70 response within 12 weeks of administration of the first dose of the JAK1 inhibitor (including at week 12). In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered in an amount sufficient to deliver 30 mg of upadacitinib freebase equivalent. In one aspect, the JAK1 inhibitor is administered orally once a day for at least 12 weeks. In one aspect, the subject is non-biologic DMARD-IR. In one aspect, the subject is bDMARD-IR.

In another aspect, provided is a method of treating PsA, including active PsA, in a population of subjects in need thereof, the method comprising administering a dose of the JAK1 inhibitor to the subjects in certain amounts and/or at certain intervals as described herein, wherein a portion of subjects in the treated population (e.g., a statistically significant population of subjects in the treated population, and/or at least 10%, at least 15%, at least 20%, at least 25%, at least 30%, at least 35%, at least 40%, or at least 45% of the subjects in the treated population) achieve an ACR20, ACR50, or ACR70 response following administration of the JAK1 inhibitor. In one aspect, subjects in the treated population achieve an ACR20, ACR50, or ACR70 response within 12 weeks of administration of the first dose of the JAK1 inhibitor (including at week 12). In one aspect, the JAK1 inhibitor is upadacitinib freebase. In one aspect, a dose of the JAK1 inhibitor is administered to the subjects in an amount sufficient to deliver 15 mg of upadacitinib freebase equivalent. In one aspect, a dose of the JAK1 inhibitor is administered to the subjects in an amount sufficient to deliver 30 mg of upadacitinib freebase equivalent. In one aspect, the JAK1 inhibitor is administered to the subjects orally once a day for at least 12 weeks.

In certain embodiments, the subject (or subjects in the treated population) achieve an ACR20 response within 12 weeks of administration of the first dose of the JAK1 inhibitor (including at week 12). In certain embodiments, the ACR20 response is maintained or improved after Week 12 by continuing to administer a daily dose of the JAK1 inhibitor. In certain embodiments, the subject (or subjects in the treated population) achieves an ACR20 response within 2 weeks of administration of the first dose (including at week 2). In certain embodiments, the subject (or subjects in the treated population) achieves an ACR20 response within 2 weeks of administration of the first dose of the JAK1 inhibitor (including at week 2), and the ACR20 response is maintained or improved after Week 2 by continuing to administer a daily dose of the JAK1 inhibitor. In certain embodiments, the subject (or subjects in the treated population) suffering from active PsA at baseline further achieve at least one result selected from the group consisting of: (a) change (improvement) from baseline in Health Assessment Questionnaire - Disability Index (HAQ-DI) within 12 weeks of administration of the first dose (including at week 12); (b) achieve Static Investigator Global Assessment (sIGA) of Psoriasis of 0 or 1 and at least a 2-point improvement from baseline at within 16 weeks of administration of the first dose (for subjects with baseline sIGA ≥ 2) (including at week 16); (c) achieve Psoriasis Area Severity Index (PASI) 75 response within 16 weeks of administration of the first dose (for subjects with ≥ 3% BSA psoriasis at baseline) (including at week 16); (d) change (improvement) from baseline in Sharp/van der Heijde Score (SHS) within 24 weeks of administration of the first dose (including at week 24); (e) achieve Minimal Disease Activity (MDA) within 24 weeks of administration of the first dose (including at week 24); (f) change (improvement) from baseline in Leeds Enthesitis Index (LEI) within 24 weeks of administration of the first dose, preferably wherein the change (improvement) is a resolution of enthesitis (LEI = 0) within 24 weeks of administration of the first dose (for subjects with baseline presence of enthesitis (LEI >0)) (including at week 24); (g) achieve ACR 20 response within 12 weeks of administration of the first dose (non-inferiority of upadacitinib vs adalimumab) (including at week 12); (h) change (improvement) from baseline in 36-Item Short Form Health Survey (SF-36) within 12 weeks of administration of the first dose (including at week 12); and (i) change (improvement) from baseline in Functional Assessment of Chronic Illness Therapy-Fatigue (FACIT-F) Questionnaire within 12 weeks of administration of the first dose (including at week 12). In certain embodiments, the subject (or subjects in the treated population) suffering from active PsA at baseline further achieve each result. In certain embodiments, the subject (or subjects in the treated population) suffering from active PsA at baseline further achieve at least one result selected from the group consisting of: (j) ACR 20 response and superiority over adalimumab (40 mg every other week) within 12 weeks of administration of the first dose (including at week 12); and (k) change (improvement) from baseline in Leeds Dactylitis Index (LDI) within 24 weeks of administration of the first dose, preferably wherein the change (improvement) is a resolution of dactylitis (LDI = 0) within 24 weeks of administration of the first dose (for subjects with baseline presence of dactylitis (LDI >0)) (including at week 24). In certain embodiments, the subject (subjects in the treated population) suffering from active PsA at baseline further achieve ACR 20 response and superiority over adalimumab (40 mg every other week) within 12 weeks of administration of the first dose (including at week 12). In one aspect, the subject (or subjects in the treated population) are non-biologic DMARD-IR at baseline. In one aspect, the subject (or subjects in the treated population) are bDMARD-IR at baseline. In one aspect, the result is maintained or improved after week 12, week 16, and/or week 24 by continuing to administer a daily dose of the JAK1 inhibitor. In one aspect, the subject (or subjects in the treated population) further achieves a Psoriasis Area Severity Index (PASI) 75 response within 16 weeks of administration of the first dose (including at week 16). In one aspect, the subject (or subjects in the treated population) further achieves a PASI 90 response within 16 weeks of administration of the first dose (including at week 16). In one aspect, the subject (or subjects in the treated population) further achieves a PASI 100 response within 16 weeks of administration of the first dose (including at week 16). In one aspect the PASI response (e.g., the PASI 75, PASI 90, or PASI 100 response) is maintained or improved after week 16 by continuing to administer a daily dose of the JAK1 inhibitor. In certain embodiments, for any of the aforementioned results achieved, the result (or results) is maintained or improved after the result (or results) is achieved by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

Further provided are methods of reducing the signs and symptoms of PsA in a subject in need thereof. In one aspect, provided is a method of reducing the signs and symptoms of PsA, including active PsA, comprising administering a dose of the JAK1 inhibitor to a subject in need thereof in certain amounts and/or at certain intervals as described herein. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered in an amount sufficient to deliver 30 mg of upadacitinib freebase equivalent. In one aspect, the JAK1 inhibitor is administered orally once a day for at least 12 weeks. In one aspect, the subject is non-biologic DMARD-IR. In one aspect, the subject is bDMARD-IR.

In another aspect, provided is a method of treating PsA, including active PsA, in a subject in need thereof, comprising administering a dose of the JAK1 inhibitor to the subject in certain amounts and/or at certain intervals as described herein, wherein the subject achieves Minimal Disease Activity (MDA) following administration of the JAK1 inhibitor. In one aspect, the JAK1 inhibitor is upadacitinib freebase, or a pharmaceutically acceptable salt thereof. In one aspect, the JAK1 inhibitor is administered in an amount sufficient to deliver 15 mg of upadacitinib freebase equivalent. In one aspect, the JAK1 inhibitor is administered in an amount sufficient to deliver 30 mg of upadacitinib freebase equivalent. In one aspect, the JAK1 inhibitor is administered orally once a day for at least 24 weeks. In one aspect, the subject achieves MDA within 24 weeks of administration of the first dose of the JAK1 inhibitor (including at week 24). In one aspect, the subject achieves MDA within 24 weeks of administration of the first dose of the JAK1 inhibitor (including at week 24), and the MDA is maintained or improved after week 24 by continuing to administer a daily dose of the JAK1 inhibitor. In one aspect, the subject further achieves a Psoriasis Area Severity Index (PASI) response selected from a PASI 75 response, a PASI 90 response, and a PASI 100 response, within 16 weeks of administration of the first dose. In one aspect, the PASI 75, PASI 90, and/or PASI 100 response is maintained or improved after Week 16 by continuing to administer the daily dose of the JAK1 inhibitor. In one aspect, the subject is non-biologic DMARD-IR. In one aspect, the subject is bDMARD-IR.

In another aspect, provided is a method of treating PsA, including active PsA, in a population of subjects in need thereof, the method comprising administering a dose of the JAK1 inhibitor to the subjects in certain amounts and/or at certain intervals as described herein, wherein a portion of subjects in the treated population (e.g., a statistically significant population of subjects in the treated population, and/or at least 10%, at least 15%, at least 20%, at least 25%, at least 30%, at least 35%, at least 40%, or at least 45% of the subjects in the treated population) achieve Minimal Disease Activity (MDA) following administration of the JAK1 inhibitor. In one aspect, the JAK1 inhibitor is upadacitinib freebase, or a pharmaceutically acceptable salt thereof. In one aspect, a dose of the JAK1 inhibitor is administered to the subjects in an amount sufficient to deliver 15 mg of upadacitinib freebase equivalent. In one aspect, a dose of the JAK1 inhibitor is administered to the subjects in an amount sufficient to deliver 30 mg of upadacitinib freebase equivalent. In one aspect, the JAK1 inhibitor is administered to the subjects orally once a day for at least 24 weeks. In one aspect, subjects in the treated population achieve MDA within 24 weeks of administration of the first dose of the JAK1 inhibitor (including at week 24). In one aspect, subjects in the treated population achieve MDA within 24 weeks of administration of the first dose of the JAK1 inhibitor (including at week 24), and the MDA is maintained or improved after week 24 by continuing to administer a daily dose of the JAK1 inhibitor. In one aspect, subjects in the treated population further achieves a Psoriasis Area Severity Index (PASI) response selected from a PASI 75 response, a PASI 90 response, and a PASI 100 response, within 16 weeks of administration of the first dose. In one aspect, the PASI 75, PASI 90, and/or PASI 100 response is maintained or improved after Week 16 by continuing to administer the daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result. In one aspect, the subjects in the treated population are non-biologic DMARD-IR at baseline. In one aspect, the subjects in the treated population are bDMARD-IR at baseline.

In one aspect, the subject (or subjects in the treated population) achieves an ACR20, ACR50, or ACR70 response within 12 weeks of administration (including at week 12) of the first dose of the JAK1 inhibitor. In one aspect, the subject (or subjects in the treated population) achieves an ACR20, ACR50, or ACR70 response within 12 weeks of administration (including at week 12) of the first dose of the JAK1 inhibitor, and the response is maintained or improved after week 12 by continuing to administer a daily dose of the JAK1 inhibitor. In one aspect, the subject (or subjects in the treated population) achieves an ACR20, ACR50, or ACR70 response within 2 weeks of administration of the first dose of the JAK1 inhibitor (including at week 2). In one aspect, the subject (or subjects in the treated population) achieves an ACR20, ACR50, or ACR70 response within 2 weeks of administration of the first dose of the JAK1 inhibitor (including at week 2), and the response is maintained or improved after week 12 by continuing to administer a daily dose of the JAK1 inhibitor. In one aspect, the subject (or subjects in the treated population) achieves an ACR20, ACR50, or ACR70 response within 2 weeks, within 4 weeks, within 8 weeks, within 12 weeks, within 16 weeks, within 20 weeks, within 24 weeks, within 28 weeks, within 32 weeks, within 36 weeks, within 44 weeks, and/or within 56 weeks of administration of the first dose of the JAK1 inhibitor (including at week 2, at week 4, at week 8, at week 12, at week 16, at week 20, at week 24, at week 28, at week 32, at week 36, at week 44, and/or at week 56). In one embodiment, the subject (or subjects in the treated population) achieves an ACR20, ACR50, or ACR70 response within 2 weeks of administration of the first dose (including at week 2), and maintains the ACR20, ACR50, or ACR70 response until at least 12 weeks after administration of the first dose (e.g., until at least week 12). In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered in an amount sufficient to deliver 30 mg of upadacitinib freebase equivalent. In one aspect, the JAK1 inhibitor is administered orally once a day for at least 2 weeks, for at least 4 weeks, for at least 8 weeks, for at least 12 weeks, at least 16 weeks, for at least 20 weeks, for at least 24 weeks, for at least 28 weeks, for at least 32 weeks, for at least 36 weeks, for at least 44 weeks, and/or for at least 56 weeks. In one embodiment, the JAK1 inhibitor is administered orally once a day for at least 12 weeks. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of a method of reducing the signs and symptoms of PsA, including active PsA, the subject (or subjects in the treated population) achieves at least one result selected from the group consisting of: an ACR20 response within 12 weeks of administration of the first dose of the JAK1 inhibitor (including at week 12); a change (improvement) from baseline in HAQ-DI within 12 weeks of administration of the first dose of the JAK1 inhibitor (including at week 12); a sIGA of 0 or 1 and at least a 2 point improvement from baseline within 16 weeks of administration of the first dose of the JAK1 inhibitor (including at week 16); PASI 75 response (for subjects with ≥3% Body Surface Area psoriasis at baseline) within 16 weeks of administration of the first dose of the JAK1 inhibitor (including at week 16); a change (improvement) from baseline in SHS within 24 weeks of administration of the first dose of the JAK1 inhibitor (including at week 24); Minimal Disease Activity within 24 weeks of administration of the first dose of the JAK1 inhibitor (including at week 24); a change (improvement) from baseline in LEI within 24 weeks of administration of the first dose of the JAK1 inhibitor (including at week 24); a change (improvement) from baseline in LDI within 24 weeks of administration of the first dose of the JAK1 inhibitor (including at week 24); a change (improvement) from baseline in SF-36 within 12 weeks of administration of the first dose of the JAK1 inhibitor (including at week 12); change (improvement) from baseline in Patient's Global Assessment of Pain (Pt Pain) (numerical rating scale) within 12 weeks of administration of the first dose of the JAK1 inhibitor (including at week 12); change (improvement) from baseline in FACIT-F within 12 weeks of administration of the first dose of the JAK1 inhibitor (including at week 12); change (improvement) from baseline in SAPS Questionnaire within 16 weeks of administration of the first dose of the JAK1 inhibitor (including at week 16); ACR50 or ACR70 response within 12 weeks of administration of the first dose of the JAK1 inhibitor (including at week 12); and ACR20 response within 2 weeks of administration of the first dose of the JAK1 inhibitor (including at week 2). In one aspect, the result is maintained or improved after week 2, week 12, week 16, or week 24 by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

Further provided are methods of inhibiting the progression of structural damage of PsA in a subject in need thereof. In one aspect, provided is a method of inhibiting the progression of structural damage of PsA, including active PsA, comprising administering a dose of the JAK1 inhibitor to a subject in need thereof in certain amounts and/or at certain intervals as described herein. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered in an amount sufficient to deliver 30 mg of upadacitinib freebase equivalent. In one aspect, the JAK1 inhibitor is administered orally once a day for at least 12 weeks. In one aspect, the subject is non-biologic DMARD-IR. In one aspect, the subject is bDMARD-IR.

Further provided are methods of improving physical function in a subject in need thereof. In one aspect, provided is a method of improving physical function of PsA, including active PsA, comprising administering a dose of the JAK1 inhibitor to a subject in need thereof in certain amounts and/or at certain intervals as described herein. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered in an amount sufficient to deliver 30 mg of upadacitinib freebase equivalent. In one aspect, the JAK1 inhibitor is administered orally once a day for at least 12 weeks. In one aspect, the subject is non-biologic DMARD-IR. In one aspect, the subject is bDMARD-IR.

Further provided are methods of preventing structural progression of PsA in a subject in need thereof. In one aspect, provided is a method of preventing structural progression of PsA, including active PsA, comprising administering a dose of the JAK1 inhibitor to a subject in need thereof in certain amounts and/or at certain intervals as described herein. In one aspect, the JAK1 inhibitor is upadacitinib freebase. In one aspect, the JAK1 inhibitor is administered in an amount sufficient to deliver 15 mg of upadacitinib freebase equivalent. In one aspect, the JAK1 inhibitor is administered in an amount sufficient to deliver 30 mg of upadacitinib freebase equivalent. In one aspect, the JAK1 inhibitor is administered orally once a day for at least 12 weeks. In one aspect, the subject is non-biologic DMARD-IR. In one aspect, the subject is bDMARD-IR.

In one aspect, the subject (or population of subjects) in need of treatment has active PsA at baseline. In one aspect, the subject (or population of subjects) in need of treatment and with active PsA at baseline further have moderately to severely active psoriatic arthritis at baseline, as determined by a physician. In one aspect, the subject (or subjects in the treated population) has a clinical diagnosis of PsA with symptoms onset at least six months prior to baseline. In one aspect, the subject (or subjects in the treated population) fulfills the Classification Criteria for PsA (CASPAR) criteria at baseline. In one embodiment, the subject (or subjects in the treated population) meets at least one criteria selected from the group consisting of: (i) >_3 tender joints (based on 68 joint counts); and (ii) >_3 swollen joints (based on 66 joint counts) at baseline. In one aspect the subject (or subjects in the treated population) has ≥3 tender joints (based on 68 joint counts); and (ii) >_3 swollen joints (based on 66 joint counts) at baseline. In certain embodiments, the subject (or subjects in the treated population) may have ≥ 5 tender joints (based on 68 joint counts) and ≥ 5 swollen joints (based on 66 joint counts) at baseline. In one aspect, the subject (or subjects in the treated population) meets at least one criteria selected from the group consisting of ≥ 1 erosion on x-ray as determined by central imaging review, and hs-CRP > laboratory defined upper limit of normal (ULN), at baseline. In one aspect, the subject (or subjects in the treated population) does not have Minimal Disease Activity (responders and non-responders) for PsA at baseline. In one aspect, the subject (or subjects in the treated population) has a diagnosis of active plaque psoriasis or a documented history of plaque psoriasis (*e.g.*, as determined by a physician) at baseline. In one aspect, the subject (or subjects in the treated population) is an adult subject. In another aspect, the subject (or subjects in the treated population) is a juvenile subject.

In one aspect, the subject (or subjects in the treated population) is non-biologic DMARD-IR at baseline. In one aspect, the subject (or subjects in the treated population) has had an inadequate response or intolerance to treatment with a non-biologic DMARD. Subjects who are non-biologic DMARD-IR include those subjects who have an intolerance to or contraindication for non-biologic DMARDs (*e.g.*, as determined by a physician), or who have had an inadequate response (*i.e.*, a lack of efficacy after a minimum 12 week duration of therapy) to previous or concomitant treatment with at least one non-biologic DMARD at maximally tolerated dose or up to a dose set forth as follows:

> i) methotrexate (MTX): ≤ 25 mg/week, or ≥ 15 to ≤ 25 mg/week, or ≥ 10 mg/week in subjects who are intolerant of MTX at doses ≥ 12.5 mg/week after complete titration;
> ii) sulfasalazine (SSZ): ≤ 3000 mg/day;
> iii) leflunomide (LEF): ≤ 20 mg/day;
> iv) apremilast: ≤ 60 mg/day;
> v) hydroxychloroquine (HCQ): ≤ 400 mg/day;
> vi) bucillamine: ≤ 300 mg/day; or
> vii) iguratimod: ≤ 50 mg/day)

for ≥ 12 weeks and at stable dose for ≥ 4 weeks prior to the baseline. In one aspect, the subject has not been exposed to any additional non-biologic DMARDs.

In certain aspects, the subject (or population of subjects) to be treated is non-biologic DMARD-IR and is further receiving at least one additional therapy. Suitable additional therapies include:

1) Stable doses of non-biologic DMARDs. In one aspect, the subject is receiving a concomitant DMARD treatment at baseline. In one aspect, the subject is on ≤ 2 non-biologic DMARDs (except the combination of MTX and leflunomide). Suitable doses for MTX, SSZ, LEF, apremilast, HCQ, bucillamine, and iguratimod are set forth above. In one aspect, the subject is not on any non-biologic DMARD other than MTX, SSZ, LEF, apremilast, HCQ, bucillamine, iguratimod, or the combination of MTX and LEF. In one embodiment, the subject is not receiving concomitant treatment of any non-biologic DMARD other than MTX, SSZ, leflunomide, apremilast, HCQ, bucillamine, or iguratimod.

2) Stable doses of NSAIDs, acetaminophen/paracetamol, low-potency opiates (tramadol or combination of acetaminophen and codeine or hydrocodone), oral corticosteroids (equivalent to prednisone ≤ 10 mg/day), or inhaled corticosteroids for stable medical conditions. In one embodiment, the subject has been receiving stable doses of such additional therapies for ≥ 1 week prior to baseline.

In one aspect, the subject (or population of subjects) to be treated is non-biologic DMARD-IR, and may discontinue treatment with at least one non-biologic DMARD prior to baseline in order to comply with the concomitant administration of non-biologic DMARD protocols set forth above (*e.g.*, concomitant administration of ≤ 2 non-biologic DMARDs (not including the combination of MTX and leflunomide), selected from MTX, SSZ, leflunomide, apremilast, HCQ, bucillamine, and iguratimod) at stable doses. In one embodiment, the subject has discontinued the treatment with the non-biologic DMARD prior to baseline for:

≥ 8 weeks for LEF if no elimination procedure was followed, or adheres to an elimination procedure (*i.e.,* 11 days with cholestyramine, or 30 days washout with activated charcoal or as per local label); or
≥ 4 weeks for all other non-biologic DMARDs.

In another aspect, the subject (or subjects in the treated population) is bDMARD-IR at baseline. In one aspect, the subject (or subjects in the treated population) has had an inadequate response or intolerance to a bDMARD. Subjects who are bDMARD-IR include those subjects who have had prior exposure to at least one bDMARD prior to baseline and have had an inadequate response due to lack of efficacy after a minimum 12 week duration of therapy, or who are intolerance to treatment with at least 1 bDMARD. In one aspect, the subject (or subjects in the treated population) has discontinued all bDMARDs prior to baseline. In certain embodiments, the subject (or subjects in the treated population) has discontinued the bDMARD prior to receiving the first dose of the JAK1 inhibitor for:

≥ 4 weeks for etanercept;
≥ 8 weeks for adalimumab, infliximab, certolizumab, golimumab, abatacept, tocilizumab, and ixekizumab;
≥ 12 weeks for ustekinumab;
≥ 16 weeks for secukinumab; or
≥ 1 year for rituximab or ≥ 6 months if B cells have returned to pre-treatment level or normal reference range (central lab) if pre-treatment levels are not available.

In certain aspects, the subject (or population of subjects) to be treated is bDMARD-IR, and is further receiving at least one additional therapy. Additional therapies include but are not limited to concomitant administration of non-biologic DMARDs and/or NSAIDs, corticosteroids, and combinations thereof. Suitable additional therapies for use in combination with the methods of described herein include:

1) Concomitant administration of ≤ 2 non-biologic DMARDs (not including the combination of MTX and leflunomide), selected from methotrexate (MTX), sulfasalazine (SSZ), leflunomide, apremilast, hydroxychloroquine (HCQ), bucillamine, and iguratimod. In one embodiment, the subject is on a stable dose of MTX (≤ 25 mg/week), SSZ (≤ 3000 mg/day), leflunomide (LEF) (≤ 20 mg/day), apremilast (≤ 60 mg/day), HCQ (≤ 400 mg/day), bucillamine (≤ 300 mg/day) or iguratimod (≤ 50 mg/day) for ≥ 12 weeks and for ≥ 4 weeks prior to the baseline. In one embodiment, the subject is not receiving concomitant treatment of any non-biologic DMARD other than MTX, SSZ, leflunomide, apremilast, HCQ, bucillamine, or iguratimod.

2) Concomitant administration of stable doses of NSAIDs, acetaminophen/paracetamol, low-potency opiates (tramadol or combination of acetaminophen and codeine or hydrocodone), oral corticosteroids (equivalent to prednisone ≤ 10 mg/day), or inhaled corticosteroids at a stable dose for ≥ 1 weeks prior to the baseline.

In one aspect, the subject (or population of subjects) to be treated is bDMARD-IR and has received previous treatment with at least one non-biologic DMARD. In one embodiment, such a subject may discontinue treatment with at least one non-biologic DMARD prior to baseline in order to comply with the concomitant administration of non-biologic DMARD protocols set forth above (*e.g.*, concomitant administration of ≤ 2 non-biologic DMARDs (not including the combination of MTX and leflunomide), selected from MTX, SSZ, leflunomide, apremilast, HCQ, bucillamine, and iguratimod) at stable doses. In one embodiment, the subject has discontinued the treatment with the non-biologic DMARD prior to baseline for:

≥ 8 weeks for LEF if no elimination procedure was followed, or adheres to an elimination procedure (*i.e.,* 11 days with cholestyramine, or 30 days washout with activated charcoal or as per local label); or
≥ 4 weeks for all other non-biologic DMARDs.

In one aspect, the subject (or subjects in the treated population) is bDMARD-IR or non-biologic DMARD-IR at baseline, and has discontinued all opiates (except for tramadol, or combination of acetaminophen and codeine or hydrocodone) at least 1 week prior to baseline. In one aspect, the subject (or subjects in the treated population) is bDMARD-IR or non-biologic DMARD-IR at baseline, and has not been previously exposed to any JAK inhibitor. In one aspect, the subject (or subjects in the treated population) is bDMARD-IR or non-biologic DMARD-IR at baseline, and is not receiving current treatment (*i.e.,* treatment at the time of administration of the JAK1 inhibitor) with >2 non-biologic DMARDs, or treatment with a DMARD other than MTX, SSZ, LEF, apremilast, HCQ, bucillamine or iguratimod, or use of MTX in combination with LEF at baseline. In one embodiment, the subject (or subjects in the treated population) is non-biologic DMARD-IR or bDMARD-IR, and does not have a history of fibromyalgia, any arthritis with onset prior to age 17 years, or a current diagnosis of inflammatory joint disease other than PsA (including, but not limited to, rheumatoid arthritis, gout, overlap connective tissue diseases, scleroderma, polymyositis, dermatomyositis, and systemic lupus erythermatosus).

In one aspect of the methods of treating PsA described herein, the subject (or subjects in the treated population) is non-biologic DMARD-IR at baseline, and alternately or additionally achieves at least one result selected from the group consisting of: a) change (improvement) from baseline in HAQ-DI within 12 weeks of administration of the first dose (including at week 12); b) sIGA of 0 or 1 and at least a 2-point improvement from baseline within 16 weeks of administration of the first dose (including at week 16); c) PASI 75 response within 16 weeks of administration of the first dose (including at week 16) (for subjects with ≥ 3% Body Surface Area (BSA) psoriasis at baseline); d) change (improvement) from baseline in SHS within 24 weeks of administration of the first dose (including at week 24); e) Minimal Disease Activity (MDA) within 24 weeks of administration of the first dose (including at week 24); f) change (improvement) from baseline in LEI within 24 weeks of administration of the first dose (including at week 24); g) change (improvement) from baseline in LDI within 24 weeks of administration of the first dose (including at week 24); h) change (improvement) from baseline in SF-36 within 12 weeks of administration of the first dose (including at week 12); i) change (improvement) from baseline in Patient's Global Assessment of Pain (Pt Pain) Numerical Rating Scale (NRS) within 12 weeks of administration of the first dose (including at week 12); j) change (improvement) from baseline in FACIT-F Questionnaire within 12 weeks of administration of the first dose (including at week 12); and k) change (improvement) from baseline in SAPS Questionnaire within 16 weeks of administration of the first dose (including at week 16). In one aspect, the result is maintained or improved after week 12, week 16, or week 24 by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of the methods of treating PsA described herein, the subject (or subjects in the treated population) is non-biologic DMARD-IR and may alternately or additionally achieve a result that is non-inferior or superior to the result achieved by administration of adalimumab. In one aspect, the result is superior or non-inferior to the result achieved when a subject is administered 40 mg doses of adalimumab every other week for 12 weeks. Examples of suitable formulations of adalimumab are described in Example 1. In one aspect, the subject achieves within 12 weeks of administration of the first dose (including at week 12) at least one result selected from the group consisting of: i) ACR20 that is non-inferior as compared to adalimumab; ii) ACR20 response that is superior as compared to adalimumab; change (improvement) from baseline in Patient's Global Assessment of Pain (Pt Pain) Numerical Rating Scale (NRS) that is superior as compared to adalimumab; iv) change (improvement) from baseline in HAQ-DI that is superior as compared to adalimumab. In one aspect, the result (or results) is maintained or improved after the result (or results) is achieved by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of the methods of treating PsA described herein, the subject (or subjects in the treated population) is non-biologic DMARD-IR at baseline, and alternately or additionally achieves least one result selected from the group consisting of: 1) ACR50 or ACR70 response rate within 12 weeks of administration of the first dose (including at week 12); and m) ACR20 response rate within 2 weeks of administration of the first dose (including at week 2). In one aspect, the result is maintained or improved after week 2 or week 12 by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of the methods of treating PsA described herein, the subject (or subjects in the treated population) is non-biologic DMARD-IR at baseline, and alternately or additionally achieves at least one result selected from the group consisting of: n) no radiographic progression, as demonstrated by change (improvement) in baseline in SHS of ≤0; o) change (improvement) from baseline in at least one individual components of ACR response selected from the group consisting of: (i) change (improvement) from baseline in Tender Joint Count (TJC68) (0 - 68); (ii) change (improvement) from baseline in Swollen Joint Count (SJC66) (0 - 66); (iii) change (improvement) from baseline in Physician Global Assessment-Disease Activity (PGA-Disease Activity) Numerical Rating Scale (NRS); (iv) change (improvement) from baseline in Patient's Global Assessment (PtGA)-Disease Activity Numerical Rating Scale (NRS); (v) change (improvement) from baseline in Patient's Global Assessment of Pain (Pt Pain) Numerical Rating Scale (NRS); (vi) change (improvement) from baseline in HAQ-DI; and (vii) change (improvement) from baseline in hs-CRP; p) ACR20, ACR50, or ACR70 response; q) change (improvement) from baseline in LDI; r) change (improvement) from baseline in dactylitis count; s) resolution of dactylitis; t) change (improvement) from baseline in LEI; u) resolution of enthesitis sites included in the LEI; v) change (improvement) from baseline in SPARCC Enthesitis Index; w) resolution of enthesitis sites included in the SPARCC Enthesitis Index; x) change (improvement) from baseline in total enthesitis count; y) resolution of enthesitis; z) PASI 75, PSA 90, or PSA 100 response in subjects with ≥ 3% Body Surface Area (BSA) psoriasis at baseline; aa) sIGA score of 0 or 1 and at least a 2-point improvement from baseline; bb) Body Surface Area with Psoriasis (BSA-PS); cc) change (improvement) from baseline in PsARC; dd) change (improvement) from baseline in DAS28 (CRP); ee) change (improvement) from baseline in DAS28 (ESR); ff) change (improvement) from baseline in PASDAS; gg) change (improvement) from baseline in DAPSA score; hh) change (improvement) from baseline in SF-36; ii) change (improvement) from baseline in FACIT-F Questionnaire; jj) change (improvement) from baseline in EQ-5D-5L Questionnaire; kk) change (improvement) from baseline in WPAI-PsA Questionnaire; ll) change (improvement) from baseline in HRU Questionnaire; mm) change (improvement) from baseline in SAPS Questionnaire; nn) change (improvement) from baseline in BASDAI; oo) BASDAI 50 response rate; pp) change (improvement) from baseline in Morning stiffness, measured as mean of BASDAI Questions 5 and 6; qq) change (improvement) from baseline in ASDAS; rr) ASDAS Inactive Disease; ss) ASDAS Major Improvement; tt) ASDAS Clinically Important Improvement; and uu) clinically meaningful improvement in HAQ-DI (>_ 0.35). In one aspect, the result (or results) is maintained or improved after achieving the result (or results) by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of the methods of treating PsA described herein, the subject (or subjects in the treated population) is bDMARD-IR at baseline, and alternately or additionally achieves least one result selected from the group consisting of: a) change (improvement) from baseline in HAQ-DI within 12 weeks of administration of the first dose (including at week 12); b) sIGA of Psoriasis of 0 or 1 and at least a 2-point improvement from baseline within 16 weeks of administration of the first dose (including at week 16); c) PASI 75 response within 16 weeks of administration of the first dose (including at week 16) for subjects with ≥ 3% Body Surface Area (BSA) psoriasis at baseline; d) Minimal Disease Activity (MDA) within 24 weeks of administration of the first dose (including at week 24); e) change (improvement) from baseline in SF-36 within 12 weeks of administration of the first dose (including at week 12); f) change (improvement) from baseline in FACIT-F Questionnaire within 12 weeks of administration of the first dose (including at week 12); and g) change (improvement) from baseline in SAPS Questionnaire within 16 weeks of administration of the first dose (including at week 16). In one aspect, the result is maintained or improved after week 12, week 16, or week 24 by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of the methods of treating PsA described herein, the subject (or subjects in the treated population) is bDMARD-IR at baseline, and alternately or additionally achieves at least one result selected from the group consisting of: h) ACR50 or ACR70 response within 12 weeks of administration of the first dose (including at week 12); and i) ACR20 response within 2 weeks of administration of the first dose (including at week 2). In one aspect, the result is maintained or improved after week 2 or week 12 by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In one aspect of the methods of treating PsA described herein, the subject (or subjects in the treated population) is bDMARD-IR at baseline, and alternately or additionally achieves at least one result selected from the group consisting of: j) change (improvement) from baseline in at least one individual component of an ACR response selected from the group consisting of: (i) change (improvement) from baseline in Tender Joint Count (TJC68) (0 - 68); (ii) change (improvement) from baseline in Swollen Joint Count (SJC66) (0 - 66); (iii) change (improvement) from baseline in Physician Global Assessment - Disease Activity (PGA-Disease Activity) Numerical Rating Scale (NRS); (iv) change (improvement) from baseline in Patient's Global Assessment (PtGA)-Disease Activity Numerical Rating Scale (NRS); (v) change (improvement) from baseline in Patient's Global Assessment of Pain (Pt Pain) Numerical Rating Scale (NRS); (vi) change (improvement) from baseline in Health Assessment Questionnaire - Disability Index (HAQ-DI); and (vii) change (improvement) from baseline in High-Sensitivity C Reactive Protein (hs-CRP); k) ACR20, ACR50, or ACR70 response rate; 1) change (improvement) from baseline in LDI; m) change (improvement) from baseline in dactylitis count; n) resolution of dactylitis; n) change (improvement) from baseline in LEI; o) resolution of enthesitis sites included in the LEI; p) change (improvement) from baseline in SPARCC Enthesitis Index; q) resolution of enthesitis sites included in the SPARCC Enthesitis Index; r) change (improvement) from baseline in total enthesitis count; s) resolution of enthesitis; t) PASI 75, PASI 90, or PASI 100 response in subjects with ≥ 3% Body Surface Area (BSA) psoriasis at baseline; u) sIGA score of 0 or 1 and at least a 2-point improvement from baseline; v) Body Surface Area with Psoriasis (BSA-PS); w) change (improvement) from baseline in PsARC; x) change (improvement) from baseline in DAS28 (CRP); y) change (improvement) from baseline in DAS28 (ESR); z) change (improvement) from baseline in PASDAS; aa) change (improvement) from baseline in DAPSA score; bb) change (improvement) from baseline in SF-36; cc) change (improvement) from baseline in FACIT-F Questionnaire; dd) change (improvement) from baseline in EQ-5D-5L Questionnaire; ee) change (improvement) from baseline in WPAI-PsA Questionnaire; ff) change (improvement) from baseline in HRU Questionnaire; gg) change (improvement) from baseline in SAPS Questionnaire; hh) change (improvement) from baseline in BASDAI; ii) BASDAI 50 response; jj) change (improvement) from baseline in Morning stiffness, measured as mean of BASDAI Questions 5 and 6; kk) change (improvement) from baseline in ASDAS; ll) ASDAS Inactive Disease; mm) ASDAS Major Improvement; nn) ASDAS Clinically Important Improvement; and oo) clinically meaningful improvement in HAQ-DI (>_ 0.35). In one aspect, the result (or results) is maintained or improved after achieving the result (or results) by continuing to administer a daily dose of the JAK1 inhibitor. In certain embodiments, for any of the aforementioned results achieved, a statistically significant population of subjects in the treated population, and/or at least 10%, at least 15%, at least 20%, at least 25%, at least 30%, at least 35%, at least 40%, or at least 45% of the subjects in the treated population, achieve at least one result.

In another aspect, provided is a method of treating active PsA in a subject in need thereof, the method comprising orally administering to the subject once a day for at least 16 weeks a dose of upadacitinib freebase, or a pharmaceutically acceptable salt thereof, in an amount sufficient to deliver 15 mg of upadacitinib freebase equivalent, wherein the subject achieves a Psoriasis Area Severity Index (PASI) 75 response within 16 weeks of administration of the first dose (including at week 16). In certain embodiments, the subject achieves a PASI 90 response within 16 weeks of administration of the first dose (including at week 16). In certain embodiments, the subject achieves a PASI 100 response within 16 weeks of administration of the first dose (including at week 16). In certain embodiments, the PASI response (e.g., the PASI 75, PASI 90, and/or PASI 100 response) is maintained or improved after week 16 by continuing to administer a daily dose of the upadacitinib freebase, or a pharmaceutically acceptable salt thereof. In one aspect, the subject is non-biologic DMARD-IR. In one aspect, the subject is bDMARD-IR.

In yet other aspects, provided is a method of treating active PsA in a subject in need thereof, the method comprising orally administering to the subject once a day for at least 16 weeks a dose of upadacitinib freebase, or a pharmaceutically acceptable salt thereof, in an amount sufficient to deliver 30 mg of upadacitinib freebase equivalent, wherein the subject achieves a Psoriasis Area Severity Index (PASI) 75 response within 16 weeks of administration of the first dose (including at week 16). In certain embodiments, the subject achieves a PASI 90 response within 16 weeks of administration of the first dose (including at week 16). In certain embodiments, the subject achieves a PASI 100 response within 16 weeks of administration of the first dose (including at week 16). In certain embodiments, the PASI response (e.g., the PASI 75, PASI 90, and/or PASI 100 response) is maintained or improved after week 16 by continuing to administer a daily dose of the upadacitinib freebase, or a pharmaceutically acceptable salt thereof. In one aspect, the subject is non-biologic DMARD-IR. In one aspect, the subject is bDMARD-IR.

In yet another aspect, provided is a method of treating active PsA in a population of subjects in need thereof, the method comprising administering a dose of upadacitinib freebase, or a pharmaceutically acceptable salt thereof, to the subjects according to the methods described herein, wherein a portion of the subjects in the treated population (e.g., a statistically significant population of the subjects in the treated population and/or at least 10%, at least 15%, at least 20%, at least 25%, at least 30%, at least 35%, at least 40%, or at least 45% of the subjects in the treated population) achieve a PASI 75 response within 16 weeks of administration of the first dose (including at week 16). In certain embodiments, subjects in the treated population achieve a PASI 90 response within 16 weeks of administration of the first dose (including at week 16). In certain embodiments, subjects in the treated population achieve a PASI 100 response within 16 weeks of administration of the first dose (including at week 16). In certain embodiments, the PASI response (e.g., the PASI 75, PASI 90, and/or PASI 100 response) is maintained or improved after week 16 by continuing to administer a daily dose of the JAK1 inhibitor. In one aspect, the upadacitinib freebase, or a pharmaceutically acceptable salt thereof, is administered in an amount sufficient to deliver 15 mg of upadacitinib freebase equivalent. In one aspect, the upadacitinib freebase, or a pharmaceutically acceptable salt thereof, is administered in an amount sufficient to deliver 30 mg of upadacitinib freebase equivalent. In one aspect, the upadacitinib freebase, or a pharmaceutically acceptable salt thereof, is administered orally once a day for at least 16 weeks. In one aspect, the subjects in the treated population are non-biologic DMARD-IR at baseline. In one aspect, the subject in the treated population are bDMARD-IR at baseline.

In certain embodiments, the subject (or subjects in the treated population) with active PsA at baseline is an adult subject. In another aspect, the subject (or subjects in the treated population) with active PsA at baseline is a juvenile subject. In certain embodiments, the subject (or subjects in the treated population) has >3% Body Surface Area with Psoriasis at baseline.

Further provided are methods of treating psoriasis (PsO), including PsO as a skin manifestation of PsA.

For example, in one aspect, provided is a method of treating active psoriasis in a subject in need thereof, the method comprising orally administering to the subject once a day for at least 16 weeks a dose of upadacitinib freebase, or a pharmaceutically acceptable salt thereof, in an amount sufficient to deliver 15 mg of upadacitinib freebase equivalent, wherein the subject achieves a Psoriasis Area Severity Index (PASI) 75 response within 16 weeks of administration of the first dose (including at week 16). In certain embodiments, the subject achieves a PASI 90 response within 16 weeks of administration of the first dose (including at week 16). In certain embodiments, the subject achieves a PASI 100 response within 16 weeks of administration of the first dose (including at week 16). In certain embodiments, the PASI response (e.g., PASI 75, PASI 90, or PASI 100 response) is maintained or improved after Week 16 by continuing to administer a daily dose of the upadacitinib freebase, or a pharmaceutically acceptable salt thereof.

In yet other aspects, provided is a method of treating active psoriasis in a subject in need thereof, the method comprising orally administering to the subject once a day for at least 16 weeks a dose of upadacitinib freebase, or a pharmaceutically acceptable salt thereof, in an amount sufficient to deliver 30 mg of upadacitinib freebase equivalent, wherein the subject achieves a Psoriasis Area Severity Index (PASI) 75 response within 16 weeks of administration of the first dose (including at week 16). In certain embodiments, the subject achieves a PASI 90 response within 16 weeks of administration of the first dose (including at week 16). In certain embodiments, the subject achieves a PASI 100 response within 16 weeks of administration of the first dose (including at week 16). In certain embodiments, the PASI response (e.g., PASI 75, PASI 90, or PASI 100 response) is maintained or improved after Week 16 by continuing to administer the daily dose of the upadacitinib freebase, or a pharmaceutically acceptable salt thereof.

In yet another aspect, provided is a method of treating active psoriasis in a population of subjects in need thereof, the method comprising administering a dose of upadacitinib freebase, or a pharmaceutically acceptable salt thereof, to the subjects according to the above methods, wherein a portion of the subjects in the treated population (e.g., a statistically significant population of the subjects in the treated population and/or at least 10%, at least 15%, at least 20%, at least 25%, at least 30%, at least 35%, at least 40%, or at least 45% of the subjects in the treated population) achieve a PASI 75 response within 16 weeks of administration of the first dose (including at week 16). In certain embodiments, subjects in the treated population achieve a PASI 90 response within 16 weeks of administration of the first dose (including at week 16). In certain embodiments, subjects in the treated population achieve a PASI 100 response within 16 weeks of administration of the first dose (including at week 16). In certain embodiments, the PASI response (e.g., PASI 75, PASI 90, or PASI 100 response) is maintained or improved after Week 16 by continuing to administer the daily dose of the upadacitinib freebase, or a pharmaceutically acceptable salt thereof. In one aspect, the upadacitinib freebase, or a pharmaceutically acceptable salt thereof, is administered in an amount sufficient to deliver 15 mg of upadacitinib freebase equivalent. In one aspect, the upadacitinib freebase, or a pharmaceutically acceptable salt thereof, is administered in an amount sufficient to deliver 30 mg of upadacitinib freebase equivalent. In one aspect, the upadacitinib freebase, or a pharmaceutically acceptable salt thereof, is administered orally once a day for at least 16 weeks. In one aspect, the subjects in the treated population are non-biologic DMARD-IR at baseline. In one aspect, the subject in the treated population are bDMARD-IR at baseline.

In certain embodiments, the subject (or subjects in the treated population) with active psoriasis at baseline is an adult subject. In another aspect, the subject (or subjects in the treated population) with active psoriasis at baseline is a juvenile subject. In certain embodiments, the subject (or subjects in the treated population) has ≥3% Body Surface Area with Psoriasis at baseline.

In certain embodiments, the active psoriasis is a skin manifestation of PsA. For example, in certain embodiments, the subject (or subjects in the treated population) in need thereof have active PsA.

Pharmaceutical Compositions

Pharmaceutical compositions comprising the JAK1 inhibitor, and which may be orally administered according to the methods described herein, may be formulated in a conventional manner using one or more pharmaceutically acceptable carriers.

Furthermore, the JAK1 inhibitor included in the composition may be a solid state form such, but not limited to, an Amorphous Freebase, a crystalline solvate or hydrate (e.g., Freebase Solvate Form A, Freebase Hydrate Form B), a crystalline hemihydrate (e.g., Freebase Hydrate Form C), a crystalline anhydrate (e.g., Freebase Anhydrate Form D), or a crystalline tartrate (e.g., Tartrate Hydrate), as described in WO 2017/066775 and WO 2018/165581.

In one embodiment, the pharmaceutical composition is a tablet dosage form. In one aspect, a film-coated tablet. In another embodiment, the pharmaceutical composition is a capsule dosage form.

In one embodiment, tablet or capsule is an extended-release formulation (also referred to herein as a modified release or sustained release formulation). Such formulations permit the sustained release of the active ingredient over an extended period of time, as compared to immediate release solid dosage forms, which permit the release of most or all of the active ingredient over a short period of time (e.g., typically around 60 minutes or less). In one aspect, the tablet comprises the JAK1 inhibitor and at least one additive selected from the group consisting of a release control polymer, a filler, a glidant, a lubricant (e.g., for use in compacting the granules), a pH modifier, a surfactant, and combinations thereof. In one aspect, the tablet comprises the JAK1 inhibitor, a release control polymer, a filler, a glidant, and a lubricant. In one aspect, the tablet comprises the JAK1 inhibitor, a release control polymer, a filler, a glidant, a lubricant, and a pH modifier. In some embodiments, the tablet further comprises a film coat. A film coat on the tablet further may contribute to the ease with which it can be swallowed. A film coat can also improve taste and provides an elegant appearance.

In certain embodiments, the release control polymer is a hydrophilic polymer. Examples of suitable release control polymers include, but are not limited, to a cellulose derivative with a viscosity of between 1000 and 150,000 mPA-s, hydroxypropylmethyl cellulose (e.g., Hypromellose 2208 or controlled release grades of hydroxypropylmethyl cellulose, including the E, F, and K series), copolymers of acrylic acid crosslinked with a polyalkenyl polyether (e.g., Carbopol® polymers), hydroxypropyl cellulose, hydroxyethyl cellulose, non-ionic homopolymers of ethylene oxide (e.g., Polyox™), water soluble natural gums of polysaccharides (e.g., xanthan gum, alginate, locust bean gum, etc.), crosslinked starch, polyvinyl acetates, polyvinylpyrrolidone, mixtures of polyvinyl acetates and polyvinyl pyrrolidone, and combinations thereof. In one embodiment, the release control polymer is selected from the group consisting of hydroxypropylmethyl cellulose, copolymers of acrylic acid crosslinked with a polyalkenyl polyether (e.g., Carbopol® polymers), and combinations thereof. Examples of suitable fillers ("bulking agents") include, but are not limited to, microcrystalline cellulose (e.g., Avicel® PH 101; Avicel® PH 102;), mannitol (e.g., Pearlitol® 100 SD or Pearlitol® 200 SD), lactose, sucrose, sorbitol, and the like. In one embodiment, the filler is selected from the group consisting of microcrystalline cellulose, mannitol, and combinations thereof. Examples of suitable glidants include, but are not limited to, silicone dioxide (e.g., colloidal silicon dioxide), calcium silicate, magnesium silicate, talc, and combinations thereof. In one embodiment, the glidant is colloidal silicone dioxide. Examples of suitable lubricants include, but are not limited to, polyethylene glycol (e.g., having a molecular weight of from 1000 to 6000), magnesium stearate, calcium stearate, sodium stearyl fumarate, talc, and the like. In one embodiment, the lubricant is magnesium stearate. Examples of suitable pH modifiers include, but are not limited to, organic acids, such as tartaric acid, citric acid, succinic acid, fumaric acid; sodium citrate; magnesium or calcium carbonate or bicarbonate; and combinations thereof. In one embodiment, the pH modifier is tartaric acid. Examples of suitable surfactants include sodium lauryl sulfate. Examples of suitable film-coats include, but are not limited to, a polymeric film-forming material such as hydroxypropyl methylcellulose, hydroxypropylcellulose, and acrylate or methacrylate copolymers. Besides a film-forming polymer, the film-coat may further comprise a plasticizer, e.g. polyethylene glycol, a surfactant, e.g. polysorbates, and optionally a pigment, e.g. titanium dioxide or iron oxides. The film-coating may also comprise talc as antiadhesive. In one embodiment, the film coat accounts for less than 5% by weight of a pharmaceutical composition.

In one embodiment, the pharmaceutical composition comprises from about 10 w/w% to about 35 w/w% of a pH modifier, and in particular, tartaric acid, fumaric acid, citric acid, succinic acid, malic acid, or combinations thereof. In other embodiments, the formulation comprises from about 20 w/w% to about 35 w/w%, or from about 20 w/w% to about 30 w/w%, or from about 20 w/w% to about 25 w/w%, or about 10 w/w%, about 15 w/w.%, about 20 w/w%, about 25 w/w% or about 30 w/w% pH modifier. In one embodiment, the pH modifier is tartaric acid.

In one embodiment, the tablet is a compressed and/or milled tablet. For example, in some embodiments, the tablet is formed by blending the composition components (e.g., including the active ingredient and at least one pharmaceutically acceptable carrier). The composition can then be either directly compressed, or one or more of the composition components can be granulated prior to compression. In one embodiment, milling is performed using a mill fitted with any suitable size screen (e.g., a fitted with a screen size of from about 600 to about 1400 μm or about 610 μm or about 1397 μm). Compression can be done in a tablet press, such as in a steel die between two moving punches.

In other embodiments, the compressed and/or milled tablet is formulated using a wet granulation process. Use of wet granulation helps reduce and/or eliminate sticking that may occur when compression without wet granulation (*e.g.*, direct compression) is used to formulate the tablets.

**EXEMPLIFICATION**

In order that this disclosure may be more fully understood, the following Examples are set forth. It should be understood that these examples are for illustrative purposes only and are not to be construed as limiting this disclosure in any manner.

**EXAMPLE 1**

Exemplary extended release upadacitinib formulations are disclosed in WO2017066775 and WO 2018/165581. The 15 mg or 30 mg upadacitinib Extended Release (ER) Wet Granulated Tablets used in the Examples as described herein are provided in the following table. The 15 mg or 30 mg upadactinib ER tablet (or matching placebo) is taken orally once daily, beginning on Day 1 (Baseline), and should be taken at approximately the same time each day, with or without food.

| Table A. Upadacitinib 15 and 30 mg Extended Release (ER) Tablets (Wet Granulated) | | | |
|---|---|---|---|
| Component | Function | 15 mg | 30 mg |
| **Tablet Core** | | | |
| **Intragranular** | | | |
| Upadacitinib* | Drug substance | 15.4 | 30.7 |
| Microcrystalline cellulose (≥65% through 75 um screen) (Avicel PH 102) | Filler | 41.2 | 82.4 |
| Hypromellose 2208 | Control release polymer and binder | 4.9 | 9.8 |
| Purified water | Wetting agent | N/A | N/A |
| **Extragranular** | | | |
| Microcrystalline cellulose (≥65% through 75 um screen) (Avicel PH 102) | Filler | 121.3 | 64.8 |
| Mannitol (Pearlitol 100 SD) | Filler | 100.6 | 100.6 |
| Tartaric acid powder | pH modifier | 96.0 | 96.0 |
| Hypromellose 2208 | Control release polymer | 91.1 | 86.2 |
| **Table A. Upadacitinib 15 and 30 mg Extended Release (ER) Tablets (Wet Granulated)** | | | |
| Component | Function | 15 mg | 30 mg |
| Colloidal Silicon Dioxide | Glidant | 2.4 | 2.4 |
| Magnesium Stearate | Lubricant | 7.2 | 7.2 |
| **Film Coat** | | | |
| OPADRY II Yellow (PVA, TiO$_2$, PEG3350, Talc, Iron Oxide Yellow) | Film coat | 14.40 | 14.40 |
| Purified water** | Processing Aid | N/A | N/A |
| *hemihydrate upadacitinib free base Form C as disclosed in WO2017066775 and WO 2018/165581 is used. 15 mg and 30 mg amount per tablet refers to the amount of anhydrous upadacitinib free base in the tablet; **removed during processing. | | | |

Adalimumab (ADA) (or matching placebo) is provided as a subcutaneous injection solution in 1 mL pre-filled syringes containing ADA 40 mg/0.8 mL (or matching placebo). ADA (or matching placebo) will be subcutaneously administered every other week at approximately the same time of day.

| Table B. | | |
|---|---|---|
| **Active Ingredient** | **Formulation** | **Strength** |
| Adalimumab (HUMIRA) | Adalimumab (Mannitol Matching Placebo), Citric acid monohydrate, Sodium citrate, Disodium phosphate dihydrate, Sodium dihydrogen phosphate dihydrate, Sodium chloride, Polysorbate 80, Water for injections, Sodium hydroxide added as necessary to adjust pH | 40 mg/ 0.8 mL |

## EXAMPLE 2

*Phase 2/3 Ankylosing Spondylitis (SELECT-AXIS 1) Clinical Study*

SELECT-AXIS 1 is a multicentre, randomised, double-blind, parallel-group, placebo-controlled, Phase 2/3, two-period study of upadacitinib (**FIG. 1**). 15 mg upadacitinib refers to the 15 mg once-daily (QD) upadacitinib drug product as described in Example 1.

Period 1 is the 14-week randomized, double-blind, parallel-group, placebo-controlled period designed to compare the safety and efficacy of upadacitinib free base 15 mg QD (once daily) versus placebo for the treatment of signs and symptoms of subjects with active AS who have had an inadequate response to at least two NSAIDs over an at least 4-week period in total at maximum recommended or tolerated doses or intolerance to or a contraindication for NSAIDs, and who are bDMARD-naïve. Period 2 is an open label long-term extension to evaluate the long-term safety, tolerability, and efficacy of upadacitinib free base 15 mg QD in subjects with AS who have completed Period 1.

X-rays of the pelvis were performed within the 35-day screening period to evaluate the SI joints to confirm the fulfillment of the modified New York Criteria for AS. X-rays of the spine were also performed within the 35-day screening period to assess for total spinal ankylosis; subjects with total spinal ankylosis were not eligible for this study. The x-rays of the spine and pelvis were not required during the Screening Period if the subject had a previous anteroposterior (AP) pelvis x-ray and lateral spine x-rays within 90 days of the Screening Period, provided that the x-rays are confirmed to be adequate for the required evaluations and are deemed acceptable by the central imaging vendor.

Subjects who met eligibility criteria were randomized in a 1:1 ratio to one of two treatment groups:

Group 1: Upadacitinib free base 15 mg QD, N = 85 (Day 1 to Week 14) → Upadacitinib free base 15 mg QD (Week 14 and thereafter)
Group 2: Placebo, N = 85 (Day 1 to Week 14) → Upadacitinib free base 15 mg QD (Week 14 and thereafter)

Starting at Week 16, subjects who did not achieve at least an ASAS 20 response at two consecutive visits had the option to add or modify doses of NSAIDs, acetaminophen/paracetamol, low potency opioid medications (tramadol or combination of acetaminophen and codeine or hydrocodone), and/or modify dose of MTX or SSZ at Week 20 or thereafter.

Starting at Week 24, subjects who still did not achieve at least an ASAS 20 response at two consecutive visits were discontinued from study drug treatment.

Subjects who completed the Week 14 visit (end of Period 1) entered the open-label long-term extension portion of the study, Period 2 (90 weeks). Subjects who were assigned to Upadacitinib in Period 1 continued to receive Upadacitinib free base 15 mg QD in an open-label manner. Subjects who were randomized to placebo at Baseline also received open-label upadacitinib free base 15 mg QD at Week 14.

### Main inclusion criteria:

1. Male or female ≥ 18 years of age.

2. Subject with a clinical diagnosis of AS and meeting the modified New York Criteria for AS.

3. Subject must have baseline disease activity as defined by having a Bath Ankylosing Spondylitis Disease Activity Index (BASDAI) score ≥ 4 and a Patient's Assessment of Total Back Pain score (Total Back Pain score) >_ 4 based on a 0 - 10 Numeric Rating Scale (NRS) at the Screening and Baseline Visits.

4. Subject has had an inadequate response to at least two NSAIDs over an at least 4-week period in total at maximum recommended or tolerated doses, or subject has an intolerance to or contraindication for NSAIDs.

5. If entering the study on concomitant methotrexate (MTX), leflunomide, sulfasalazine (SSZ), and/or hydroxychloroquine, subject must be on a stable dose of MTX (<_ 25 mg/week) and/or SSZ (<_ 3 g/day) and/or hydroxychloroquine (<_ 400 mg/day) or leflunomide (<_ 20 mg/day) for at least 28 days prior to the Baseline Visit. A combination of up to two background conventionalsynthetic disease modifying anti-rheumatic drugs (csDMARDs) is allowed EXCEPT the combination of MTX and leflunomide.

6. If entering the study on concomitant oral corticosteroids, subject must be on a stable dose of prednisone (<_ 10 mg/day), or oral corticosteroid equivalents, for at least 14 days prior to the Baseline Visit.

7. If entering the study on concomitant NSAIDs, tramadol, combination of acetaminophen and codeine or hydrocodone, and/or non-opioid analgesics, subject must be on stable dose(s) for at least 14 days prior to the Baseline Visit.

**Main Exclusion criteria:**

1. Patients with total spinal ankylosis were ineligible.
2. Prior exposure to any Janus kinase (JAK) inhibitor (including but not limited to tofacitinib, baricitinib, and filgotinib).
3. Prior exposure to any biologic therapy with a potential therapeutic impact on spondyloarthritis (SpA).
4. Intra-articular joint injections, spinal/paraspinal injection(s), or parenteral administration of corticosteroids within 28 days prior to the Baseline Visit. Inhaled or topical corticosteroids are allowed.
5. Subject on any other DMARDs (other than those allowed), thalidomide, or apremilast within 28 days or five half-lives (whichever is longer) of the drug prior to the Baseline Visit.
6. Subject on opioid analgesics (except for combination acetaminophen/codeine or acetaminophen/hydrocodone which are allowed) or use of inhaled marijuana within 14 days prior to the Baseline Visit.
7. Subject has a history of inflammatory arthritis of different etiology other than axial SpA (including but not limited to rheumatoid arthritis [RA], psoriatic arthritis [PsA], mixed connective tissue disease, systemic lupus erythematosus, reactive arthritis, scleroderma, polymyositis, dermatomyositis, fibromyalgia), or any arthritis with onset prior to 17 years of age.

**Primary Endpoint**

The primary efficacy endpoint is ASAS 40 response at Week 14.

**Key Secondary Endpoints**

The key multiplicity adjusted secondary efficacy endpoints at Week 14 are:

1. Change from Baseline in Ankylosing Spondylitis Disease Activity Score (ASDAS(CRP));
2. Change from Baseline in MRI Spondyloarthritis Research Consortium of Canada (SPARCC) score (Spine);
3. Proportion of subjects with BASDAI 50 response (defined as 50% improvement in the Bath AS Disease Activity Index);
4. Change from Baseline in AS quality of life (ASQoL);
5. Proportion of subjects with ASAS partial remission (PR) (defined as an absolute score of $\leq$ 2 units for each of the four domains identified in ASAS 40);
6. Change from Baseline in BASFI;
7. Change from Baseline in BASMI$_{lin}$;
8. Change from Baseline in Maastricht Ankylosing Spondylitis Enthesitis Score (MASES) *(i.e.,* for subjects with baseline enthesitis);
9. Change from Baseline in Work Productivity and Activity Impairment (WPAI) (the overall work impairment due to SpA);
10. Change from Baseline in ASAS HI.

Additional key secondary endpoints are:

1. ASAS 20 response at Week 14.
2. Change from Baseline in MRI SPARCC score (SI joints) at Week 14.

Additional endpoints are the following measurements assessed in subjects treated with upadacitinib versus placebo at scheduled time points other than those specified for the primary and key secondary variables:

1. Proportion of subjects with ASAS 20 response;
2. Proportion of subjects with ASAS 40 response;
3. Proportion of subjects with ASAS PR;
4. Proportion of subjects with ASAS 5/6 (20% improvement from Baseline in five out of the following six domains: BASFI, patient's assessment of total back pain, PtGA, inflammation [mean of Questions 5 and 6 of the BASDAI] lateral lumbar flexion from BASMI$_{lin}$, and high sensitivity CRP [hsCRP]);
5. Proportion of subjects with Inactive Disease based on ASDAS(CRP) and ASDAS(ESR) (ASDAS score < 1.3);
6. Proportion of subjects with Major Improvement based on ASDAS(CRP) and ASDAS(ESR) (a change from Baseline $\leq$ -2.0);
7. Proportion of subjects with Clinically Important Improvement based on ASDAS(CRP) and ASDAS(ESR) (a change from Baseline $\leq$ -1.1);
8. Proportion of subjects with Resolution of dactylitis (*i.e.,* for subjects with baseline presence of dactylitis);
9. Change from Baseline in ASAS HI;
10. Change from Baseline in ASDAS(CRP) and ASDAS(ESR) respectively;
11. Change from Baseline in AsQoL;
12. Change from Baseline in BASDAI;
13. Change from Baseline in BASFI;
14. Change from Baseline in BASMI$_{lin}$;
15. Change from Baseline in CRP;
16. Change from Baseline in Total dactylitis count (*i.e.,* for subjects with baseline presence of dactylitis);
17. Change from Baseline in FACIT-F;
18. Change from Baseline in ISI;
19. Change from Baseline in MASES (*i.e.,* for subjects with baseline MASES > 0);
20. Change from Baseline in Modified Stoke Ankylosing Spondylitis Spine Score (mSASSS) score with conventional radiograph;
21. Change from Baseline in MRI SPARCC score of SI joints;
22. Change from Baseline in MRI SPARCC score of Spine;
23. Change from Baseline in Patient's Assessment of Total Back Pain NRS score 0 - 10;
24. Change from Baseline in Patient's Assessment of Nocturnal Back Pain NRS score 0 - 10;
25. Change from Baseline in Patient's Global Assessment of Pain NRS score 0 - 10;
26. Change from Baseline in Physician's Global Assessment of Disease Activity NRS score 0 - 10;
27. Change from Baseline in Inflammation (mean of Questions 5 and 6 of BASDAI NRS scores 0 - 10);
28. Change from Baseline in Patient's assessment of total back pain (BASDAI Question 2 NRS score 0- 10);
29. Change from Baseline in Peripheral pain/swelling (BASDAI Question 3 NRS score 0 - 10);
30. Change from Baseline in Duration of morning stiffness (BASDAI Question 6 NRS score 0 - 10);
31. Change from Baseline in Patient's Global Assessment of Disease Activity NRS score 0 - 10;
32. Change from Baseline in TJC68 and SJC66;
33. Change from Baseline in WPAI (all 4 dimension scores);
34. Change from Baseline in Categories in ISI.

See, *e.g.,* Machado et al., Ann Rheum Dis (2018) 77: 1539-40; Maksymowych et al., Arthritis Rheum (2005) 53: 502-9.

## Analysis Windows

For each protocol-specified study visit, a target study day will be identified to represent the corresponding visit along with a window around the target day. Windows will be selected in a non-overlapping fashion so that a collection date does not fall into multiple visit windows. If a subject has two or more actual visits in one visit window, the visit closest to the target day will be used for analysis. If two visits are equidistant from the target day, then the later visit will be used for analysis.

**Statistical analyses**

The planned sample size of 170 for this study (with 1:1 randomisation ratio) was determined to provide ≥90% power for detecting a 26% difference in ASAS40 response rate (assuming a placebo ASAS40 response rate of 20%). Power and sample size calculations were performed at a two-sided significance level of 0·05 accounting for a 10% dropout rate. The full analysis set included all randomised patients who received at least one dose of study drug. The safety analysis set included all patients who received at least one dose of study drug. The SPARCC MRI assessment population was pre-specified in the statistical analysis plan (baseline included MRI data ≤3 days after first dose of study drug, and week 14 included MRI data up to first dose of Period 2 study drug; first dose in Period 2 was at week 14). A supplemental post hoc SPARCC MRI analysis was conducted to include all MRI data collected at nominal visits at baseline and week 14. Cumulative probability plots were generated to illustrate MRI SPARCC score changes on a patient level.

In the full analysis set, the primary endpoint was compared between the upadacitinib and the placebo group using the Cochran-Mantel-Haenszel test, adjusting for the stratification factor of screening hsCRP level. Non-responder imputation was used for handling missing data. Similar analyses as for the primary endpoint were conducted for secondary efficacy binary endpoints. For continuous secondary efficacy endpoints, comparisons between the upadacitinib and the placebo group were performed using mixed model for repeated measures with treatment group, visit, and treatment-by-visit interaction as fixed effects and the corresponding baseline value and the stratification factor of screening hsCRP level as the covariates. In order to preserve the overall type I error rate at $\alpha$=0.05 level, a step-down approach was used to test the primary and multiplicity controlled key secondary endpoints. The testing sequence includes a group of endpoints tested by the Hochberg procedure, including BASDAI50, ASQoL, ASAS PR, BASFI, BASMI, MASES, and WPAI (**FIG. 2**). See, *e.g.,* Hochberg Y, Tamhane AC. Multiple comparison procedures: John Wiley & Sons, Inc., 1987.

**Week 14 Results**

Between October 24, 2017, and September 10, 2018, 395 patients were assessed for eligibility, and 187 were enrolled into the study. Of the 395 patients screened, 208 (52.7%) did not meet eligibility criteria and were excluded from the study (main reason for screening failure was not meeting the radiographic criterion of the modified New York criteria for AS). The remaining 187 patients who met eligibility criteria were randomised to placebo (n=94) or upadacitinib (n=93). Overall, 95.2% of patients completed Period 1 through week 14 on study drug (placebo, 89/94 [94.7%]; upadacitinib, 89/93 [95.7%]); one patient in the placebo group discontinued study drug but completed Period 1 visits. The most common primary reason for study drug discontinuation by week 14 were adverse events in the placebo group (n=3 [3.2%]) and adverse events (n=2 [2.2%]) and withdrawal of consent (n=2 [2.2%]) in the upadacitinib group.

Mean age was 45.4 years, mean duration from onset of symptoms was 14.4 years, and mean duration since diagnosis was 6.9 years. Most patients were male (132 [70.6%]), were human leukocyte antigen (HLA) B27 positive (143 [76.5%]) and were receiving concomitant NSAIDs at baseline (150 [80.2%]). Baseline disease characteristics were generally balanced between the two groups. Key demographics and baseline characteristics of the patients are summarized in the below Table.

| Table C. Key demographics and baseline characteristics of patients | | |
|---|---|---|
| **Key Demographic and Baseline Characteristics Mean (SD) or n (%)** | **PLACEBO N=94** | **UPADACITINIB 15 MG QD N=93** |
| **Male** | 69 (73.4) | 63 (67.7) |
| **Age (Yrs)** | 44 (12.1) | 47 (12.8) |
| **HLA-B27 Positive** | 73 (77.7) | 70 (75.3) |
| **White** | 76 (80.9) | 79 (84.9) |
| **Region** | | |
| **North America** | 10 (10.6) | 9 (9.7) |
| **South/Central America** | 0 | 0 |
| **Western Europe** | 33 (35.1) | 30 (32.3) |
| **Eastern Europe** | 34 (36.2) | 36 (38.7) |
| **Asia[a]** | 14 (14.9) | 12 (12.9) |
| **Other[b]** | 3 (3.2) | 6 (6.5) |

(continued)

| Table C. Key demographics and baseline characteristics of patients | | |
|---|---|---|
| Duration of AS Diagnosis (Yrs) | 6.0 (6.79) | 7.8 (10.64) |
| Duration of AS Symptom (Yrs) | 14.0 (9.86) | 14.8 (11.64) |
| NSAID Use at Baseline | 80 (85.1) | 70 (75.3) |
| Prior NSAID Use | 94 (100) | 92 (98.9) |
| csDMARDs Use at Baseline | 17 (18.1) | 13 (14.0) |
| BASDAI | 6.5 (1.56) | 6.3 (1.76) |
| Total back pain (NRS 0-10) | 6.7 (1.78) | 6.8 (1.77) |
| Patient Global Assessment (NRS 0-10) | 6.8 (1.66) | 6.6 (1.81) |
| ASDAS(CRP) | 3.7 (0.74) | 3.5 (0.76) |
| BASFI (Function) | 5.5 (2.17) | 5.4 (2.36) |
| BASMI (Mobility) | 3.5 (1.48) | 3.7 (1.45) |
| Presence of Enthesitis (MASES > 0) | 55 (58.5) | 54 (58.1) |
| MASES Score[c] | 3.7 (2.71) | 3.9 (2.79) |
| MRI Spine SPARCC[d] | 11.9 (14.52) | 10.4 (14.36) |
| MRI Sacroiliac Joint SPARCC[d] | 5.4 (8.55) | 7.9 (10.91) |
| hsCRP at Screening (mg/L) | 11.7 (11.11) | 9.6 (12.57) |
| hsCRP >ULN at Screening | 68 (72.3) | 67 (72.0) |
| AS QoL | 10.3 (4.65) | 10.0 (5.27) |
| WPAI - Overall Work Impairment[e] | 53.3 (24.64) | 54.3 (28.10) |
| ASAS Health Index | 8.2 (3.84) | 8.6 (4.12) |
| a. South Korea and Japan.<br>b. New Zealand and Australia.<br>c. Summarized for subjects with presence of enthesitis at baseline.<br>d. Summarized for subjects whose baseline MRI data up to 3 days post first dose of study drug.<br>e. Summarized for subjects employed at baseline. | | |

The study met its primary endpoint, with statistically significantly more patients treated with upadacitinib versus placebo achieving ASAS40 response at week 14 (48/93 [51.6%] vs 24/94 [25.5%]; p=0 0003) with a treatment difference (95% CI) of 26.1% (12.6-39.5%) (**FIG. 3A**). A significant difference for upadacitinib versus placebo in ASAS40 (**FIG. 4A**) and the mean change for each of its four individual domains (**FIG. 4B-4E**) was observed as early as the first post-baseline visit (week 2), and this difference was maintained consistently through week 14, with week 14 achieving a statistically significant difference in the multiplicity-controlled analysis. Accounting for multiplicity adjustment, change from baseline to week 14 in ASDAS (CRP) (**FIG. 3C**), SPARCC MRI spine (**FIG. 3B**), and BASFI (**FIG. 3C**) and proportion of patients who achieved BASDAI50 (**FIG. 3A**) and ASAS PR (**FIG. 3A**) were statistically significant for upadacitinib versus placebo. Upadacitinib versus placebo mean (95% CI) change from baseline to week 14 was -1.45 (-1.62 to - 1.28) versus -0.54 (-0.71 to -0.37; treatment difference, -0.91 [-1.14 to -0.68; p<0.0001]) for ASDAS (CRP) and -2.29 (-2.73 to -1.85) versus -1.30 (-1.74 to -0.86; treatment difference, -1.00 [-1.60 to -0.39; p=0.0013]) for BASFI. BASDAI50 was achieved by 42/93 (45.2%) patients treated with upadacitinib versus 22/94 (23.4%) patients in the placebo group (treatment difference, 21.8% [8.5-35.0%; p=0.0016]) and ASAS PR was achieved by 18/93 (19.4%) patients in the upadacitinib group versus 1/94 (1.1%) in the placebo group (treatment difference, 18.3% [10.0-26.6%; p<0.0001]).

For the other multiplicity-controlled key efficacy endpoints, statistical significance based on multiplicity adjustment was not met per the Hochberg procedure. Consistent improvement was observed in patients receiving upadacitinib versus placebo with nominal p values <0.05 for MASES (p=0.0488), BASMI (p=0.0296), ASQoL (p=0.0156), and ASAS HI (p=0.0073) at week 14, except for WPAI (**FIG. 3C**).

The additional key secondary efficacy endpoints, ASAS20 and SPARCC MRI SI joint score, also improved with upadacitinib versus placebo based on nominal p values (**FIG. 3A and 3B**). ASAS20 was achieved by 60/93 (64.5%) patients treated with upadacitinib versus 38/94 (40.4%) of patients in the placebo group at week 14 (treatment difference, 24.1% [10.2-38.0%; p=0.0010]).

For the SPARCC MRI outcomes, change from baseline to week 14 in SPARCC MRI spine was -6.93 (-8.58 to -5.28) for upadacitinib versus -0.22 (-2.01 to 1.57) for placebo (treatment difference, -6.71 [-9.01 to -4.41; significant in multiplicity-controlled analysis, p<0.0001]) and change from baseline to week 14 in SPARCC MRI SI joint was -3.91 (-5.05 to -2.77) for upadacitinib versus -0.22 (-1.47 to -1.04) for placebo (treatment difference, -3.69 [-5.31 to -2.08; p<0.0001]; **FIG. 3B**). The supplemental MRI analysis conducted in all patients with available data confirmed the results of the primary SPARCC MRI analysis for both the spine and SI joints (**FIG. 5**). The cumulative probability plots of change in SPARCC scores demonstrated that SPARCC MRI spine and SI joint scores improved from baseline to week 14 to a greater extent in patients receiving upadacitinib compared with placebo; results for the primary MRI analyses (**FIG. 6A-6B**) and supplemental MRI analyses (**FIG. 6B-6C**) were consistent. Table D below summarizes the primary and key secondary efficacy endpoints at Week 14. Table E below provides additional efficacy measurements at Week 14.

| Table D. Primary and Key Secondary Efficacy Endpoints at Week 14[a] | | | |
|---|---|---|---|
| | **Endpoint** | **PLACEBO** <br> **N=94** | **UPADACITINIB** <br> **15 MG QD** <br> **N =93** |
| Primary | ASAS40 | 25.5% | 51.6% |
| Multiplicity Adjusted Key Secondary | ASDAS(CRP) | -0.54 | -1.45 |
| | MRI Spine SPARCC[b] | -0.22 | -6.93 |
| | BASDAI50 | 23.4% | 45.2% |
| | AS QoL | -2.7 | -4.2 |
| | ASAS Partial Remission | 1.1% | 19.4% |
| | BASFI (Function) | -1.30 | -2.29 |
| | BASMI (Mobility) | -0.1 | -0.4 |
| | MASES (Enthesitis)[c] | -1.4 | -2.3 |
| | WPAI - Overall Work Impairment[d] | -12.6 | -18.1 |
| | ASAS Health Index | -1.4 | -2.8 |
| Other Key Secondary | ASAS20 | 40.4% | 64.5% |
| | MRI SI Joints SPARCC[c] | -0.22 | -3.91 |
| a. Results for binary endpoints are based on NRI analysis. Analyses for all continuous endpoints are for the change from baseline value. Results for continuous endpoints are based on MMRM or ANCOVA analysis. | | | |
| b. Summarized for subjects whose baseline MRI data up to 3 days post first dose of study drug and week 14 data up to the first dose of period 2 study drug. | | | |
| c. Summarized for subjects with presence of enthesitis at baseline. | | | |
| d. Summarized for subjects employed at baseline. | | | |

The proportions of patients who achieved ASDAS LDA, ASDAS ID, ASDAS CII, and ASDAS MI were greater (nominal p<0.0001) for upadacitinib versus placebo at week 14 (**FIG. 7A**). These results are summarized in the below Table. Improvement in the mean ASDAS (**FIG. 7B**) and the individual ASDAS components (**FIG. 8A-8D**) was seen as early as week 2 with continued improvement up to week 14 with upadacitinib.

[a] post-hoc analysis

| Table E. Additional Efficacy Measurements at Week 14 | | |
|---|---|---|
| Endpoint | PLACEBO (N=94) | UPADACITINIB 15 MG QD (N=93) |
| ASDAS ID | 0% | 16.1% |
| ASDAS LDA[a] | 10.6% | 49.5% |
| ASDAS MI | 5.3% | 32.3% |
| ASDAS CII | 18.1% | 52.7% |

Patients treated with the upadacitinib 15 mg QD dose showed greater improvement in back pain as assessed by the Total Back Pain component of ASAS response compared to placebo at Week 14. Improvement in the overall level of neck, back, or hip pain was demonstrated using BASDAI Question 2. Improvements were also demonstrated for peripheral pain and swelling (assessed by BASDAI question 3 on overall pain in joints other than in the neck, back, or hips) and nocturnal back pain. Improvements in total and nocturnal back pain were observed as early as Week 2.

No serious infections, herpes zoster, malignancy, venous thromboembolic events, or deaths were reported in Period 1. The proportion of patients with adverse events was higher in the upadacitinib group 58/93 (62.4%) versus placebo group 52/94 (55.3%). One serious adverse event in each group was reported during Period 1: cardiovascular disorder /circulation dysregulation in the placebo group (patient was not feeling well and was hospitalised but no significant findings were obtained) and worsening of spinal osteoarthritis in the upadacitinib group in a patient with a history of spondylosis and disc protrusion in the cervical spine. The proportion of patients with adverse events leading to discontinuation of study drug (upadacitinib, 2/93 [2.2%]; placebo, 3/94 [3.2%]) and infections (upadacitinib, 19/93 [20.4%]; placebo, 26/94 [27.7%]) was similar for both treatment groups. The most common adverse event in the upadacitinib group was blood creatine phosphokinase (CPK) increased (8/93 [8.6%] patients vs 2/94 [2.1%] patients in the placebo group), with four events (vs one with placebo) assessed by the investigator to be possibly related to study drug; all patients were asymptomatic with elevations <4× ULN, except for one patient in the placebo group with muscle pain and an increase to 4.3× ULN. Most of these events were reversible without study drug interruption (6/8 with upadacitinib, 1/2 with placebo). One patient in the upadacitinib group who already had grade 2 neutropenia at baseline experienced a mild adverse event of grade 2 neutropenia.

Seven patients reported hepatic disorder adverse events (upadacitinib, 5/93 [5.4%]; placebo, 2/94 [2.1%]); none resulted in study drug discontinuation, and all were asymptomatic alanine aminotransferase or aspartate aminotransferase increases, with associated elevations <2× ULN in 6/7 and an elevation <3× ULN in the remaining patient. No differences in mean haemoglobin levels were observed throughout the 14-week period in either group.

Increases from baseline to week 14 in low-density lipoprotein cholesterol (0.318 mmol/L) and high-density lipoprotein (HDL) cholesterol (0.263 mmol/L) were observed in the upadacitinib group versus the placebo group (-0.083 and 0.010 mmol/L, respectively); however, no changes in the total cholesterol/HDL ratio were observed (upadacitinib, -0.071 mmol/L; placebo, - 0.083 mmol/L).

**Week 14 Results Discussion**

The SELECT-AXIS 1 is the first clinical trial of upadacitinib in AS and demonstrated consistent efficacy results supported by multiplicity-controlled endpoints. The study met its primary endpoint of ASAS40 response at week 14 (51.6% vs 25.5%) as well as several multiplicity-controlled secondary endpoints reflecting statistically significant improvement in disease activity (ASAS PR, BASDAI50, ASDAS), function (BASFI), and MRI outcomes (SPARCC MRI spine). The other multiplicity-controlled secondary endpoints did not meet significance in the multiplicity testing but demonstrated consistent improvements for ASQoL, BASMI, MASES, and ASAS HI, with upadacitinib versus placebo (nominal p<0.05), with the exception of WPAI.

A rapid onset of response to upadacitinib free base 15 mg QD treatment was observed for ASAS40 and ASDAS composite scores and their individual domains of disease activity (e.g., back pain, PtGA, morning stiffness, function, and serum markers of inflammation [hsCRP]), with responses observed as early as week 2 (first post-baseline visit) and consistently maintained through week 14. The results of upadacitinib on improving the signs and symptoms of AS are further confirmed by a significant reduction of active inflammation on MRI for both the spine and the SI joints.

In addition, outcomes related to clinically relevant treatment goals of remission or low disease activity, such as ASDAS ID or LDA, were also achieved, with 50% of patients reaching ASDAS LDA (difference vs placebo, 39%). See, e.g., Smolen et al., Ann Rheum Dis 2018; 77: 3-17. Of note, the placebo response rates for ASAS20 and ASAS40 in this study were similar to rates observed in recent clinical studies of AS; differences in ASAS40 response with upadacitinib versus placebo were p<0·05 (based on nominal p values) as early as week 2 and maintained throughout 14 weeks. See *e.g.*, van der Heijde et al., Ann Rheum Dis 2017; 76: 1340-47; van der Heijde et al., Lancet 2018; 392: 2378-87; van der Heijde et al., Lancet 2018; 392: 2441-51; Landewe et al., Ann Rheum Dis 2014; 73: 39-47. Interestingly, mean changes from baseline to week 14 in the MRI SPARCC scores for the spine and SI joints in the placebo group were quite small.

The study results are in line with findings from two Phase 2/3 JAK inhibitor studies in patients with active AS. See, *e.g.*, van der Heijde et al., Ann Rheum Dis 2017; 76: 1340-47; van der Heijde et al., Lancet 2018; 392: 2378-87. Together with these findings, the SELECT-AXIS 1 results further support that JAK inhibitors could represent an effective treatment option for AS. Currently, only TNF-α and IL-17 inhibition have been proven to be effective in axSpA; but, these cytokines are not directly blocked by JAK inhibitors including upadacitinib. See, *e.g.*, Furst and Louie, Arthritis Res Ther 2019; 21: 135. However, emerging data from upadacitinib RA studies suggest that selective inhibition of JAK1 may result in the secondary inhibition of additional pathways that do not depend on JAK1 signalling, such as TNF-α and IL-12. See, *e.g.*, Sornasse et al., Ann Rheum Dis 2019; 78: 365-66. Also, other JAK1-associated pathways, including IL-7 and IL-22, have been described in preclinical studies, but further research is needed to evaluate the mechanism of action of JAK inhibitors in axSpA. See, *e.g.*, Veale et al., Rheumatology (Oxford) 2019; 58: 197-205; Gracey et al., Ann Rheum Dis 2016; 75: 2124-32.

The proportion of patients with adverse events was generally similar in the upadacitinib and placebo groups, and no new safety findings were observed compared with previous upadacitinib phase 3 RA studies. See, *e.g.*, Burmester et al., Lancet 2018; 391: 2503-12; Genovese et al., Lancet 2018; 391: 2513-24; Fleischmann R, Pangan AL, Song I, et al. Upadacitinib versus placebo or adalimumab in patients with rheumatoid arthritis and an inadequate response to methotrexate: results of a phase 3, double-blind, randomized controlled trial. Arthritis Rheumatol 2019; doi: 10.1002/art.41032. [Epub ahead of print]; Cohen et al., Ann Rheum Dis (2019) 78. No serious infections, malignancies, anaemia, lymphopenia, herpes zoster, renal dysfunction, adjudicated major adverse cardiovascular events, venous thromboembolic events, or deaths were reported, and haemoglobin levels remained consistent throughout the study.

A higher proportion of patients in the upadacitinib group experienced adverse events of CPK elevation, all of which were asymptomatic and most were mild and reversible without study drug interruption. One patient in the placebo group experienced symptoms (muscle pain) in the setting of elevated CPK and permanently discontinued study drug. In the two previous JAK inhibitor studies, elevations in CPK were also observed. See *e.g.*, van der Heijde et al., Ann Rheum Dis 2017; 76: 1340-47; van der Heijde et al., Lancet 2018; 392: 2378-87. Additional data are needed to better understand the safety profile of upadacitinib in axSpA.

JAK inhibitors, such as upadacitinib, could help address the unmet need in axSpA treatment given that only approximately half of bDMARD-naive patients achieve an ASAS40 response and even less achieve remission with TNF or IL-17 inhibitor treatment. See, *e.g.*, Sieper et al., Ann Rheum Dis 2017; 76: 571-92; Deodhar et al., Arthritis Rheumatol 2019; 71: 599-611; van der Heijde et al., Lancet 2018; 392: 2441-51; Landewe et al., Ann Rheum Dis 2014; 73: 39-47; Lie E et al., Ann Rheum Dis 2011; 70: 157-63; Glintborg et al., Ann Rheum Dis 2013; 72: 1149-55. Furthermore, fewer patients are expected to achieve the treatment goal of sustained remission/LDA, and response rates are even lower in patients with AS who have not responded to bDMARD therapy. See, *e.g.*, Sieper et al., Lancet 2017; 390: 73-84; Sieper et al., Ann Rheum Dis 2017; 76: 571-92; Deodhar et al., Arthritis Rheumatol 2019; 71: 599-611. Furthermore, some patients with axSpA may not be eligible for or might have contraindications common to IL-17 and TNF inhibitor therapy, such as allergic reactions and injection site pain, or specific to TNF inhibitors, such as congestive heart failure and concomitant demyelinating disease. See, *e.g.*, Cortese et al., Mult Scler Relat Disord2019; 35: 193-95. The use of IL-17 inhibitors is also not recommended for patients with concomitant inflammatory bowel disease. See, *e.g.,* van der Heijde et al., Ann Rheum Dis 2017; 76: 978-91; Fragoulis et al., World J Gastroenterol 2019; 25: 2162-76. Because patients with AS are typically younger and may have more active lifestyles, a treatment option administered orally may be particularly important in this patient population. See *e.g.*, Alten et al., Patient Prefer Adherence 2016; 10: 2217-28. Considering these unmet needs, the findings of the SELECT-AXIS 1 study, which demonstrated that upadacitinib treatment effects are within the range observed with bDMARDs and other JAK inhibitors in AS, support further investigation of upadacitinib for AS. See, *e.g.*, Sieper et al., Ann Rheum Dis 2017; 76: 571-92; Deodhar et al., Arthritis Rheumatol 2019; 71: 599-611; van der Heijde et al., Ann Rheum Dis 2017; 76: 1340-47; van der Heijde et al., Lancet 2018; 392: 2378-87; van der Heijde et al., Lancet 2018; 392: 2441-51; Landewe et al., Ann Rheum Dis 2014; 73: 39-47.

This study is not without limitations. The focus on patients with AS who were bDMARD-naive allowed for a focused evaluation of benefit and risk in a homogeneous population, but the safety and efficacy of upadacitinib in patients with AS who are bDMARD-IR or in patients with non-radiographic axSpA has not yet been evaluated, and further studies are needed in these patient populations. Furthermore, only one dose of upadacitinib was evaluated in this study, and thus there are no data to confirm whether a higher dose could have resulted in greater efficacy. Lastly, only 14-week, short-term data are reported here, but the long-term efficacy and safety of upadacitinib will be collected in the ongoing SELECT-AXIS 1 extension period for up to 2 years.

In conclusion, oral upadacitinib free base 15 mg QD significantly improved disease activity, function, and MRI-detected axial inflammation in patients with active AS after 14 weeks of treatment. The incidence of adverse events was similar with upadacitinib and placebo, and no new safety signals were observed compared with previous studies in RA. Overall, these results support the further investigation of upadacitinib for the treatment of AS/axSpA.

**Year 1 Results**

The phase 2/3 SELECT-AXIS 1 study included a randomized, placebo-controlled, 14-week period followed by 90-week open-label extension; reported here are data through week 64.

The study enrolled adults (>_18 years) with active AS who had an inadequate response to ≥2 non-steroidal anti-inflammatory drugs therapy (or intolerance to or contraindication for NSAIDs) and were biologic disease-modifying antirheumatic drugs naive, and who met the modified New York criteria based on independent central reading of radiographs of the sacroiliac joints and who had active disease at baseline defined as Bath Ankylosing Spondylitis Disease Activity Index (BASDAI) score ≥4 and patient's assessment of back pain score ≥4 (numeric rating scale [NRS], 0-10) at screening and baseline visit. Patients receiving a stable dose of concomitant conventional synthetic disease-modifying antirheumatic drugs (DMARDs), oral glucocorticoids, NSAIDs and analgesics were eligible; patients with prior exposure to JAK inhibitors or biologic DMARDs with potential impact on spondyloarthritis were excluded. Of the 187 patients randomized to Period 1, 178 (continuous upadacitinib, n=89; placebo switch, n=89) completed week 14 on study drug and entered the open-label extension; 160 patients (continuous upadacitinib n=78 [83.9%]; placebo switch, n=82 [87.2%]) completed week 64. Lack of efficacy (n=10) and AEs (n=4) were the most common reasons for discontinuation of study drug between weeks 14 and 64. In the continuous upadacitinib and the placebo-to-upadacitinib switch groups, mean duration since AS symptom onset was 14.8 and 14.0 years, mean duration since diagnosis was 7.8 and 6.0 years, mean ASDAS was 3.5 and 3.7, and mean hsCRP levels were 9.6 and 11.4 mg/L, respectively. Concomitant medications included NSAIDs (76% and 86%), conventional synthetic DMARDs (14% and 18%), and glucocorticoids (6% and 13%, respectively).

Efficacy was assessed based on percentage of patients achieving ASAS20 response, ASAS40 response, ASAS partial remission, BASDAI50, and Ankylosing Spondylitis Disease Activity Score (ASDAS) inactive disease (ID; <1.3), low disease activity (LDA; <2.1), major improvement (MI; decrease from baseline ≥2.0), and clinically important improvement (CII; decrease from baseline ≥1.1) through 64 weeks. In addition, change from baseline in ASDAS based on C-reactive protein (ASDAS-CRP), Bath Ankylosing Spondylitis Functional Index (BASFI), and linear Bath Ankylosing Spondylitis Metrology Index (BASMI) through 64 weeks and Maastricht Ankylosing Spondylitis Enthesitis Score (MASES), Work Productivity and Activity Impairment (WPAI; on a scale of 0-100), ASAS Health Index (HI), and AS quality of life (ASQoL) through 52 were assessed.

ASAS20 and ASAS40 responses were defined as ≥20% or ≥40% improvement and an absolute improvement of ≥1 or ≥2 units (on an NRS scale of 0-10), respectively, from baseline in ≥3 of the following 4 domains (with no worsening of ≥20% and ≥1 unit or no worsening at all, respectively, in the remaining domain): Patient Global Assessment of disease activity (PtGA), patient assessment of back pain, BASFI, and inflammation defined as the mean of BASDAI questions 5 and 6 (severity and duration of morning stiffness). ASAS partial remission was defined as an absolute score of ≤2 units for each of the 4 domains identified for ASAS40 response. ASDAS-CRP consists of patient-reported outcomes about back pain (BASDAI item 2), peripheral pain/swelling (BASDAI item 3), duration of morning stiffness (BASDAI item 6), the PtGA, and CRP.

The percentage of patients achieving the primary efficacy endpoint of ASAS40 at week 14 continued to increase throughout the study in the continuous upadacitinib group: 85% (95% CI, 77%-93%) of patients achieved ASAS40 at week 64 in the as-observed analysis and 72% (63%-81%) in the NRI analysis (FIG. 3D). An analogous pattern of improvement was observed in the ASAS20 (94% [88%-99%] as-observed analysis and 80% [71%-88%] NRI analysis) (FIG. 3E), ASAS partial remission (46% [35%-57%] as-observed analysis and 40% [30%-50%] NRI analysis) (FIG. 3F), and BASDAI50 endpoints (82% [74%-91%] as-observed analysis and 70% [61%-79%] NRI analysis) (FIG. 3G). Patients who switched from placebo to upadacitinib at week 14 showed a speed of onset and magnitude of responses comparable with patients who were initially randomized to upadacitinib (responses at week 64 were ASAS40: 81% [72%-89%] as-observed analysis and 70% [61%-80%] NRI analysis; ASAS20: 96% [92%-100%] as-observed analysis and 83% [75%-91%] NRI analysis; ASAS partial remission: 39% [29%-50%] as-observed analysis and 34% [25%-44%] NRI analysis; and BASDAI50: 77% [68%-86%] as-observed analysis and 67% [58%-77%] NRI analysis). Likewise, the percentage of patients achieving ASDAS ID (**FIG. 3H**), ASDAS LDA (**FIG. 3I**), ASDAS MI (**FIG. 3J**), and ASDAS CII (**FIG. 3K**) continued to improve throughout the study in the continuous upadacitinib group; patients who switched to upadacitinib from placebo at week 14 showed a rapid onset of response for theses endpoints, with responses at week 64 similar to those observed in patients on continuous upadacitinib.

Mean changes from baseline to 1 year in disease activity (ASDAS), physical function (BASFI), patient assessment of pain and disease activity (PtGA), and inflammation (hsCRP) showed consistent improvement or sustained maintenance throughout the study in the continuous upadacitinib group; a similar magnitude of improvement was seen in the placebo-to-upadacitinib switch group after initiation of upadacitinib at week 14. Analogous patterns of improvement were shown in assessments of quality of life (ASQoL and ASAS HI), spinal mobility (BASMI), and enthesitis (MASES) over time, as well as in measurements of back pain, nocturnal back pain, BASDAI Q2 (back pain) and BASDAI Q5/6. Among patients who were employed at baseline, the mean (95% CI) WPAI overall work impairment score continued to improve throughout the study in the continuous upadacitinib group (from -20.5 [-27.1, -14.0] at week 14 to -35.6 [-43.2, -28.0] at week 52; as-observed analysis) and placebo-to-upadacitinib switch group (from -12.3 [-19.8, -4.8] at week 14 to -27.7 [-35.4, -20.0] at week 52).

A significantly higher proportion of patients receiving upadacitinib versus placebo achieved ≥30% and ≥50% reduction in Patient's Global Assessment (PGA) of pain and back pain as early as week 2, and ≥70% reduction as early as week 4, and efficacy achieved was sustained thereafter. See, e.g., **FIGS. 3L-3N.** Patients who switched from placebo to open-label upadacitinib at week 14 generally reached the same level of pain reduction after week 14 as those initially randomized to upadacitinib.

## Upadacitinib as a Promising Oral Therapy in AS and nr-AxSpA

SELECT-AXIS 1, the first study to report long-term data with a JAK inhibitor in AS, showed that upadacitinib 15 mg QD therapy led to sustained and consistent efficacy up to and including Week 64 in both NRI and as-observed analyses in patients with active AS who had an inadequate response to NSAIDs. Improvements were seen in disease activity measures (ASDAS, BASDAI, ASAS, and their components), inflammation (hsCRP), physical function (BASFI), quality of life (ASQoL, ASAS HI), and other aspects of disease (BASMI, MASES) with continuous upadacitinib therapy. In patients who switched from placebo to upadacitinib at week 14, a similar speed of onset and magnitude of efficacy response was observed up to and including Week 64 compared with those who received continuous upadacitinib starting at Week 0. Of note, approximately 40%-45% of patients receiving upadacitinib reached remission based on the more difficult to achieve endpoints ASAS partial remission (PR) or ASDAS inactive disease (ID) up to and including Week 64, and >80% were in a state of ASDAS low disease activity (LDA).

The below Table provides placebo corrected data for upadacitinib at Week 14, biologics Ixekizumab and Adalimumab at Week 16, and JAK small molecule inhibitors Tofacitinib and Filgotinib at Week 12 for key primary and secondary endpoints. While this data is not a head to head comparison, the placebo corrected response calculated for upadacitinib for the more difficult to achieve endpoints ASAS PR, ASDAS ID, and ASDAS LDA shows decided promise over the efficacy demonstrated by the other two JAK small molecule inhibitors, with a remarkable efficacy only comparable to that demonstrated with the biologics. Furthermore, this efficacy, once achieved at Week 14, was sustained or improved over time, with long term efficacy in these difficult to achieve endpoints (including ASDAS major improvement (MI) and ASDAS clinically important improvement (CII)), sustained or improved up to and including Week 64. Coupled with the fact that upadacitinib is well tolerated with no new or unexpected safety findings (particularly compared to the other JAK inhibitors), the data suggests upadacitinib will be a promising new safe oral therapy for AS patients, especially for those AS patients patients who have active disease and inadequate response to NSAIDs.

| Table F. Placebo Corrected Responses (% response / placebo response, p value) | | | | | |
|---|---|---|---|---|---|
| | **Upadacitinib** | **Ixekizumab vs. Adalimumab H2H Study** | | **Tofacitinib** | **Filgotinib** |
| **Endpoint** | **15 mg QD** Week 14 (naiive) | **Ixekizumab Q4W** Week 16 (TNF naiive) | **ADA 40 mg EOW** Week 16 (TNF naiive) | **5mg BID Week 12 (bDMARD-naiive)** | **200mg QD Week 12 (Mixed)** |
| ASAS20 | **24.1%** (64.5% / 40.4%, p<0.001) | **24%** (64%/40%, p=0.0015) | **19%** (59%/ 40%, p=0.0075) | **39.6%** (80.8%/ 41.2%, p≤0.001) | **36.2%** (75.9%/ 39.7%, p<0.0001) |
| ASAS40 | **26.1%** (51.6%/ 25.5%, p<0.001*) | **30%** (48%/ 18%, p<0.0001,) | **18%** (36%/ 18%, p=0.0053) | **26.6%** (46.2%/ 19.6%, p≤0.01) | **18.9%** (37.9%/ 19%, p=0.0189) |
| BASDAI50 | **21.8%** (45.2%/ 23.4%, p=0.002*) | **25%** (42%/ 17%, p=0.0003) | **15%** (32%/ 17%, p=0.0119) | **18.8%** (42.3%/ 23.5%, p≤0.05) | NA |

(continued)

| Table F. Placebo Corrected Responses (% response / placebo response, p value) | | | | | |
|---|---|---|---|---|---|
| | **Upadacitinib** | **Ixekizumab vs. Adalimumab H2H Study** | | **Tofacitinib** | **Filgotinib** |
| **Endpoint** | **15 mg QD** Week 14 (naiive) | **Ixekizumab Q4W** Week 16 (TNF naiive) | **ADA 40 mg EOW** Week 16 (TNF naiive) | **5mg BID Week 12** (bDMARD-naiive) | **200mg QD Week 12** (Mixed) |
| ASAS Partial Remisson (PR) | **18.3%** (19.4%/ 1.1%, p<0.001*) | NA | NA | **7.4%** (19.2%/ 11.8%, NS) | **8.7%** (12.1%/ 3.4%, p=0.1028) |
| ASDAS Inactive Disease (ID) | **16.1%** (16.1%/ 0%, p<0.001) | **14%** (16%/ 2%, p=0.0074) | **14%** (16%/ 2%, p=0.0087) | **5.7%** (13.5%/ 7.8%, NS) | **5%** (5%/ 0%, p=0.092) |
| ASDAS low disease activity (LDA) | **38.9%** (49.5%/ 10.6%, p<0.001) | **30%** (43%/13%, p<0.0001) | **25%** (38%/ 13%, p=0.0002) | **34.3%** (53.9%/ 19.6%, p≤0.001) | NA |
| NS: non-significant; NA: not available; UPA p values are nominal, unless *significant after multiplicity-adjustment; Ixekizumab bDMARD-naïve AS COAST-V Study. Van der Heijde et al. Lancet 2018; 392: 2441-51; Tofacitinib study: van der Heijde D, et al. ARD 2017;0: 1-8.; Filgotinib study: van der Heijde et al. Lancet 2018; 392: 2378-87. | | | | | |

## EXAMPLE 3

*A Phase 3 Randomized, Placebo-Controlled, Double-Blind Program to Evaluate Efficacy and Safety of Upadacitinib in Adult Subjects with Axial Spondyloarthritis (SELECT AXIS 2)*

The safety and efficacy data from the Phase 2/3 study (as described in Examples 2) show a favorable benefit risk profile for upadacitinib and support the continued investigation of upadacitinib in adult subjects with active axSpA who had an inadequate response to biologic disease-modifying anti-rheumatic drug therapy (bDMARD-IR) (Study 1) and in adult subjects with active nr-axSpA (Study 2).
The Phase 3 clinical study plan is set forth in **FIG. 9.**

## Adult subjects with active ankylosing spondylitis (AS) who had an inadequate response to biologic disease-modifying anti-rheumatic drug therapy (bDMARD-IR) (Study 1)

To evaluate the safety, tolerability, and efficacy of upadacitinib compared with placebo on reduction of signs and symptoms in adult subjects with active ankylosing spondylitis (AS) who had an inadequate response to biologic disease-modifying anti-rheumatic drug therapy (bDMARD-IR).
Study 1 (main study) is comprised of a 35-day Screening Period; a 14-week randomized, double-blind, parallel-group, placebo-controlled period (the Double-Blind Period); a 90-week open-label, long-term extension period (the Open-Label Extension Period); and a 30-day Follow-Up Visit (F/U Visit). Group 1: upadacitinib free base 15 mg QD; Group 2: placebo QD. Subjects in the placebo group will be switched to upadacitinib free base 15 mg QD at Week 14 in the Open-Label Extension Period for Study 1.
Eligible subjects will be adult females and males who are at least 18 years of age at Screening with a clinical diagnosis of AS, meet the modified New York Criteria for AS, and are without total spinal ankylosis (Study 1, bDMARD-IR AS). Eligible study subjects must have a Bath Ankylosing Spondylitis Disease Activity Index score ≥ 4 and a Patient's Assessment of Total Back Pain score (Total Back Pain score) >_ 4 based on a 0 - 10 numerical rating scale at the Screening and Baseline Visits.
For Study 1 (bDMARD-IR AS), subjects with prior exposure to 1 biologic disease-modifying anti-rheumatic drug (bDMARD) (either 1 tumor necrosis factor [TNF] inhibitor or 1 interleukin [IL]-17 inhibitor) may be enrolled, and the subject must have discontinued the bDMARD due to either intolerance or lack of efficacy. Prior exposure to a 2nd bDMARD is allowed for no more than 30% of subjects if the reason for discontinuation was not due to lack of efficacy. Subjects who have had lack of efficacy to both a TNF inhibitor and IL-17 inhibitor are not eligible.

**Study 1 Primary endpoint**

The primary endpoint is the proportion of subjects achieving an ASAS40 response at Week 14. Secondary endpoints for Study 1 are described below.

**Adult subjects with active non-radiographic axial spondyloarthritis (nr-axSpA) (Study 2)**

To evaluate the safety, tolerability, and efficacy of upadacitinib compared with placebo on reduction of signs and symptoms in adult subjects with active non-radiographic axial spondyloarthritis (nr-axSpA).

Study 2 (nr-axSpA) is comprised of a 35-day Screening Period; a 52-week randomized, double-blind, parallel-group, placebo-controlled period (the Double-Blind Period); a 52-week open-label, long-term extension period (the Open-Label Extension Period); and a 30-day F/U Visit. Group 1: upadacitinib free base 15 mg QD; Group 2: placebo QD.

Subjects in the placebo group will be switched to upadacitinib free base 15 mg QD at Week 52 in the Open-Label Extension Period for Study 2 (nr-axSpA).

Eligible subjects will be adult females and males who are at least 18 years of age at Screening with a clinical diagnosis of nr-axSpA fulfilling the 2009 ASAS classification criteria for axSpA but not meeting the radiologic criterion of the modified New York criteria for AS and have objective signs of active inflammation on magnetic resonance imaging of sacroiliac joints or based on high sensitivity C-reactive protein > upper limit of normal (Study 2, nr-axSpA). Eligible study subjects must have a Bath Ankylosing Spondylitis Disease Activity Index score $\geq 4$ and a Patient's Assessment of Total Back Pain score (Total Back Pain score) $\geq 4$ based on a 0 - 10 numerical rating scale at the Screening and Baseline Visits. Subject must have objective signs of active inflammation on MRI of SI joints or hsCRP > ULN at screening.

For Study 2 (nr-axSpA), subjects with prior failure of nonsteroidal anti-inflammatory drugs (NSAIDs) may be enrolled, and prior treatment with at most 1 bDMARD (either 1 TNF inhibitor or 1 IL-17 inhibitor) is allowed in a subset of subjects (at least 25%, but not exceeding 35% of total enrolled subjects). Subjects with prior exposure to 1 bDMARD (either 1 tumor necrosis factor [TNF] inhibitor or 1 interleukin [IL]-17 inhibitor) may be enrolled, and the subject must have discontinued the bDMARD due to either intolerance or lack of efficacy. Prior exposure to a 2nd bDMARD is allowed for no more than 30% of subjects if the reason for discontinuation was not due to lack of efficacy. Subjects who have had lack of efficacy to both a TNF inhibitor and IL-17 inhibitor are not eligible.

**Study 2 Primary endpoints**

The primary endpoint for European Union [EU]/European Medicines Agency [EMA] regulatory purposes is the proportion of subjects achieving an ASAS40 response at Week 14.

The primary endpoint for US/Food and Drug Administration [FDA] regulatory purposes is the proportion of subjects achieving an ASAS40 response at Week 52.

Secondary endpoints for Study 2 are described below.

**Study 1: bDMARD-IR AS specific criteria**

1. Subject must have a clinical diagnosis of AS and subjects must meet the modified New York criteria for AS.
2. Subject must not have total spinal ankylosis.
3. Subjects with prior exposure to 1 bDMARD (either 1 tumor necrosis factor [TNF] inhibitor or 1 interleukin [IL]-17 inhibitor) may be enrolled, and the subject must have discontinued the bDMARD due to either intolerance or lack of efficacy. Prior exposure to a 2nd bDMARD is allowed for no more than 30% of subjects if the reason for discontinuation was not due to lack of efficacy. Subjects who have had lack of efficacy to both a TNF inhibitor and IL-17 inhibitor are not eligible.

**Study 2: nr-axSpA-specific criteria**

1. Subject must have a clinical diagnosis of nr-axSpA fulfilling the 2009 ASAS classification criteria for axSpA but not meeting the radiologic criterion of the modified New York criteria for AS.
2. Subjects with or without prior exposure to a bDMARD may be enrolled.
For the subset of subjects with prior bDMARD exposure (at least 25%, but not exceeding 35% of total enrolled subjects), prior treatment with at most 1 bDMARD (either 1 TNF inhibitor or 1 IL-17 inhibitor) is allowed, and the subject must have discontinued the bDMARD due to either intolerance or lack of efficacy. Subjects who have had lack of efficacy to both a TNF inhibitor and IL-17 inhibitor are not eligible.

3. Subject must have objective signs of active inflammation on MRI of SI joints or hsCRP > ULN at screening.

**The following eligibility criteria are applicable for Study 1 and Study 2:**

1. Subject must be an adult male or female, at least 18 years of age at Screening.

2. Subject must meet the following scores at Screening and Baseline Visits: BASDAI score $\geq$ 4 and Total Back Pain score $\geq$ 4 based on a 0 - 10 NRS.

3. Subject has had an inadequate response to at least 2 NSAIDs over an at least 4-week period in total at maximum recommended or tolerated doses, or subject has an intolerance to or contraindication for NSAIDs.

4. The washout period for bDMARDs prior to the first dose of study drug is specified below:

- $\geq$ 4 weeks for etanercept;
- $\geq$ 8 weeks for adalimumab, infliximab, certolizumab, golimumab, abatacept, tocilizumab, and ixekizumab;
- $\geq$ 12 weeks for ustekinumab;
- $\geq$ 16 weeks for secukinumab;
- $\geq$ 1 year for rituximab OR $\geq$ 6 months if B cells have returned to pre-treatment level or normal reference range (central lab) if pre-treatment levels are not available;
- $\geq$ 12 weeks or at least 5 times the mean terminal elimination half-life, whichever is longer, for other bDMARDs.

5. If entering the study on the following concomitant csDMARDs (MTX (<_ 25 mg/week); or Sulfasalazine (SSZ) (<_ 3 g/day); or Hydroxychloroquine (<_ 400 mg/day); or Chloroquine (<_ 400 mg/day); or Leflunomide ($\leq$ 20 mg/day); or Apremilast ($\leq$ 60 mg/day)), subject must be on a stable dose as indicated below for at least 28 days prior to the Baseline Visit. A combination of up to 2 background csDMARDs is allowed EXCEPT the combination of methotrexate (MTX) and leflunomide.

6. If entering the study on concomitant oral corticosteroids, subject must be on a stable dose of prednisone (<_ 10 mg/day) or oral corticosteroid equivalent for at least 14 days prior to the Baseline Visit.

7. If entering the study on concomitant NSAIDs, tramadol, combination of acetaminophen/paracetamol and codeine or combination of acetaminophen/paracetamol and hydrocodone, and/or non-opioid analgesics, subject must be on stable dose(s) for at least 14 days prior to the Baseline Visit.

8. Subject must not have been exposed to any JAK inhibitor.

9. Subject must not have used the following prohibited concomitant treatments within the specified timeframe prior to Baseline Visit:

- Intra-articular joint injections, spinal/paraspinal injection(s), or parenteral administration of corticosteroids within 28 days prior to the Baseline Visit. Inhaled or topical corticosteroids are allowed;
- Any other csDMARDs (other than those allowed per eligibility criterion), including thalidomide, within 28 days or 5 half-lives (whichever is longer) of the drug prior to the Baseline Visit;
- Opioid analgesics (except for combination of acetaminophen/paracetamol and codeine or combination of acetaminophen/paracetamol and hydrocodone which are allowed) within 14 days prior to the Baseline Visit.

10. Subject must not have received a live vaccine within 28 days (or longer if required locally) prior to the first dose of study drug or have expected need of live vaccination during study participation including at least 30 days (or longer if required locally) after the last dose of study drug.

11. Subject must have no systemic use of known strong cytochrome P450 3A (CYP3A) inhibitors from Screening through the end of study drug administration or strong CYP3A inducers 30 days prior to study drug administration through the end of study drug administration. Subjects must not use herbal therapies or other traditional medicines with unknown effects on CYP3A from Screening through the end of study drug administration.

12. Subject must not have been treated with any investigational drug of chemical or biologic nature within a minimum of 30 days or 5 half-lives of the drug (whichever is longer) prior to the first dose of study drug or is currently enrolled in another interventional study.

13. Subject must not have a history of an allergic reaction or significant sensitivity to constituents of the study drug (and its excipients) and/or other products in the same class.

## Study 1 and Study 2 Secondary Endpoints

The key multiplicity-controlled secondary endpoints at Week 14 are as follows:

**Table G.**

| | Study 1 (active AS) | Study 2 (active nr-axSpA) |
|---|---|---|
| 1 | Change from Baseline in Ankylosing Spondylitis Disease Activity Score (ASDAS) at Week 14 | Change from Baseline in Ankylosing Spondylitis Disease Activity Score (ASDAS) at Week 14 |
| 2 | Change from Baseline in magnetic resonance imaging (MRI) Spondyloarthritis Research Consortium of Canada (SPARCC) score (spine) at Week 14 (MRI-Spine SPARCC) | Change from Baseline in magnetic resonance imaging (MRI) Spondyloarthritis Research Consortium of Canada (SPARCC) score (SI joints) at Week 14 (MRI-Joints SPARCC) |
| 3 | Proportion of subjects with ASAS partial remission (PR) (an absolute score of $\leq 2$ units for each of the 4 domains identified in ASAS40) at Week 14 | Proportion of subjects with ASAS partial remission (PR) (an absolute score of $\leq 2$ units for each of the 4 domains identified in ASAS40) at Week 14 |
| 4 | Proportion of subjects with Bath Ankylosing Spondylitis Disease Activity Index (BASDAI) 50 response at Week 14 | Proportion of subjects with Bath Ankylosing Spondylitis Disease Activity Index (BASDAI) 50 response at Week 14 |
| 5 | Change from Baseline in Bath Ankylosing Spondylitis Functional Index (BASFI) at Week 14 (Function) | Change from Baseline in Bath Ankylosing Spondylitis Functional Index (BASFI) at Week 14 (Function) |
| 6 | Change from Baseline in Ankylosing Spondylitis Quality of Life (ASQoL) at Week 14 | Change from Baseline in Ankylosing Spondylitis Quality of Life (ASQoL) at Week 14 |
| 7 | Change from Baseline in ASAS Health Index (HI) at Week 14 | Change from Baseline in ASAS Health Index (HI) at Week 14 |
| 8 | Change from Baseline in Maastricht Ankylosing Spondylitis Enthesitis Score (MASES) at Week 14 (Enthesitis) | Change from Baseline in Maastricht Ankylosing Spondylitis Enthesitis Score (MASES) at Week 14 (Enthesitis) |
| 9 | Change from Baseline in Linear Bath Ankylosing Spondylitis Metrology Index (BASMI$_{lin}$) at Week 14 (Mobility) | Change from Baseline in Linear Bath Ankylosing Spondylitis Metrology Index (BASMI$_{lin}$) at Week 14 (Mobility) |

Additional key secondary endpoints at Week 14 include:

**Table H.**

| | Study 1 (active AS) | Study 2 (active nr-axSpA) |
|---|---|---|
| 1 | Proportion of subjects with ASAS20 response at Week 14 | Proportion of subjects with ASAS20 response at Week 14 |
| 2 | Change from Baseline in MRI SPARCC score (SI joints) at Week 14 | Change from Baseline in MRI SPARCC score (spine) at Week 14 |
| 3 | | Proportion of subjects rescued between Week 24 and Week 52 |

## Additional Study 1 and Study 2 Endpoints

Additional endpoints are the following measurements assessed at time points other than those specified for the primary and key secondary variables are as follows:

| Table I. | | | |
|---|---|---|---|
| | | **Study 1 (active AS)** | **Study 2 (active nr-axSpA)** |
| | 1 | Proportion of subjects with ASAS20 response | Proportion of subjects with ASAS20 response |
| | 2 | Proportion of subjects with ASAS40 response | Proportion of subjects with ASAS40 response |
| | 3 | Proportion of subjects with ASAS PR | Proportion of subjects with ASAS PR |
| | 4 | Proportion of subjects with ASDAS Inactive Disease (ASDAS score < 1.3) | Proportion of subjects with ASDAS Inactive Disease (ASDAS score < 1.3) |
| | 5 | Proportion of subjects with ASDAS Low Disease (ASDAS score < 2.1) | Proportion of subjects with ASDAS Low Disease (ASDAS score < 2.1) |
| | 6 | Proportion of subjects with ASDAS Major Improvement (a change from Baseline of $\leq$ - 2.0) | Proportion of subjects with ASDAS Major Improvement (a change from Baseline of $\leq$ -2.0) |
| | 7 | Proportion of subjects with ASDAS Clinically Important Improvement (a change from Baseline of $\leq$ -1.1) | Proportion of subjects with ASDAS Clinically Important Improvement (a change from Baseline of $\leq$ -1.1) |
| | 8 | Proportion of subjects with Discontinuation of opioids among subjects with opioid use at Baseline | Proportion of subjects with Discontinuation of opioids among subjects with opioid use at Baseline |
| | 9 | Change from Baseline in ASAS HI | Change from Baseline in ASAS HI |
| | 10 | Change from Baseline in ASDAS | Change from Baseline in ASDAS |
| | 11 | Change from Baseline in ASQoL | Change from Baseline in ASQoL |
| | 12 | Change from Baseline in BASDAI and BASDAI Questions including mean of question 5 and 6 of the BASDAI | Change from Baseline in BASDAI and BASDAI Questions including mean of question 5 and 6 of the BASDAI |
| | 13 | Change from Baseline in BASFI | Change from Baseline in BASFI |
| | 14 | Change from Baseline in BASMIlin | Change from Baseline in $BASMI_{lin}$ |
| | 15 | Change from Baseline in High sensitivity C-reactive protein (hsCRP) | Change from Baseline in hsCRP |
| | 16 | Change from Baseline in Functional Assessment of Chronic Illness Therapy-Fatigue (FACIT-F) | Change from Baseline in FACIT-F |
| | 17 | Change from Baseline in EuroQoL-5D-5L (EQ-5D-5L) | Change from Baseline in EQ-5D-5L |
| | 18 | Change from Baseline in MASES | Change from Baseline in MASES |
| | 19 | Change from Baseline in Modified Stoke Ankylosing Spondylitis Spine Score (mSASSS) with conventional radiograph | Change from Baseline in mSASSS with conventional radiograph |
| | 20 | Change from Baseline in MRI SPARCC score of SI joints | Change from Baseline in MRI SPARCC score of SI joints |
| | 21 | Change from Baseline in MRI SPARCC score of spine | Change from Baseline in MRI SPARCC score of spine |
| | 22 | Change from Baseline in Patient's Assessment of Total Back Pain (Total Back Pain score); | Change from Baseline in Total Back Pain; |
| | 23 | Change from Baseline in Patient's Assessment of Nocturnal Back Pain (Nocturnal Back Pain) | Change from Baseline in Nocturnal Back Pain |
| | 24 | Change from Baseline in Patient's Global Assessment of Pain (Pain) | Change from Baseline in Pain; |

(continued)

| | Table I. | |
|---|---|---|
| | **Study 1 (active AS)** | **Study 2 (active nr-axSpA)** |
| 25 | Change from Baseline in Physician's Global Assessment of Disease Activity (PGA) | Change from Baseline in PGA; |
| 26 | Change from Baseline in Patient's Global Assessment of Disease Activity (PtGA) | Change from Baseline in PtGA |
| 27 | Change from Baseline in 36-Item Short Form Health Survey (SF-36) | Change from Baseline in SF-36 |
| 28 | Change from Baseline in Tender joint count (TJC) and swollen joint count (SJC); | Change from Baseline in TJC and SJC |
| 29 | Change from Baseline in Work Productivity and Activity Impairment (WPAI) | Change from Baseline in WPAI |
| 30 | Change from Baseline in Change of NSAID score | Change from Baseline in Change of NSAID score |
| 31 | Change from Baseline in Physical Activity Assessment (step count, physical activity, and spinal range of motion tasks) as measured by a wearable device (in countries where the digital health technology device is approved) | |

## EXAMPLE 4

*Phase 3 Protocol in subjects with active Psoriatic Arthritis and a previous inadequate response to at least one non-biologic Disease Modifying Anti-Rheumatic Drug (non-biologic DMARD) (SELECT-PSA1)*

The Phase 3 Study Design for SELECT-PSA1 is provided in FIG. 10.

15 mg or 30 mg upadacitinib refers to the 15 mg and 30 mg once-daily (QD) upadacitinib drug product as described in Example 1.

This is a Phase 3 multicenter study that includes two periods. Period 1 is 56 weeks in duration and includes a 24-week randomized, double-blind, parallel-group, placebo-controlled and active comparator-controlled period followed by an additional 32 weeks of blinded, active comparator-controlled treatment (Weeks 24 - 56). Period 1 is designed to compare the safety, tolerability, and efficacy of upadacitinib free base 15 mg QD and 30 mg QD versus placebo and versus adalimumab 40 mg every other week (eow) in subjects with moderately to severely active PsA and have an inadequate response to non-biologic DMARDs (DMARD-IR). Period 1 is also designed to compare the efficacy of upadacitinib free base 15 mg QD and 30 mg QD versus placebo for the prevention of structural progression. Period 2 is an open-label (blinded until the last subject completes the last visit of Period 1), long-term extension of up to a total treatment duration of approximately 3 years to evaluate the safety, tolerability and efficacy of upadacitinib free base 15 mg QD and 30 mg QD in subjects with moderately to severely active PsA who have completed Period 1.

The study duration includes a 35-day screening period; a 56-week blinded period which includes 24 weeks of double-blind, placebo-controlled and active comparator controlled treatment followed by 32 weeks of active comparator controlled treatment (Period 1); a long-term extension period of up to a total treatment duration of approximately 3 years ([blinded until the last subject completes the last visit of Period 1] Period 2); a 30-day follow-up call or visit; and a 70-day follow-up call.

Subjects who met eligibility criteria were stratified by extent of psoriasis (>_ 3% body surface area [BSA] or < 3% BSA), current use of at least 1 DMARD, presence of dactylitis, and presence of enthesitis, except for subjects from China and Japan, where randomization for each country was stratified by extent of psoriasis (>_ 3% body surface area [BSA] or < 3% BSA) only, and then randomized in a 2:2:2:1:1 ratio to one of five treatment groups:

Group 1: upadacitinib 15 mg QD (N = 430)
Group 2: upadacitinib free base 30 mg QD (N = 423)
Group 3: adalimumab (ADA) (40 mg eow) (N = 429)
Group 4: Placebo followed by upadacitinib free base 15 mg QD (N = 211)
Group 5: Placebo followed by upadacitinib free base 30 mg QD (N = 212)

No more than approximately 15% of subjects were enrolled with concomitant use of hydroxychloraquine, sulfasalazine, bucillamine, or iguratimod.

Subjects received both oral study drug QD (upadacitinib free base 15 mg, upadacitinib free base 30 mg, or matching placebo) and subcutaneous study drug eow (either ADA 40 mg or matching placebo) until all subjects completed Period 1 (Week 56) and sites and subjects are unblinded to study treatment.

Subjects who were assigned to placebo at Baseline were preassigned to receiving either upadacitinib free base 15 mg QD or upadacitinib free base 30 mg QD starting at Week 24 in a 1:1 ratio. Subjects who complete the Week 56 visit (end of Period 1) will enter the long-term extension portion of the study, Period 2 (total study duration up to approximately 3 years). Subjects will continue study treatment as assigned in Period 1. Subjects who are assigned to the upadacitinib free base 15 mg QD, upadacitinib free base 30 mg QD, or adalimumab 40 mg eow will continue to receive upadacitinib free base 15 mg QD, upadacitinib free base 30 mg QD, or adalimumab 40 mg eow, respectively, in a blinded manner. When the last subject completes the last visit of Period 1 (Week 56), study drug assignment in both periods will be unblinded to the sites, and subjects will be dispensed study drug in an open-label fashion until the completion of Period 2.

Subjects must have had inadequate response to ≥ 1 non-biologic DMARD (MTX, SSZ, LEF, apremilast, bucillamine or iguratimod) or an intolerance to or contraindication for DMARDs prior to the Screening visit. No background non-biologic DMARD therapy is required during participation in this study. For subjects who are on non-biologic DMARD therapy at baseline (MTX, SSZ, LEF, apremilast, hydroxychloroquine (HCQ), bucillamine or iguratimod), non-biologic DMARDs should have been started ≥ 12 weeks prior to the baseline visit, must be at stable dose for ≥ 4 weeks prior to the first dose of study drug and remain on a stable dose through Week 36 of the study; the non-biologic DMARD dose may be decreased only for safety reasons. In addition, all subjects taking MTX should take a dietary supplement of oral folic acid (or equivalent) throughout study participation.

At Week 16, rescue therapy will be offered to subjects classified as non-responders (defined as not achieving at least 20% improvement in either or both tender joint count (TJC) and swollen joint count (SJC) at both Week 12 and Week 16) as follows: 1) add or modify doses of non-biologic DMARDs, NSAIDs, acetaminophen/paracetamol, low potency opioid medications (tramadol or combination of acetaminophen and codeine or hydrocodone), oral corticosteroids and/or 2) receive 1 intra-articular, trigger point or tender point, intra-bursa, or intra-tendon sheath corticosteroid injection for 1 peripheral joint, 1 trigger point, 1 tender point, 1 bursa, or 1 enthesis (Rescue Therapy).

After the last subject completes the Week 24 study visit, an unblinded analysis will be conducted for the purpose of initial regulatory submission. To maintain integrity of the trial during the blinded 56-week period, study sites and subjects will remain blinded until all subjects have reached Week 56. A second unblinded analysis may be conducted for regulatory purposes after all subjects have completed Period 1. A final analysis will be conducted after all subjects have completed Period 2.

## Primary and Secondary Endpoints

The primary efficacy endpoint is the proportion of subjects achieving American College of Rheumatology (ACR) 20% response rate at Week 12. The primary and secondary clinical endpoints for this study (SELECT-PSA1 clinical study; Example 4) and the SELECT-PSA2 clinical study (Example 5) are further described in Example 6.

## Study Population

Patients with active psoriatic arthritis and a previous inadequate response to at least one non-biologic disease-modifying anti-rheumatic drug (non-biologic DMARD or DMARD).

## Main Inclusion criteria include:

1. Adult male or female, >_ 18 years old at Screening.
2. Clinical diagnosis of PsA with symptom onset at least 6 months prior to the Screening Visit and fulfillment of the Classification Criteria for PsA (CASPAR) criteria.
3. Subject has active disease at Baseline defined as ≥ 3 tender joints (based on 68 joint counts) and ≥ 3 swollen joints (based on 66 joint counts) at Screening and Baseline Visits.
4. Presence of either at Screening:

> ≥ 1 erosion on x-ray as determined by central imaging review or;
> hs-CRP > laboratory defined upper limit of normal (ULN).

5. Diagnosis of active plaque psoriasis or documented history of plaque psoriasis.

6. Subject has had an inadequate response (lack of efficacy after a minimum 12 week duration of therapy) to previous or current treatment with at least 1 non-biologic DMARD at maximally tolerated dose or up to dose defined in Inclusion Criterion 7 [(inadequate response to MTX is defined as $\geq$ 15 to $\leq$ 25 mg/week; or $\geq$ 10 mg/week in subjects who are intolerant of MTX at doses $\geq$ 12.5 mg/week after complete titration; for subjects in China, Taiwan, and Japan inadequate response to MTX is defined as $\geq$ 7.5 mg/week), SSZ, LEF, cyclosporine, apremilast, bucillamine or iguratimod)], or subject has an intolerance to or contraindication for DMARDs.

7. Subject who is on current treatment with concomitant non-biologic DMARDs at study entry must be on $\leq$ 2 non-biologic DMARDs (except the combination of MTX and leflunomide) at the following doses: MTX ($\leq$ 25 mg/week), SSZ ($\leq$ 3000 mg/day), leflunomide (LEF) ($\leq$ 20 mg/day), apremilast ($\leq$ 60 mg/day), HCQ ($\leq$ 400 mg/day), bucillamine ($\leq$ 300 mg/day) or iguratimod ($\leq$ 50 mg/day) for $\geq$ 12 weeks and at stable dose for $\geq$ 4 weeks prior to the Baseline Visit. No other DMARDs are permitted during the study. Subjects who need to discontinue DMARDs prior to the Baseline Visit to comply with this inclusion criterion must follow the procedure specified below or at least five times the mean terminal elimination half-life of a drug:

$\geq$ 8 weeks for LEF if no elimination procedure was followed, or adhere to an elimination procedure (*i.e.*, 11 days with cholestyramine, or 30 days washout with activated charcoal or as per local label);
$\geq$ 4 weeks for all others

8. Stable doses of non-steroidal anti-inflammatory drugs (NSAIDs), acetaminophen/paracetamol, low-potency opiates (tramadol or combination of acetaminophen and codeine or hydrocodone), oral corticosteroids (equivalent to prednisone $\leq$ 10 mg/day), or inhaled corticosteroids for stable medical conditions are allowed, but must have been at a stable dose for $\geq$ 1 week prior to the Baseline Visit.

9. Subjects must have discontinued all opiates (except for tramadol, or combination of acetaminophen and codeine or hydrocodone) at least 1 week and oral traditional Chinese medicine for at least 4 weeks prior to the first dose of study drug.

10. Where mandated by local requirements only, treatment with at least one of the following background medications is required: non-biologic DMARDs, NSAIDs, acetaminophen, low potency opiates (tramadol or combination of acetaminophen codeine or hydrocodone), or oral corticosteroids at doses.

## Main Exclusion Criteria include:

1. Prior exposure to any Janus Kinase (JAK) inhibitor (including but not limited to ruxolitinib, tofacitinib, baricitinib, and filgotinib).

2. Current treatment with > 2 non-biologic DMARDs or use of DMARDs other than MTX, SSZ, LEF, apremilast, HCQ, bucillamine or iguratimod or use of MTX in combination with LEF at Baseline.

3. History of fibromyalgia, any arthritis with onset prior to age 17 years, or current diagnosis of inflammatory joint disease other than PsA (including, but not limited to rheumatoid arthritis, gout, overlap connective tissue diseases, scleroderma, polymyositis, dermatomyositis, systemic lupus erythermatosus). Prior history of reactive arthritis or axial spondyloarthritis including ankylosing spondylitis and non-radiographic axial spondyloarthritis is permitted if documentation of change in diagnosis to PsA or additional diagnosis of PsA is made. Prior history of fibromyalgia is permitted if documentation of change in diagnosis to PsA or documentation that the diagnosis of fibromyalgia was made incorrectly.

## Analysis Windows

The following rules will be applied to assign actual subject visits to protocol-specified visits. For each protocol-specified study visit, a target study day will be identified to represent the corresponding visit along with a window around the target day. Windows will be selected in a non-overlapping fashion so that a collection date does not fall into multiple visit windows. If a subject has two or more actual visits in one visit window, the visit closest to the target day will be used for analysis. If two visits are equidistant from the target day, then the later visit will be used for analysis.

## Results up to and including Week 24

As noted above, after the last subject completed the Week 24 study visit, an unblinded analysis was conducted for the purpose of initial regulatory submission.

The study met the primary endpoint ACR20 at Week 12. At week 12, the primary outcome of ACR20 response was achieved by 153 (36.2%) patients receiving placebo versus 303 (70.6%; difference: 34.5%; 95% CI: 28.2 to 40.7; P<0.001) receiving upadacitinib 15mg and 332 (78.5%; difference: 42.3%; 95% CI: 36.3 to 48.3; P<0.001) receiving upadacitinib 30mg; 279 (65.0%) patients receiving adalimumab achieved ACR20 response rates versus upadacitinib 15mg (difference: 5.6%; 95% CI: -0.6 to 11.8) and 30mg (difference: 13.5%; 95% CI: 7.5 to 19.4; P<0.001. Upadacitinib 30mg was superior to adalimumab; the 15mg dose was not superior to adalimumab, which prevented the testing of significance for secondary endpoints lower in the testing hierarchy. The efficacy of upadacitinib freebase 15 mg was demonstrated regardless of subgroups evaluated including baseline BMI, baseline hsCRP, and number of prior non-biologic DMARDs (<_ 1 or >1).

The study also showed statistical significance for the non-inferiority (NI) of ACR20 at Week 12 compared to adalimumab for both upadacitinib free base doses. After achieving the non-inferiority (NI) comparisons, superiority tests compared to adalimumab were conducted. Upadacitinib free base 30 mg showed superiority vs. adalimumab in ACR20 at Week 12.

The results for key secondary endpoints versus placebo were consistent with that of the primary endpoint. At week 12, treatment differences (95% CI) in ACR50 response versus placebo for upadacitinib 15mg and 30mg were 24.3% (18.7 to 29.9) and 38.5% (32.8 to 44.3), respectively and versus adalimumab were 0.0% (-6.5 to 6.5) and 14.2% (7.6 to 20.9), respectively. Treatment differences (95% CI) in ACR70 response versus placebo for upadacitinib 15mg and 30mg were 13.3% (9.5 to 17.0) and 22.9% (18.5 to 27.3), respectively and versus adalimumab were 1.9% (-2.9 to 6.6) and 11.5% (6.3 to 16.8), respectively. At week 24, the percentage of patients achieving ACR20/50/70 responses were in the same direction as the primary endpoint for both upadacitinib doses versus placebo and adalimumab; no clinical inferences can be drawn from these data. At week 24, the percentage of patients achieving Minimal Disease Activity (MDA) was significantly greater with both upadacitinib doses versus placebo (P<0.001 for both comparisons) and was in the same direction as the primary outcome versus adalimumab.

At week 16, a significantly greater percentage of patients achieved Psoriasis Area Severity Index of at least 75% (PASI 75) and Static Investigator Global Assessment of Psoriasis of 0 or 1 and at least a 2-point improvement from baseline (sIGA 0/1) response with both upadacitinib doses versus placebo (P<0.001 for each comparison). The percentage of patients achieving PASI 75 response was in the same direction as the primary endpoint for both upadacitinib doses versus adalimumab, and for sIGA 0/1 response with upadacitinib 30mg; the percentage of sIGA 0/1 responders was not significantly different for upadacitinib 15mg versus adalimumab. Greater improvement in patient-reported psoriasis symptoms, as measured by the Self-Assessment of Psoriasis Symptoms (SAPS), was also observed at Week 16 in patients treated with upadacitinib freebase 15 mg QD compared to placebo. Statistically greater efficacy was observed with each upadacitinib dose versus placebo for change from baseline in HAQ-DI, SF-36 PCS, and FACIT-F at week 12 (P<0.001 for each comparison). The proportion of HAQ-DI responders (>_ 0.35 improvement from baseline in HAQ-DI score) at Week 12 was 58% in patients receiving upadacitinib freebase 15 mg QD and 33% in patients receiving placebo.

Health-related quality of life was assessed by SF-36. Patients receiving upadacitinib freebase 15 mg QD experienced significantly greater improvement from baseline in the Physical Component Summary score compared to placebo at Week 12. Greater improvement was also observed in the Mental Component Summary score and all 8 domains of SF-36 (Physical Functioning, Bodily Pain, Vitality, Social Functioning, Role Physical, General Health, Role Emotional, and Mental Health) compared to placebo..

At week 24, significantly more upadacitinib-treated patients achieved resolution of enthesitis versus placebo (P<0.001 for each comparison); upadacitinib 15mg was not significantly different to adalimumab for resolution of enthesitis and results with upadacitinib 30mg were in the same direction as the primary endpoint. Rates for resolution of dactylitis are presented in the table below.

The study also demonstrated significantly greater efficacy in PsA signs and symptoms as well as inhibition of radiographic progression compared to placebo. For example, at week 24, upadacitinib-treated patients exhibited significantly less radiographic progression versus placebo-treated patients as demonstrated by the change from baseline in modified total Sharp/van der Heijde Score (mTSS; P<0.001) (mTSS equivalent to SHS); mean progression was not different for upadacitinib and adalimumab. Statistically significant results were also achieved for both erosion and joint space narrowing scores for the 15 mg QD dosage. The proportion of patients with no radiographic progression (mTSS change ≤ 0) was higher with upadacitinib freebase 15 mg QD (93%) compared to placebo (89%) at Week 24. These results are summarized below in the table below.

A summary of primary and key secondary efficacy results are presented in the below Tables (Parts 1 and 2). See also **FIGS. 11A-11B** for a listing of the ranked secondary endpoints, Parts 1 and 2. The time course of the ACR20 response and associated 95% confidence interval (CI) up to and including Week 24 is presented in **FIG. 12A,** and results for additional key secondary endpoints are presented in **FIGS. 12B-12BB.** It was observed that this clinical efficacy achieved was sustained with continued daily dosing.

| Table J. Demographics and Characteristics at Baseline | | | | |
|---|---|---|---|---|
| | **Placebo N=423** | **Upadacitinib 15mg QD N=429** | **Upadacitinib 30mg QD N=423** | **Adalimumab 40 mg EOW N=429** |
| Female, n (%) | 211 (49.9) | 238 (55.5) | 236 (55.8) | 222 (51.7) |
| Age (years) | 50.4 ± 12.2 | 51.6 ± 12.2 | 49.9 ± 12.4 | 51.4 ± 12.0 |
| White race, n (%) | 377 (89.1) | 386 (90.0) | 377 (89.1) | 375 (87.4) |
| BMI ≥25 kg/m$^2$, n (%) | 329 (77.8) | 342 (79.7) | 319 (75.4) | 334 (77.9) |
| Duration since PsA diagnosis (years) | 6.2 ± 7.0 | 6.2 ± 7.4 | 5.9 ± 6.4 | 5.9 ± 7.1 |
| Any non-biologic DMARD at baseline, n (%) | 347 (82.0) | 353 (82.3) | 346 (81.8) | 347 (80.9) |
| MTX alone | 267 (63.1) | 279 (65) | 268 (63.4) | 270 (62.9) |
| MTX + another non-biologic DMARD | 26 (6.1) | 20 (4.7) | 27 (6.4) | 16 (3.7) |
| Non-biologic DMARD other than MTX | 54 (12.8) | 54 (12.6) | 51 (12.1) | 61 (14.2) |
| Glucocorticoid use at baseline, n (%) | 70 (16.5) | 73 (17.0) | 71 (16.8) | 72 (16.8) |
| TJC68 | 20.0 ± 14.3 | 20.4 ± 14.7 | 19.4 ± 13.3 | 20.1 ± 13.8 |
| SJC66 | 11.0 ± 8.2 | 11.6 ± 9.3 | 10.6 ± 7.1 | 11.6 ± 8.8 |
| hs-CRP > ULN [a], n (%) | 324 (76.6) | 324 (75.5) | 324 (76.6) | 308 (71.8) |
| HAQ-DI | 1.12 ± 0.6 | 1.15 ± 0.7 | 1.09 ± 0.6 | 1.12 ± 0.6 |
| Patient's Assessment of Pain (NRS 0-10) | 6.1 ± 2.1 | 6.2 ± 2.1 | 5.9 ± 2.1 | 6.0 ± 2.1 |
| BSA-psoriasis ≥ 3%, n (%) PASI (for baseline BSA-psoriasis ≥3%) | 211(49.9) 11.2 ± 11.4 | 214 (49.9) 9.78 ± 10.0 | 210 (49.6) 9.5 ± 8.8 | 211 (49.2) 9.4 ± 8.5 |
| sIGA of Psoriasis score, n (%)     0     1     2     3     4 | 24 (5.7) 86 (20.3) 167 (39.5) 119 (28.1) 27 (6.4) | 34 (7.9) 73 (17.0) 170 (39.6) 133 (31.0) 19 (4.4) | 21 (5.0) 78 (18.4) 173 (40.9) 128 (30.3) 23 (5.4) | 34 (7.9) 65 (15.2) 181 (42.2) 132 (30.8) 17 (4.0) |
| Presence of enthesitis (defined as LEI > 0), n (%) | 241 (57.0) | 270 (62.9) | 267 (63.1) | 265 (61.8) |
| Presence of dactylitis (defined as LDI > 0), n (%) | 126 (29.8) | 136 (31.7) | 127 (30.0) | 127 (29.6) |
| Values are mean ± SD unless noted. [a]ULN >2.87 mg/L; Permitted concomitant non-biologic DMARDs included: methotrexate, sulfasalazine, leflunomide, apremilast, hydroxychloroquine, bucillamine, and iguratimod. | | | | |

(continued)

| Table J. Demographics and Characteristics at Baseline | | | | |
|---|---|---|---|---|
| | Placebo N=423 | Upadacitinib 15mg QD N=429 | Upadacitinib 30mg QD N=423 | Adalimumab 40 mg EOW N=429 |
| BMI, body mass index; BSA, body surface area; DMARD, disease-modifying anti-rheumatic drug; EOW, every other week; HAQ-DI, Health Assessment Questionnaire - Disability Index; hs-CRP, high-sensitivity C-reactive protein; LDI, Leeds Dactylitis Index; LEI, Leeds Enthesitis Index; MTX, methotrexate; NRS, numeric rating scale; NSAID, nonsteroidal anti-inflammatory drug; PASI, psoriasis area severity index; PsA, psoriatic arthritis; QD, once daily; SD, standard deviation; ULN, upper limit normal; SD, standard deviation; sIGA, Static Investigator Global Assessment of Psoriasis; SJC, swollen joint count; TJC, tender joint count. | | | | |

| Table K. Primary and Ranked Secondary Efficacy Endpoints - Part 1 | | | | | | |
|---|---|---|---|---|---|---|
| Summary Statistics % or LS Mean (P-Value) | | Endpoint[a] | PLACEBO N = 423 | ADA 40 MG EOW N = 429 | UPA 15 MG QD N = 429 | UPA 30 MG QD N=423 |
| Primary | | ACR20 (Wk12) | 36.2% | 65.0% | 70.6% (<0.0001*) | 78.5% (<0.0001*) |
| Ranked Secondary Endpoints (Part 1) | 1 | HAQ-DI (Wk12) | -0.14 | -0.34 | -0.42 (<0.0001*) | -0.47 (<0.0001*) |
| | 2 | sIGA (Wk16)[b] | 10.9% | 38.5% | 41.9% (<0.0001*) | 54.0% (<0.0001*) |
| | 3 | PASI 75 (Wk16)[c] | 21.3% | 53.1% | 62.6% (<0.0001*) | 62.4% (<0.0001*) |
| | 4 | SHS (mTSS) (Wk24) | 0.25 | 0.01 | -0.04 (0.0002*) | 0.03 (0.0069*) |
| | 5 | MDA (Wk24) | 12.3% | 33.3% | 36.6% (<0.0001*) | 45.4% (<0.0001*) |
| | 6 | Enthesitis Resolution (Wk 24)[d] | 32.4% | 47.2% | 53.7% (<0.0001*) | 57.7% (<0.0001*) |
| | 7 | ACR 20 NI vs ADA[e] (Wk12) | 36.2% | 65.0% | 70.6% (<0.0001*) | 78.5% (<0.0001*) |
| | 8 | SF-36 PCS (Wk12) | 3.19 | 6.82 | 7.86 (<0.0001*) | 8.90 (<0.0001*) |
| | 9 | FACIT-F (Wk12) | 2.8 | 5.7 | 6.3 (<0.0001*) | 7.1 (<0.0001*) |
| Note: The nominal p-values are provided in parentheses. * Achieved statistical significance using graphical multiple testing procedure controlling the overall type I error rate at 2-sided 0.0499 level. Note that 0.0001 was spent at the interim futility analysis. | | | | | | |

a. Results for binary endpoints are based on NRI analysis. Results for MDA and enthesitis resolution at week 24 are based on non-responder imputation with additional rescue handling, where subjects rescued at Week 16 are imputed as non-responders. Results for continuous endpoints are based on MMRM model with fixed effects of treatment, visit, treatment-by-visit interaction, the stratification factor of current DMARD use (yes/no) and baseline measurement. Results for SHS are based on ANCOVA with linear extrapolation for missing data and rescue handling.

b. Summarized for subjects with baseline sIGA ≥ 2; $N_{(pbo)}$ = 313, $N_{(ADA)}$ = 330, $N_{(upa15)}$ = 322, $N_{(upa30)}$ = 324.

c. Summarized for subjects with baseline BSA affected by psoriasis ≥ 3%; N(pbo) = 211, N(ADA) = 211, N(upa15) = 214, N(upa30) = 210.

d. Summarized for subjects with baseline LEI >0; N(pbo) = 241, N(ADA) = 265, N(upa15) = 270, N(upa30) = 267.

e. Non-inferiority test of upadacitinib vs adalimumab, preserving 50% of adalimumab effect.

| Table L. Ranked Secondary Efficacy Endpoints in Part 2 and Other Key Secondary Endpoints | | | | | | |
|---|---|---|---|---|---|---|
| Summary Statistics % or LS Mean (P-Value) | | Endpoint[a] | PLACEBO N=423 | ADA 40 MG EOW N=429 | UPA 15 MG QD N=429 | UPA 30 MG QD N=423 |
| Ranked Secondary Endpoints (Part 2) | 10 | ACR 20 Sup. vs ADA[b] (Wk12) | 36.2% | 65.0% | 70.6% (0.0815) | 78.5% (<0.0001*) |
| | 11 | Dactylitis Resolution (Wk24)[c] | 39.7% | 74.0% | 76.5% (<0.0001) | 79.5% (<0.0001) |
| | 12 | Pain Sup. vs ADA[b] (Wk12) | -0.9 | -2.3 | -2.3 (0.8970) | -2.7 (0.0028) |
| | 13 | HAQ-DI Sup. vs ADA[b] (Wk12) | -0.14 | -0.34 | -0.42 (0.0162) | -0.47 (<0.0001) |
| | 14 | SAPS (Wk16) | -8.2 | -22.7 | -25.3 (<0.0001) | -28.1 (<0.0001) |
| Other Key Secondary | | ACR50 (Wk12) | 13.2% | 37.5% | 37.5% (<0.0001) | 51.8% (<0.0001) |
| | | ACR70 (Wk12) | 2.4% | 13.8% | 15.6% (<0.0001) | 25.3% (<0.0001) |
| | | ACR20 (Wk2) | 12.1% | 30.3% | 28.2% (<0.0001) | 38.3% (<0.0001) |
| Note: The nominal p-values are provided in parentheses. * Statistical significance using graphical multiple testing procedure controlling overall type I error rate. | | | | | | |

a. Results for binary endpoints are based on NRI analysis. Results for dactylitis resolution at week 24 is based on non-responder imputation with additional rescue handling, where subjects rescued at Week 16 are imputed as non-responders. Results for continuous endpoints are based on MMRM model with fixed effects of treatment, visit, treatment-by-visit interaction, the stratification factor of current DMARD use (yes/no) and baseline measurement.
b. Superiority test of upadacitinib vs. adalimumab.
c. Summarized for subjects with baseline LDI >0; N(pbo) = 126, N(ADA) = 127, N(upa15) = 136, N(upa30) = 127.

| Table M. Additional Efficacy Endpoints for SELECT-PSA1: Summarized for subjects with baseline BSA affected by psoriasis $\geq$ 3%[a] | | | | |
|---|---|---|---|---|
| Endpoint | PLACEBO N = 211 | ADA 40 MG EOW N = 211 | UPA 15 MG QD N = 214 | UPA 30 MG QD N = 210 |
| PASI 90 (Wk16) | 12.3% | 38.9% | 38.3% (<0.0001) | 49.5% (<0.0001) |
| PASI 100 (Wk16) | 7.1% | 19.9% | 23.8% (<0.0001) | 33.8% (<0.0001) |

a. Full analysis set: N (placebo) = 423, N(ADA) = 429, N(UPA15) = 429, N(UPA30) = 423.

Additional data for ACR20, ACR50, and ACR70 response at Week 24, and MDA, resolution of enthesitis (LEI = 0), and resolution of dactylitis (LDI = 0) at week 12 is set forth in the below Table.

| Table N. Additional Clinical Responses | | |
|---|---|---|
| Endpoint | Placebo (N = 423) % | UPA 15 mg QD (N = 429) |
| ACR20 (Wk 24) | 45 | 73 |
| ACR50 (Wk 24) | 19 | 52 |
| ACR70 (Wk 24) | 5 | 29 |
| MDA (Wk 12) | 6 | 25 |
| Enthesitis resolution[a] (Wk 12) | 33 | 47 |
| Dactylitis resolution[b] (Wk 12) | 42 | 74 |
| Patients who discontinued randomized treatment or were missing data at week of evaluation were imputed as non-responders in the analyses. For MDA, resolution of enthesitis, and resolution of dactylitis at Week 24, the subjects rescued at Week 16 were imputed as non-responders in the analyses. [a] In patients with enthesitis at baseline (n=241 and 270, respectively) [b] In patients with dactylitis at baseline (n=126 and 136) | | |

Treatment with upadacitinib freebase 15 mg resulted in improvements in individual ACR components, including tender/painful and swollen joint counts, patient and physician global assessments of disease activity, HAQ-DI, pain assessment, and hsCRP compared to placebo. These results are shown in **Figs. 12F-12K and 12Q**. Onset of efficacy was seen as early as Week 2 for ACR20 and its components.

Inhibition of progression of structural damage was assessed radiographically and expressed as the change from baseline in modified Total Sharp Score (mTSS) and its components, the erosion score and the joint space narrowing score, at Week 24. Treatment with upadacitinib freebase 15 mg QD resulted in significantly greater inhibition of the progression of structural joint damage compared to placebo at Week 24. Statistically significant results were also achieved for both erosion and joint space narrowing scores. The proportion of patients with no radiographic progression (mTSS change $\leq 0$) was higher with upadacitinib freebase 15 mg QD (93%) compared to placebo (89%) at Week 24. These results are summarized in **FIG. 12Z.**

The safety profile of upadacitinib in this trial was generally similar to the safety profile observed in other upadacitinib clinical trials. Through week 24, the rates of treatment-emergent adverse events and serious adverse events, including serious infections, were similar with placebo, adalimumab, and upadacitinib 15mg but higher with upadacitinib 30mg. The most common AE was upper respiratory tract infection. Rates of serious infections were 0.9%, 0.7%, 1.2%, and 2.6% with placebo, adalimumab, upadacitinib 15mg and 30mg, respectively. Up to week 24, treatment-emergent opportunistic infections included one event of candida urethritis with upadacitinib 15mg, and one event each of pneumocystis jirovecii pneumonia and cytomegalovirus with upadacitinib 30mg. There were 3, 4, and 5 cases of herpes zoster reported with placebo and upadacitinib 15mg and 30mg, respectively. One malignancy occurred in each of the placebo and upadacitinib 15mg arms, and 3 malignancies were reported in each of the upadacitinib 30mg and adalimumab arms. No major adverse cardiovascular events (MACE) were reported with upadacitinib. One event of deep vein thrombosis was reported with placebo and 2 events with adalimumab; one event of pulmonary embolism was reported with upadacitinib 30mg. One death, adjudicated to unknown cause, was reported with placebo.

Mean hemoglobin, neutrophil, lymphocyte, and platelet levels remained within normal limits from baseline through week 24 in all treatment arms. Adverse events of anemia and lymphopenia were reported at similar rates for upadacitinib 15mg and placebo and more often with upadacitinib 30mg. Adverse events of neutropenia were more common with upadacitinib compared to placebo. Overall, the frequency of Grade $\geq 3$ laboratory abnormalities was $\leq 2.1\%$; most resolved without interruption in therapy. No patients had Grade 4 decreases in hematology parameters. One patient each reported isolated Grade 3 decrease in hemoglobin or platelets after discontinuation with upadacitinib 30mg. The frequency of Grade 3 neutrophil and lymphocytes decreases was higher for upadacitinib 30mg compared to other arms.

Hepatic disorder adverse events were reported in 3.8%, 15.6%, 9.1%, and 12.3% of the placebo, adalimumab, upadacitinib 15mg and 30mg groups, respectively. Most alanine aminotransferase (ALT) or aspartate aminotransferase (AST) increases were mild to moderate (Grade 2 or less). Grade 3 elevations in ALT occurred in 1.7%, 0.9%, 0.9%, and 1.2% with placebo, adalimumab, upadacitinib 15mg and 30mg, respectively. Grade 3 elevations in AST occurred in 0.5%, 0.2%, 0%, and 0.7% with placebo, adalimumab, and upadacitinib 15mg and 30mg, respectively. No Grade 4 elevations in ALT values were reported; 1 patient in the upadacitinib 30mg group had a Grade 4 increase in AST at a single time point. No Hy's law cases were reported. Grade 3 or 4 increases in creatine phosphokinase (CPK) values were more common with upadacitinib and were reported in <2% of patients; no patients experienced rhabdomyolysis.

Mean increases in low-density lipoprotein cholesterol (LDL-C) and high-density lipoprotein cholesterol (HDL-C) were observed with upadacitinib in comparison to placebo. LDL-C:HDL-C and total cholesterol:HDL-C ratios did not change through week 24.

**Upadacitinib as a Promising Oral Therapy for the Treatment of PsA**

A review of the placebo corrected data for upadacitinib and the JAK small molecule inhibitor Tofacitinib (approved for the lower (5 mg BID) dose in the treatment of PsA) for key primary and secondary endpoints, while not a head to head comparison, suggests that upadacitinib 15 mg QD and 30 mg QD shows decided promise for the more difficult to achieve endpoints of minimal disease activity (MDA), as well as psoriasis endpoints PASI 75 or PASI 90, as well as certain ACR components (e.g., ACR20/50/70). It was observed that this clinical efficacy achieved was sustained with continued daily dosing.

| Table O. Placebo Corrected Responses (% response / placebo response) | | | | | | | |
|---|---|---|---|---|---|---|---|
| **Endpoint** | **Upadacitinib H2H Adalimumab Select PsA1** | | | **Tofacitinib Opal Broaden** | | **Ixekizumamb Spirit P1** | |
| | **Upa 15 mg QD Week 12/16/24** | **Upa 30 mg QD Week 12/16/24** | **Adalimumab Week 12/24** | **Tofa 5 mg BID Week 12** | **Tofa 10 mg BID Week 12** | **IXE 80 mg Q4W Week 24** | **IXE 80 mg Q2W Week 24** |
| ACR20 | Week 12 34% (70.6 % / 36.2%, p ≤ 0.001) | Week 12 42% (78.5 % / 36.2%, p ≤ 0.001) | Week 12 29% (65 % / 36.2%) | 17% (50 % / 33%, p ≤ 0.05) | 28% (61 % / 33%, p ≤ 0.001) | 28% (57.9 % / 30.2%, p ≤ 0.001) | 32% (62.1 % / 30.2%, p ≤ 0.001) |
| ACR50 | Week 12 24% (37.5 % / 13.2%, p ≤ 0.001) | Week 12 39% (51.8 % / 13.2%, p ≤ 0.001) | Week 12 24% (37.5 % / 13.2%) | 18% (28 % / 10%, p ≤ 0.001) | 30% (40 % / 10%, p ≤ 0.001) | 25% (40.2 % / 15.1%, p ≤ 0.001) | 32% (46.6 % / 15.1%, p ≤ 0.001) |
| ACR70 | Week 12 13% (15.6 % / 2.4%, p ≤ 0.001) | Week 12 23% (25.3 % / 2.4%, p ≤ 0.001) | Week 12 11.4% (13.8 % / 2.4%) | 12% (17 % / 5%, p ≤ 0.01) | 9% (14 % / 5%, p ≤ 0.05) | 18% (23.4 % / 5.7%, p ≤ 0.001) | 28% (34.0 % / 5.7%, p ≤ 0.001) |
| MDA | Week 24 24% (36.6 % / 12.3%, p ≤ 0.001) | Week 24 33% (45.4 % / 12.3%, p ≤ 0.001) | Week 24 21% (33.3 % / 12.3%) | 19% (26 % / 7%) | 18% (25 % / 7%) | 15% (29.9 % / 15.1%, p ≤ 0.01) | 26% (40.8 % / 15.1%, p ≤ 0.01) |
| PASI 75 | Week 16 41% (62.6 % / 21.3%, p ≤ 0.001) | Week 16 41% (62.4 % / 21.3%, p ≤ 0.001) | Week 16 31.8% (53.1 % / 21.3%) | 28% (43 % / 15%, p ≤ 0.001) | 29% (44 % / 15%, p ≤ 0.001) | 61% (71.2 % / 10.4%, p ≤ 0.001) | 69% (79.9 % / 10.4%, p ≤ 0.001) |
| PASI 90 | Week 16 26% (38.3 % / 12.3%, p ≤ 0.001) | Week 16 37% (49.5 % / 12.3%, p ≤ 0.001) | Week 16 17.6% (38.9 % / 21.3%) | 17% (27% / 7%, p ≤ 0.01) | 27% (27% / 7%, p ≤ 0.001) | 50% (56.2% / 6%, p ≤ 0.001) | 52% (67.8% / 6%, p ≤ 0.001) |
| Tofacitinib study: Mease P et al. N Engl J Med 2017;377:1537-1550 (5 mg BID dose is approved in the United States for treatment of PsA; 10 mg BID dose is not approved in the United States); Ixekizumab study: Mease PJ et al. Ann Rheum Dis 2017;76:79-87. | | | | | | | |

**EXAMPLE 5**

*Phase 3 Protocol in Subjects with active Psoriatic Arthritis and a previous inadequate response to at least one biologic Disease Modifying Anti-Rheumatic Drug (bDMARD) (SELECT PSA2)*

The Phase 3 Study Design for SELECT-PSA2 is provided in **FIG. 12.**

15 mg or 30 mg upadacitinib refers to the 15 mg and 30 mg once-daily (QD) upadacitinib drug product as described in Example 1.

This is a Phase 3 multicenter study that includes two periods. Period 1 is 56 weeks in duration and includes a 24-week randomized, double-blind, parallel-group, placebo-controlled period followed by an additional 32 weeks of blinded treatment (Weeks 24 - 56). Period 1 is designed to compare the safety, tolerability, and efficacy of upadacitinib free base 15 mg QD and 30 mg QD versus placebo in subjects with moderately to severely active PsA who have an inadequate response to at least one Biological Disease Modifying Anti-Rheumatic Drug (bDMARD) (PsA Bio-IR). Period 2 is an open label (blinded until the last subject completes the last visit of Period 1) long-term extension of up to a total treatment duration of approximately 3 years to evaluate the safety, tolerability and efficacy of upadacitinib free base 15 mg QD, and 30 mg QD, in subjects with PsA who have completed Period 1.

The study duration includes a 35-day screening period; a 56-week blinded period which includes 24 weeks of double-blind, placebo-controlled treatment followed by 32 weeks of treatment blinded to dose of upadacitinib (Period 1); a long-term extension period of up to a total treatment duration of approximately 3 years ([blinded until the last subject completes the last visit of Period 1] Period 2); and a 30-day follow-up call or visit.

Subjects who met eligibility criteria were stratified by extent of psoriasis (>_ 3% body surface area [BSA] or < 3% BSA), current use of at least 1 DMARD (yes or no), and number of prior failed (had an inadequate response to) biologic DMARDs (1 vs > 1), except for subjects from Japan, for which randomization were stratified by extent of psoriasis (>_ 3% body surface area [BSA] or < 3% BSA) only. Subjects were randomized in a 2:2:1:1 ratio to one of four treatment groups:

Group 1: upadacitinib free base 15 mg QD (N = 211)
Group 2: upadacitinib free base 30 mg QD (N = 219)
Group 3: Placebo followed by upadacitinib free base 15 mg QD (N = 106)
Group 4: Placebo followed by upadacitinib free base 30 mg QD (N = 106)

No more than approximately 40% of subjects were enrolled with < 3% BSA extent of psoriasis and no more than approximately 30% of subjects were enrolled with prior failure of more than 1 biologic DMARD.

Subjects received oral study drug QD (upadacitinib free base 15 mg, upadacitinib free base 30 mg, or matching placebo) until the end of the study or until they discontinue study drug.

Subjects who were assigned to placebo at Baseline were preassigned to receiving either upadacitinib free base 15 mg QD or upadacitinib free base 30 mg QD starting at Week 24 in a 1:1 ratio. Subjects who completed the Week 56 visit (end of Period 1) entered the long-term extension portion of the study, Period 2 (total treatment up to approximately 3 years). Subjects continued study treatment as assigned in Period 1. Subjects continued to receive upadacitinib free base 15 mg QD or upadacitinib free base 30 mg QD, respectively, in a blinded manner until the last subject completed the last visit of Period 1 (Week 56), when study drug assignment in both periods will be unblinded to the sites, and subjects will be dispensed study drug in an open-label fashion until the completion of Period 2.

Subjects must have had inadequate response to ≥ 1 bDMARD prior to the Screening visit and must have discontinued all bDMARDs prior to the first dose of study drug. No background non-biologic DMARD therapy was required during participation in this study. For subjects who are on non-biologic DMARD therapy at baseline (methotrexate (MTX), sulfasalazine (SSZ), leflunomide (LEF), apremilast, hydroxychloroquine (HCQ), bucillamine or iguratimod), non-biologic DMARDs should have been started ≥ 12 weeks prior to baseline visit, must be at stable dose for ≥ 4 weeks prior to the first dose of study drug and remain at stable dose through Week 36 of the study; the non-biologic DMARD dose may be decreased only for safety reasons. In addition, all subjects taking MTX should take a dietary supplement of oral folic acid (or equivalent) throughout study participation.

At Week 16, rescue therapy will be offered to subjects classified as non-responders (defined as not achieving at least 20% improvement in either or both tender joint count (TJC) and swollen joint count (SJC) at both Week 12 and Week 16) as follows: 1) add or modify doses of non-biologic DMARDs, NSAIDs, acetaminophen/paracetamol, low potency opioid medications (tramadol or combination of acetaminophen and codeine or hydrocodone), oral corticosteroids and/or 2) receive 1 intra-articular, trigger point or tender point, intra-bursa, or intra-tendon sheath corticosteroid injection for 1 peripheral joint, 1 trigger point, 1 tender point, 1 bursa, or 1 enthesis (Rescue Therapy).

At Week 24, all subjects allocated to placebo at Baseline will be switched to blinded upadacitinib (randomized at baseline to either 15 mg QD or 30 mg QD) treatment regardless of clinical response.

After the last subject completes the Week 24 study visit, an unblinded analysis will be conducted for the purpose of initial regulatory submission. To maintain integrity of the trial during the blinded 56-week period study sites and subjects will remain blinded until all subjects have reached Week 56. A second unblinded analysis may be conducted for regulatory purposes after all subjects have completed Period 1. A final analysis will be conducted after all subjects have completed Period 2.

### Primary and Secondary Endpoints

The primary efficacy endpoint is the proportion of subjects achieving American College of Rheumatology (ACR) 20% response rate at Week 12. The primary and secondary clinical endpoints for the SELECT-PSA1 clinical study (Example 4) and this study (SELECT-PSA2 clinical study; Example 5) are further described in Example 6.

### Study Population

Patients with active psoriatic arthritis and a previous inadequate response to at least one biologic DMARD.

### Inclusion criteria include:

1. Adult male or female, at least ≥ 18 years old at Screening.
2. Clinical diagnosis of PsA with symptom onset at least 6 months prior to the Screening Visit and fulfillment of the Classification Criteria for PsA (CASPAR).
3. Subject has active disease at Baseline defined as ≥ 3 tender joints (based on 68 joint counts) and ≥ 3 swollen joints (based on 66 joint counts) at Screening and Baseline Visits.
4. Diagnosis of active plaque psoriasis or documented history of plaque psoriasis.
5. Subject has had an inadequate response (lack of efficacy after a minimum 12 week duration of therapy) or intolerance to treatment with at least 1 bDMARD.
6. Subjects must have discontinued all bDMARDs prior to the first dose of study drug. Subjects who need to discontinue bDMARDs prior to the Baseline Visit to comply with this inclusion criterion must follow the procedure specified below or at least five times the mean terminal elimination half-life of a drug:

- ≥ 4 weeks for etanercept;
- ≥ 8 weeks for adalimumab, infliximab, certolizumab, golimumab, abatacept, tocilizumab, and ixekizumab;
- ≥ 16 weeks for secukinumab;
- ≥ 12 weeks for ustekinumab;
- ≥ 1 year for rituximab OR ≥ 6 months if B cells have returned to pretreatment level or normal reference range (local lab) if pretreatment levels are not available.

7. Subject who is on current treatment with concomitant non-biologic DMARDs at study entry must be on ≤ 2 non-biologic DMARDs (except the combination of MTX and leflunomide) at the following doses: MTX (<_ 25 mg/week), SSZ (≤ 3000 mg/day), leflunomide (LEF) (<_ 20 mg/day), apremilast (<_ 60 mg/day), HCQ (<_ 400 mg/day), bucillamine (≤ 300 mg/day) and iguratimod (<_ 50 mg/day) for ≥ 12 weeks and at stable dose for ≥ 4 weeks prior to the Baseline Visit. No other DMARDs are permitted during the study.

- Subjects who need to discontinue DMARDs prior to the Baseline Visit to comply with this inclusion criterion must follow the procedure specified below or at least five times the mean terminal elimination half-life of a drug:

    ≥ 8 weeks for LEF if no elimination procedure was followed, or adhere to an elimination procedure (*i.e.*, 11 days with cholestyramine, or 30 days washout with activated charcoal or as per local label);
    ≥ 4 weeks for all others.

8. Stable doses of NSAIDs, acetaminophen/paracetamol, low-potency opiates (tramadol or combination of acetaminophen and codeine or hydrocodone), oral corticosteroids (equivalent to prednisone ≤ 10 mg/day), or inhaled corticosteroids for stable medical conditions are allowed, but must have been at a stable dose for ≥ 1 weeks prior to the Baseline Visit.
9. Subjects must have discontinued all opiates (except for tramadol or combination of acetaminophen and codeine or hydrocodone) at least 1 week and oral Traditional Chinese Medicine for at least 4 weeks prior to the first dose of study drug.

**Exclusion Criteria include:**

1. Prior exposure to any Janus Kinase (JAK) inhibitor (including but not limited to ruxolitinib, tofacitinib, baricitinib, and filgotinib)
2. Current treatment with > 2 non-biologic DMARDs or use of DMARDs other than MTX, SSZ, LEF, apremilast, HCQ, bucillamine or iguratimod or use of MTX in combination with LEF at Baseline
3. History of fibromyalgia, any arthritis with onset prior to age 17 years, or current diagnosis of inflammatory joint disease other than PsA (including, but not limited to rheumatoid arthritis, gout, overlap connective tissue diseases, scleroderma, polymyositis, dermatomyositis, systemic lupus erythematosus). Prior history of reactive arthritis or axial spondyloarthritis including ankylosing spondylitis and non-radiographic axial spondyloarthritis is permitted if documentation of change in diagnosis to PsA or additional diagnosis of PsA is made. Prior history of fibromyalgia is permitted if documentation of change in diagnosis to PsA or documentation that the diagnosis of fibromyalgia was made incorrectly.

**Analysis Windows**

For each protocol-specified study visit, a target study day will be identified to represent the corresponding visit along with a window around the target day. Windows will be selected in a non-overlapping fashion so that a collection date does not fall into multiple visit windows. If a subject has two or more actual visits in one visit window, the visit closest to the target day will be used for analysis. If two visits are equidistant from the target day, then the later visit will be used for analysis.

**Results up to and including** Week 24

As noted above, after the last subject completed the Week 24 study visit, an unblinded analysis was conducted for the purpose of initial regulatory submission.

A total of 642 subjects were randomized in this study, and 641 subjects received double-blind study treatment, out of which 591 (92.1%) completed study drug through Week 12, and 543 (84.6%) completed study drug through Week 24.

The study met all primary and key secondary endpoints, with upadacitinib 15mg QD and 30 mg QD demonstrating significantly greater efficacy in PsA compared with placebo. See below Tables T,U, and V1, as well as **FIGS. 14A-BB.** The time course of the primary endpoint ACR20 up to and including Week 24 is provided in **FIG. 14A,** and key secondary endpoints at ACR50, ACR70, and Proportion of Patients Achieving Minimal Disease Activity (MDA) Over 24 Weeks are provided in **FIGS. 14B-14D.** The time course of the change in baseline in Leeds Enthesitis Index (LEI) and subjects with resolution of enthesitis (LEI = 0) up to and including Week 24 is presented in **FIGS. 14E-14G.** The time course of the change in baseline in Leeds Dactylitis Index (LDI) and subjects with resolution of dactylitis (LDI = 0) up to and including Week 24 is presented in **FIGS. 14H-14I.** Data for other key secondary endpoints is provided in **FIG. 14J.**

All p-values were statistically significant for both upadacitinib doses as compared to placebo (graphical multiple testing procedure controlling the overall type I error rate at the 0.05 level).

The primary analysis showed a significantly higher response rate in both upadacitinib treatment groups as compared to the placebo group for the primary endpoint. At week 12, significantly more patients achieved an ACR20 response in the upadacitinib 15mg and 30mg arms versus the placebo arm (56.9%, 63.8%, and 24.1%, respectively; p<0.001 for both upadacitinib arms versus placebo). By week 2, ACR20 response was achieved by more upadacitinib 15mg- and 30mg-treated patients (nominal p<0.001). The proportion of patients with ACR20 response continued to increase over time in both treatment groups with the plateau of response observed at week 12 for the upadacitinib 30mg group, whereas the proportion of patients with ACR20 response in the upadacitinib 15mg group increased through week 20, approximating the response rate in the 30mg dose group by the end of the placebo-controlled period. Response rates for upadacitinib 15mg and upadacitinib 30mg were 44.9% and 64.8% in the subgroup of patients who had failed >1 biologic DMARD and 55.8% and 66.7% in the subgroup of patients that were on monotherapy; these responses were similar to results in the overall population. Additionally, improvements in ACR50 and ACR70 were observed with both upadacitinib doses versus placebo at week 12. From week 2 through week 24, improvement from baseline in all components of ACR response were observed with upadacitinib 15mg or 30mg versus placebo. The efficacy of upadacitinib freebase 15 mg was demonstrated regardless of subgroups evaluated including baseline BMI, baseline hsCRP, and number of prior non-biologic DMARDs (<_ 1 or >1).

The 15mg and 30mg doses of upadacitinib showed greater improvement versus placebo with respect to all key secondary endpoints. By week 12 and through week 24, improvement in psoriasis was observed with both upadacitinib doses versus placebo as measured by PASI 75/90/100 (at week 16, p<0.001 for PASI 75 and nominal p<0.001 for PASI 90/100; nominal p<0.001 for all the other time points) and sIGA 0/1 (p<0.001 at week 16; nominal p<0.001 for weeks 12 and 24). The changes from baseline in SAPS were greater for both upadacitinib arms versus placebo at weeks 16 (p<0.001) and 24 (nominal p<0.001). Improvements in physical function were observed in patients on both doses of upadacitinib versus placebo based on the mean change from baseline in HAQ-DI from week 2 through week 24 (p<0.001 at week 12) and SF-36 PCS at weeks 12 (p<0.001) and 24 (nominal p<0.001). The proportion of HAQ-DI responders (>_ 0.35 improvement from baseline in HAQ-DI score) at Week 12 was 45% in patients receiving upadacitinib freebase 15 mg QD and 27% in patients receiving placebo. Patients on both doses of upadacitinib reported improvements in fatigue as assessed by FACIT-F versus placebo at weeks 12 (p<0.001) and 24 (nominal p<0.001). Mean improvements from baseline in morning stiffness were observed at weeks 12 and 24 (nominal p<0.001). Resolution of enthesitis using both the LEI and the SPARCC enthesitis index and of dactylitis was reported in a higher proportion of patients on either dose of upadacitinib versus placebo from week 12 to week 24 (nominal p<0.001). A higher proportion of patients receiving either dose of upadacitinib achieved MDA through week 24 versus placebo (p<0.001 at week 24; nominal p<0.001 for weeks 12 and 16). Mean changes from baseline in the DAPSA score were greater with both upadacitinib doses versus placebo through week 24 (nominal p<0.001 for all time points).

Health-related quality of life was assessed by SF-36. Patients receiving upadacitinib freebase 15 mg QD experienced significantly greater improvement from baseline in the Physical Component Summary score compared to placebo at Week 12. Greater improvement was also observed in the Mental Component Summary score and all 8 domains of SF-36 (Physical Functioning, Bodily Pain, Vitality, Social Functioning, Role Physical, General Health, Role Emotional, and Mental Health) compared to placebo.

| Table P. Demographics and Characteristics at Baseline | | | |
|---|---|---|---|
| | Placebo N=212 | Upadacitinib 15mg QD N=211 | Upadacitinib 30mg QD N=218 |
| Female, n (%) | 120 (56.6) | 113 (53.6) | 115 (52.8) |
| Age (years) | 54.1 ± 11.5 | 53.0 ± 12.0 | 53.0 ± 11.9 |
| Race, n (%) | | | |
|     White | 186 (87.7) | 183 (86.7) | 196 (89.9) |
|     Black or African American | 7 (3.3) | 5 (2.4) | 5 (2.3) |
|     American Indian/Alaska Native | 0 | 3 (1.4) | 0 |
|     Native Hawaiian or other Pacific Islander | 1 (0.5) | 1 (0.5) | 1 (0.5) |
|     Asian | 17 (8.0) | 19 (9.0) | 16 (7.3) |
|     Multiple | 1 (0.5) | 0 | 0 |
| Duration of PsA symptoms (years) | 14.6 ± 11.7 | 12.2 ± 8.8 | 13.3 ± 10.8 |
| Duration since PsA diagnosis (years) | 11.0 ± 10.3 | 9.6 ± 8.4 | 9.7 ± 8.7 |
| Number of prior failed biologic DMARDs, n (%) | | | |
|     0a | 18 (8.5) | 16 (7.6) | 17 (7.8) |
|     1 | 135 (63.7) | 126 (59.7) | 130 (59.6) |
|     2 | 35 (16.5) | 35 (16.6) | 46 (21.1) |
|     ≥3 | 24(11.3) | 34 (16.1) | 25 (11.5) |
| Monotherapy, n (%) | 112 (52.8) | 113 (53.6) | 120 (55.0) |
| Any non-biologic DMARD at baseline, n (%) | | | |
|     MTX alone | 75 (35.4) | 74 (35.1) | 73 (33.5) |
|     MTX + another non-biologic DMARD | 7 (3.3) | 6 (2.8) | 5 (2.3) |

(continued)

| Table P. Demographics and Characteristics at Baseline | | | | |
|---|---|---|---|---|
| | | Placebo N=212 | Upadacitinib 15mg QD N=211 | Upadacitinib 30mg QD N=218 |
| | Non-biologic DMARD other than MTX | 18 (8.5) | 18 (8.5) | 20 (9.2) |
| MTX dose for patients with concomitant MTX alone at baseline (mg/week) | | | | |
| | Mean | 16.26 | 15.06 | 16.76 |
| | Median | 17.50 | 15.00 | 17.50 |
| Steroid use at baseline, n (%) | | 24 (11.3) | 22 (10.4) | 13 (6.0) |
| NSAID use at baseline, n (%) | | 125 (59.0) | 124 (58.8) | 129 (59.2) |
| RF status positive, n (%) | | 6 (2.8) | 11 (5.2) | 8 (3.7) |
| Anti-CCP status positive, n (%) | | 10 (4.7) | 7 (3.3) | 5 (2.3) |
| TJC68 | | $25.3 \pm 17.6$ | $24.9 \pm 17.3$ | $24.2 \pm 15.9$ |
| SJC66 | | $12.0 \pm 8.9$ | $11.3 \pm 8.2$ | $12.9 \pm 9.4$ |
| hs-CRP > ULN[b] (mg/L), n (%) | | 121 (57.1) | 126 (59.7) | 128 (58.7) |
| hs-CRP (mg/L) | | $10.4 \pm 18.5$ | $11.2 \pm 18.5$ | $10.5 \pm 17.2$ |
| HAQ-DI | | $1.23 \pm 0.7$ | $1.10 \pm 0.6$ | $1.19 \pm 0.7$ |
| Patient's assessment of pain (NRS 0-10) | | $6.6 \pm 2.1$ | $6.4 \pm 2.1$ | $6.2 \pm 2.2$ |
| BSA - psoriasis $\geq$ 3%, n (%) | | 131 (61.8) | 130 (61.6) | 131 (60.1) |
| | PASI (for baseline BSA-Ps $\geq$ 3%) | $11.7 \pm 11.4$ | $10.1 \pm 9.2$ | $8.9 \pm 9.1$ |
| BSA-psoriasis > 0%, n (%) | | 198 (93.4) | 202 (95.7) | 202 (92.7) |
| BSA-psoriasis (for baseline > 0%) | | $12.8 \pm 18.4$ | $10.0 \pm 15.7$ | $10.0 \pm 15.8$ |
| sIGA of psoriasis score, n (%) | | | | |
| | 0 | 17 (8.0) | 9(4.3) | 16 (7.3) |
| | 1 | 32 (15.1) | 31 (14.7) | 38 (17.4) |
| | 2 | 59 (27.8) | 82 (38.9) | 78 (35.8) |
| | 3 | 88 (41.5) | 78 (37.0) | 77 (35.3) |
| | 4 | 16 (7.5) | 11 (5.2) | 9 (4.1) |
| Presence of enthesitis | LEI > 0, n (%) | 144 (67.9) | 133 (63.0) | 152 (69.7) |
| | SPARCC Enthesitis Index > 0, n (%) | 173 (81.6) | 172 (81.5) | 179 (82.1) |
| Presence of dactylitis (defined as LDI > 0), n (%) | | 64 (30.2) | 55 (26.1) | 50 (22.9) |
| Morning stiffness score[c] | | $5.8 \pm 2.5$ | $6.0 \pm 2.5$ | $5.7 \pm 2.7$ |
| Values are mean $\pm$ SD unless noted. [a]Patients with intolerance but not inadequate response to a biologic DMARD. [b]ULN =2.87 mg/L; [c]Morning stiffness score is the mean of BASDAI questions 5 and 6; [d]Based on investigator opinion. | | | | |

(continued)

| Table P. Demographics and Characteristics at Baseline | | | |
|---|---|---|---|
| | Placebo N=212 | Upadacitinib 15mg QD N=211 | Upadacitinib 30mg QD N=218 |
| Anti-CCP, anti-cyclic citrullinated peptide; ASDAS, Ankylosing Spondylitis Disease Activity Score; BASDAI, Bath Ankylosing Spondylitis Disease Activity Index; BSA, body surface area; DMARD, disease-modifying anti-rheumatic drug; HAQ-DI, Health Assessment Questionnaire-Disability Index; hs-CRP, high-sensitivity C-reactive protein; LDI, Leeds Dactylitis Index; LEI, Leeds Enthesitis Index; MTX, methotrexate; NRS, numeric rating scale; NSAID, nonsteroidal anti-inflammatory drug; PASI, Psoriasis Area Severity Index; Ps, psoriasis; PsA, psoriatic arthritis; QD, once daily; RF, rheumatoid factor; ULN, upper limit normal; SD, standard deviation; sIGA, Static Investigator Global Assessment; SJC, swollen joint count; SPARCC, Spondyloarthritis Research Consortium of Canada; TJC, tender joint count. | | | |

| Table Q. Primary and Key Secondary Efficacy Endpoints | | | | | |
|---|---|---|---|---|---|
| Summary Statistics % or LS Mean (P-Value) | | Endpoint[a] | PLACEBO N = 212 | UPA 15 MG QD N = 211 | UPA 30 MG QD N = 218 |
| Primary | | ACR20 (Wk12) | 24.1% | 56.9% (<0.0001*) | 63.8% (<0.0001*) |
| Ranked Key Secondary | 1. | HAQ-DI (Wk12) | -0.10 | -0.30 (<0.0001*) | -0.41 (<0.0001*) |
| | 2. | sIGA (Wk16)[b] | 9.2% | 36.8% (<0.0001*) | 40.2% (<0.0001*) |
| | 3. | PASI 75 (Wk16)[c] | 16.0% | 52.3% (<0.0001*) | 56.5% (<0.0001*) |
| | 4. | SF-36 PCS (Wk12) | 1.62 | 5.2 (<0.0001*) | 7.1 (<0.0001*) |
| | 5. | FACIT-F (Wk12) | 1.3 | 5.0 (<0.0001*) | 6.1 (<0.0001 *) |
| | 6. | MDA (Wk24) | 2.8% | 25.1% (<0.0001*) | 28.9% (<0.0001*) |
| | 7. | SAPS (Wk16) | -1.5 | -24.4 (<0.0001*) | -29.7 (<0.0001*) |
| Other Key Secondary | | ACR50 (Wk12) | 4.7% | 31.8% (<0.0001) | 37.6% (<0.0001) |
| | | ACR70 (Wk12) | 0.5% | 8.5% (<0.0001) | 16.5% (<0.0001) |
| | | ACR20 (Wk2) | 10.8% | 32.7% (<0.0001) | 33.5% (<0.0001) |
| Note: The nominal p-values are provided in parentheses. * Statistical significance using graphical multiple testing procedure controlling overall type I error rate at the 0.05 level. | | | | | |

a. Results for binary endpoints are based on NRI (net reclassification improvement) analysis. Results for minimal disease activity (MDA) at week 24 are based on non-responder imputation with additional rescue handling, where subjects rescued at Week 16 are imputed as non-responders. Results for continuous endpoints are based on mixed model for repeated measures (MMRM) model with fixed effects of treatment, visit, treatment-by-visit interaction, the stratification factor of current DMARD use (yes/no) and baseline measurement.

b. Summarized for subjects with baseline sIGA ≥ 2.

c. Summarized for subjects with baseline BSA affected by psoriasis ≥ 3%.

| Table R. Additional Efficacy Endpoints for SELECT-PSA2: Summarized for subjects with baseline BSA affected by psoriasis ≥ 3%[a] | | | |
|---|---|---|---|
| Endpoint | PLACEBO N = 131 | UPA 15 MG QD N = 130 | UPA 30 MG QD N = 131 |
| PASI 90 (Wk16) | 8.4% | 34.6% (<0.0001) | 45.0% (<0.0001) |
| PASI 100 (WK16) | 6.1% | 25.4% (<0.0001) | 32.1% (<0.0001) |

a. Full analysis set: N(PBO) = 212, N(UPA15) = 211, N(UPA30) = 218

| Table S. Exploratory endpoints | | | |
|---|---|---|---|
| Endpoint | PLACEBO | UPA 15 MG QD | UPA 30 MG QD |
| Resolution of enthesitis at Week 12 (defined as LEI = 0) | 20.1% (N = 144) | 39.1% (<0.0001) (N = 133) | 48.0% (<0.0001) (N = 152) |
| Resolution of dactylitis at Week 12 (defined as LDI = 0) | 35.9% (N = 64) | 63.6% (<0.0001) (N = 55) | 76.0% (<0.0001) (N = 50) |

Additional data for ACR20, ACR50, and ACR70 response at Week 24, MDA at Week 12, and resolution of enthesitis (LEI = 0), and resolution of dactylitis (LDI = 0) at Week 24 is set forth in the below Table.

| Table T. Additional Clinical Responses | | |
|---|---|---|
| Endpoint | Placebo (N = 212) % | UPA 15 mg QD (N = 211) % |
| ACR20 (Wk 24) | 20 | 59 |
| ACR50 (Wk 24) | 9 | 38 |
| ACR70 (Wk 24) | 1 | 19 |
| MDA (Wk 12) | 4 | 17 |
| Enthesitis resolution[a] (Wk 24) | 15 | 43 |
| Dactylitis resolution[b] (Wk 24) | 28 | 58 |
| Patients who discontinued randomized treatment or were missing data at week of evaluation were imputed as non-responders in the analyses. For MDA, resolution of enthesitis, and resolution of dactylitis at Week 24, the subjects rescued at Week 16 were imputed as non-responders in the analyses. [a] In patients with enthesitis at baseline (n=144 and 133, respectively) [b] In patients with dactylitis at baseline (n=64 and 55) | | |

Treatment with upadacitinib freebase 15 mg QD resulted in improvements in individual ACR components, including tender/painful and swollen joint counts, patient and physician global assessments of disease activity, HAQ-DI, pain assessment, and hsCRP compared to placebo. These results are summarized in **FIGs. 14N-14S** and **14V.** Onset of efficacy was seen as early as Week 2 for ACR20 and its components.

Through week 24, the rate of overall treatment-emergent AEs (TEAEs) was higher in the upadacitinib 30mg arm and rates of serious AEs (SAEs) and TEAEs leading to discontinuation of trial drug were higher with both upadacitinib doses versus placebo.

The most commonly reported TEAEs were upper respiratory tract infection and nasopharyngitis in upadacitinib-treated patients. SAEs were reported in four (1.9%) patients on placebo, twelve (5.7%) on upadacitinib 15mg, and eighteen (8.3%) on upadacitinib 30mg. Serious infections occurred in one patient each (0.5%) on placebo and upadacitinib 15mg and six (2.8%) patients on upadacitinib 30mg. Pneumonia was the most frequently reported serious infection (one patient on upadacitinib 15mg and three patients on upadacitinib 30mg). Up to week 24, treatment-emergent opportunistic infections, excluding tuberculosis and herpes zoster, included one event each of candidiasis of the trachea and oropharyngeal candidiasis, both with upadacitinib 30mg. Herpes zoster was reported in two, three, and eight patients in the placebo, upadacitinib 15mg and 30mg arms, respectively; none of the cases were serious. One patient on upadacitinib 15mg and two patients on upadacitinib 30mg had cutaneous disseminated herpes zoster. No cases of herpes zoster with central nervous system involvement were observed. Hepatic disorders were reported in three (1.4%) patients on placebo, four (1.9%) on upadacitinib 15mg, and eighteen (8.3%) on upadacitinib 30mg; most were asymptomatic liver enzyme elevations.

Malignancies were reported in three patients in each upadacitinib arm (upadacitinib 15mg: one basal cell carcinoma, one prostate cancer, one rectal cancer; upadacitinib 30mg: one rectal adenocarcinoma, one ovarian and endometrial cancer, and one basal cell carcinoma) and none in the placebo arm. The time to event onset for these malignant events was <6 months.

There were no adjudicated gastrointestinal perforations reported through week 24. One case of major adverse cardiovascular event (MACE; 0.5%, non-fatal myocardial infarction) and one case of venous thromboembolic event (VTE; 0.5%; pulmonary embolism) were reported in the upadacitinib 15mg arm; both patients had at least one risk factor (e.g., obesity, hypertension, or hypercholesterolemia) for MACE or VTE, respectively. Over the 24-week period, one death was reported in the placebo arm related to a motor vehicle accident.

Generally, mean hemoglobin, neutrophil, lymphocyte, and platelet levels remained within normal limits from baseline through week 24 in all treatment arms. There were two patients with Grade 3 decreases in hemoglobin values in the upadacitinib 30mg arm. Grade 3 decreases in neutrophils were reported in one patient on placebo (0.5%), two patients on upadacitinib 15mg (1.0%), and four patients on upadacitinib 30mg (1.8%). No patients had Grade 4 decreases in platelets, leukocytes, neutrophils, or lymphocytes.

Isolated Grade 3 increases in alanine aminotransferase or aspartate aminotransferase were observed in $\leq 1\%$ of the patients among the treatment arms, and no Grade 4 increases were observed. No Hy's law cases were reported. Grade 3 increases in CPK values were reported in one (0.5%), one (0.5%), and five (2.3%) patients in the placebo, and upadacitinib 15mg and 30mg arms, respectively. Grade 4 increases in CPK values were reported in two patients with placebo and one patient with upadacitinib 15mg. None led to discontinuation of trial drug and there were no events of rhabdomyolysis. Slight mean elevations in low-density lipoprotein cholesterol (LDL-C) and high-density lipoprotein cholesterol (HDL-C) were observed in the upadacitinib arms versus the placebo arm. The ratios of LDL-C:HDL-C and Total cholesterol: HDL-C generally remained constant through week 24.

### Upadacitinib as a Promising Therapy for the Treatment of PsA

A review of the placebo corrected data for upadacitinib and the JAK small molecule inhibitor Tofacitinib (approved for the lower (5 mg BID) dose in the treatment of PsA) for key primary and secondary endpoints, while not a head to head comparison, suggests that upadacitinib 15 mg QD and 30 mg QD shows decided promise for the more difficult to achieve endpoints of minimal disease activity (MDA), as well as psoriasis endpoints PASI 75 or PASI 90, as well as certain ACR components (e.g., ACR20/50/70). Furthermore, it was observed that this efficacy, once achieved, was sustained or improved over time.

| Table U. Placebo Corrected Responses (% response / placebo response) | | | | | | |
|---|---|---|---|---|---|
| Endpoint | Upadacitinib Select PsA2 | | Tofacitinib Opal Beyond | | Ixekizumamb Spirit P2 | |
| | Upa 15 mg QD Week 12/16/24 | Upa 30 mg QD Week 12/16/24 | Tofa 5 mg BID Week 12 | Tofa 10 mg BID Week 12 | IXE 80 mg Q4W Week 24 | IXE 80 mg Q2W Week 24 |
| ACR20 | Week 12 33% (56.9 % / 24.1%, p ≤ 0.001) | Week 12 40% (63.8 % / 24.1 %, p ≤ 0.001) | 26% (50 % / 24 %, p ≤ 0.001) | 23% (47 % / 24 %, p ≤ 0.001) | 34% (53 % / 19%, p ≤ 0.001) | 29% (48 % / 19%, p ≤ 0.001) |

(continued)

| Table U. Placebo Corrected Responses (% response / placebo response) | | | | | | |
|---|---|---|---|---|---|---|
| Endpoint | Upadacitinib Select PsA2 | | Tofacitinib Opal Beyond | | Ixekizumamb Spirit P2 | |
| | Upa 15 mg QD Week 12/16/24 | Upa 30 mg QD Week 12/16/24 | Tofa 5 mg BID Week 12 | Tofa 10 mg BID Week 12 | IXE 80 mg Q4W Week 24 | IXE 80 mg Q2W Week 24 |
| ACR50 | Week 12 27% (31.8 % / 4.7%, p ≤ 0.001) | Week 12 37.6% (37.6 % / 4.7%, p ≤ 0.001) | 15% (30 % / 15 %, p ≤ 0.01) | 13% (28 % / 15 %, p ≤ 0.01) | 30% (35 % / 5%, p ≤ 0.001) | 28% (33 % / 5%, p ≤ 0.001) |
| ACR70 | Week 12 8% (8.5 % / 0.5%, p ≤ 0.001) | Week 12 16% (16.5 % / 0.5%, p ≤ 0.001) | 7% (17 % / 10%) | 4% (14 % / 10 %) | 22% (22 % / 0%, p ≤ 0.001) | 12% (12 % / 0%, p ≤ 0.001) |
| PASI 75 | Week 16 36% (52 % / 16%, p ≤ 0.001) | Week 16 41% (57 % / 16%, p ≤ 0.001) | 7% (21 % / 14%) | 29% (43 % / 14%) | 41% (56 % / 15%, p ≤ 0.001) | 45% (60 % / 15%, p ≤ 0.001) |
| MDA | Week 24 22% (25 % / 3%, p ≤ 0.001) | Week 24 26% (29 % / 3%, p ≤ 0.001) | 8% (23 % / 15%) | 6% (21 % / 15%) | 25% (28 % / 3%, p ≤ 0.001) | 21% (24 % / 3%, p ≤ 0.001) |
| Tofacitinib study: Gladman D et al. N Engl J Med 2017;377:1525-1536 (5 mg BID dose is approved in the United States for treatment of PsA; 10 mg BID dose is not approved in the United States); Ixekizumab study: Nash P et al. Lancet 2017;389:2317-2327. | | | | | | |

## EXAMPLE 6

*SELECT-PSA1 (Example 4) and SELECT-PSA2 (Example 5) Clinical Endpoints*

### Primary Endpoint for PsAl and PsA2 Studies

The primary efficacy endpoint is the proportion of subjects achieving American College of Rheumatology (ACR) 20% response rate at Week 12. ACR20 response rate was determined based on a 20% or greater improvement in tender and swollen joint counts (TJC and SJC) and ≥ 3 of the 5 measures of Patient's Global Assessment of Pain (Pt Pain) Numerical Rating Scale (NRS), Patient's Global Assessment (PtGA)-Disease Activity Numerical Rating Scale (NRS), Physician Global Assessment (PGA)-Disease Activity Numerical Rating Scale (NRS), Health Assessment Questionnaire - Disability Index (HAQ-DI), or High-Sensitivity C Reactive Protein (hs-CRP).

### Key Secondary Endpoints for PsAl and PsA2 Studies

The key multiplicity adjusted secondary efficacy endpoints (each dose of upadacitinib versus placebo unless noted) are:

| Table V. | | |
|---|---|---|
| | PsA-1 Study Key Secondary Endpoints | PsA-2 Study Key Secondary Endpoints |
| 1 | Change from baseline in Health Assessment Questionnaire - Disability Index (HAQ-DI) at Week 12; | Change from baseline in Health Assessment Questionnaire - Disability Index (HAQ-DI) at Week 12; |

(continued)

| Table V. | | |
| --- | --- | --- |
| | **PsA-1 Study Key Secondary Endpoints** | **PsA-2 Study Key Secondary Endpoints** |
| 2 | Proportion of subjects achieving a static Investigator Global Assessment (sIGA) of Psoriasis of 0 or 1 and at least a 2-point improvement from baseline at Week 16 (for subjects with baseline sIGA≥2); | Proportion of subjects achieving a static Investigator Global Assessment (sIGA) of Psoriasis of 0 or 1 and at least a 2-point improvement from baseline at Week 16 (for subjects with baseline sIGA≥2); |
| 3 | Psoriasis Area Severity Index (PASI) 75 response at Week 16 (for subjects with ≥ 3% Body Surface Area (BSA) psoriasis at baseline); | Psoriasis Area Severity Index (PASI) 75 response at Week 16 (for subjects with ≥ 3% Body Surface Area (BSA) psoriasis at baseline); |
| 4 | Change from baseline in Sharp/van der Heijde Score (SHS) at Week 24; | |
| 5 | Proportion of subjects achieving MDA (Minimal Disease Activity) at Week 24; | Proportion of subjects achieving MDA (Minimal Disease Activity) at Week 24; |
| 6 | Change from baseline in Leeds Enthesitis Index (LEI) at Week 24; | |
| 7 | Change from baseline in Leeds Dactylitis Index (LDI) at Week 24; | |
| 8 | American College of Rheumatology (ACR) 20 response rate at Week 12 (non-inferiority of upadacitinib vs adalimumab); | |
| 9 | Change from baseline in 36-Item Short Form Health Survey (SF-36) at Week 12; | Change from baseline in 36-Item Short Form Health Survey (SF-36) at Week 12; |
| 10 | American College of Rheumatology (ACR) 20 response rate at Week 12 (superiority of upadacitinib vs. adalimumab); | |
| 11 | Change from baseline in Patient's Global Assessment of Pain Numerical Rating Scale (NRS) at Week 12 (superiority of upadacitinib vs. adalimumab); | |
| 12 | Change from baseline in Health Assessment Questionnaire - Disability Index (HAQ-DI) at Week 12 (superiority of upadacitinib vs. adalimumab); | |
| 13 | Change from baseline in Functional Assessment of Chronic Illness Therapy-Fatigue (FACIT-Fatigue) Questionnaire at Week 12; and | Change from baseline in Functional Assessment of Chronic Illness Therapy-Fatigue (FACIT-Fatigue) Questionnaire at Week 12; and |
| 14 | Change from baseline in Self-Assessment of Psoriasis Symptoms (SAPS) Questionnaire at Week 16. | Change from baseline in Self-Assessment of Psoriasis Symptoms (SAPS) Questionnaire at Week 16 |

Additional key secondary efficacy endpoints (each dose of upadacitinib versus placebo) are:

| Table W. | | |
| --- | --- | --- |
| | **PsA-1 Study Additional Key Secondary Endpoints** | **PsA-2 Study Additional Key Secondary Endpoints** |
| 1 | ACR50/70 response at Week 12; | ACR50/70 response at Week 12; |
| 2 | ACR20 response at Week 2 | ACR20 response at Week 2 |

The proportion of subjects achieving Minimal Disease Activity (MDA) are determined based on subjects fulfilling 5 of 7 outcome measures: TJC ≤ 1; SJC ≤ 1; PASI ≤ 1 or BSA-Ps ≤ 3%; Patient's Global Assessment of Pain (Pt Pain)

NRS $\leq$ 1.5 (0-10 NRS); PtGA-Disease Activity NRS $\leq$ 2 (0-10 NRS); HAQ-DI score $\leq$ 0.5; and LEI $\leq$ 1. See, *e.g.,* Coates LC, Kavanaugh A, Mease PJ, et al; Group for Research and Assessment of Psoriasis and Psoriatic Arthritis (GRAPPA) 2015 treatment recommendations for psoriatic arthritis. Arthritis Rheumatol. 2016;68(5):1060-71.

ACR20/50/70 response rates are determined based on 20%/50%/70% or greater improvement in TJC and SJC and $\geq$ 3 of the 5 measures of Patient's Global Assessment of Pain (Pt Pain) NRS, PtGA-Disease Activity NRS, PGA-Disease Activity NRS, HAQ-DI, or hs-CRP.

**Additional Secondary Endpoints for PsAl and PsA2 Studies**

The following outcome measures were assessed in subjects treated with upadacitinib as compared to placebo and adalimumab at scheduled time points other than those specified for the primary and key secondary variables:

**Table X.**

| | **PsA-1 Study Additional Secondary Endpoints** | **PsA-2 Study Additional Secondary Endpoints** |
|---|---|---|
| 1 | Proportion of subjects with no radiographic progression (defined as change from baseline in SHS $\leq$ 0); | |
| 2 | Change from baseline in individual components of ACR response: (i) Change from baseline in Tender Joint Count (TJC) (0 - 68); (ii) Change from baseline in Swollen Joint Count (SJC) (0 - 66); (iii) Change from baseline in Physician Global Assessment (PGA) - Disease Activity Numerical Rating Scale (NRS); (iv) Change from baseline in Patient's Global Assessment (PtGA)-Disease Activity Numerical Rating Scale (NRS); (v) Change from baseline in Patient's Global Assessment of Pain Numerical Rating Scale (NRS); (vi) Change from baseline in Health Assessment Questionnaire - Disability Index (HAQ-DI); (vii) Change from baseline in High-Sensitivity C Reactive Protein (hs-CRP); | Change from baseline in individual components of ACR response: (i) Change from baseline in Tender Joint Count (TJC) (0 - 68); (ii) Change from baseline in Swollen Joint Count (SJC) (0 - 66); (iii) Change from baseline in Physician Global Assessment (PGA) - Disease Activity Numerical Rating Scale (NRS); (iv) Change from baseline in Patient's Global Assessment (PtGA)-Disease Activity Numerical Rating Scale (NRS); (v) Change from baseline in Patient's Global Assessment of Pain Numerical Rating Scale (NRS); (vi) Change from baseline in Health Assessment Questionnaire - Disability Index (HAQ-DI); (vii) Change from baseline in High-Sensitivity C Reactive Protein (hs-CRP); |
| 3 | ACR 20/50/70 response rates; | ACR 20/50/70 response rates; |
| 4 | Change from baseline in Leeds Dactylitis Index (LDI); | Change from baseline in Leeds Dactylitis Index (LDI); |
| 5 | Change from baseline in dactylitis count; | Change from baseline in dactylitis count; |
| 6 | Proportion of subjects with resolution of dactylitis; | Proportion of subjects with resolution of dactylitis; |
| 7 | Change from baseline in Leeds Enthesitis Index (LEI); | Change from baseline in Leeds Enthesitis Index (LEI); |
| 8 | Proportion of subjects with resolution of enthesitis sites included in the Leeds Enthesitis Index (LEI); | Proportion of subjects with resolution of enthesitis sites included in the Leeds Enthesitis Index (LEI); |
| 9 | Change from baseline in Spondyloarthritis Research Consortium of Canada (SPARCC) Enthesitis Index; | Change from baseline in Spondyloarthritis Research Consortium of Canada (SPARCC) Enthesitis Index; |
| 10 | Proportion of subjects with resolution of enthesitis sites included in the Spondyloarthritis Research Consortium of Canada (SPARCC) Enthesitis Index; | Proportion of subjects with resolution of enthesitis sites included in the Spondyloarthritis Research Consortium of Canada (SPARCC) Enthesitis Index; |
| 11 | Change from baseline in total enthesitis count; | Change from baseline in total enthesitis count; |
| 12 | Proportion of subjects with resolution of enthesitis; | Proportion of subjects with resolution of enthesitis; |
| 13 | PASI 75/90/100 response rates (for subjects with $\geq$ 3% Body Surface Area (BSA) psoriasis at baseline); | PASI 75/90/100 response rates (for subjects with $\geq$ 3% Body Surface Area (BSA) psoriasis at baseline); |
| 14 | Proportion of subjects achieving a static Investigator Global Assessment of Psoriasis (sIGA) score of 0 or 1 and at least a 2-point improvement from baseline; | Proportion of subjects achieving a static Investigator Global Assessment of Psoriasis (sIGA) score of 0 or 1 and at least a 2-point improvement from baseline; |

(continued)

| Table X. | | |
|---|---|---|
| | **PsA-1 Study Additional Secondary Endpoints** | **PsA-2 Study Additional Secondary Endpoints** |
| 15 | BSA-Ps; | BSA-Ps; |
| 16 | Change from baseline in Modified Psoriatic Arthritis Response Criteria (PsARC); | Change from baseline in Modified Psoriatic Arthritis Response Criteria (PsARC); |
| 17 | Change from baseline in Disease Activity Score 28 (DAS28) (CRP); | Change from baseline in Disease Activity Score 28 (DAS28) (CRP); |
| 18 | Change from baseline in DAS28 (ESR); | Change from baseline in DAS28 (ESR); |
| 19 | Change from baseline in PsA Disease Activity Score (PASDAS); | Change from baseline in PsA Disease Activity Score (PASDAS); |
| 20 | Change from baseline in Disease Activity In Psoriatic Arthritis (DAPSA) score; | Change from baseline in Disease Activity In Psoriatic Arthritis (DAPSA) score; |
| 21 | Change from baseline in Short Form 36 (SF-36) Health Questionnaire; | Change from baseline in Short Form 36 (SF-36) Health Questionnaire; |
| 22 | Change from baseline in Functional Assessment of Chronic Illness Therapy (FACIT)-Fatigue Questionnaire; | Change from baseline in Functional Assessment of Chronic Illness Therapy (FACIT)-Fatigue Questionnaire; |
| 23 | Change from baseline in EuroQol-5-Dimensions-5-Levels (EQ-5D-5L) Questionnaire; | Change from baseline in EuroQol-5-Dimensions-5-Levels (EQ-5D-5L) Questionnaire; |
| 24 | Change from baseline in Work Productivity and Activity Impairment (WPAI) Questionnaire; | Change from baseline in Work Productivity and Activity Impairment (WPAI) Questionnaire; |
| 25 | Change from baseline in Health Resource Utilization (HRU) Questionnaire; | Change from baseline in Health Resource Utilization (HRU) Questionnaire; |
| 26 | Change from baseline in Self-Assessment of Psoriasis Symptoms (SAPS) Questionnaire; | Change from baseline in Self-Assessment of Psoriasis Symptoms (SAPS) Questionnaire; |
| 27 | Change from baseline in Bath Ankylosing Spondylitis Disease Activity Index (BASDAI); | Change from baseline in Bath Ankylosing Spondylitis Disease Activity Index (BASDAI); |
| 28 | Bath Ankylosing Spondylitis Disease Activity Index (BASDAI) 50 response rates; | Bath Ankylosing Spondylitis Disease Activity Index (BASDAI) 50 response rates; |
| 29 | Change from baseline in Morning stiffness (mean of BASDAI Questions 5 and 6); | Change from baseline in Morning stiffness (mean of BASDAI Questions 5 and 6); |
| 30 | Change from baseline in Ankylosing Spondylitis Disease Activity Score (ASDAS); | Change from baseline in Ankylosing Spondylitis Disease Activity Score (ASDAS); |
| 21 | Proportion of subjects with Ankylosing Spondylitis Disease Activity Score (ASDAS) Inactive Disease; | Proportion of subjects with Ankylosing Spondylitis Disease Activity Score (ASDAS) Inactive Disease; |
| 32 | Proportion of subjects with Ankylosing Spondylitis Disease Activity Score (ASDAS) Major Improvement; | Proportion of subjects with Ankylosing Spondylitis Disease Activity Score (ASDAS) Major Improvement; |
| 33 | Proportion of subjects with Ankylosing Spondylitis Disease Activity Score (ASDAS) Clinically Important Improvement; | Proportion of subjects with Ankylosing Spondylitis Disease Activity Score (ASDAS) Clinically Important Improvement; |
| 34 | Proportion of subjects achieving a clinically meaningful improvement in Health Assessment Questionnaire - Disability Index (HAQ-DI) (>_ 0.35). | Proportion of subjects achieving a clinically meaningful improvement in Health Assessment Questionnaire - Disability Index (HAQ-DI) (>_ 0.35). |

OTHER EMBODIMENTS

This application refers to various issued patent, published patent applications, journal articles, and other publications, each of which is incorporated herein by reference.

The foregoing has been described of certain non-limiting embodiments of the present disclosure. Those of ordinary skill in the art will appreciate that various changes and modifications to this description may be made without departing from the spirit or scope of the present disclosure, as defined in the following claims.

The present invention is now described with reference to the following clauses:

1. A method of treating active ankylosing spondylitis in a subject in need thereof, the method comprising orally administering to the subject once a day for at least 14 weeks a dose of upadacitinib freebase, or a pharmaceutically acceptable salt thereof, in an amount sufficient to deliver 15 mg of upadacitinib freebase equivalent, wherein the subject achieves an Assessment of SpondyloArthritis International Society 40 (ASAS40) response within 14 weeks of administration of the first dose.

2. The method of clause 1, wherein when the method is used to treat a population of subjects, at least 10% of the subjects in the treated population achieve an ASAS40 response within 14 weeks of administration of the first dose.

3. The method of clauses 1 or 2, wherein the subject or subjects in the treated population further achieve within 14 weeks of administration of the first dose at least one result selected from the group consisting of:

   a. improvement from baseline in Ankylosing Spondylitis Disease Activity Score (ASDAS);
   b. improvement from baseline in magnetic resonance imaging (MRI) Spondyloarthritis Research Consortium of Canada (SPARCC) score for spine (MRI-Spine SPARCC);
   c. ASAS partial remission (PR);
   d. Bath Ankylosing Spondylitis Disease Activity Index 50 (BASDAI50) response;
   e. improvement from baseline in Bath Ankylosing Spondylitis Functional Index (BASFI);
   f. ASDAS low disease activity (LDA);
   g. ASDAS inactive disease (ID);
   h. ASDAS major improvement (MI); and
   i. ASDAS clinically important improvement (CII).

4. The method of clause 3, wherein the subject or subjects in the treated population further achieve within 14 weeks of administration of the first dose each result.

5. The method of any one of clauses 1-4, wherein the subject or subjects in the treated population fulfill the 1984 modified New York Criteria for ankylosing spondylitis at baseline.

6. The method of any one of clauses 1-4, wherein the subject or subjects in the treated population fulfill the 2009 ASAS classification criteria at baseline.

7. The method of any one of clauses 1-6, wherein the subject or subjects in the treated population meet at least one criteria at baseline selected from the group consisting of:

   a. a Bath Ankylosing Spondylitis Disease Activity Index (BASDAI) score ≥4;
   b. an Ankylosing Spondylitis Disease Activity Score (ASDAS) of ≥2.1; and
   c. a Patient's Assessment of Total Back Pain (Total Back Pain score) of ≥4 based on a 0-10 numerical rating scale.

8. The method of any one of clauses 1-7, wherein the subject or subjects in the treated population are biologic disease-modifying anti-rheumatic drug (bDMARD) naive at baseline.

9. The method of any one of clauses 1-7, wherein the subject or subjects in the treated population have had an inadequate response or intolerance to a biologic disease-modifying anti-rheumatic drug (bDMARD) at baseline.

10. The method of clause 9, wherein prior to administration of the first dose, the subject or subjects in the treated population have been administered one bDMARD, and discontinued use of the bDMARD due to intolerance or lack of efficacy at baseline.

11. The method of clause 10, wherein the bDMARD is a tumor necrosis factor (TNF) inhibitor or an interleukin (IL)-17 inhibitor.

12. The method of any one of clauses 1-11, wherein the subject or subjects in the treated population have had an inadequate response or intolerance to at least two NSAIDs, intolerance to NSAIDS, and/or contraindication for NSAIDs at baseline.

13. A method of treating active non-radiographic axial spondyloarthritis in a subject in need thereof, the method comprising orally administering to the subject once a day for at least 14 weeks a dose of upadacitinib freebase, or a pharmaceutically acceptable salt thereof, in an amount sufficient to deliver 15 mg of upadacitinib freebase equivalent, wherein the subject achieves an ASAS40 response within 14 weeks of administration of the first dose.

14. A method of treating active non-radiographic axial spondyloarthritis in a subject in need thereof, the method comprising orally administering to the subject once a day for at least 52 weeks a dose of upadacitinib freebase, or a pharmaceutically acceptable salt thereof, in an amount sufficient to deliver 15 mg of upadacitinib freebase equivalent, wherein the subject achieves an ASAS40 response within 52 weeks of administration of the first dose.

15. The method of clauses 13 or 14, wherein the subject fulfills at baseline the 2009 ASAS classification criteria for axial spondyloarthritis, but does not meet the radiologic criteria of the 1984 modified New York criteria for ankylosing spondylitis.

16. The method of clauses 13 or 14, wherein the subject meets at least one criteria at baseline selected from the group consisting of:

a. Bath Ankylosing Spondylitis Disease Activity Index (BASDAI) score of $\geq 4$;
b. an Ankylosing Spondylitis Disease Activity Score (ASDAS) of $\geq 2.1$;
c. a Patient's Assessment of Total Back Pain (Total Back Pain score) of $\geq 4$ based on a 0 - 10 numerical rating scale; and
d. an objective sign of inflammatory activity selected from the group consisting of:

i. an objective sign of active inflammation on MRI of sacroiliac (SI) joints, and
ii. high-sensitivity C reactive protein > upper limit of normal (ULN).

17. The method of any one of clauses 13-16, wherein the subject is bDMARD naive at baseline.

18. The method of any one of clauses 13-16, wherein the subject has had an inadequate response or intolerance to a bDMARD at baseline.

19. The method of clause 18, wherein prior to administration of the first dose, the subject has been administered one bDMARD, and discontinued use of the bDMARD due to intolerance or lack of efficacy at baseline.

20. The method of clause 19, wherein the bDMARD is a tumor necrosis factor (TNF) inhibitor or an interleukin (IL)-17 inhibitor.

21. The method of any one of clause 13-20, wherein the subject has had an inadequate response or intolerance to at least 2 NSAIDs, has an intolerance to NSAIDS, and/or has a contraindication for NSAIDs at baseline.

22. The method of any one of clauses 13-20, wherein the subject achieves within 14 weeks of administration of the first dose at least one additional result selected from the group consisting of:

a. improvement from baseline in Ankylosing Spondylitis Disease Activity Score (ASDAS);
b. improvement from baseline in magnetic resonance imaging (MRI) Spondyloarthritis Research Consortium of Canada (SPARCC) score for SI joints (MRI-SIjoints SPARCC);
c. ASAS partial remission (PR);
d. Bath Ankylosing Spondylitis Disease Activity Index (BASDAI) 50 response;
e. improvement from baseline in Bath Ankylosing Spondylitis Functional Index (BASFI);
f. improvement from baseline in Ankylosing Spondylitis Quality of Life (ASQoL);
g. improvement from baseline in ASAS Health Index (HI);
h. improvement from baseline in Maastricht Ankylosing Spondylitis Enthesitis Score (MASES); and
i. improvement from baseline in Linear Bath Ankylosing Spondylitis Metrology Index (BASMI$_{lin}$).

23. A method of treating active psoriatic arthritis in a subject in need thereof, the method comprising orally administering to the subject once a day for at least 12 weeks a dose of upadacitinib freebase, or a pharmaceutically acceptable salt thereof, in an amount sufficient to deliver 15 mg of upadacitinib freebase equivalent, wherein the subject achieves an American College of Rheumatology 20% (ACR20) response within 12 weeks of administration of the first dose.

24. A method of treating active psoriatic arthritis in a subject in need thereof, the method comprising orally administering to the subject once a day for at least 12 weeks a dose of upadacitinib freebase, or a pharmaceutically acceptable salt thereof, in an amount sufficient to deliver 30 mg of upadacitinib free base equivalent, wherein the subject achieves an American College of Rheumatology 20% (ACR20) response within 12 weeks of administration of the first dose.

25. The method of clauses 23 or 24, wherein when the method is used to treat a population of subjects, at least 10% of the subjects in the treated population achieve an ACR20 response within 12 weeks of administration of the first dose.

26. The method of any one of clauses 23-25, wherein the subject or subjects in the treated population suffering from active PsA at baseline further achieve at least one result selected from the group consisting of:

a. improvement from baseline in Health Assessment Questionnaire - Disability Index (HAQ-DI) within 12 weeks of administration of the first dose;

b. achieve Static Investigator Global Assessment (sIGA) of Psoriasis of 0 or 1 and at least a 2-point improvement from baseline at within 16 weeks of administration of the first dose (for subjects with baseline sIGA $\geq$ 2);

c. achieve Psoriasis Area Severity Index (PASI) 75 response within 16 weeks of administration of the first dose (for subjects with $\geq$ 3% BSA psoriasis at baseline);

d. improvement from baseline in Sharp/van der Heijde Score (SHS) within 24 weeks of administration of the first dose;

e. achieve Minimal Disease Activity (MDA) within 24 weeks of administration of the first dose;

f. improvement from baseline in Leeds Enthesitis Index (LEI) within 24 weeks of administration of the first dose, preferably wherein the improvement is a resolution of enthesitis (LEI = 0) within 24 weeks of administration of the first dose (for subjects with baseline presence of enthesitis (LEI >0));

g. achieve ACR 20 response within 12 weeks of administration of the first dose (non-inferiority of upadacitinib vs adalimumab);

h. improvement from baseline in 36-Item Short Form Health Survey (SF-36) within 12 weeks of administration of the first dose; and

i. improvement from baseline in Functional Assessment of Chronic Illness Therapy-Fatigue (FACIT-F) Questionnaire within 12 weeks of administration of the first dose.

27. The method of clause 26, wherein the subject or subjects in the treated population suffering from active PsA at baseline further achieve each result.

28. The method of any one of clauses 23-27, wherein the subject or subjects in the treated population suffering from active PsA at baseline further achieve at least one result selected from the group consisting of:

a. ACR 20 response and superiority over adalimumab (40 mg every other week) within 12 weeks of administration of the first dose; and

b. improvement from baseline in Leeds Dactylitis Index (LDI) within 24 weeks of administration of the first dose, preferably wherein the improvement is a resolution of dactylitis (LDI = 0) within 24 weeks of administration of the first dose (for subjects with baseline presence of dactylitis (LDI >0)).

29. The method of clause 28, wherein the subject subjects in the treated population suffering from active PsA at baseline further achieve ACR 20 response and superiority over adalimumab (40 mg every other week) within 12 weeks of administration of the first dose.

30. The method of clause 29, wherein the subject or subjects in the treated population suffering from active PsA at baseline are orally administered once a day for at least 12 weeks a dose of upadacitinib freebase, or a pharmaceutically acceptable salt thereof, in an amount sufficient to deliver 30 mg of upadacitinib freebase equivalent.

31. The method of any one of clauses 23-30, wherein the subject or subjects in the treated population achieve an ACR 50% response (ACR50) within 12 weeks of administration of the first dose.

32. The method of any one of clauses 23-30, wherein the subject or subjects in the treated population achieve an ACR 70% response (ACR70) within 12 weeks of administration of the first dose.

33. The method of any one of clauses 23-32, wherein the subject or subjects in the treated population fulfill the Classification Criteria for Psoriatic Arthritis (CASPAR) criteria at baseline.

34. The method any one of clauses 23-33, wherein the subject or subjects in the treated population have at least one criteria selected from the group consisting of $\geq$ 3 tender joints (based on 68 joint counts) and $\geq$ 3 swollen joints (based on 66 joint counts) at baseline.

35. The method of clause 34, wherein the subject or subjects in the treated population have $\geq$ 5 tender joints (based on 68 joint counts) and $\geq$ 5 swollen joints (based on 66 joint counts) at baseline.

36. The method of any one of clauses 23-35, wherein the subject or subjects in the treated population have at least one criteria selected from the group consisting of $\geq$ 1 erosion on x-ray as determined by central imaging review, and hs-CRP > laboratory defined upper limit of normal (ULN) at baseline.

37. The method of any one of clauses 23-36, wherein the subject or subjects in the treated population have a diagnosis of active plaque psoriasis or the subject has a documented history of plaque psoriasis at baseline.

38. The method of any one of clauses 23-36, wherein the subject or subjects in the treated population have had an inadequate response or intolerance to at least one biologic disease-modifying anti-rheumatic drug (bDMARD) at baseline.

39. The method of clause 38, wherein the subject or subjects in the treated population have discontinued all bDMARDs prior to administration of the first dose.

40. The method of any one of clauses 23-36, wherein the subject or subjects in the treated population have had an inadequate response or intolerance to previous or concurrent treatment with at least one non-biologic DMARD, or an intolerance to or contraindication for non-biologic DMARDs at baseline.

41. The method of any one of clauses 23-40, wherein the subject or subjects in the treated population have moderately to severely active psoriatic arthritis at baseline.

42. A method of treating active psoriasis in a subject in need thereof, the method comprising orally administering to the subject once a day for at least 16 weeks a dose of upadacitinib freebase, or a pharmaceutically acceptable salt thereof, in an amount sufficient to deliver 15 mg of upadacitinib freebase equivalent, wherein the subject achieves a Psoriasis Area Severity Index (PASI) 75 response within 16 weeks of administration of the first dose.

43. A method of treating active psoriasis in a subject in need thereof, the method comprising orally administering to the subject once a day for at least 16 weeks a dose of upadacitinib freebase, or a pharmaceutically acceptable salt thereof, in an amount sufficient to deliver 30 mg of upadacitinib freebase equivalent, wherein the subject achieves a Psoriasis Area Severity Index (PASI) 75 response within 16 weeks of administration of the first dose.

44. The method of clauses 42 or 43, wherein when the method is used to treat a population of subjects, at least 10% of the subjects in the treated population a PASI 75 response within 16 weeks of administration of the first dose.

45. The method of any one of clauses 1-44 or 46-77, wherein the subject is an adult subject, or the subjects in the treated population are adult subjects.

46. The method of any one of clauses 1-12, wherein the ASAS40 is maintained or improved after Week 64 by continuing to administer the daily dose.

47. The method of any one of clauses 1-12, wherein the subject or subjects in the treated population further achieves ASAS40 within 2 weeks of administration of the first dose.

48. The method of any one of clauses 1-12, wherein the subject or subjects in the treated population further achieves ASAS40 within 2 weeks of administration of the first dose, and wherein the ASAS40 is maintained or improved after Week 14 by continuing to administer the daily dose.

49. A method of treating active ankylosing spondylitis in a subject in need thereof, the method comprising orally administering to the subject once a day for at least 14 weeks a dose of upadacitinib freebase, or a pharmaceutically acceptable salt thereof, in an amount sufficient to deliver 15 mg of upadacitinib freebase equivalent, wherein the subject achieves ASAS partial remission (PR), ASDAS low disease activity (LDA), ASDAS inactive disease (ID), ASDAS major improvement (MI), and/or ASDAS clinically important improvement (CII) within 14 weeks of administration of the first dose.

50. The method of clause 49, wherein when the method is used to treat a population of subjects, at least 10% of the subjects in the treated population achieve ASAS partial remission (PR), ASDAS low disease activity (LDA), ASDAS inactive disease (ID), ASDAS major improvement (MI), and/or ASDAS clinically important improvement (CII) within 14 weeks of administration of the first dose.

51. The method of clauses 49 or 50, wherein the subject or subjects in the treated population further achieve within 14 weeks of administration of the first dose each result.

52. The method of any one of clauses 49-51, wherein the subject or subjects in the treated population fulfill the 1984 modified New York Criteria for ankylosing spondylitis at baseline.

53. The method of any one of clauses 49-51, wherein the subject or subjects in the treated population fulfill the 2009 ASAS classification criteria at baseline.

54. The method of any one of clauses 49-53, wherein the subject or subjects in the treated population meet at least one criteria at baseline selected from the group consisting of:

d. a Bath Ankylosing Spondylitis Disease Activity Index (BASDAI) score $\geq 4$;

e. an Ankylosing Spondylitis Disease Activity Score (ASDAS) of $\geq 2.1$; and

f. a Patient's Assessment of Total Back Pain (Total Back Pain score) of $\geq 4$ based on a 0-10 numerical rating scale.

55. The method of any one of clauses 49-54, wherein the subject or subjects in the treated population are biologic disease-modifying anti-rheumatic drug (bDMARD) naive at baseline.

56. The method of any one of clauses 49-55, wherein the subject or subjects in the treated population have had an inadequate response or intolerance to a biologic disease-modifying anti-rheumatic drug (bDMARD) at baseline.

57. The method of clause 56, wherein prior to administration of the first dose, the subject or subjects in the treated population have been administered one bDMARD, and discontinued use of the bDMARD due to intolerance or lack of efficacy.

58. The method of clause 57, wherein the bDMARD is a tumor necrosis factor (TNF) inhibitor or an interleukin (IL)-17 inhibitor.

59. The method of any one of clauses 49-58, wherein the subject or subjects in the treated population have had an inadequate response or intolerance to at least two NSAIDs, intolerance to NSAIDS, and/or contraindication for NSAIDs at baseline.

60. The method of any one of clauses 49-59, wherein the ASAS partial remission (PR), ASDAS low disease activity (LDA), ASDAS inactive disease (ID), ASDAS major improvement (MI), and/or ASDAS clinically important improvement (CII) is maintained or improved after Week 14 by continuing to administer the daily dose.

61. The method of any one of clauses 49-60, wherein the subject or subjects in the treated population further achieve ASAS partial remission (PR), ASDAS low disease activity (LDA), ASDAS inactive disease (ID), ASDAS major improvement (MI), and/or ASDAS clinically important improvement (CII) within 2 weeks of administration of the first dose.

62. The method of any one of clauses 23-41, wherein the ACR score is maintained or improved after Week 12 by continuing to administer the daily dose.

63. The method of any one of clauses 23-41 or 62, wherein the subject or subjects in the treated population further achieve ACR20 within 2 weeks of administration of the first dose.

64. The method of any one of clauses 42-44, wherein the subject or subjects in the treated population achieve a Psoriasis Area Severity Index (PASI) 90 response within 16 weeks of administration of the first dose.

65. The method of any one of clauses 42-44 or 64, wherein the PASI response is maintained or improved after Week 16 by continuing to administer the daily dose.

66. A method of treating active psoriatic arthritis in a subject in need thereof, the method comprising orally administering to the subject once a day for at least 24 weeks a dose of upadacitinib freebase, or a pharmaceutically acceptable salt thereof, in an amount sufficient to deliver 15 mg of upadacitinib freebase equivalent, wherein the subject achieves Minimal Disease Activity (MDA) within 24 weeks of administration of the first dose.

67. A method of treating active psoriatic arthritis in a subject in need thereof, the method comprising orally administering to the subject once a day for at least 24 weeks a dose of upadacitinib freebase, or a pharmaceutically acceptable salt thereof, in an amount sufficient to deliver 30 mg of upadacitinib freebase equivalent, wherein the subject achieves Minimal Disease Activity (MDA) within 24 weeks of administration of the first dose.

68. The method of clauses 66 or 67, wherein when the method is used to treat a population of subjects, at least 10% of the subjects in the treated population achieve Minimal Disease Activity (MDA) within 24 weeks of administration of the first dose.

69. The method of any one of clauses 66-68, wherein the subject or subjects in the treated population further achieves a Psoriasis Area Severity Index (PASI) response selected from a PASI 75 response, a PASI 90 response, and a PASI 100 response, within 16 weeks of administration of the first dose, and the PASI response is maintained or improved after Week 16 by continuing to administer the daily dose.

70. The method of any one of clauses 23-42, wherein the subject or subjects in the treated population achieve a Psoriasis Area Severity Index (PASI) 75 response within 16 weeks of administration of the first dose.

71. The method of any one of clauses 23-42 or 70, wherein the subject or subjects in the treated population achieve a Psoriasis Area Severity Index (PASI) 90 response within 16 weeks of administration of the first dose.

72. The method of any one of clauses 23-42 or 70-71, wherein the subject or subjects in the treated population achieve a Psoriasis Area Severity Index (PASI) 100 response within 16 weeks of administration of the first dose.

73. A method of treating active psoriatic arthritis in a subject in need thereof, the method comprising orally administering to the subject once a day for at least 16 weeks a dose of upadacitinib freebase, or a pharmaceutically acceptable salt thereof, in an amount sufficient to deliver 15 mg of upadacitinib freebase equivalent, wherein the subject achieves a Psoriasis Area Severity Index (PASI) 75 response within 16 weeks of administration of the first dose.

74. A method of treating active psoriatic arthritis in a subject in need thereof, the method comprising orally administering to the subject once a day for at least 16 weeks a dose of upadacitinib freebase, or a pharmaceutically acceptable salt thereof, in an amount sufficient to deliver 30 mg of upadacitinib free base equivalent, wherein the subject achieves a Psoriasis Area Severity Index (PASI) 75 response within 16 weeks of administration of the first dose.

75. The method of clauses 73 or 74, wherein when the method is used to treat a population of subjects, at least 10% of the subjects in the treated population achieve a PASI 75 response within 16 weeks of administration of the first dose.

76. The method of any one of clauses 73-75, wherein the subject or subjects in the treated population achieve a Psoriasis Area Severity Index (PASI) 90 response within 16 weeks of administration of the first dose.

77. The method of any one of clauses 73-76, wherein the subject or subjects in the treated population achieve a Psoriasis Area Severity Index (PASI) 100 response within 16 weeks of administration of the first dose.

**Claims**

1.  Upadacitinib freebase, or a pharmaceutically acceptable salt thereof, for use in a method of treating active psoriasis in a subject in need thereof, the method comprising orally administering to the subject once a day for at least 16 weeks a dose of upadacitinib freebase, or a pharmaceutically acceptable salt thereof, in an amount sufficient to deliver 15 mg of upadacitinib freebase equivalent, wherein the subject achieves a Psoriasis Area Severity Index (PASI) 75 response within 16 weeks of administration of the first dose.

2.  Upadacitinib freebase, or a pharmaceutically acceptable salt thereof, for use according to claim 1, wherein the subject is an adult subject.

3.  Upadacitinib freebase, or a pharmaceutically acceptable salt thereof, for use according to claim 1, wherein the PASI response is maintained or improved after Week 16 by continuing to administer the daily dose.

4.  Upadacitinib freebase, or a pharmaceutically acceptable salt thereof, for use according to claim 2, wherein the PASI response is maintained or improved after Week 16 by continuing to administer the daily dose.

5.  Upadacitinib freebase, or a pharmaceutically acceptable salt thereof, for use according to claim 1, wherein the subject or subjects in the treated population achieve a Psoriasis Area Severity Index (PASI) 90 response within 16 weeks of administration of the first dose.

6.  Upadacitinib freebase, or a pharmaceutically acceptable salt thereof, for use according to claim 5, wherein the subject is an adult subject.

7.  Upadacitinib freebase, or a pharmaceutically acceptable salt thereof, for use according to claim 5, wherein the PASI response is maintained or improved after Week 16 by continuing to administer the daily dose.

8.  Upadacitinib freebase, or a pharmaceutically acceptable salt thereof, for use according to claim 6, wherein the PASI response is maintained or improved after Week 16 by continuing to administer the daily dose.

9.  Upadacitinib freebase, or a pharmaceutically acceptable salt thereof, for use according to of claim 1, wherein the subject or subjects in the treated population achieve a Psoriasis Area Severity Index (PASI) 100 response within 16 weeks of administration of the first dose.

10. Upadacitinib freebase, or a pharmaceutically acceptable salt thereof, for use according to claim 9, wherein the subject is an adult subject.

11. Upadacitinib freebase, or a pharmaceutically acceptable salt thereof, for use according to claim 9, wherein the PASI response is maintained or improved after Week 16 by continuing to administer the daily dose.

12. Upadacitinib freebase, or a pharmaceutically acceptable salt thereof, for use according to claim 10, wherein the PASI response is maintained or improved after Week 16 by continuing to administer the daily dose.

FIG. 1

Upadacitinib 15 mg QD vs Placebo
$\alpha = 0.05$

| Endpoint | Nominal p value | Assigned $\alpha$ |
|---|---|---|
| ASAS40 | 0.0003* | 0.05 |
| ASDAS | <0.0001* | 0.05 |
| SPARCC MRI Spine | <0.0001* | 0.05 |
| WPAI - Overall Work Impairment | 0.1900 | 0.05 |
| MASES (Enthesitis) | 0.0488 | 0.025 |
| BASMI (Mobility) | 0.0296 | 0.017 |
| ASQoL | 0.0156 | 0.013 |
| BASDAI50 | 0.0016* | 0.010 |
| BASFI (Function) | 0.0013* | 0.008 |
| ASAS PR | <0.0001* | 0.007 |
| ASAS HI | 0.0073 | 0 |

FIG. 2

FIG. 3A

FIG. 3B

FIG. 3C

EP 4 356 963 A2

Patients achieving ASAS40 up to and including Week 64

| AO:% (n/N) | W2 | W14 | W24 | W40 | W52 | W64 |
|---|---|---|---|---|---|---|
| | 16.7 (15/90) | 54.0 (47/87) | 73.8 (62/84) | 79.0 (64/81) | 80.2 (65/81) | 84.8 (67/79) |
| | 1.1 (1/90) | 27.6 (24/87) | 66.7 (60/90) | 78.6 (66/84) | 77.4 (65/84) | 80.5 (66/82) |
| NRI: % | 16.1 | 51.6 | 66.7 | 68.8 | 69.9 | 72.0 |
| | 1.1 | 25.5 | 63.8 | 69.1 | 69.1 | 70.2 |

—□— **Continuous upadacitinib 15mg QD (AO)**   – –□– – Placebo → upadacitinib 15mg QD (AO)

—○— **Continuous upadacitinib 15mg QD (NRI)**   – –○– – Placebo → upadacitinib 15mg QD (NRI)

## FIG. 3D

Patients achieving ASAS20 up to and including Week 64

ASAS20

Response (95% CI)

96% (92%-100%)
**94% (88%-99%)**
83% (75%-91%)
**80% (71%-88%)**

| | W2 | W14 | W24 | W40 | W52 | W64 |
|---|---|---|---|---|---|---|
| AO:% (n/N) | **40.0 (36/90)** | **67.8 (59/87)** | **84.5 (71/84)** | **96.3 (78/81)** | **87.7 (71/81)** | **93.7 (74/79)** |
| | 13.3 (12/90) | 42.5 (37/87) | 86.7 (78/90) | 94.0 (79/84) | 94.0 (79/84) | 96.3 (79/82) |
| NRI: % | **38.7** | **64.5** | **76.3** | **83.9** | **76.3** | **79.6** |
| | 12.8 | 40.4 | 83.0 | 83.0 | 84.0 | 83.0 |

—□— **Continuous upadacitinib 15mg QD (AO)**   - -□- - Placebo → upadacitinib 15mg QD (AO)

—○— **Continuous upadacitinib 15mg QD (NRI)**   - -○- - Placebo → upadacitinib 15mg QD (NRI)

## FIG. 3E

Patients achieving ASAS Partial Remission(PR) up to and including Week 64

ASAS PR

Response (95% CI)

**46% (35%-57%)**
39% (29%-50%)
**40% (30%-50%)**
34% (25%-44%)

| | W2 | W14 | W24 | W40 | W52 | W64 |
|---|---|---|---|---|---|---|
| AO:% (n/N) | **6.5 (6/90)** | **19.3 (17/88)** | **34.1 (29/85)** | **47.6 (39/82)** | **50.0 (41/82)** | **46.3 (37/80)** |
| | 0 (0/90) | 1.1 (1/87) | 25.6 (23/90) | 39.3 (33/84) | 45.2 (38/84) | 39.0 (32/82) |
| NRI: % | **6.5** | **19.4** | **31.2** | **41.9** | **44.1** | **39.8** |
| | 0 | 1.1 | 24.5 | 35.1 | 40.4 | 34.0 |

—□— **Continuous upadacitinib 15mg QD (AO)**        – –□– –  Placebo → upadacitinib 15mg QD (AO)

—○— **Continuous upadacitinib 15mg QD (NRI)**       – –○– –  Placebo → upadacitinib 15mg QD (NRI)

FIG. 3F

EP 4 356 963 A2

Patients achieving BASDAI50 Responses up to and including Week 64

BASDAI50

Response (95% CI)

**82% (74%-91%)**
77% (68%-86%)
**70% (61%-79%)**
67% (58%-77%)

|  | W2 | W14 | W24 | W40 | W52 | W64 |
|---|---|---|---|---|---|---|
| **AO:% (n/N)** | **15.4 (14/91)** | **48.3 (42/87)** | **72.6 (61/84)** | **84.0 (68/81)** | **77.8 (63/81)** | **82.3 (65/79)** |
|  | 6.7 (6/90) | 25.3 (22/87) | 68.9 (62/90) | 75.0 (63/84) | 76.2 (64/84) | 76.2 (63/82) |
| **NRI: %** | **15.1** | **45.2** | **65.6** | **73.1** | **67.7** | **69.9** |
|  | 6.4 | 23.4 | 66.0 | 66.0 | 68.1 | 67.0 |

—□— **Continuous upadacitinib 15mg QD (AO)**   – –□– – Placebo → upadacitinib 15mg QD (AO)

—○— **Continuous upadacitinib 15mg QD (NRI)**   – –○– – Placebo → upadacitinib 15mg QD (NRI)

FIG. 3G

Patients achieving ASDAS inactive disease (ID) up to and including Week 64

ASDAS ID

Response (95% CI)

44% (33%-55%)
**42% (31%-53%)**
36% (27%-46%)
**34% (25%-44%)**

| | W2 | W14 | W24 | W40 | W52 | W64 |
|---|---|---|---|---|---|---|
| AO:% (n/N) | **5.4 (5/92)** | **17.4 (15/86)** | **25.9 (21/81)** | **39.5 (32/81)** | **46.2 (36/78)** | **41.6 (32/77)** |
| | 0 (0/90) | 0 (0/84) | 28.2 (24/85) | 39.2 (31/79) | 41.5 (34/82) | 43.6 (34/78) |
| NRI: % | **5.4** | **16.1** | **22.6** | **34.4** | **38.7** | **34.4** |
| | 0 | 0 | 25.5 | 33.0 | 36.2 | 36.2 |

—□— **Continuous upadacitinib 15mg QD (AO)**   – –□– – Placebo → upadacitinib 15mg QD (AO)

—○— **Continuous upadacitinib 15mg QD (NRI)**   – –○– – Placebo → upadacitinib 15mg QD (NRI)

FIG. 3H

EP 4 356 963 A2

Patients achieving ASDAS low disease activity (LDA) up to and including Week 64

FIG. 3I

EP 4 356 963 A2

Patients achieving ASDAS major improvement (MI) up to and including Week 64

**ASDAS MI**

Response (95% CI)

56% (45%-67%)
**57% (45%-68%)**
47% (37%-57%)
**46% (36%-56%)**

| | W2 | W14 | W24 | W40 | W52 | W64 |
|---|---|---|---|---|---|---|
| AO:% (n/N) | **6.7 (6/90)** | **35.3 (30/85)** | **40.0 (32/80)** | **52.5 (42/80)** | **55.8 (43/77)** | **56.6 (43/76)** |
| | 0 (0/90) | 6.0 (5/84) | 45.9 (39/85) | 50.6 (40/79) | 52.4 (43/82) | 56.4 (44/78) |
| NRI: % | **6.5** | **32.3** | **34.4** | **45.2** | **46.2** | **46.2** |
| | 0 | 5.3 | 41.5 | 42.6 | 45.7 | 46.8 |

—□— **Continuous upadacitinib 15mg QD (AO)**  − □ − − Placebo → upadacitinib 15mg QD (AO)

—○— **Continuous upadacitinib 15mg QD (NRI)**  − ○ − − Placebo → upadacitinib 15mg QD (NRI)

## FIG. 3J

Patients achieving ASDAS clinically important improvement (CII) up to and including Week 64

| AO:% (n/N) | W2 | W14 | W24 | W40 | W52 | W64 |
|---|---|---|---|---|---|---|
| | **44.4 (40/90)** | **57.6 (49/85)** | **76.3 (61/80)** | **86.3(69/80)** | **85.7 (66/77)** | **84.2 (64/76)** |
| | 5.6 (5/90) | 20.2 (17/84) | 82.4 (70/85) | 88.6 (70/79) | 90.2 (74/82) | 87.2 (68/78) |
| NRI: % | **43.0** | **52.7** | **65.6** | **74.2** | **71.0** | **68.8** |
| | 5.3 | 18.1 | 74.5 | 74.5 | 78.7 | 72.3 |

—□— **Continuous upadacitinib 15mg QD (AO)**      − −□− − Placebo → upadacitinib 15mg QD (AO)

—⊖— **Continuous upadacitinib 15mg QD (NRI)**      − −⊖− − Placebo → upadacitinib 15mg QD (NRI)

## FIG. 3K

EP 4 356 963 A2

Proportion of Patients with $\geq$ 30% Reduction from Baseline in Pain Through 64 Weeks

30% Reduction

95%
89%
83%
76%

UPA, n=   92   90   88   88   85   84   82   82   80
Placebo, n=   90   91   87   90   90   87   84   84   82

—○—Placebo →--△--upadacitinib (AO)    —□— Continuous upadacitinib (AO)

—●—Placebo →--▲--upadacitinib (NRI/MMRM)    —■— Continuous upadacitinib (NRI/MMRM)

FIG. 3L

EP 4 356 963 A2

Proportion of Patients with ≥ 50% Reduction from Baseline in Pain Through 64 Weeks

**FIG. 3M**

Proportion of Patients with ≥ 70% Reduction from Baseline in Pain Through 64 Weeks

FIG. 3N

EP 4 356 963 A2

FIG. 4A

FIG. 4B

FIG. 4C

FIG. 4D

FIG. 4E

FIG. 5

FIG. 6B

Change in SPARCC MRI SI Joint Score
Cumulative Percentile
○ Placebo, n=59
● Upadacitinib, n=68

FIG. 6A

Change in SPARCC MRI Spine Score
Cumulative Percentile
○ Placebo, n=60
● Upadacitinib, n=68

118

FIG. 6D

FIG. 6C

FIG. 7A

EP 4 356 963 A2

FIG. 7B

FIG. 7C

**FIG. 8A**

**FIG. 8B**

Peripheral Pain / Swelling

FIG. 8C

Morning Stiffness

FIG. 8D

| Screening Period (up to 35 days) | 14-week double-blind, randomized placebo-controlled | 90-week open-label extension | 30-day Follow-up Period |
|---|---|---|---|

**Study 1: bDMARD-IR (N=386*)**

Meets eligibility criteria for bDMARD-IR AS

1:1

| Placebo N=193* | → | UPA 15 mg QD | → |
| UPA 15 mg QD N=193* | → | UPA 15 mg QD | → |

ASAS 40 Week 14 primary endpoint in Study 1

| 52-week double-blind, randomized, placebo-controlled | 52-week open-label extension |

**Study 2: nr-axSpA (N=304)**

Meets eligibility criteria for nr-axSpA

1:1

| Placebo N=152 | → | UPA 15 mg QD | → |
| UPA 15 mg QD N=152 | → | UPA 15 mg QD | → |

0 1 2 4 8 12 14 18 24 32 40 52 64 76 88 104

MRI Spine and SI Joints X-ray of SI Joints and Spine

ASAS40 Week 14 Primary Endpoint for EMA in Study 2

MRI Spine and SI Joints

ASAS40 Week 52 Primary Endpoint for FDA in Study 2

MRI Spine and SI Joints X-ray of Spine

## FIG. 9

FIG. 10

## Part 1

**Low Dose**

$$\frac{\alpha}{2}$$

ACR20

1

HAQ-DI

1

sIGA (W16)

1

PASI75 (W16)

1

SHS(W24)

1

MDA(W24)

1

Resolution of Enthesitis (W24)

1

Non-inferiority ACR20 vs Humira

$\frac{1}{2}$

SF-36-PCS

1

FACIT-F

**High Dose**

$$\frac{\alpha}{2}$$

ACR20

1

HAQ-DI

1

sIGA (W16)

1

PASI75 (W16)

1

SHS(W24)

1

MDA(W24)

1

Resolution of Enthesitis (W24)

1

Non-inferiority ACR20 vs Humira

$\frac{1}{2}$

SF-36-PCS

1

FACIT-F

1/2     1/2

1        1

# FIG. 11A

Part 2

Superiority Testing Sequence at level $\alpha$

Tested if ALL higher ranked hypotheses in low
dose and high dose of ABT-494 are rejected

```
┌─────────────────────┐        ┌─────────────────────┐
│  Superiority ACR20  │───────▶│  Superiority ACR20  │
│  High Dose vs Humira│        │  Low Dose vs Humira │
└─────────────────────┘        └─────────────────────┘
                                         │
                                         ▼
┌──────────────────────────┐   ┌──────────────────────────┐
│ Resolution of Dactylitis │   │ Resolution of Dactylitis │
│ (W24) High Dose vs Placebo│──▶│ (W24) Low Dose vs Placebo│
└──────────────────────────┘   └──────────────────────────┘
                                         │
                                         ▼
┌─────────────────────┐        ┌─────────────────────┐
│  Superiority Pain   │───────▶│  Superiority Pain   │
│  High Dose vs Humira│        │  Low Dose vs Humira │
└─────────────────────┘        └─────────────────────┘
                                         │
                                         ▼
┌─────────────────────┐        ┌─────────────────────┐
│ Superiority HAQ-DI  │───────▶│ Superiority HAQ-DI  │
│ High Dose vs Humira │        │ Low Dose vs Humira  │
└─────────────────────┘        └─────────────────────┘
                                         │
                                         ▼
┌─────────────────────┐        ┌─────────────────────┐
│     SAPs(W16)       │───────▶│     SAPs(W16)       │
│ High Dose vs Placebo│        │ Low Dose vs Placebo │
└─────────────────────┘        └─────────────────────┘
```

# FIG. 11B

FIG. 12A

FIG. 12B

Non-inferiority of Upadacitinib versus Adalimumab in ACR20 at Week 12

FIG. 12E

LS Mean Change from Baseline Over 24 Weeks in Core Components
of the ACR Criteria: tender joint counts (TJC68)

FIG. 12F

LS Mean Change from Baseline Over 24 Weeks in Core Components
of the ACR Criteria: swollen joint count (SJC66)

FIG. 12G

LS Mean Change from Baseline Over 24 Weeks in Core Components of
the ACR Criteria: Physician's global assessment of disease activity (PhGA)

FIG. 12H

LS Mean Change from Baseline Over 24 Weeks in Core Components of
the ACR Criteria: patient global assessment of disease activity (PtGA)

FIG. 12I

LS Mean Change from Baseline Over 24 Weeks in Core Components of the ACR Criteria: Pain (score of 0 indicates "no pain" and a score of 10 indicates "worst possible pain")

**FIG. 12J**

LS Mean Change from Baseline Over 24 Weeks in Core Components of the ACR Criteria: high-sensitivity C-reactive protein (hs-CRP)

**FIG. 12K**

Proportion of Patients Achieving Psoriasis Area Severity Index
PASI75 Over 24 Weeks

FIG. 12L

Proportion of Patients Achieving Psoriasis Area Severity Index
PASI90 Over 24 Weeks

FIG. 12M

Proportion of Patients Achieving Psoriasis Area Severity Index
PASI100 Over 24 Weeks

FIG. 12N

Proportion of Patients Achieving sIGA Over 24 Weeks

FIG. 12O

Change from Baseline Over 24 Weeks in Self-Assessment of Psoriasis Symptoms (SAPS)

FIG. 12P

Change from baseline Over 24 Weeks in Health assessment
questionnaire disability index (HAQ-DI)

FIG. 12Q

Change from baseline Over 24 Weeks in SF-36 Physical
Component Summary Score

FIG. 12R

Change from baseline Over 24 Weeks in Functional Assessment
of Chronic Illness Therapy-Fatigue (FACIT-F)

FIG. 12S

Change from Baseline Over 24 Weeks in Morning Stiffness
(Mean of BASDAI Questions 5 and 6)

FIG. 12T

Change from Baseline Over 24 Weeks in Morning Stiffness
Severity (BASDAI Question 5)

FIG. 12U

Change from Baseline Over 24 Weeks in Morning Stiffness
Duration (BASDAI Question 6)

FIG. 12V

Proportion of Patients with Resolution of Enthesis Over
24 weeks by Leeds Enthesitis Indices (LEI)

FIG. 12W

Proportion of Patients with Resolution of Enthesis Over 24 Weeks by
Spondyloarthritis Research Consortium of Canada (SPARCC)

FIG. 12X

Proportion of Patients with Resolution of Dactylitis by Leeds
Dactylitis Index (LDI) Over 24 Weeks

FIG. 12Y

Change from Baseline at Week 24 in Radiographic Endpoints

FIG. 12Z

Placebo     Upadacitinib 30mg QD
Upadacitinib 15mg QD     Adalimumab 40mg EOW

Proportion of Patients with No
Radiographic Progression mTSS ≤0.0

**FIG. 12AA**

Proportion of Patients with No
Radiographic Progression mTSS ≤0.5

**FIG. 12BB**

Percentage of Patients Achieving ≥ 30% Reduction in Pain
in SELECT-PsA 1 Over 24 Weeks.

FIG. 12CC

Percentage of Patients Achieving ≥ 50% Reduction in Pain
in SELECT-PsA 1 Over 24 Weeks.

FIG. 12DD

Percentage of Patients Achieving ≥ 70% Reduction in Pain
in SELECT-PsA 1 Over 24 Weeks.

FIG. 12EE

FIG. 13

Proportions of Patients Achieving ACR20
Response Over 24 weeks

FIG. 14A

Proportions of Patients Achieving ACR50
Response Over 24 weeks

FIG. 14B

Proportions of Patients Achieving ACR70
Response Over 24 weeks

FIG. 14C

Proportions of Patients Achieving Minimal
Disease Activity (MDA) Over 24 Weeks

FIG. 14D

FIG. 14E

FIG. 14F

Proportions of Patients with Resolution of Enthesitis Over 24 Weeks
by Spondyloarthritis Research Consortium of Canada (SPARCC)

FIG. 14G

CHANGE FROM BASELINE IN LDI

FIG. 14H

Proportions of Patients with Resolution of Dactylitis by Leeds
Dactylitis Index (LDI)

FIG. 14I

Proportions of Patients Achieving PASI75 Response
Over 24 Weeks

FIG. 14J

Proportions of Patients Achieving PASI90
Response Over 24 Weeks

FIG. 14K

Proportions of Patients Achieving PASI100 Response
Over 24 Weeks

FIG. 14L

FIG. 14M

FIG. 14N

Change from Baseline Over 24 Weeks in
swollen joint count (SJC66)

FIG. 14O

Change from Baseline Over 24 Weeks in
Physician's global assessment of disease activity (PhGA)

FIG. 14P

Change from Baseline Over 24 Weeks in
patient's global assessment of disease activity (PtGA)

FIG. 14Q

Change from Baseline Over 24 Weeks in Pain

FIG. 14R

Change from Baseline Over 24 Weeks in
high-sensitivity C-reactive protein (hs-CRP)

FIG. 14S

Proportion of Patients Achieving Static Investigator Global
Assessment (sIGA) 0/1 Over 24 Weeks

FIG. 14T

## Change from Baseline Over 24 Weeks in Self-Assessment of Psoriasis Symptoms (SAPS)

−♦− Placebo  −■− Upadacitinib 15mg QD  −▲− Upadacitinib 30mg QD

## FIG. 14U

## Change from Baseline Over 24 Weeks in Health assessment questionnaire disability index (HAQ-DI)

−♦− Placebo  −■− Upadacitinib 15mg QD  −▲− Upadacitinib 30mg QD

## FIG. 14V

### Change from Baseline Over 24 Weeks in SF-36 Physical Component Summary

**FIG. 14W**

### Change from Baseline Over 24 Weeks in Functional Assessment of Chronic Illness Therapy-Fatigue (FACIT-F)

**FIG. 14X**

FIG. 14Y

FIG. 14Z

FIG. 14AA

FIG. 14BB

Percentage of Patients Achieving ≥ 50% Reduction in Pain
in SELECT-PsA 2 Over 24 Weeks.
(*Statistically significant at 0.05 level)

FIG. 14CC

Percentage of Patients Achieving ≥ 70% Reduction in Pain
in SELECT-PsA 2 Over 24 Weeks.
(*Statistically significant at 0.05 level)

FIG. 14DD

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63032042
- US 62968849
- US 62927548
- US 62908163
- WO 2017066775 A
- WO 2018165581 A

**Non-patent literature cited in the description**

- **PODDUBNYY ; SIEPER.** *Curr Opin Rheumatol,* 2014, vol. 26, 377-383
- **VAN DER HEIJDE D et al.** *Ann Rheum Dis.,* 2017, vol. 76, 978-991
- **WARD et al.** *Arthritis Rheumatol.,* 2016, vol. 68, 282-298
- **SIEPER ; PODDUBNYY.** *Lancet,* 2017, vol. 390, 73-84
- **SIEPER et al.** *Ann Rheum Dis.,* 2017, vol. 76, 571-592
- **RUDWALEIT et al.** *Arthritis Res Ther.,* 2010, vol. 12, R117
- **DEODHAR et al.** *Arthritis Rheumatol.,* 2019, vol. 71, 599-611
- **GOSSEC et al.** *Ann Rheum Dis.,* 2016, vol. 75, 499-510
- **COATES et al.** *Arthritis Rheumatol.,* 2016, vol. 68, 1060-71
- **ALAMANOS et al.** *J Rheumatol.,* 2003, vol. 30, 2641-2644
- **SAVOLAINEN et al.** *J Rheumatol.,* 2003, vol. 30, 2460-8
- **SANDBORN.** *Dig Dis,* 2010, vol. 28, 536-42
- **SABER et al.** *Arthritis Res Therapy,* 2010, vol. 12, R94
- **PERROTTA et al.** *J Rheumatol.,* 2016, vol. 43, 350-5
- **SIEPER et al.** *Ann Rheum Dis,* 2009, vol. 68 (II), ii1-ii44
- **VAN DER HEIJDE et al.** *Arth. Care & Res.,* 2012, vol. 64, 1919-1922
- **VAN DER HEIJDE et al.** *Ann Rheum Dis,* 2008, vol. 67, 489-93
- **BOZEK ; REICH.** *Adv. Clin. Exp. Med.,* 2017, vol. 26, 851-856
- **PODDUBNYY et al.** *Ann. Rheum. Dis.,* 2010, vol. 69, 1338-1341
- **DOUGADOS et al.** *Ann Rheum Dis,* 2011, vol. 70, 249-251
- **FELDMAN et al.** *J Invest Dermatol.,* 1996, vol. 106 (1), 183-6
- **PETTY et al.** *J Rheumatol.,* 2004, vol. 31, 390-2
- **AVIEL et al.** *Pediatric Rheumatology,* 2013, vol. 11, 11
- **ZISMAN et al.** *J. Rheum.,* 2017, vol. 44, 342-351
- **RUDWALEIT et al.** *Ann. Rheum. Dis.,* 2009, vol. 68, 777-783
- **DEODHAR et al.** *Arth. & Rheum.,* 2014, vol. 66, 2649-2656
- **VAN DER LINDEN et al.** *Arthritis and Rheumatism,* 1984, vol. 27, 361-368
- **DEODHAR et al.** *, Arth. & Rheum.,* 2014, vol. 66, 2649-2656
- **VAN DER HEIJDE et al.** *Ann Rheum Dis,* 2017, vol. 76, 978-991
- **SIEPER ; PODDUBNYY.** *Lancet,* 2017, 73-84
- **VAN DER HEIJDE et al.** *Ann Rheum Dis.,* 2017, vol. 76, 978-991
- **SIEPER et al.** *Ann. Rheum. Dis.,* 2009, vol. 68 (2), 1-44
- **TAYLOR et al.** *Arthritis and Rheumatism,* 2006, vol. 54, 2665-2673
- **STAHL ; WERMUTH.** Handbook of Pharmaceutical Salts: Properties, Selection, and Use. Wiley-VCH, 2002
- Handbook of Pharmaceutical Excipients. Pharmaceutical Press, 2012
- **SIEPER et al.** *Ann. Rheum. Dis,* 2014, vol. 73, 95-100
- **SIEPER et al.** *Ann. Rheum. Dis.,* 2015, vol. 74, 1051-1057
- **DEODHAR et al.** *Arthritis and Rheumatology,* 2019, vol. 71, 258-270
- **SONG et al.** *Ann. Rheum. Dis.,* 2011, vol. 70, 1108-1110
- **GHORESCHI et al.** *Immunol Rev.,* 2009, vol. 228, 273-87
- **VAN DER HEIJDE et al.** *Lancet,* 2018, vol. 392, 2441-2451

- **VAN DER HEIJDE D et al.** *Ann. Rheum. Dis,* 2017, 1-8
- **VAN DER HEIJDE et al.** *Lancet,* 2018, 2378-2387
- **NESTLE et al.** *N. Engl. J. Med.,* 2009, vol. 361, 496-509
- **MEASE.** *Curr. Opin. Rheumatol.,* 2015, vol. 27, 127-33
- **GLADMAN et al.** *New England Journal of Medicine,* 2017, vol. 377, 1525-1536
- **MEASE P et al.** *N Engl J Med,* 2017, vol. 377, 1537-1550
- **MACHADO et al.** *Ann Rheum Dis,* 2018, vol. 77, 1539-40
- **MAKSYMOWYCH et al.** *Arthritis Rheum,* 2005, vol. 53, 502-9
- **HOCHBERG Y ; TAMHANE AC.** Multiple comparison procedures. John Wiley & Sons, Inc, 1987
- **SMOLEN et al.** *Ann Rheum Dis,* 2018, vol. 77, 3-17
- **VAN DER HEIJDE et al.** *Ann Rheum Dis,* 2017, vol. 76, 1340-47
- **VAN DER HEIJDE et al.** *Lancet,* 2018, vol. 392, 2378-87
- **VAN DER HEIJDE et al.** *Lancet,* 2018, vol. 392, 2441-51
- **LANDEWE et al.** *Ann Rheum Dis,* 2014, vol. 73, 39-47
- **FURST ; LOUIE.** *Arthritis Res Ther,* 2019, vol. 21, 135
- **SORNASSE et al.** *Ann Rheum Dis,* 2019, vol. 78, 365-66
- **VEALE et al.** *Rheumatology (Oxford),* 2019, vol. 58, 197-205
- **GRACEY et al.** *Ann Rheum Dis,* 2016, vol. 75, 2124-32
- **BURMESTER et al.** *Lancet,* 2018, vol. 391, 2503-12
- **GENOVESE et al.** *Lancet,* 2018, vol. 391, 2513-24
- **FLEISCHMANN R ; PANGAN AL ; SONG I et al.** Upadacitinib versus placebo or adalimumab in patients with rheumatoid arthritis and an inadequate response to methotrexate: results of a phase 3, double-blind, randomized controlled trial. *Arthritis Rheumatol,* 2019
- **COHEN et al.** *Ann Rheum Dis,* 2019, vol. 78
- **SIEPER et al.** *Ann Rheum Dis,* 2017, vol. 76, 571-92
- **DEODHAR et al.** *Arthritis Rheumatol,* 2019, vol. 71, 599-611
- **LIE E et al.** *Ann Rheum Dis,* 2011, vol. 70, 157-63
- **GLINTBORG et al.** *Ann Rheum Dis,* 2013, vol. 72, 1149-55
- **SIEPER et al.** *Lancet,* 2017, vol. 390, 73-84
- **CORTESE et al.** *Mult Scler Relat Disord,* 2019, vol. 35, 193-95
- **VAN DER HEIJDE et al.** *Ann Rheum Dis,* 2017, vol. 76, 978-91
- **FRAGOULIS et al.** *World J Gastroenterol,* 2019, vol. 25, 2162-76
- **ALTEN et al.** *Patient Prefer Adherence,* 2016, vol. 10, 2217-28
- **VAN DER HEIJDE D et al.** *ARD,* 2017, vol. 0, 1-8
- **MEASE PJ et al.** *Ann Rheum Dis,* 2017, vol. 76, 79-87
- **GLADMAN D et al.** *N Engl J Med,* 2017, vol. 377, 1525-1536
- **NASH P et al.** *Lancet,* 2017, vol. 389, 2317-2327
- **COATES LC ; KAVANAUGH A ; MEASE PJ et al.** Group for Research and Assessment of Psoriasis and Psoriatic Arthritis (GRAPPA) 2015 treatment recommendations for psoriatic arthritis. *Arthritis Rheumatol,* 2016, vol. 68 (5), 1060-71